# EUROPEAN PATENT APPLICATION

(11) **EP 4 549 452 A1**
(43) Date of publication of application: **07.05.2025**
(21) Application number: 23834781.9
(22) Date of filing: 30.06.2023
(51) Int. Cl.: C07K 14/165, C07K 19/00, C12N 15/50, A61K 39/215, A61P 31/14

(54) **PHARMACEUTICAL COMPOSITION FOR RESISTING INFECTION WITH SARS-COV-2 OR MUTANT THEREOF, AND COMBINED DRUG THEREOF**

(30) Priority: 07.07.2022 CN 202210802239; 15.12.2022 CN 202211618137
(71) Applicant: WestVac Biopharma Co., Ltd., Chengdu, Sichuan 610000 (CN)
(72) Inventor: WEI, Xiawei, Chengdu, Sichuan 610065 (CN); LU, Guangwen, Chengdu, Sichuan 610065 (CN); CHENG, Ping, Chengdu, Sichuan 610065 (CN); YANG, Li, Chengdu, Sichuan 610065 (CN); LI, Jiong, Chengdu, Sichuan 610065 (CN); YANG, Jingyun, Chengdu, Sichuan 610065 (CN); WANG, Wei, Chengdu, Sichuan 610065 (CN); WEI, Yuquan, Chengdu, Sichuan 610065 (CN); WANG, Zhenling, Chengdu, Sichuan 610065 (CN); SHEN, Guobo, Chengdu, Sichuan 610065 (CN); ZHAO, Zhiwei, Chengdu, Sichuan 610065 (CN); YANG, Jinliang, Chengdu, Sichuan 610065 (CN)
(74) Representative: Gulde & Partner
(86) International application number: PCT/CN2023/105178
(87) International publication number: WO 2024/008014

(57) **Abstract**

Provided are a pharmaceutical composition for resisting infection with SARS-CoV-2 or a mutant thereof, and a combined drug thereof. To solve the problem of the lack of effective prevention and treatment drugs for infection with SARS-CoV-2 or a mutant virus thereof, provided are a recombinant protein vaccine and/or an adenovirus vaccine for preventing and/or treating an infection with SARS-CoV-2 or a mutant thereof, and in particular, provided are a nasal spray administration compound formulation containing active ingredients of two vaccines, i.e., a recombinant protein vaccine and an adenovirus vaccine, and a combination of the two vaccines for nasal spray administration, which can induce generation of strong antibody and cellular immune responses in vivo and block the binding of a protein S of SARS-CoV-2 to an ACE2 receptor of a host cell, thus enabling a host to resist coronavirus infection. Particularly, the present invention has good prevention and treatment effects on various mutant viruses.

## Description

### Technical Field

The present invention relates to a pharmaceutical composition for resisting infection with SARS-CoV-2 or a mutant thereof and a combined drug thereof, and in particular, to a broad-spectrum nasal spray of compound preparation for resisting infection with SARS-CoV-2 or a mutant thereof and a combined drug thereof, which belongs to the medical field.

### Background

SARS-CoV-2 is a novel betacoronavirus named by the World Health Organization. The virus has an envelope and the particles are round or oval, and often polymorphic, with a diameter of 60nm-140nm. Its genetic characteristics are significantly different from those of SARS-CoV and MERS-CoV, and SARS-CoV-2 is a branch of novel coronavirus that has not been previously found in humans. At present, there are five major types of mutated novel coronavirus: Alpha, Beta, Gamma, Delta, and Omicron. Omicron variant strains are further divided into several substrains, such as BA.1, BA.2, BA.2.12.1, BA.4, and BA.5. Up to now, the novel coronavirus SARS-CoV-2 has infected 545 million people worldwide, including more than 6.3 million of fatality.

The main structural proteins of SARS-CoV-2 include Spike (S), Envelop (E), Membrane (M) and Nucleocapsid (N). Among them, S protein plays a key role in virus infection and virulence. It is often used as an antigen in vaccines. As the SARS-CoV-2 variant strain contains multiple mutation sites and the S protein of the virus also contains multiple mutation sites, the variant strain can escape antibodies stimulated by the SARS-COV-2 prototype strain (namely, "novel coronavirus wild strain") vaccine to a certain extent, resulting in the ineffectiveness or reduced protection of the SARS-COV-2 vaccine, which brings great pressure to the prevention and control of the SARS-COV-2 pandemic. Therefore, the development of a vaccine against various variants of SARS-CoV-2 virus, especially a broad spectrum vaccine against various variants of SARS-CoV-2 virus, is very important for the prevention and treatment of novel coronavirus pneumonia.

At present, hundreds of companies and units around the world are developing SARS-COV-2 vaccines, which mainly involve in five technical routes of inactivated vaccines, recombinant protein vaccines, adenovirus vector vaccines, attenuated influenza virus vector vaccines, and nucleic acid vaccines (including mRNA vaccines and DNA vaccines). Meanwhile, a variety of vaccines have been launched. Vaccination routes mainly include subcutaneous injection, intradermal injection, intramuscular injection, oral administration and inhalation (including nasal spray). Many viruses such as influenza virus and novel coronavirus are mainly diffused through a respiratory tract, and nasal spray or inhalation vaccines are developed through simulation of the natural infection process of human body. Therefore, it is important to develop vaccines that can induce strong mucosal immunity to effectively prevent the invasion and infection of these viruses from the respiratory tract.

### Summary

The present invention is aimed to solve one of the technical problems existing in the prior art. For this purpose, the present invention is to provide a nasal spray of compound preparation containing a recombinant protein vaccine and an adenovirus vaccine for resisting infection with SARS-CoV-2 or a mutant thereof, and a combined nasal spray.

The present invention provides a pharmaceutical composition for preventing and/or treating infection with SARS-CoV-2 or a mutant thereof, where the pharmaceutical composition is a compound preparation containing active ingredients of a recombinant protein vaccine and/or an adenovirus vaccine for resisting infection with SARS-CoV-2 or the mutant thereof. Further, the compound preparation also contains other pharmaceutically acceptable excipients or complementary ingredients.

The present invention further provides a combined drug for preventing and/or treating infection with SARS-CoV-2 or a mutant thereof, where the combined drug contains a recombinant protein vaccine and an adenovirus vaccine for resisting infection with SARS-CoV-2 or the mutant thereof, and the recombinant protein vaccine and the adenovirus vaccine are administered separately or simultaneously.

Further, the pharmaceutical composition or the combined drug is an intramuscular injection, a nasal drop, a spray, a nasal spray or inhalation; and preferably, the pharmaceutical composition or the combined drug is a nasal spray.

Further, the recombinant protein vaccine and/or the adenovirus vaccine contain/contains a protein and/or a protein precursor for resisting infection with SARS-CoV-2 or the mutant thereof.

Further, the protein and/or the protein precursor contain/contains a full-length S protein or a protein formed by at least one RBD sequence and/or at least one HR sequence in the S protein of SARS-CoV-2 or the mutant thereof;
preferably, the RBD sequence is shown in SEQ ID No.1; or the RBD sequence is a variant that has homology and same or similar biological activity with SEQ ID No.1 and is obtained by substitution and/or deletion and/or insertion of at least one amino acid in the SEQ ID No.1 sequence; preferably, the RBD sequence is a variant obtained by substitution and/or deletion and/or insertion of 1-400 amino acids in the SEQ ID No.1 sequence; and preferably, the RBD sequence is a variant obtained by substitution and/or deletion and/or insertion of 5-30 amino acids in the SEQ ID No.1 sequence.

Further, the protein formed by the RBD sequence and the HR sequence in the S protein are capable of spontaneously forming a trimer.

Further, the homologous amino acid sequence is selected from at least one of RBD sequences of Alpha, Beta, Gamma, Delta or Omicron.

Further, protein precursor is that a signal peptide and/or a protein tag are/is bonded to the protein for resisting infection with SARS-CoV-2 or the mutant thereof; preferably, the signal peptide comprises the signal peptide of the S protein or the mutant thereof and/or a human tPA signal peptide additionally provided outside the forehead of the self-provided signal peptide; preferably, the protein tag is selected from at least one of a histidine tag, a thioredoxin tag, a glutathione transferase tag, a ubiquitin-like modified protein tag, a maltose-binding protein tag, a c-Myc protein tag or a Avi tag protein tag; and more preferably, the protein tag is a Trx tag and/or a 6His tag.

Further, the protein for resisting infection with SARS-CoV-2 or the mutant thereof is further bonded to a protease recognition zone for removing the protein tag; and preferably, the protease is selected from at least one of enterokinase, TEV protease, thrombin, coagulation factor Xa, carboxypeptidase A or rhinovirus 3c protease.

Further, the amino acid sequence of the protein and/or the protein precursor are/is selected from at least one of SEQ ID No.1, SEQ ID No.2, SEQ ID No.3, SEQ ID No.4, SEQ ID No.5, SEQ ID No.6, SEQ ID No.7, SEQ ID No.8, SEQ ID No.9, SEQ ID No.18, SEQ ID No.20, SEQ ID No.22, SEQ ID No.40, SEQ ID No.43, SEQ ID No.46, SEQ ID No.49, SEQ ID No.52, SEQ ID No.55, SEQ ID No.58, SEQ ID No.61, SEQ ID No.64, SEQ ID No.67, SEQ ID No.70, or SEQ ID No.73.

Further, a nucleotide sequence for encoding the amino acid sequence is shown in SEQ ID No.10, SEQ ID No.11, SEQ ID No.12, SEQ ID No.13, SEQ ID No.14, SEQ ID No.15, SEQ ID No.16, SEQ ID No.17, SEQ ID No.19, SEQ ID No.21, SEQ ID No.23, SEQ ID No.41, SEQ ID No.42, SEQ ID No.44, SEQ ID No.45, SEQ ID No.47, SEQ ID No.48, SEQ ID No.50, SEQ ID No.51, SEQ ID No.53, SEQ ID No.54, SEQ ID No.56, SEQ ID No.57, SEQ ID No.59, SEQ ID No.60, SEQ ID No.62, SEQ ID No.63, SEQ ID No.65, SEQ ID No.66, SEQ ID No.68, SEQ ID No.69, SEQ ID No.71, SEQ ID No.72, SEQ ID No.74, or SEQ ID No.75.

Further, the recombinant protein vaccine and/or the adenovirus vaccine contain/contains a nucleic acid for resisting infection with SARS-CoV-2 or the mutant thereof.

Further, the nucleotide sequence of the nucleic acid is shown in SEQ ID No.24, SEQ ID No.26, SEQ ID No.28, SEQ ID No.30, SEQ ID No.32, SEQ ID No.34, SEQ ID No.36, SEQ ID No.38, SEQ ID No.41, SEQ ID No.42, SEQ ID No.44, SEQ ID No.45, SEQ ID No.47, SEQ ID No.48, SEQ ID No.50, SEQ ID No.51, SEQ ID No.53, SEQ ID No.54, SEQ ID No.56, SEQ ID No.57, SEQ ID No.59, SEQ ID No.60, SEQ ID No.62, SEQ ID No.63, SEQ ID No.65, SEQ ID No.66, SEQ ID No.68, SEQ ID No.69, SEQ ID No.71, SEQ ID No.72, SEQ ID No.74, SEQ ID No.75, or SEQ ID No.76.

Further, the nucleotide sequence is obtained based on the encoded amino acid sequence SEQ ID No.25, SEQ ID No.27, SEQ ID No.29, SEQ ID No.31, SEQ ID No.33, SEQ ID No.35, SEQ ID No.37, SEQ ID No.39, SEQ ID No.40, SEQ ID No.43, SEQ ID No.46, SEQ ID No.49, SEQ ID No.52, SEQ ID No.55, SEQ ID No.58, SEQ ID No.61, SEQ ID No.64, SEQ ID No.67, SEQ ID No.70, SEQ ID No.73, or SEQ ID No.77 through optimization of codons or cells; and further, the cells are mammalian cells CHO or insect cells.

The present invention further provides a recombinant vector or an adenovirus vector, where the recombinant vector or the adenovirus vector contains a polynucleotide sequence in the recombinant protein vaccine or the adenovirus vaccine in the pharmaceutical composition or the combined drug, and the polynucleotide sequence is selected from at least one of SEQ ID No.10, SEQ ID No.11, SEQ ID No.12, SEQ ID No.13, SEQ ID No.14, SEQ ID No.15, SEQ ID No.16, SEQ ID No.17, SEQ ID No.19, SEQ ID No.21, SEQ ID No.23, SEQ ID No.24, SEQ ID No.26, SEQ ID No.28, SEQ ID No.30, SEQ ID No.32, SEQ ID No.34, SEQ ID No.36, SEQ ID No.38, SEQ ID No.41, SEQ ID No.42, SEQ ID No.44, SEQ ID No.45, SEQ ID No.47, SEQ ID No.48, SEQ ID No.50, SEQ ID No.51, SEQ ID No.53, SEQ ID No.54, SEQ ID No.56, SEQ ID No.57, SEQ ID No.59, SEQ ID No.60, SEQ ID No.62, SEQ ID No.63, SEQ ID No.65, SEQ ID No.66, SEQ ID No.68, SEQ ID No.69, SEQ ID No.71, SEQ ID No.72, SEQ ID No.74, SEQ ID No.75, or SEQ ID No.76.

Further, the recombinant vector is selected from at least one of an insect baculovirus expression vector, a mammalian cell expression vector, an Escherichia coli expression vector or a yeast expression vector; preferably, the insect baculovirus expression vector is pFastBac1; preferably, the mammalian cell expression vector is an CHO cell expression vector; further preferably, the CHO cell expression vector is pTT5 or FTP-002; preferably, the Escherichia coli expression vector is pET32a; and preferably, the yeast expression vector is pPICZaA.

Further, the adenovirus vector is a human replication-deficient recombinant adenovirus vector; preferably, the adenovirus vector is selected from a human type 5, type 35 or type 26 replication-deficient adenovirus or/and a chimpanzee type AdC68 or AdC7 replication-deficient adenovirus, and more preferably, is selected from a human type 5 replication-deficient adenovirus with combined deletion of E1 and E3.

The present invention also provides a host cell containing the recombinant vector or the adenovirus vector described above.

Further, the host cell is selected from at least one of insect cells, mammalian cells, Escherichia coli or yeasts; preferably, the insect cells are selected from at least one of sf9 cells, sf21 cells or Hi5 cells; and preferably, the mammalian cells are CHO cells or HEK293 cells.

Further, the recombinant protein vaccine and/or the adenovirus vaccine further comprise/comprises a pharmaceutically acceptable excipient or a complementary ingredient.

Further, the complementary ingredient is an immunologic adjuvant; preferably, the immunologic adjuvant is selected from at least one of a squalene oil-in-water emulsion, aluminum salt, calcium salt, plant saponin, plant polysaccharide, monophosphoryl lipid A, muramyl dipeptide, muramyl tripeptide, bacterial toxin, GM-CSF cytokines, lipid or cationic liposome materials;
further, the immunologic adjuvant satisfies at least one the squalene oil-in-water emulsion being MF59, the aluminum salt being selected from at least one of aluminum hydroxide or alum, the calcium salt being tricalcium phosphate, the plant saponin being QS-21 or ISCOM, the plant polysaccharide being astragalus polysaccharide, the bacterial toxin being selected from at least one of recombinant cholera toxin or diphtheria toxin, the lipid being selected from at least one of phosphatidylethanolamine, phosphatidylcholine, cholesterol or dioleoyl phosphatidylethanolamine, or the cationic liposome material being selected from at least one of (2,3-dioleoxypropyl) trimethyl ammonium chloride, N-[1-(2,3-dioleoyl chloride) propyl ]-N,N,N-trimethylamine chloride, cationic cholesterol, dimethyl-2, 3-dioleoxypropyl-2-(2-arginine formyl amino) ethyl ammonium trimethyldodecylammonium bromide, dodecyltrimethylammonium bromide, tetradecyltrimethylammonium bromide, hexadecyltrimethylammonium bromide, dimethyldioctadecylammonium bromide or CpG ODN.

Further, the vaccine preparations of the recombinant protein vaccine and the adenovirus vaccine are an intramuscular injection, an injection, a nasal drop, a spray, a nasal spray or inhalation; and preferably, the vaccine is a nasal spray.

The present invention further provides use of the pharmaceutical composition or the combined drug in preparation of a drug for preventing and/or treating infection with SARS-CoV-2 or a mutant thereof.

The present invention further provides use of the pharmaceutical composition or the combined drug in preparation of a drug for treating and/or preventing SARS-COV-2 variant strain infection or pathopoiesis.

Further, the SARS-COV-2 variant strain comprises at least one of Alpha, Beta, Gamma, Delta or Omicron.

The present invention provides use of the pharmaceutical composition or the combined drug in preparation of a drug for treating and/or preventing influenza virus or other respiratory virus infection or pathopoiesis.

The present invention further provides a preparation method of the protein, and the method comprises the following steps: culturing the host cell to express the required protein or precursor, and then recovering the required protein.

The present invention further provides a preparation method of adenovirus, and the method comprises the following steps: constructing a plasmid vector containing the polynucleotide; transfecting the host cell and culturing the transfected host cell; obtaining the recombinant adenovirus; extending the culture of the recombinant adenovirus; and purifying the recombinant adenovirus.

The polynucleotide sequence is selected from at least one of SEQ ID No.10, SEQ ID No.11, SEQ ID No.12, SEQ ID No.13, SEQ ID No.14, SEQ ID No.15, SEQ ID No.16, SEQ ID No.17, SEQ ID No.19, SEQ ID No.21, SEQ ID No.23, SEQ ID No.24, SEQ ID No.26, SEQ ID No.28, SEQ ID No.30, SEQ ID No.32, SEQ ID No.34, SEQ ID No.36, SEQ ID No.38, SEQ ID No.41, SEQ ID No.42, SEQ ID No.44, SEQ ID No.45, SEQ ID No.47, SEQ ID No.48, SEQ ID No.50, SEQ ID No.51, SEQ ID No.53, SEQ ID No.54, SEQ ID No.56, SEQ ID No.57, SEQ ID No.59, SEQ ID No.60, SEQ ID No.62, SEQ ID No.63, SEQ ID No.65, SEQ ID No.66, SEQ ID No.68, SEQ ID No.69, SEQ ID No.71, SEQ ID No.72, SEQ ID No.74, SEQ ID No.75, or SEQ ID No.76.

The present invention further provides a preparation method of the recombinant protein, and the method comprises the following steps: constructing a recombinant vector containing the polynucleotide, and immunizing a human body to produce the protein.

Further, the vector is selected from at least one of mRNA, DNA vaccine, adenovirus, vaccinia Ankara virus, or adeno-associated virus.

The present invention further provides a second pharmaceutical composition that contains the recombinant protein vaccine and an inactivated virus vaccine as active ingredients. Further, the inactivated virus vaccine is an inactivated influenza virus vaccine.

The present invention further provides a third pharmaceutical composition that contains the adenovirus vaccine and an influenza virus recombinant protein vaccine as active ingredients.

The present invention further provides use of the second or third pharmaceutical composition in preparation of a drug for treating and/or preventing respiratory virus infection or pathopoiesis.

In the three pharmaceutical compositions of the present invention, the active ingredients are combined in any combination ratio.

Further, in the pharmaceutical compositions, the volume ratio of the recombinant protein vaccine to the adenovirus vaccine is 1-5: 1-5.

The present invention further provides a second combined drug, where the recombinant protein vaccine and the inactivated virus vaccine are administered separately or simultaneously. Further, the inactivated virus vaccine is an inactivated influenza virus vaccine.

The present invention further provides a third combined drug, where the adenovirus vaccine and the influenza virus recombinant protein vaccine are administered separately or simultaneously.

Preferably, the influenza virus is selected from influenza A virus and/or influenza B virus.

Further preferably, the influenza A virus is influenza A (H1N1) virus.

SEQ ID No.1: original strain (Wuhan strain)

VQPTESIVRFPNITNLCPFGEVFNATRFASVYAWNRKRISNCVADYSVLYNSASFSTFKC

YGVSPTKLNDLCFTNVYADSFVIRGDEVRQIAPGQTGKIADYNYKLPDDFTGCVIAWNSNN

LDSKVGGNYNYLYRLFRKSNLKPFERDISTEIYQAGSTPCNGVEGFNCYFPLQSYGFQPTNG

VGYQPYRVVVLSFELLHAPATVCGPKKSTNLVKNKCVNFNFNG

SEQ ID No.1 shares a same amino acid sequence with sites 320-545 in the S protein of SARS-CoV-2.

SEQ ID No.2: RBD-HR-Beta sequence
MLLVNQSHQGFNKEHTSKMVSAIVLYVLLAAAAHSAFAADSIHIKDSDDLKNRLAEA
GDKLVVIDFMATWCGPCKMIGPKLDEMANEMSDSIVVLKVDVDECEDIATEYNINSMPTFV
FVKNSKKIEEFSGANVDKLRNTIIKLKLAGSGSGHMHHHHHHSSGDDDDKVQPTESIVRFP
NITNLCPFGEVFNATRFASVYAWNRKRISNCVADYSVLYNSASFSTFKCYGVSPTKLNDLCF
TNVYADSFVIRGDEVRQIAPGQTGNIADYNYKLPDDFTGCVIAWNSNNLDSKVGGNYNYR
YRLFRKSNLKPFERDISTEIYQAGSTPCNGVKGFNCYFPLQSYGFQPTYGVGYQPYRVVVLS
FELLHAPATVCGPKKSTNLVKNKSVNFNFNGLYENQKLIANQFNSAIGKIQDSLSSTASALG
KLQDVVNQNAQALNTLVKQLKNHTSPDVDLGDISGINASVVNIQKEIDRLNEVAKNLNESL
IDLQEL

The above sequence consists of signal peptide-Trx tag-6His tag-EK restriction enzyme cutting site-RBD sequence-HR1 sequence-HR2 sequence. For the RBD sequence, the amino acid at the site 98 of the SEQ ID No.1 sequence is replaced by K with N, the amino acid at the site 133 is replaced by L with R, the amino acid at the site 165 is replaced by E with K, the amino acid at the site 182 is replaced by N with Y, and the amino acid at the site 219 is replaced by C with S.

SEQ ID No.3: RBD-HR-Delta sequence
MLLVNQSHQGFNKEHTSKMVSAIVLYVLLAAAAHSAFAADSIHIKDSDDLKNRLAEA
GDKLVVIDFMATWCGPCKMIGPKLDEMANEMSDSIVVLKVDVDECEDIATEYNINSMPTFV

FVKNSKKIEEFSGANVDKLRNTIIKLKLAGSGSGHMHHHHHHSSGDDDDKVQPTESIVRFP
NITNLCPFGEVFNATRFASVYAWNRKRISNCVADYSVLYNSASFSTFKCYGVSPTKLNDLCF
TNVYADSFVIRGDEVRQIAPGQTGKIADYNYKLPDDFTGCVIAWNSNNLDSKVGGNYNYR
YRLFRKSNLKPFERDISTEIYQAGSKPCNGVEGFNCYFPLQSYGFQPTNGVGYQPYRVVVLS
FELLHAPATVCGPKKSTNLVKNKSVNFNFNGLYENQKLIANQFNSAIGKIQDSLSSTASALG
KLQDVVNQNAQALNTLVKQLKNHTSPDVDLGDISGINASVVNIQKEIDRLNEVAKNLNESL
IDLQEL

The above sequence consists of signal peptide-Trx tag-6His tag-EK restriction enzyme cutting site-RBD sequence-HR1 sequence-HR2 sequence. For the RBD sequence, the amino acid at the site 133 of the SEQ ID No.1 sequence is replaced by L with R, the amino acid at the site 159 is replaced by T with K, and the amino acid at the site 219 is replaced by C with S.

SEQ ID No.4: RBD-HR- Omicron BA.1

MLLVNQSHQGFNKEHTSKMVSAIVLYVLLAAAAHSAFAADSIHIKDSDDLKNRLAEA

GDKLVVIDFMATWCGPCKMIGPKLDEMANEMSDSIVVLKVDVDECEDIATEYNINSMPTFV

FVKNSKKIEEFSGANVDKLRNTIIKLKLAGSGSGHMHHHHHHSSGDDDDKVQPTESIVRFP

NITNLCPFDEVFNATRFASVYAWNRKRISNCVADYSVLYNLAPFFTFKCYGVSPTKLNDLCF

TNVYADSFVIRGDEVRQIAPGQTGNIADYNYKLPDDFTGCVIAWNSNKLDSKVSGNYNYLY

RLFRKSNLKPFERDISTEIYQAGNKPCNGVAGFNCYFPLRSYSFRPTYGVGHQPYRVVVLSF

ELLHAPATVCGPKKSTNLVKNKSVNFNFNGLYENQKLIANQFNSAIGKIQDSLSSTASALGK

LQDVVNQNAQALNTLVKQLKNHTSPDVDLGDISGINASVVNIQKEIDRLNEVAKNLNESLI

DLQEL

The above sequence consists of signal peptide-Trx tag-6His tag-EK restriction enzyme cutting site-RBD sequence-HR1 sequence-HR2 sequence. The RBD sequence is obtained by mutating five or more amino acid sequences in the SEQ ID No.1 sequence.

SEQ ID No.5: RBD-HR-Omicron BA.2
MLLVNQSHQGFNKEHTSKMVSAIVLYVLLAAAAHSAFAADSIHIKDSDDLKNRLAEA
GDKLVVIDFMATWCGPCKMIGPKLDEMANEMSDSIVVLKVDVDECEDIATEYNINSMPTFV
FVKNSKKIEEFSGANVDKLRNTIIKLKLAGSGSGHMHHHHHHSSGDDDDKVQPTESIVRFP
NITNLCPFDEVFNATRFASVYAWNRKRISNCVADYSVLYNFAPFFAFKCYGVSPTKLNDLCF

TNVYADSFVIRGNEVSQIAPGQTGNIADYNYKLPDDFTGCVIAWNSNKLDSKVGGNYNYL
YRLFRKSNLKPFERDISTEIYQAGNKPCNGVAGFNCYFPLRSYGFRPTYGVGHQPYRVVVLS
FELLHAPATVCGPKKSTNLVKNKSVNFNFNGLYENQKLIANQFNSAIGKIQDSLSSTASALG
KLQDVVNQNAQALNTLVKQLKNHTSPDVDLGDISGINASVVNIQKEIDRLNEVAKNLNESL
IDLQEL

The above sequence consists of signal peptide-Trx tag-6His tag-EK restriction enzyme cutting site-RBD sequence-HR1 sequence-HR2 sequence. The RBD sequence is obtained by mutating five or more amino acid sequences in the SEQ ID No.1 sequence.

SEQ ID No.6: RBD-HR-Omicron BA.4/.5

MLLVNQSHQGFNKEHTSKMVSAIVLYVLLAAAAHSAFAADSIHIKDSDDLKNRLAEA

GDKLVVIDFMATWCGPCKMIGPKLDEMANEMSDSIVVLKVDVDECEDIATEYNINSMPTFV

FVKNSKKIEEFSGANVDKLRNTIIKLKLAGSGSGHMHHHHHHSSGDDDDKVQPTESIVRFP

NITNLCPFDEVFNATRFASVYAWNRKRISNCVADYSVLYNFAPFFAFKCYGVSPTKLNDLCF

TNVYADSFVIRGNEVSQIAPGQTGNIADYNYKLPDDFTGCVIAWNSNKLDSKVGGNYNYR

YRLFRKSNLKPFERDISTEIYQAGNKPCNGVAGVNCYFPLQSYGFRPTYGVGHQPYRVVVL

SFELLHAPATVCGPKKSTNLVKNKSVNFNFNGLYENQKLIANQFNSAIGKIQDSLSSTASALG

KLQDVVNQNAQALNTLVKQLKNHTSPDVDLGDISGINASVVNIQKEIDRLNEVAKNLNESL

IDLQEL

The above sequence consists of signal peptide-Trx tag-6His tag-EK restriction enzyme cutting site-RBD sequence-HR1 sequence-HR2 sequence. The RBD sequence is obtained by mutating five or more amino acid sequences in the SEQ ID No.1 sequence.

SEQ ID No.7: RBD-HR-Omicron BA2.12.1
MLLVNQSHQGFNKEHTSKMVSAIVLYVLLAAAAHSAFAADSIHIKDSDDLKNRLAEA
GDKLVVIDFMATWCGPCKMIGPKLDEMANEMSDSIVVLKVDVDECEDIATEYNINSMPTFV
FVKNSKKIEEFSGANVDKLRNTIIKLKLAGSGSGHMHHHHHHSSGDDDDKVQPTESIVRFP
NITNLCPFDEVFNATRFASVYAWNRKRISNCVADYSVLYNFAPFFAFKCYGVSPTKLNDLCF
TNVYADSFVIRGNEVSQIAPGQTGNIADYNYKLPDDFTGCVIAWNSNKLDSKVGGNYNYQ
YRLFRKSNLKPFERDISTEIYQAGNKPCNGVAGFNCYFPLRSYGFRPTYGVGHQPYRVVVLS
FELLHAPATVCGPKKSTNLVKNKSVNFNFNGLYENQKLIANQFNSAIGKIQDSLSSTASALG

KLQDVVNQNAQALNTLVKQLKNHTSPDVDLGDISGINASVVNIQKEIDRLNEVAKNLNESL
IDLQEL

The above sequence consists of signal peptide-Trx tag-6His tag-EK restriction enzyme cutting site-RBD sequence-HR1 sequence-HR2 sequence. The RBD sequence is obtained by mutating five or more amino acid sequences in the SEQ ID No.1 sequence.

SEQ ID No.8: RBD-HR- BF.7
MLLVNQSHQGFNKEHTSKMVSAIVLYVLLAAAAHSAFAADSIHIKDSDDLKNRLAEA
GDKLVVIDFMATWCGPCKMIGPKLDEMANEMSDSIVVLKVDVDECEDIATEYNINSMPTFV
FVKNSKKIEEFSGANVDKLRNTIIKLKLAGSGSGHMHHHHHHSSGDDDDKVQPTESIVRFP
NITNLCPFDEVFNATTFASVYAWNRKRISNCVADYSVLYNFAPFFAFKCYGVSPTKLNDLCF
TNVYADSFVIRGNEVSQIAPGQTGNIADYNYKLPDDFTGCVIAWNSNKLDSKVGGNYNYR
YRLFRKSNLKPFERDISTEIYQAGNKPCNGVAGVNCYFPLQSYGFRPTYGVGHQPYRVVVL
SFELLHAPATVCGPKKSTNLVKNKSVNFNFNGLYENQKLIANQFNSAIGKIQDSLSSTASALG
KLQDVVNQNAQALNTLVKQLKNHTSPDVDLGDISGINASVVNIQKEIDRLNEVAKNLNESL
IDLQEL

The above sequence consists of signal peptide-Trx tag-6His tag-EK restriction enzyme cutting site-RBD sequence-HR1 sequence-HR2 sequence. The RBD sequence is obtained by mutating five or more amino acid sequences in the SEQ ID No.1 sequence.

SEQ ID No.9: RBD-HR-BQ.1

MLLVNQSHQGFNKEHTSKMVSAIVLYVLLAAAAHSAFAADSIHIKDSDDLKNRLAEA

GDKLVVIDFMATWCGPCKMIGPKLDEMANEMSDSIVVLKVDVDECEDIATEYNINSMPTFV

FVKNSKKIEEFSGANVDKLRNTIIKLKLAGSGSGHMHHHHHHSSGDDDDKVQPTESIVRFP

NITNLCPFDEVFNATRFASVYAWNRKRISNCVADYSVLYNFAPFFAFKCYGVSPTKLNDLCF

TNVYADSFVIRGNEVSQIAPGQTGNIADYNYKLPDDFTGCVIAWNSNKLDSTVGGNYNYR

YRLFRKSKLKPFERDISTEIYQAGNKPCNGVAGVNCYFPLQSYGFRPTYGVGHQPYRVVVL

SFELLHAPATVCGPKKSTNLVKNKSVNFNFNGLYENQKLIANQFNSAIGKIQDSLSSTASALG

KLQDVVNQNAQALNTLVKQLKNHTSPDVDLGDISGINASVVNIQKEIDRLNEVAKNLNESL

IDLQEL

The above sequence consists of signal peptide-Trx tag-6His tag-EK restriction enzyme cutting site-RBD sequence-HR1 sequence-HR2 sequence. The RBD sequence is obtained by mutating five or more amino acid sequences in the SEQ ID No.1 sequence.

SEQ ID No.10 (nucleotide sequence encoding SEQ ID No.2):
ATGCTACTAGTAAATCAGTCACACCAAGGCTTCAATAAGGAACACACAAGCAAGAT
GGTGAGCGCTATCGTGCTGTACGTCCTGCTGGCCGCTGCTGCTCACAGCGCTTTCGCTG
CTGACTCCATCCACATCAAGGACAGCGACGACCTGAAGAACCGTCTGGCCGAGGCCGG
TGACAAGCTGGTCGTCATCGACTTCATGGCCACTTGGTGCGGTCCTTGCAAGATGATCG
GCCCTAAGCTGGACGAGATGGCTAACGAGATGTCCGACAGCATCGTGGTCCTGAAGGTG
GACGTCGACGAGTGCGAGGACATCGCCACCGAATACAACATCAACAGCATGCCCACCTT
CGTGTTCGTGAAGAACAGCAAGAAGATCGAGGAATTTTCCGGCGCTAACGTCGACAAG
CTGCGTAACACCATCATCAAGCTGAAGCTGGCCGGCTCCGGCTCCGGCCACATGCATCA
CCACCACCACCATTCCTCCGGTGACGACGACGACAAGGTGCAGCCAACCGAATCTATCG
TCAGATTCCCAAACATCACTAACCTGTGCCCTTTCGGAGAGGTGTTCAACGCTACCAGG
TTCGCCAGCGTCTACGCTTGGAACCGCAAGCGTATCAGCAACTGCGTCGCCGACTACTC
TGTGCTGTACAACTCCGCTAGCTTCTCTACTTTCAAGTGCTACGGCGTGTCACCTACCAA
GCTGAACGACCTGTGCTTCACTAACGTCTACGCCGACTCCTTCGTGATCCGCGGAGACG
AAGTCCGTCAGATCGCTCCTGGACAGACCGGAAATATCGCTGACTACAACTACAAGCTG
CCAGACGACTTCACTGGCTGCGTGATCGCTTGGAACTCAAACAACCTGGACTCCAAGG
TCGGTGGCAACTACAACTACCGGTACAGGCTGTTCAGAAAGTCAAACCTGAAGCCTTTC
GAGCGCGACATCTCAACCGAAATCTACCAGGCTGGTTCCACTCCCTGCAACGGTGTGAA
GGGCTTCAACTGCTACTTCCCCCTGCAGTCCTACGGTTTCCAGCCAACCTATGGAGTCGG
TTACCAGCCTTACCGTGTGGTCGTGCTGAGCTTCGAACTGCTCCACGCTCCTGCTACTGT
GTGCGGTCCCAAGAAGTCTACTAACCTGGTCAAAAACAAAAGCGTCAACTTCAACTTC
AACGGTCTGTACGAGAACCAGAAGCTGATCGCTAACCAGTTCAACAGCGCCATCGGCA
AGATCCAGGACTCACTGTCATCCACTGCCTCCGCTCTGGGAAAGCTGCAGGACGTCGTG
AACCAGAACGCCCAGGCTCTGAACACCCTGGTCAAGCAGCTGAAGAACCACACCTCTC
CTGACGTCGACCTGGGCGACATCAGCGGAATCAACGCTTCTGTCGTGAACATCCAGAAG
GAGATCGACCGCCTGAACGAAGTGGCCAAGAACCTGAACGAATCCCTGATTGACCTCC
AAGAACTCTAA

Signal peptide coding sequence - Trx tag coding sequence - 6His tag coding sequence - EK restriction enzyme cutting site coding sequence - RBD coding sequence - HR1 coding sequence - HR2 coding sequence

SEQ ID No.11 (nucleotide sequence encoding SEQ ID No.3):
ATGCTACTAGTAAATCAGTCACACCAAGGCTTCAATAAGGAACACACAAGCAAGAT
GGTGAGCGCTATCGTGCTGTACGTCCTGCTGGCCGCTGCTGCTCACAGCGCTTTCGCTG
CTGACTCCATCCACATCAAGGACAGCGACGACCTGAAGAACCGTCTGGCCGAGGCCGG
TGACAAGCTGGTCGTCATCGACTTCATGGCCACTTGGTGCGGTCCTTGCAAGATGATCG
GCCCTAAGCTGGACGAGATGGCTAACGAGATGTCCGACAGCATCGTGGTCCTGAAGGTG
GACGTCGACGAGTGCGAGGACATCGCCACCGAATACAACATCAACAGCATGCCCACCTT
CGTGTTCGTGAAGAACAGCAAGAAGATCGAGGAATTTTCCGGCGCTAACGTCGACAAG
CTGCGTAACACCATCATCAAGCTGAAGCTGGCCGGCTCCGGCTCCGGCCACATGCATCA
CCACCACCACCATTCCTCCGGTGACGACGACGACAAGGTGCAGCCAACCGAATCTATCG
TCAGATTCCCAAACATCACTAACCTGTGCCCTTTCGGAGAGGTGTTCAACGCTACCAGG
TTCGCCAGCGTCTACGCTTGGAACCGCAAGCGTATCAGCAACTGCGTCGCCGACTACTC
TGTGCTGTACAACTCCGCTAGCTTCTCTACTTTCAAGTGCTACGGCGTGTCACCTACCAA
GCTGAACGACCTGTGCTTCACTAACGTCTACGCCGACTCCTTCGTGATCCGCGGAGACG
AAGTCCGTCAGATCGCTCCTGGACAGACCGGAAAGATCGCTGACTACAACTACAAGCT
GCCAGACGACTTCACTGGCTGCGTGATCGCTTGGAACTCAAACAACCTGGACTCCAAG
GTCGGTGGCAACTACAACTACCGGTACAGGCTGTTCAGAAAGTCAAACCTGAAGCCTTT
CGAGCGCGACATCTCAACCGAAATCTACCAGGCTGGTTCCAAGCCCTGCAACGGTGTGG
AGGGCTTCAACTGCTACTTCCCCCTGCAGTCCTACGGTTTCCAGCCAACCAACGGAGTC
GGTTACCAGCCTTACCGTGTGGTCGTGCTGAGCTTCGAACTGCTCCACGCTCCTGCTACT
GTGTGCGGTCCCAAGAAGTCTACTAACCTGGTCAAAAACAAAAGCGTCAACTTCAACT
TCAACGGTCTGTACGAGAACCAGAAGCTGATCGCTAACCAGTTCAACAGCGCCATCGGC
AAGATCCAGGACTCACTGTCATCCACTGCCTCCGCTCTGGGAAAGCTGCAGGACGTCGT
GAACCAGAACGCCCAGGCTCTGAACACCCTGGTCAAGCAGCTGAAGAACCACACCTCT
CCTGACGTCGACCTGGGCGACATCAGCGGAATCAACGCTTCTGTCGTGAACATCCAGAA
GGAGATCGACCGCCTGAACGAAGTGGCCAAGAACCTGAACGAATCCCTGATTGACCTC
CAAGAACTCTAA

Signal peptide coding sequence - Trx tag coding sequence - 6His tag coding sequence - EK restriction enzyme cutting site coding sequence - RBD coding sequence - HR1 coding sequence - HR2 coding sequence

SEQ ID No.12 (nucleotide sequence encoding SEQ ID No.4):
ATGCTACTAGTAAATCAGTCACACCAAGGCTTCAATAAGGAACACACAAGCAAGAT
GGTGAGCGCTATCGTGCTGTACGTCCTGCTGGCCGCTGCTGCTCACAGCGCTTTCGCTG
CTGACTCCATCCACATCAAGGACAGCGACGACCTGAAGAACCGTCTGGCCGAGGCCGG
TGACAAGCTGGTCGTCATCGACTTCATGGCCACTTGGTGCGGTCCTTGCAAGATGATCG
GCCCTAAGCTGGACGAGATGGCTAACGAGATGTCCGACAGCATCGTGGTCCTGAAGGTG
GACGTCGACGAGTGCGAGGACATCGCCACCGAATACAACATCAACAGCATGCCCACCTT
CGTGTTCGTGAAGAACAGCAAGAAGATCGAGGAATTTTCCGGCGCTAACGTCGACAAG
CTGCGTAACACCATCATCAAGCTGAAGCTGGCCGGCTCCGGCTCCGGCCACATGCATCA
CCACCACCACCATTCCTCCGGTGACGACGACGACAAGGTGCAGCCAACCGAATCTATCG
TCAGATTCCCAAACATCACTAACCTGTGCCCTTTCGATGAGGTGTTCAACGCTACCAGGT
TCGCCAGCGTCTACGCTTGGAACCGCAAGCGTATCAGCAACTGCGTCGCCGACTACTCT
GTGCTGTACAACCTCGCTCCTTTCTTCACTTTCAAGTGCTACGGCGTGTCACCTACCAAG
CTGAACGACCTGTGCTTCACTAACGTCTACGCCGACTCCTTCGTGATCCGCGGAGACGA
AGTCCGTCAGATCGCTCCTGGACAGACCGGAAATATCGCTGACTACAACTACAAGCTGC
CAGACGACTTCACTGGCTGCGTGATCGCTTGGAACTCAAACAAGCTGGACTCCAAGGT
CAGTGGCAACTACAACTACCTGTACAGGCTGTTCAGAAAGTCAAACCTGAAGCCTTTCG
AGCGCGACATCTCAACCGAAATCTACCAGGCTGGTAATAAGCCCTGCAACGGTGTGGCT
GGCTTCAACTGCTACTTCCCCCTGAGATCCTACTCTTTCCGACCAACCTACGGAGTCGGT
CATCAGCCTTACCGTGTGGTCGTGCTGAGCTTCGAACTGCTCCACGCTCCTGCTACTGTG
TGCGGTCCCAAGAAGTCTACTAACCTGGTCAAAAACAAAAGCGTCAACTTCAACTTCA
ACGGTCTGTACGAGAACCAGAAGCTGATCGCTAACCAGTTCAACAGCGCCATCGGCAA
GATCCAGGACTCACTGTCATCCACTGCCTCCGCTCTGGGAAAGCTGCAGGACGTCGTGA
ACCAGAACGCCCAGGCTCTGAACACCCTGGTCAAGCAGCTGAAGAACCACACCTCTCC
TGACGTCGACCTGGGCGACATCAGCGGAATCAACGCTTCTGTCGTGAACATCCAGAAG
GAGATCGACCGCCTGAACGAAGTGGCCAAGAACCTGAACGAATCCCTGATTGACCTCC
AAGAACTCTAA

Signal peptide coding sequence - Trx tag coding sequence - 6His tag coding sequence - EK restriction enzyme cutting site coding sequence - RBD coding sequence - HR1 coding sequence - HR2 coding sequence

SEQ ID No.13 (nucleotide sequence encoding SEQ ID No.5):
ATGCTGCTGGTGAACCAGAGCCACCAGGGTTTCAACAAGGAACACACTAGCAAGA
TGGTGTCTGCTATCGTCCTCTACGTGCTGCTCGCCGCCGCTGCTCACTCAGCTTTCGCCG
CTGACTCTATCCACATCAAGGACTCCGACGACCTCAAGAACCGTTTGGCCGAAGCCGGC
GACAAGCTGGTCGTTATCGACTTCATGGCTACCTGGTGTGGCCCTTGCAAGATGATCGG
ACCTAAGCTGGACGAAATGGCTAACGAGATGAGCGACAGCATCGTCGTTCTGAAGGTG
GACGTCGACGAATGTGAGGACATCGCCACTGAGTACAACATCAACAGCATGCCTACCTT
CGTTTTCGTGAAGAACTCTAAGAAGATCGAGGAGTTCTCTGGTGCCAACGTGGACAAG
CTCCGTAACACCATCATCAAGCTCAAGCTGGCCGGCAGCGGTAGCGGTCACATGCACCA
CCACCACCACCATTCAAGCGGCGACGACGACGACAAGGTGCAGCCAACCGAATCAATC
GTCCGCTTCCCAAACATCACTAACCTGTGCCCATTCGACGAGGTGTTCAACGCCACCCG
TTTCGCCTCTGTCTACGCTTGGAACCGCAAGCGCATCTCCAACTGTGTCGCCGACTACA
GCGTGCTGTACAACTTCGCTCCTTTCTTCGCATTCAAGTGTTACGGAGTGTCCCCTACCA
AGCTCAACGATTTGTGCTTCACAAACGTGTACGCTGACAGCTTCGTGATCCGTGGTAAC
GAGGTTTCTCAAATCGCCCCTGGTCAGACAGGTAACATCGCTGACTACAACTACAAGCT
CCCAGACGACTTCACTGGCTGTGTTATCGCTTGGAACTCAAACAAGCTGGACTCTAAGG
TGGGTGGTAACTACAACTACCTCTACCGTCTGTTCCGCAAGAGCAACCTCAAGCCATTC
GAACGTGACATCAGCACAGAGATCTACCAAGCCGGTAACAAGCCCTGCAACGGCGTTG
CCGGATTCAACTGCTACTTCCCCCTCCGTTCCTACGGATTCCGCCCAACCTACGGCGTGG
GTCACCAGCCATACCGCGTGGTTGTGCTGAGCTTCGAACTGCTGCACGCCCCCGCTACC
GTGTGTGGCCCTAAGAAGAGCACTAACCTCGTTAAGAACAAGTCCGTGAACTTCAACTT
CAACGGTCTCTACGAAAACCAGAAGCTGATCGCTAACCAGTTCAACTCCGCCATCGGCA
AGATCCAGGACTCCCTGTCCTCCACAGCTTCCGCTCTGGGCAAGCTGCAGGACGTTGTG
AACCAAAACGCTCAGGCCCTGAACACACTGGTGAAGCAACTGAAGAACCACACCAGC
CCCGACGTTGACCTGGGTGACATCAGCGGTATCAACGCTAGCGTGGTGAACATCCAGAA
GGAGATCGACAGGCTGAACGAAGTGGCTAAGAACTTGAACGAGAGCCTCATCGACCTG
CAGGAACTGTAA

Signal peptide coding sequence - Trx tag coding sequence - 6His tag coding sequence - EK restriction enzyme cutting site coding sequence - RBD coding sequence - HR1 coding sequence - HR2 coding sequence

SEQ ID No.14 (nucleotide sequence encoding SEQ ID No.6):
ATGCTGCTGGTGAACCAGAGCCACCAGGGTTTCAACAAGGAACACACTAGCAAGA
TGGTGTCTGCTATCGTCCTCTACGTGCTGCTCGCCGCCGCTGCTCACTCAGCTTTCGCCG
CTGACTCTATCCACATCAAGGACTCCGACGACCTCAAGAACCGTTTGGCCGAAGCCGGC
GACAAGCTGGTCGTTATCGACTTCATGGCTACCTGGTGTGGCCCTTGCAAGATGATCGG
ACCTAAGCTGGACGAAATGGCTAACGAGATGAGCGACAGCATCGTCGTTCTGAAGGTG
GACGTCGACGAATGTGAGGACATCGCCACTGAGTACAACATCAACAGCATGCCTACCTT
CGTTTTCGTGAAGAACTCTAAGAAGATCGAGGAGTTCTCTGGTGCCAACGTGGACAAG
CTCCGTAACACCATCATCAAGCTCAAGCTGGCCGGCAGCGGTAGCGGTCACATGCACCA
CCACCACCACCATTCAAGCGGCGACGACGACGACAAGGTGCAGCCAACCGAATCAATC
GTCCGCTTCCCAAACATCACTAACCTGTGCCCATTCGACGAGGTGTTCAACGCCACCCG
TTTCGCCTCTGTCTACGCTTGGAACCGCAAGCGCATCTCCAACTGTGTCGCCGACTACA
GCGTGCTGTACAACTTCGCTCCTTTCTTCGCATTCAAGTGTTACGGAGTGTCCCCTACCA
AGCTCAACGATTTGTGCTTCACAAACGTGTACGCTGACAGCTTCGTGATCCGTGGTAAC
GAGGTTTCTCAAATCGCCCCTGGTCAGACAGGTAACATCGCTGACTACAACTACAAGCT
CCCAGACGACTTCACTGGCTGTGTTATCGCTTGGAACTCAAACAAGCTGGACTCTAAGG
TGGGTGGTAACTACAACTACCGGTACCGTCTGTTCCGCAAGAGCAACCTCAAGCCATTC
GAACGTGACATCAGCACAGAGATCTACCAAGCCGGTAACAAGCCCTGCAACGGCGTTG
CCGGAGTCAACTGCTACTTCCCCCTCCAGTCCTACGGATTCCGCCCAACCTACGGCGTG
GGTCACCAGCCATACCGCGTGGTTGTGCTGAGCTTCGAACTGCTGCACGCCCCCGCTAC
CGTGTGTGGCCCTAAGAAGAGCACTAACCTCGTTAAGAACAAGTCCGTGAACTTCAACT
TCAACGGTCTCTACGAAAACCAGAAGCTGATCGCTAACCAGTTCAACTCCGCCATCGGC
AAGATCCAGGACTCCCTGTCCTCCACAGCTTCCGCTCTGGGCAAGCTGCAGGACGTTGT
GAACCAAAACGCTCAGGCCCTGAACACACTGGTGAAGCAACTGAAGAACCACACCAG
CCCCGACGTTGACCTGGGTGACATCAGCGGTATCAACGCTAGCGTGGTGAACATCCAGA
AGGAGATCGACAGGCTGAACGAAGTGGCTAAGAACTTGAACGAGAGCCTCATCGACCT
GCAGGAACTGTAA

Signal peptide coding sequence - Trx tag coding sequence - 6His tag coding sequence - EK restriction enzyme cutting site coding sequence - RBD coding sequence - HR1 coding sequence - HR2 coding sequence

SEQ ID No.15 (nucleotide sequence encoding SEQ ID No.7):
ATGCTGCTGGTGAACCAGAGCCACCAGGGTTTCAACAAGGAACACACTAGCAAGA
TGGTGTCTGCTATCGTCCTCTACGTGCTGCTCGCCGCCGCTGCTCACTCAGCTTTCGCCG
CTGACTCTATCCACATCAAGGACTCCGACGACCTCAAGAACCGTTTGGCCGAAGCCGGC
GACAAGCTGGTCGTTATCGACTTCATGGCTACCTGGTGTGGCCCTTGCAAGATGATCGG
ACCTAAGCTGGACGAAATGGCTAACGAGATGAGCGACAGCATCGTCGTTCTGAAGGTG
GACGTCGACGAATGTGAGGACATCGCCACTGAGTACAACATCAACAGCATGCCTACCTT
CGTTTTCGTGAAGAACTCTAAGAAGATCGAGGAGTTCTCTGGTGCCAACGTGGACAAG
CTCCGTAACACCATCATCAAGCTCAAGCTGGCCGGCAGCGGTAGCGGTCACATGCACCA
CCACCACCACCATTCAAGCGGCGACGACGACGACAAGGTGCAGCCAACCGAATCAATC
GTCCGCTTCCCAAACATCACTAACCTGTGCCCATTCGACGAGGTGTTCAACGCCACCCG
TTTCGCCTCTGTCTACGCTTGGAACCGCAAGCGCATCTCCAACTGTGTCGCCGACTACA
GCGTGCTGTACAACTTCGCTCCTTTCTTCGCATTCAAGTGTTACGGAGTGTCCCCTACCA
AGCTCAACGATTTGTGCTTCACAAACGTGTACGCTGACAGCTTCGTGATCCGTGGTAAC
GAGGTTTCTCAAATCGCCCCTGGTCAGACAGGTAACATCGCTGACTACAACTACAAGCT
CCCAGACGACTTCACTGGCTGTGTTATCGCTTGGAACTCAAACAAGCTGGACTCTAAGG
TGGGTGGTAACTACAACTACCAGTACCGTCTGTTCCGCAAGAGCAACCTCAAGCCATTC
GAACGTGACATCAGCACAGAGATCTACCAAGCCGGTAACAAGCCCTGCAACGGCGTTG
CCGGATTCAACTGCTACTTCCCCCTCCGTTCCTACGGATTCCGCCCAACCTACGGCGTGG
GTCACCAGCCATACCGCGTGGTTGTGCTGAGCTTCGAACTGCTGCACGCCCCCGCTACC
GTGTGTGGCCCTAAGAAGAGCACTAACCTCGTTAAGAACAAGTCCGTGAACTTCAACTT
CAACGGTCTCTACGAAAACCAGAAGCTGATCGCTAACCAGTTCAACTCCGCCATCGGCA
AGATCCAGGACTCCCTGTCCTCCACAGCTTCCGCTCTGGGCAAGCTGCAGGACGTTGTG
AACCAAAACGCTCAGGCCCTGAACACACTGGTGAAGCAACTGAAGAACCACACCAGC
CCCGACGTTGACCTGGGTGACATCAGCGGTATCAACGCTAGCGTGGTGAACATCCAGAA
GGAGATCGACAGGCTGAACGAAGTGGCTAAGAACTTGAACGAGAGCCTCATCGACCTG
CAGGAACTGTAA

Signal peptide coding sequence - Trx tag coding sequence - 6His tag coding sequence - EK restriction enzyme cutting site coding sequence - RBD coding sequence - HR1 coding sequence - HR2 coding sequence

SEQ ID No.16 (nucleotide sequence encoding SEQ ID No.8):
ATGCTGCTGGTGAACCAGAGCCACCAGGGTTTCAACAAGGAACACACTAGCAAGA
TGGTGTCTGCTATCGTCCTCTACGTGCTGCTCGCCGCCGCTGCTCACTCAGCTTTCGCCG
CTGACTCTATCCACATCAAGGACTCCGACGACCTCAAGAACCGTTTGGCCGAAGCCGGC
GACAAGCTGGTCGTTATCGACTTCATGGCTACCTGGTGTGGCCCTTGCAAGATGATCGG
ACCTAAGCTGGACGAAATGGCTAACGAGATGAGCGACAGCATCGTCGTTCTGAAGGTG
GACGTCGACGAATGTGAGGACATCGCCACTGAGTACAACATCAACAGCATGCCTACCTT
CGTTTTCGTGAAGAACTCTAAGAAGATCGAGGAGTTCTCTGGTGCCAACGTGGACAAG
CTCCGTAACACCATCATCAAGCTCAAGCTGGCCGGCAGCGGTAGCGGTCACATGCACCA
CCACCACCACCATTCAAGCGGCGACGACGACGACAAGGTGCAGCCAACCGAATCAATC
GTCCGCTTCCCAAACATCACTAACCTGTGCCCATTCGACGAGGTGTTCAACGCCACCCG
TTTCGCCTCTGTCTACGCTTGGAACCGCAAGCGCATCTCCAACTGTGTCGCCGACTACA
GCGTGCTGTACAACTTCGCTCCTTTCTTCGCATTCAAGTGTTACGGAGTGTCCCCTACCA
AGCTCAACGATTTGTGCTTCACAAACGTGTACGCTGACAGCTTCGTGATCCGTGGTAAC
GAGGTTTCTCAAATCGCCCCTGGTCAGACAGGTAACATCGCTGACTACAACTACAAGCT
CCCAGACGACTTCACTGGCTGTGTTATCGCTTGGAACTCAAACAAGCTGGACTCTAAGG
TGGGTGGTAACTACAACTACCGGTACCGTCTGTTCCGCAAGAGCAACCTCAAGCCATTC
GAACGTGACATCAGCACAGAGATCTACCAAGCCGGTAACAAGCCCTGCAACGGCGTTG
CCGGAGTCAACTGCTACTTCCCCCTCCAGTCCTACGGATTCCGCCCAACCTACGGCGTG
GGTCACCAGCCATACCGCGTGGTTGTGCTGAGCTTCGAACTGCTGCACGCCCCCGCTAC
CGTGTGTGGCCCTAAGAAGAGCACTAACCTCGTTAAGAACAAGTCCGTGAACTTCAACT
TCAACGGTCTCTACGAAAACCAGAAGCTGATCGCTAACCAGTTCAACTCCGCCATCGGC
AAGATCCAGGACTCCCTGTCCTCCACAGCTTCCGCTCTGGGCAAGCTGCAGGACGTTGT
GAACCAAAACGCTCAGGCCCTGAACACACTGGTGAAGCAACTGAAGAACCACACCAG
CCCCGACGTTGACCTGGGTGACATCAGCGGTATCAACGCTAGCGTGGTGAACATCCAGA
AGGAGATCGACAGGCTGAACGAAGTGGCTAAGAACTTGAACGAGAGCCTCATCGACCT
GCAGGAACTGTAA

Signal peptide coding sequence - Trx tag coding sequence - 6His tag coding sequence - EK restriction enzyme cutting site coding sequence - RBD coding sequence - HR1 coding sequence - HR2 coding sequence

SEQ ID No.17 (nucleotide sequence encoding SEQ ID No.9):
ATGCTGCTGGTGAACCAGAGCCACCAGGGTTTCAACAAGGAACACACTAGCAAGATG
GTGTCTGCTATCGTCCTCTACGTGCTGCTCGCCGCCGCTGCTCACTCAGCTTTCGCCGCT
GACTCTATCCACATCAAGGACTCCGACGACCTCAAGAACCGTTTGGCCGAAGCCGGCG
ACAAGCTGGTCGTTATCGACTTCATGGCTACCTGGTGTGGCCCTTGCAAGATGATCGGAC
CTAAGCTGGACGAAATGGCTAACGAGATGAGCGACAGCATCGTCGTTCTGAAGGTGGA
CGTCGACGAATGTGAGGACATCGCCACTGAGTACAACATCAACAGCATGCCTACCTTCG
TTTTCGTGAAGAACTCTAAGAAGATCGAGGAGTTCTCTGGTGCCAACGTGGACAAGCTC
CGTAACACCATCATCAAGCTCAAGCTGGCCGGCAGCGGTAGCGGTCACATGCACCACCA
CCACCACCATTCAAGCGGCGACGACGACGACAAGGTGCAGCCAACCGAATCAATCGTC
CGCTTCCCAAACATCACTAACCTGTGCCCATTCGACGAGGTGTTCAACGCCACCCGTTT
CGCCTCTGTCTACGCTTGGAACCGCAAGCGCATCTCCAACTGTGTCGCCGACTACAGCG
TGCTGTACAACTTCGCTCCTTTCTTCGCATTCAAGTGTTACGGAGTGTCCCCTACCAAGC
TCAACGATTTGTGCTTCACAAACGTGTACGCTGACAGCTTCGTGATCCGTGGTAACGAG
GTTTCTCAAATCGCCCCTGGTCAGACAGGTAACATCGCTGACTACAACTACAAGCTCCC
AGACGACTTCACTGGCTGTGTTATCGCTTGGAACTCAAACAAGCTGGACTCTACGGTGG
GTGGTAACTACAACTACCGGTACCGTCTGTTCCGCAAGAGCAAGCTCAAGCCATTCGAA
CGTGACATCAGCACAGAGATCTACCAAGCCGGTAACAAGCCCTGCAACGGCGTTGCCG
GAGTCAACTGCTACTTCCCCCTCCAGTCCTACGGATTCCGCCCAACCTACGGCGTGGGT
CACCAGCCATACCGCGTGGTTGTGCTGAGCTTCGAACTGCTGCACGCCCCCGCTACCGT
GTGTGGCCCTAAGAAGAGCACTAACCTCGTTAAGAACAAGTCCGTGAACTTCAACTTCA
ACGGTCTCTACGAAAACCAGAAGCTGATCGCTAACCAGTTCAACTCCGCCATCGGCAAG
ATCCAGGACTCCCTGTCCTCCACAGCTTCCGCTCTGGGCAAGCTGCAGGACGTTGTGAA
CCAAAACGCTCAGGCCCTGAACACACTGGTGAAGCAACTGAAGAACCACACCAGCCCC
GACGTTGACCTGGGTGACATCAGCGGTATCAACGCTAGCGTGGTGAACATCCAGAAGG
AGATCGACAGGCTGAACGAAGTGGCTAAGAACTTGAACGAGAGCCTCATCGACCTGCA
GGAACTGTAA

Signal peptide coding sequence - Trx tag coding sequence - 6His tag coding sequence - EK restriction enzyme cutting site coding sequence - RBD coding sequence - HR1 coding sequence - HR2 coding sequence

### Construction mode of M65: RBD (BA.1)-HR1-RBD (Delta)-HR2-RBD (Beta)

**The amino acid sequence constructed for M65 is:**
**SEQ ID No.18: RBD (BA.1)-HR1-RBD (Delta)-HR2-RBD (Beta)** 
MDAMKRGLCCVLLLCGAVFVSPVQPTESIVRFPNITNLCPFDEVFNATRFASVYAWNR
KRISNCVADYSVLYNLAPFFTFKCYGVSPTKLNDLCFTNVYADSFVIRGDEVRQIAPGQTGN
IADYNYKLPDDFTGCVIAWNSNKLDSKVSGNYNYLYRLFRKSNLKPFERDISTEIYQAGNK
PCNGVAGFNCYFPLRSYSFRPTYGVGHQPYRVVVLSFELLHAPATVCGPKKSTNLVKNKSV
NFNFNGLYENQKLIANQFNSAIGKIQDSLSSTASALGKLQDVVNQNAQALNTLVKQLVQPT
ESIVRFPNITNLCPFGEVFNATRFASVYAWNRKRISNCVADYSVLYNSASFSTFKCYGVSPTK
LNDLCFTNVYADSFVIRGDEVRQIAPGQTGKIADYNYKLPDDFTGCVIAWNSNNLDSKVGG
NYNYRYRLFRKSNLKPFERDISTEIYQAGSKPCNGVEGFNCYFPLQSYGFQPTNGVGYQPY
RVVVLSFELLHAPATVCGPKKSTNLVKNKSVNFNFNGKNHTSPDVDLGDISGINASVVNIQK
EIDRLNEVAKNLNESLIDLQELGKYVQPTESIVRFPNITNLCPFGEVFNATRFASVYAWNRKR
ISNCVADYSVLYNSASFSTFKCYGVSPTKLNDLCFTNVYADSFVIRGDEVRQIAPGQTGNIA
DYNYKLPDDFTGCVIAWNSNNLDSKVGGNYNYLYRLFRKSNLKPFERDISTEIYQAGSTPC
NGVKGFNCYFPLQSYGFQPTYGVGYQPYRVVVLSFELLHAPATVCGPKKSTNLVKNKSVN
FNFNG
tPA signal peptide - RBD sequence of OmicronBA.1 - HR1 sequence - RBD sequence of Delta - HR2 sequence - RBD sequence of Beta
**SEQ ID No.19 (nucleotide sequence encoding SEQ ID No.18):**

ATGGACGCCATGAAGAGAGGCCTGTGCTGCGTGCTGCTCCTGTGCGGCGCCGTGTT
CGTGAGCCCCGTGCAACCCACCGAAAGCATTGTGCGGTTTCCCAATATCACCAACCTCT
GTCCTTTCGACGAGGTGTTCAACGCCACAAGATTTGCTAGCGTGTACGCCTGGAACCGG
AAGAGAATCTCCAATTGTGTCGCCGACTATAGCGTCCTCTATAACCTGGCCCCCTTCTTC
ACATTCAAGTGCTACGGCGTGTCCCCCACAAAGCTGAACGACCTGTGCTTTACCAATGT
GTACGCCGACAGCTTCGTCATCAGAGGGGACGAGGTCCGGCAGATCGCTCCTGGGCAG
ACCGGCAATATCGCCGACTACAACTATAAGCTCCCTGACGACTTCACCGGGTGCGTCATT

GCTTGGAACTCCAACAAGCTGGATTCCAAAGTGAGCGGGAACTACAACTATCTCTACAG
ACTGTTCAGAAAGAGCAACCTGAAACCTTTCGAAAGAGACATCTCCACAGAGATCTAC
CAAGCCGGCAACAAGCCCTGCAACGGCGTGGCCGGCTTTAATTGTTACTTCCCCCTCAG
AAGCTACAGCTTCAGACCCACCTACGGCGTGGGCCATCAGCCCTATAGAGTCGTGGTGC
TGTCCTTCGAACTGCTGCACGCCCCCGCCACCGTGTGTGGCCCCAAGAAAAGCACAAA
TCTGGTCAAGAATAAGAGCGTCAATTTTAATTTCAACGGCCTGTACGAGAATCAGAAGC
TGATCGCCAATCAGTTCAACAGCGCCATCGGCAAGATCCAAGACAGCCTGAGCAGCAC
CGCTAGCGCCCTGGGCAAGCTGCAAGACGTGGTGAATCAGAACGCCCAAGCCCTGAAC
ACCCTGGTGAAGCAGCTGGTGCAGCCCACCGAATCCATTGTCAGATTCCCCAACATCAC
CAACCTGTGCCCCTTTGGCGAAGTGTTTAACGCCACCCGGTTCGCTAGCGTGTATGCCTG
GAACCGGAAGAGAATTAGCAACTGCGTGGCCGATTACAGCGTGCTCTATAACAGCGCTA
GCTTCAGCACCTTTAAGTGCTATGGCGTGAGCCCTACCAAGCTGAATGACCTCTGCTTCA
CCAACGTGTATGCTGATAGCTTCGTGATCAGAGGCGACGAAGTGAGACAGATCGCCCCT
GGGCAGACCGGCAAGATTGCCGACTACAATTATAAGCTGCCTGATGATTTCACCGGCTG
CGTGATCGCCTGGAACAGCAACAACCTGGATTCCAAGGTCGGGGGCAATTACAATTACA
GATACAGACTCTTCAGAAAGTCCAACCTGAAGCCCTTCGAGAGAGACATCAGCACCGA
GATCTATCAAGCCGGGAGCAAGCCCTGTAACGGCGTGGAGGGCTTCAATTGCTACTTTC
CCCTGCAGTCCTATGGGTTTCAGCCCACCAACGGGGTGGGCTATCAGCCCTATAGAGTC
GTCGTCCTCAGCTTCGAACTGCTGCACGCTCCTGCTACAGTGTGCGGGCCCAAGAAGAG
CACCAATCTCGTCAAGAATAAAAGCGTCAACTTCAACTTCAACGGCAAGAACCACACA
AGCCCCGACGTGGACCTGGGCGACATCAGCGGCATCAACGCTAGCGTGGTGAACATTC
AGAAGGAGATCGACAGACTGAACGAGGTGGCCAAGAACCTGAACGAGAGCCTGATCG
ACCTGCAAGAGCTGGGCAAGTACGTGCAGCCCACCGAGAGCATCGTGAGATTCCCCAAT
ATCACAAATCTCTGCCCTTTTGGCGAGGTGTTTAACGCTACAAGATTCGCTAGCGTCTAC
GCCTGGAACAGAAAACGGATCAGCAATTGTGTGGCCGACTATAGCGTGCTGTACAATTC
CGCTAGCTTCAGCACATTCAAATGCTACGGGGTGTCCCCCACCAAGCTGAACGATCTCT
GCTTCACCAACGTCTACGCCGACAGCTTCGTGATTAGAGGCGACGAGGTCAGACAAATC
GCCCCTGGGCAGACCGGCAACATCGCTGACTATAATTACAAGCTGCCTGACGATTTCAC
CGGGTGTGTGATCGCCTGGAACTCCAACAATCTGGACTCCAAGGTGGGGGGCAACTATA
ACTACCTGTACAGACTCTTTAGAAAGAGCAATCTCAAGCCCTTCGAACGGGATATTAGC
ACCGAAATTTACCAAGCCGGCTCCACACCCTGTAACGGCGTGAAGGGGTTCAACTGCTA

TTTCCCCCTGCAGAGCTACGGCTTTCAGCCCACCTACGGCGTGGGGTATCAGCCTTACCG
GGTGGTCGTGCTCAGCTTCGAGCTGCTGCATGCTCCTGCCACCGTCTGTGGGCCTAAGA
AGAGCACCAACCTGGTGAAGAACAAATCCGTGAACTTCAACTTTAACGGCTGA

**Construction of M65-BA.4/5: replacement of BA.1RBD in M65 with RBD sequence of BA.4/5, and construction mode thereof: RBD (BA.4/5)-HR1-RBD (Delta)-HR2-RBD (WT)**

**The amino acid sequence constructed for M65-BA.4/5 is:**
**EQ ID No.20: RBD (BA.4/5)-HR1-RBD (Delta)-HR2-RBD (WT)** 
MDAMKRGLCCVLLLCGAVFVSPVQPTESIVRFPNITNLCPFDEVFNATRFASVYAWNR
KRISNCVADYSVLYNFAPFFAFKCYGVSPTKLNDLCFTNVYADSFVIRGNEVSQIAPGQTGNI
ADYNYKLPDDFTGCVIAWNSNKLDSKVGGNYNYRYRLFRKSNLKPFERDISTEIYQAGNKP
CNGVAGVNCYFPLQSYGFRPTYGVGHQPYRVVVLSFELLHAPATVCGPKKSTNLVKNKSV
NFNFNGLYENQKLIANQFNSAIGKIQDSLSSTASALGKLQDVVNQNAQALNTLVKQLVQPT
ESIVRFPNITNLCPFGEVFNATRFASVYAWNRKRISNCVADYSVLYNSASFSTFKCYGVSPTK
LNDLCFTNVYADSFVIRGDEVRQIAPGQTGKIADYNYKLPDDFTGCVIAWNSNNLDSKVGG
NYNYRYRLFRKSNLKPFERDISTEIYQAGSKPCNGVEGFNCYFPLQSYGFQPTNGVGYQPY
RVVVLSFELLHAPATVCGPKKSTNLVKNKSVNFNFNGKNHTSPDVDLGDISGINASVVNIQK
EIDRLNEVAKNLNESLIDLQELGKYVQPTESIVRFPNITNLCPFGEVFNATRFASVYAWNRKR
ISNCVADYSVLYNSASFSTFKCYGVSPTKLNDLCFTNVYADSFVIRGDEVRQIAPGQTGKIA
DYNYKLPDDFTGCVIAWNSNNLDSKVGGNYNYLYRLFRKSNLKPFERDISTEIYQAGSTPC
NGVEGFNCYFPLQSYGFQPTNGVGYQPYRVVVLSFELLHAPATVCGPKKSTNLVKNKSVNF
NFNG
tPA signal peptide - RBD sequence of OmicronBA.4/5 - HR1 sequence - RBD sequence of Delta - HR2 sequence - RBD sequence of WT.

**SEQ ID No.21 (nucleotide sequence encoding SEQ ID No.20):**

ATGGACGCCATGAAGAGAGGCCTGTGCTGCGTGCTGCTCCTGTGCGGCGCCGTGTT
CGTGAGCCCCGTGCAACCCACCGAGAGCATTGTGAGATTCCCCAATATCACCAACCTCT
GCCCCTTCGACGAAGTGTTTAACGCCACAAGATTCGCTAGCGTCTACGCTTGGAATAGA
AAGCGGATCAGCAACTGCGTCGCTGATTACTCCGTGCTGTACAACTTCGCCCCCTTCTTC
GCTTTCAAGTGTTACGGGGTGAGCCCTACCAAGCTGAACGACCTCTGTTTCACCAATGT

GTACGCCGATAGCTTCGTGATCCGGGGCAACGAAGTGAGCCAAATCGCCCCCGGCCAA
ACCGGCAATATCGCTGACTACAACTACAAGCTCCCTGATGACTTCACCGGCTGTGTGATC
GCTTGGAACAGCAACAAGCTGGACAGCAAAGTGGGCGGGAATTATAACTACAGATACC
GGCTCTTCCGGAAGAGCAACCTCAAGCCTTTTGAGCGGGACATCTCCACCGAAATCTAC
CAAGCCGGCAATAAACCCTGCAATGGCGTGGCCGGCGTGAACTGCTACTTCCCTCTGCA
AAGCTACGGCTTTAGACCCACCTACGGCGTGGGCCATCAGCCCTACAGAGTGGTCGTGC
TCAGCTTTGAGCTGCTCCACGCTCCTGCTACAGTCTGCGGCCCTAAAAAGAGCACAAAC
CTCGTCAAGAACAAAAGCGTGAACTTCAACTTTAACGGCCTGTATGAGAATCAGAAGCT
GATCGCCAATCAGTTCAACAGCGCCATCGGCAAGATCCAAGACAGCCTGAGCAGCACC
GCTAGCGCCCTGGGCAAGCTGCAAGACGTGGTGAATCAGAACGCCCAAGCCCTGAACA
CCCTGGTGAAGCAGCTGGTGCAGCCTACCGAGTCCATCGTGCGGTTCCCCAACATCACA
AACCTGTGTCCCTTTGGGGAAGTCTTTAACGCCACCCGGTTCGCTAGCGTGTATGCCTGG
AATAGAAAGAGAATTAGCAATTGCGTGGCTGACTATAGCGTGCTGTACAACAGCGCCTC
CTTCTCCACATTTAAGTGCTACGGCGTGAGCCCCACAAAGCTGAATGATCTCTGCTTCAC
CAACGTGTACGCCGACAGCTTCGTCATTAGAGGCGATGAAGTGAGACAGATCGCTCCTG
GGCAGACCGGCAAAATTGCTGACTACAACTATAAGCTGCCCGACGATTTCACCGGCTGC
GTGATTGCCTGGAACAGCAATAACCTGGACAGCAAGGTCGGCGGCAACTATAACTACAG
ATACAGACTGTTCCGGAAGTCCAACCTGAAGCCCTTCGAGAGAGACATCTCCACCGAGA
TCTATCAAGCCGGCAGCAAGCCCTGCAACGGCGTGGAGGGCTTTAACTGCTATTTCCCC
CTGCAGAGCTACGGCTTTCAGCCTACAAACGGCGTGGGGTATCAGCCCTACAGAGTCGT
GGTCCTGAGCTTTGAACTGCTCCATGCTCCCGCTACCGTGTGTGGCCCCAAGAAGAGCA
CCAACCTCGTGAAAAACAAGTCCGTGAATTTCAACTTCAACGGGAAGAACCACACAAG
CCCCGACGTGGACCTGGGCGACATCAGCGGCATCAACGCTAGCGTGGTGAACATTCAG
AAGGAGATCGACAGACTGAACGAGGTGGCCAAGAACCTGAACGAGAGCCTGATCGAC
CTGCAAGAGCTGGGCAAGTACGTGCAGCCCACAGAGAGCATCGTGCGGTTCCCCAACA
TCACCAATCTGTGTCCCTTCGGCGAGGTCTTCAATGCCACCCGGTTCGCTAGCGTGTATG
CTTGGAACAGAAAACGGATCAGCAATTGCGTGGCCGACTACAGCGTGCTGTATAATAGC
GCTAGCTTCAGCACCTTCAAATGTTATGGCGTCTCCCCCACCAAGCTGAATGACCTGTGT
TTCACAAACGTGTATGCCGACTCCTTCGTGATCAGAGGCGACGAGGTCAGACAGATTGC
CCCTGGGCAGACCGGCAAGATCGCCGACTACAACTACAAACTGCCCGATGACTTCACCG
GCTGCGTGATCGCCTGGAATTCCAACAATCTGGACTCCAAAGTGGGCGGGAACTACAAC

TACCTGTACCGGCTGTTTAGAAAAAGCAACCTGAAACCTTTCGAGCGGGATATTAGCAC
AGAAATCTATCAAGCCGGGAGCACCCCCTGCAATGGGGTGGAGGGCTTCAATTGCTACT
TCCCCCTGCAATCCTATGGGTTTCAGCCCACCAACGGCGTGGGCTACCAACCCTATAGAG
TGGTGGTCCTCAGCTTTGAGCTGCTGCACGCCCCCGCCACCGTGTGCGGGCCCAAAAA
GAGCACCAACCTGGTCAAGAACAAGTCCGTCAACTTTAACTTTAACGGCTGA

**Construction of M65-HR-opt: further optimization of HR1 and HR2 sequences in M65, and construction mode thereof: RBD (BA.4/5)-HR1 (opt)-RBD (Delta)-HR2 (opt)-RBD (WT)**

**The amino acid sequence constructed for M65-HR-opt is:**
**SEQ ID No.22: RBD (BA.4/5)-HR1 (opt)-RBD (Delta)-HR2 (opt)-RBD (WT)** 
MDAMKRGLCCVLLLCGAVFVSPVQPTESIVRFPNITNLCPFDEVFNATRFASVYAWNR
KRISNCVADYSVLYNFAPFFAFKCYGVSPTKLNDLCFTNVYADSFVIRGNEVSQIAPGQTGNI
ADYNYKLPDDFTGCVIAWNSNKLDSKVGGNYNYRYRLFRKSNLKPFERDISTEIYQAGNKP
CNGVAGVNCYFPLQSYGFRPTYGVGHQPYRVVVLSFELLHAPATVCGPKKSTNLVKNKSV
NFNFNGTQNVLYENQKLIANQFNSAIGKIQDSLSSTASALGKLQDVVNQNAQALNTLVKQL
SSNFGAISSVLNDILSRLDKVEVQPTESIVRFPNITNLCPFGEVFNATRFASVYAWNRKRISNC
VADYSVLYNSASFSTFKCYGVSPTKLNDLCFTNVYADSFVIRGDEVRQIAPGQTGKIADYN
YKLPDDFTGCVIAWNSNNLDSKVGGNYNYRYRLFRKSNLKPFERDISTEIYQAGSKPCNGV
EGFNCYFPLQSYGFQPTNGVGYQPYRVVVLSFELLHAPATVCGPKKSTNLVKNKSVNFNFN
GVYDPLQPELDSFKEELDKYFKNHTSPDVDLGDISGINASVVNIQKEIDRLNEVAKNLNESLI
DLQELGKYGKYEQYIKWPWYIWLGFIAGVQPTESIVRFPNITNLCPFGEVFNATRFASVYAW
NRKRISNCVADYSVLYNSASFSTFKCYGVSPTKLNDLCFTNVYADSFVIRGDEVRQIAPGQT
GKIADYNYKLPDDFTGCVIAWNSNNLDSKVGGNYNYLYRLFRKSNLKPFERDISTEIYQAG
STPCNGVEGFNCYFPLQSYGFQPTNGVGYQPYRVVVLSFELLHAPATVCGPKKSTNLVKNK
SVNFNFNG
tPA signal peptide - RBD sequence of OmicronBA.4/5 - HR1 sequence - RBD sequence of Delta - HR2 sequence - RBD sequence of WT.
**SEQ ID No.23 (nucleotide sequence encoding SEQ ID No.22):** 
ATGGACGCCATGAAGAGAGGCCTGTGCTGCGTGCTGCTCCTGTGCGGCGCCGTGTT
CGTGAGCCCCGTGCAACCTACCGAGAGCATTGTCCGGTTCCCCAATATTACCAACCTCTG
 
CCCCTTCGACGAGGTCTTTAACGCTACAAGATTCGCCTCCGTGTACGCCTGGAACCGGA
AGAGAATCAGCAATTGCGTCGCCGACTACTCCGTGCTCTACAACTTTGCCCCCTTCTTCG
CTTTCAAGTGCTACGGCGTGAGCCCCACAAAGCTCAACGACCTGTGCTTCACCAACGTG
TACGCCGACAGCTTTGTCATCAGAGGCAACGAGGTGAGCCAAATTGCCCCTGGCCAAA
CCGGCAACATCGCCGATTACAACTACAAGCTCCCCGACGACTTCACCGGCTGTGTGATT
GCTTGGAACAGCAATAAGCTCGACAGCAAGGTGGGGGGCAACTACAATTACCGGTACC
GGCTGTTCCGGAAGTCCAATCTGAAGCCTTTCGAGAGAGACATCAGCACCGAGATCTAC
CAAGCCGGGAACAAACCCTGCAATGGCGTGGCCGGCGTGAACTGCTACTTCCCTCTGC
AGAGCTACGGGTTTCGGCCCACCTACGGCGTGGGCCACCAACCCTACAGAGTGGTGGT
CCTGAGCTTCGAGCTGCTGCACGCCCCTGCTACAGTGTGTGGCCCCAAGAAAAGCACC
AATCTCGTGAAGAACAAGAGCGTCAACTTCAATTTTAACGGCACACAGAACGTGCTGTA
CGAGAATCAGAAGCTGATCGCCAATCAGTTCAACAGCGCCATCGGCAAGATCCAAGAC
AGCCTGAGCAGCACCGCTAGCGCCCTGGGCAAGCTGCAAGACGTGGTGAATCAGAACG
CCCAAGCCCTGAACACCCTGGTGAAGCAGCTGAGCAGCAACTTCGGCGCCATCAGCAG
CGTGCTGAACGACATCCTGAGCAGACTGGACAAGGTGGAGGTGCAGCCTACAGAGTCC
ATCGTCAGATTCCCCAACATCACCAACCTGTGCCCCTTCGGCGAGGTGTTTAATGCCACA
CGGTTCGCTAGCGTGTACGCCTGGAATCGGAAGCGGATCTCCAACTGCGTCGCTGACTA
TTCCGTGCTCTATAACTCCGCTAGCTTCAGCACATTTAAGTGCTATGGGGTGTCCCCCAC
CAAGCTGAATGACCTGTGCTTCACAAACGTCTACGCCGACTCCTTTGTCATCCGGGGCG
ACGAAGTCCGGCAGATCGCCCCTGGGCAGACCGGCAAAATCGCTGACTACAACTACAA
ACTGCCCGATGACTTCACCGGCTGTGTGATCGCCTGGAACTCCAACAATCTGGACTCCA
AGGTCGGCGGCAATTACAATTACAGATACAGACTGTTCAGAAAATCCAACCTGAAACCC
TTCGAGCGGGACATTTCCACAGAGATCTATCAAGCCGGCAGCAAACCTTGCAACGGCGT
GGAGGGGTTCAACTGCTATTTTCCCCTGCAGAGCTACGGGTTTCAGCCCACCAACGGCG
TCGGCTACCAACCCTATCGGGTGGTCGTGCTGAGCTTCGAACTCCTGCACGCTCCCGCC
ACAGTCTGCGGGCCTAAAAAGAGCACCAACCTGGTGAAGAATAAATCCGTCAATTTCAA
CTTCAACGGCGTGTACGACCCCCTGCAGCCCGAGCTGGACAGCTTCAAGGAGGAGCTG
GACAAGTACTTCAAGAACCACACAAGCCCCGACGTGGACCTGGGCGACATCAGCGGCA
TCAACGCTAGCGTGGTGAACATTCAGAAGGAGATCGACAGACTGAACGAGGTGGCCAA
GAACCTGAACGAGAGCCTGATCGACCTGCAAGAGCTGGGCAAGTACGGCAAGTACGAG
CAGTACATCAAGTGGCCCTGGTACATCTGGCTGGGCTTCATCGCCGGCGTGCAGCCCAC
 
CGAGAGCATCGTCCGGTTTCCCAACATTACCAACCTGTGTCCCTTCGGCGAGGTCTTCAA
TGCCACACGGTTCGCTAGCGTCTATGCCTGGAACCGGAAGAGAATTAGCAACTGCGTGG
CCGACTATAGCGTCCTGTACAACAGCGCTAGCTTCTCCACCTTCAAGTGTTATGGCGTGT
CCCCCACCAAGCTGAATGACCTCTGTTTTACCAATGTCTATGCCGACAGCTTCGTGATCA
GAGGCGATGAAGTGAGACAGATCGCCCCTGGGCAGACCGGGAAGATCGCCGACTACAA
CTATAAGCTCCCCGATGACTTCACCGGCTGCGTCATTGCTTGGAACTCCAATAATCTGGA
CAGCAAGGTGGGGGGCAATTATAACTACCTGTATCGGCTGTTCAGAAAGAGCAACCTGA
AGCCTTTTGAGAGAGATATCAGCACCGAAATTTATCAAGCCGGGAGCACCCCCTGCAAC
GGGGTGGAAGGCTTCAACTGTTACTTCCCCCTGCAGAGCTACGGCTTTCAGCCTACCAA
CGGGGTGGGGTATCAGCCTTATAGAGTCGTGGTCCTGTCCTTCGAGCTGCTGCACGCCC
CCGCCACAGTGTGCGGGCCCAAGAAGAGCACCAATCTGGTGAAAAACAAAAGCGTCA
ACTTCAATTTCAACGGCTGA
**SEQ ID No.24: Nucleotide sequence of S antigen of wild Ad-S vaccine** 
ATGTTCGTGTTCCTGGTGCTGCTGCCCCTGGTGAGCAGCCAGTGTGTGAACCTGACA
ACAAGGACCCAGCTGCCTCCCGCCTACACCAACAGCTTCACAAGGGGCGTGTACTACCC
TGACAAGGTGTTCAGGAGCAGCGTGCTGCACTCCACACAGGACCTGTTCCTGCCCTTCT
TCTCCAACGTGACATGGTTCCACGCCATCCACGTGTCCGGCACCAACGGCACAAAGAGA
TTCGACAACCCCGTGCTGCCTTTCAACGACGGCGTGTACTTCGCCTCCACAGAGAAGTC
CAACATCATCAGGGGCTGGATCTTCGGCACAACACTGGACAGCAAGACACAGAGCCTG
CTGATCGTGAACAACGCCACAAACGTGGTCATTAAGGTGTGTGAGTTCCAGTTCTGCAA
CGACCCCTTCCTGGGCGTGTACTATCACAAGAACAACAAGAGCTGGATGGAGAGCGAG
TTCAGGGTGTACTCCAGCGCCAACAACTGTACATTCGAGTACGTGTCCCAGCCTTTCCTG
ATGGACCTGGAGGGCAAGCAGGGCAACTTCAAGAACCTGAGGGAGTTCGTGTTCAAGA
ACATCGACGGCTACTTCAAGATCTACTCCAAGCACACCCCCATCAACCTGGTGAGAGAC
CTGCCTCAGGGCTTCTCCGCCCTGGAGCCTCTGGTGGACCTGCCCATCGGCATCAACAT
CACAAGATTCCAGACACTGCTGGCCCTGCACAGAAGCTACCTGACCCCCGGCGATTCCA
GCAGCGGCTGGACCGCTGGCGCCGCTGCTTACTACGTGGGCTACCTGCAGCCCAGGACA
TTCCTGCTGAAGTACAACGAGAACGGCACAATCACCGATGCCGTGGATTGCGCCCTGGA
TCCTCTGAGCGAGACCAAGTGTACACTGAAGTCCTTCACAGTGGAGAAGGGCATCTACC
AGACCTCCAACTTCAGAGTGCAGCCCACAGAGAGCATCGTGAGATTCCCTAACATCACA
 
AACCTGTGCCCTTTCGGCGAGGTGTTCAACGCCACAAGGTTCGCCAGCGTGTACGCCTG
GAACAGAAAGAGGATCAGCAACTGCGTGGCCGATTACAGCGTGCTGTACAACTCCGCC
TCCTTCTCCACATTCAAGTGCTACGGCGTGTCCCCCACCAAGCTGAACGATCTGTGTTTC
ACCAACGTGTACGCCGACTCCTTCGTGATCAGAGGCGACGAGGTGAGGCAGATCGCCC
CCGGACAGACCGGCAAGATCGCCGATTACAACTACAAGCTGCCCGATGACTTCACCGGC
TGTGTGATCGCCTGGAACTCCAACAACCTGGATTCCAAGGTGGGCGGCAACTACAACTA
CCTGTACAGACTGTTCAGAAAGTCCAACCTGAAGCCCTTCGAGAGAGATATCTCCACCG
AGATCTACCAGGCCGGCTCCACACCTTGTAACGGCGTGGAGGGCTTCAACTGCTACTTC
CCCCTGCAGAGCTACGGCTTCCAGCCTACCAACGGCGTGGGCTACCAGCCTTACAGAGT
GGTGGTGCTGTCCTTCGAGCTGCTGCACGCCCCCGCCACAGTGTGTGGCCCAAAGAAG
TCCACCAACCTGGTGAAGAACAAGTGTGTGAACTTCAACTTCAACGGCCTGACCGGCA
CAGGCGTGCTGACCGAGAGCAACAAGAAGTTCCTGCCTTTCCAGCAGTTCGGCAGAGA
CATCGCCGATACCACAGACGCCGTGAGAGACCCTCAGACCCTGGAGATCCTGGATATCA
CCCCTTGCAGCTTCGGCGGCGTGTCCGTGATCACCCCTGGCACAAACACCAGCAACCA
GGTGGCCGTGCTGTACCAGGACGTGAACTGTACAGAGGTGCCCGTGGCCATCCACGCC
GACCAGCTGACCCCCACATGGAGAGTGTACTCCACCGGCTCCAACGTGTTCCAGACAA
GGGCCGGCTGCCTGATCGGCGCCGAGCATGTGAACAACAGCTACGAGTGTGACATCCCT
ATCGGCGCCGGCATCTGCGCCAGCTACCAGACCCAGACCAACAGCCCCAGGAGGGCCA
GATCCGTGGCCAGCCAGAGCATCATCGCCTACACCATGTCCCTGGGCGCCGAGAACAGC
GTGGCCTACTCCAACAACAGCATCGCCATCCCCACCAACTTCACAATCTCCGTGACCAC
CGAGATCCTGCCCGTGAGCATGACCAAGACATCCGTGGATTGTACCATGTACATCTGCGG
CGACAGCACAGAGTGCTCCAACCTGCTGCTGCAGTACGGCTCCTTCTGTACCCAGCTGA
ACAGGGCCCTGACAGGCATCGCCGTGGAGCAGGACAAGAACACCCAGGAGGTGTTCG
CCCAGGTGAAGCAGATCTACAAGACACCTCCCATCAAGGACTTCGGCGGCTTCAACTTC
TCCCAGATCCTGCCCGACCCTTCCAAGCCCTCCAAGAGATCCTTCATCGAGGACCTGCT
GTTCAACAAGGTGACCCTGGCCGATGCCGGCTTCATCAAGCAGTACGGCGACTGCCTGG
GCGACATCGCCGCCAGAGACCTGATCTGCGCCCAGAAGTTCAACGGCCTCACCGTGCT
GCCCCCTCTGCTGACCGATGAGATGATCGCCCAGTACACCAGCGCCCTGCTGGCCGGAA
CAATCACCAGCGGCTGGACATTCGGCGCCGGCGCTGCTCTGCAGATCCCTTTCGCCATG
CAGATGGCCTACAGGTTCAACGGCATCGGCGTGACCCAGAACGTGCTGTACGAGAACC
AGAAGCTGATCGCCAACCAGTTCAACAGCGCCATCGGCAAGATCCAGGATTCCCTGAGC
 
AGCACAGCCAGCGCCCTGGGCAAGCTGCAGGATGTGGTGAACCAGAACGCCCAGGCCC
TGAACACACTGGTGAAGCAGCTGAGCAGCAACTTCGGCGCCATCAGCAGCGTGCTCAA
CGACATCCTGAGCAGACTGGATAAGGTGGAGGCCGAGGTGCAGATCGATAGACTGATCA
CCGGCAGGCTGCAGTCCCTGCAGACATACGTGACCCAGCAGCTGATCAGAGCCGCCGA
GATCAGGGCCAGCGCCAACCTGGCCGCCACCAAGATGTCCGAGTGTGTGCTGGGCCAG
AGCAAGAGGGTGGACTTCTGTGGCAAGGGCTACCACCTGATGTCCTTCCCTCAGTCCGC
CCCCCACGGCGTGGTGTTCCTGCACGTGACATACGTGCCTGCCCAGGAGAAGAACTTCA
CAACAGCCCCTGCCATCTGTCACGACGGCAAGGCCCACTTCCCCAGGGAGGGCGTGTT
CGTGAGCAACGGCACCCACTGGTTCGTGACCCAGAGGAACTTCTACGAGCCTCAGATCA
TCACAACCGATAACACATTCGTGAGCGGCAACTGTGATGTGGTCATTGGCATCGTGAAC
AATACCGTGTACGATCCCCTGCAGCCTGAGCTGGACTCCTTCAAGGAGGAGCTGGATAA
GTACTTCAAGAACCACACCTCCCCCGACGTGGATCTGGGCGACATTAGCGGCATCAACG
CCTCCGTGGTGAACATCCAGAAGGAGATCGACAGGCTGAACGAGGTGGCCAAGAACCT
GAACGAGTCCCTGATCGATCTGCAGGAGCTGGGCAAGTACGAGCAGTACATCAAGTGG
CCCTGGTACATCTGGCTGGGCTTCATCGCCGGCCTGATCGCCATCGTGATGGTGACAATC
ATGCTGTGTTGCATGACATCCTGTTGTTCCTGTCTGAAGGGCTGTTGTAGCTGTGGCTCC
TGTTGCAAGTTCGACGAGGATGACTCCGAGCCCGTGCTGAAGGGCGTGAAGCTGCACT
ACACCTGA
**SEQ ID No.25: Amino acid sequence of S antigen of wild Ad-S vaccine** 











 









*
**SEQ ID No.26: Nucleotide sequence of S antigen of Ad-S-Beta vaccine** 
ATGTTCGTGTTCCTGGTGCTGCTGCCCCTGGTGAGCAGCCAATGCGTGAACCTGAC
CACAAGAACACAGCTGCCCCCCGCCTACACCAACAGCTTCACAAGAGGCGTGTATTATC
CCGACAAGGTGTTCAGAAGCAGCGTGCTGCACAGCACCCAAGACCTGTTTCTGCCTTTT
TTCAGCAACGTGACCTGGTTCCACGCCATCCACGTGAGCGGCACCAACGGCACCAAGA
GATTCGCCAACCCCGTGCTGCCCTTCAACGACGGCGTGTACTTCGCTAGCACCGAGAAG
AGCAACATCATCAGAGGCTGGATCTTCGGCACCACCCTGGATAGCAAGACACAGAGCCT
GCTGATCGTGAACAACGCCACCAACGTGGTGATCAAGGTGTGCGAGTTTCAGTTCTGCA
ACGACCCCTTCCTGGGCGTGTATTACCACAAGAACAACAAGAGCTGGATGGAGAGCGA
GTTCAGAGTCTACAGCAGCGCCAACAACTGCACCTTCGAGTACGTGAGCCAACCCTTCC
TGATGGACCTGGAGGGCAAGCAAGGCAATTTCAAGAACCTGAGAGAGTTCGTGTTCAA
GAACATCGACGGCTACTTCAAGATCTACAGCAAGCACACCCCCATCAACCTGGTGAGAG
GCCTGCCCCAAGGCTTCAGCGCCCTGGAGCCCCTGGTGGACCTGCCCATCGGCATCAAC
ATCACAAGATTTCAGACACTGCACCGGTCCTATCTCACCCCTGGCGACAGCAGCAGCGG
GTGGACCGCTGGCGCCGCCGCTTACTACGTGGGCTACCTGCAGCCTAGAACCTTCCTGC
TGAAGTACAACGAGAACGGCACCATTACCGACGCCGTGGACTGCGCCCTGGACCCCCT
GAGCGAGACCAAGTGCACCCTGAAGAGCTTCACCGTGGAGAAGGGCATCTATCAGACA
AGCAACTTCAGAGTGCAGCCCACCGAGAGCATCGTGAGATTCCCCAACATCACCAACCT
GTGCCCCTTCGGCGAGGTGTTCAACGCCACAAGATTCGCTAGCGTGTACGCCTGGAACC
GGAAAAGAATCAGCAACTGCGTGGCCGACTACAGCGTGCTGTACAACAGCGCTAGCTT
 
CAGCACCTTCAAGTGCTACGGCGTGAGCCCCACCAAGCTGAACGACCTGTGCTTCACC
AACGTGTACGCCGACAGCTTCGTGATCAGAGGCGACGAGGTGAGACAGATCGCCCCCG
GGCAGACCGGCAACATCGCCGACTACAACTACAAGCTGCCCGACGACTTCACCGGCTG
CGTGATCGCCTGGAACAGCAATAACCTGGACAGCAAGGTGGGCGGCAACTACAACTAC
CTGTACAGACTGTTCAGAAAGAGCAACCTGAAGCCCTTCGAGAGAGACATCAGCACCG
AGATCTACCAAGCCGGCAGCACCCCCTGCAACGGCGTGAAGGGCTTCAACTGCTACTTC
CCCCTGCAGAGCTACGGCTTTCAGCCCACCTACGGCGTGGGCTATCAGCCCTACAGAGT
GGTCGTGCTGAGCTTCGAGCTGCTGCACGCCCCTGCCACCGTGTGTGGCCCCAAGAAG
AGCACCAACCTGGTGAAGAACAAGTGCGTGAACTTCAACTTCAACGGGCTGACCGGGA
CCGGCGTGCTGACCGAGAGCAACAAGAAGTTTCTGCCCTTTCAACAGTTCGGCAGAGA
CATCGCCGACACCACCGACGCCGTCCGGGACCCTCAGACCCTGGAGATCCTGGACATCA
CCCCCTGCTCCTTCGGCGGCGTGAGCGTGATCACCCCCGGCACCAACACAAGCAACCA
AGTGGCCGTGCTGTACCAAGGCGTGAACTGCACCGAGGTGCCCGTGGCCATCCACGCC
GATCAGCTGACCCCCACCTGGAGAGTGTACAGCACCGGCAGCAACGTGTTTCAGACAA
GAGCCGGCTGCCTGATCGGCGCCGAGCACGTGAACAACAGCTACGAGTGCGACATCCC
CATCGGCGCCGGCATCTGCGCTAGCTATCAGACACAGACCAACAGCCCTAGAAGAGCTA
GAAGCGTGGCTAGCCAAAGCATCATCGCCTACACCATGAGCCTGGGCGTGGAGAACAG
CGTGGCCTACAGCAACAACAGCATCGCCATCCCCACCAACTTCACCATCAGCGTGACCA
CCGAAATCCTGCCCGTGAGCATGACCAAGACAAGCGTGGACTGCACCATGTACATCTGC
GGCGACAGCACCGAGTGCAGCAACCTGCTCCTGCAGTACGGCAGCTTCTGCACACAGC
TGAACAGAGCCCTGACCGGCATCGCCGTGGAGCAAGACAAGAACACCCAAGAGGTGTT
CGCCCAAGTGAAGCAGATCTACAAGACCCCCCCCATCAAGGACTTCGGCGGCTTCAACT
TCAGCCAAATTCTGCCCGACCCTAGCAAGCCTAGCAAGAGAAGCTTCATCGAGGACCTG
CTGTTCAACAAGGTGACCCTGGCCGACGCCGGCTTCATCAAGCAGTACGGCGACTGCCT
GGGCGATATCGCTGCTAGAGACCTGATCTGCGCTCAGAAGTTCAACGGCCTGACCGTGC
TCCCCCCCCTGCTGACCGACGAGATGATCGCTCAGTACACAAGCGCTCTGCTCGCCGGG
ACCATCACATCCGGGTGGACATTCGGGGCCGGCGCTGCCCTGCAGATCCCCTTCGCCAT
GCAGATGGCCTACAGATTCAACGGCATCGGCGTGACACAGAACGTGCTGTACGAGAATC
AGAAGCTGATCGCCAATCAGTTCAACAGCGCCATCGGCAAGATCCAAGACAGCCTGAG
CAGCACCGCTAGCGCCCTGGGCAAGCTGCAAGACGTGGTGAATCAGAACGCCCAAGCC
CTGAACACCCTGGTGAAGCAGCTGAGCAGCAACTTCGGCGCCATCAGCTCCGTGCTGA
 
ACGACATCCTGAGCAGACTGGACCCCCCCGAGGCCGAGGTGCAGATTGACAGACTGAT
CACCGGCAGACTGCAGAGCCTGCAGACCTACGTGACACAGCAGCTGATCAGAGCCGCC
GAGATCAGAGCTAGCGCCAACCTGGCCGCCACCAAGATGAGCGAGTGCGTGCTGGGGC
AGAGCAAGAGAGTGGACTTCTGCGGCAAGGGCTACCACCTGATGAGCTTCCCTCAGAG
CGCCCCCCACGGCGTGGTGTTCCTGCACGTGACCTACGTGCCCGCCCAAGAGAAGAAC
TTCACCACCGCCCCCGCCATCTGCCACGACGGCAAGGCCCACTTCCCTAGAGAGGGCGT
GTTCGTGAGCAACGGCACCCACTGGTTCGTGACACAGAGAAACTTCTACGAGCCTCAG
ATCATCACCACCGACAACACCTTCGTGAGCGGCAACTGCGACGTGGTGATCGGCATTGT
GAACAATACCGTGTACGACCCCCTGCAGCCCGAGCTGGACAGCTTCAAGGAGGAGCTG
GACAAGTACTTCAAGAACCACACAAGCCCCGACGTGGACCTGGGCGACATTAGCGGCA
TCAACGCTAGCGTGGTGAACATTCAGAAGGAGATCGACAGACTGAACGAGGTGGCCAA
GAACCTGAACGAGAGCCTGATCGACCTGCAAGAGCTGGGCAAGTACGAGCAGTACATC
AAGTGGCCCTGGTACATCTGGCTGGGCTTCATCGCCGGCCTGATCGCCATCGTGATGGTG
ACCATCATGCTGTGCTGCATGACAAGCTGTTGCAGCTGCCTGAAGGGCTGCTGCAGCTG
CGGGAGCTGCTGCAAGTTCGACGAGGACGACAGCGAGCCCGTGCTGAAGGGCGTGAA
GCTGCACTACACCTGA
**SEQ ID No.27: Amino acid sequence of S antigen of Ad-S-Beta vaccine** 
MFVFLVLLPLVSSQCVNLTTRTQLPPAYTNSFTRGVYYPDKVFRSSVLHSTQDLFLPFFS
NVTWFHAIHVSGTNGTKRFANPVLPFNDGVYFASTEKSNIIRGWIFGTTLDSKTQSLLIVNN
ATNVVIKVCEFQFCNDPFLGVYYHKNNKSWMESEFRVYSSANNCTFEYVSQPFLMDLEGK
QGNFKNLREFVFKNIDGYFKIYSKHTPINLVRGLPQGFSALEPLVDLPIGINITRFQTLHRSYL
TPGDSSSGWTAGAAAYYVGYLQPRTFLLKYNENGTITDAVDCALDPLSETKCTLKSFTVEK
GIYQTSNFRVQPTESIVRFPNITNLCPFGEVFNATRFASVYAWNRKRISNCVADYSVLYNSAS
FSTFKCYGVSPTKLNDLCFTNVYADSFVIRGDEVRQIAPGQTGNIADYNYKLPDDFTGCVIA
WNSNNLDSKVGGNYNYLYRLFRKSNLKPFERDISTEIYQAGSTPCNGVKGFNCYFPLQSYG
FQPTYGVGYQPYRVVVLSFELLHAPATVCGPKKSTNLVKNKCVNFNFNGLTGTGVLTESNK
KFLPFQQFGRDIADTTDAVRDPQTLEILDITPCSFGGVSVITPGTNTSNQVAVLYQGVNCTEV
PVAIHADQLTPTWRVYSTGSNVFQTRAGCLIGAEHVNNSYECDIPIGAGICASYQTQTNSPR
RARSVASQSIIAYTMSLGVENSVAYSNNSIAIPTNFTISVTTEILPVSMTKTSVDCTMYICGDS
TECSNLLLQYGSFCTQLNRALTGIAVEQDKNTQEVFAQVKQIYKTPPIKDFGGFNFSQILPDP
 
SKPSKRSFIEDLLFNKVTLADAGFIKQYGDCLGDIAARDLICAQKFNGLTVLPPLLTDEMIAQ
YTSALLAGTITSGWTFGAGAALQIPFAMQMAYRFNGIGVTQNVLYENQKLIANQFNSAIGKI
QDSLSSTASALGKLQDVVNQNAQALNTLVKQLSSNFGAISSVLNDILSRLDPPEAEVQIDRLI
TGRLQSLQTYVTQQLIRAAEIRASANLAATKMSECVLGQSKRVDFCGKGYHLMSFPQSAPH
GVVFLHVTYVPAQEKNFTTAPAICHDGKAHFPREGVFVSNGTHWFVTQRNFYEPQIITTDN
TFVSGNCDVVIGIVNNTVYDPLQPELDSFKEELDKYFKNHTSPDVDLGDISGINASVVNIQK
EIDRLNEVAKNLNESLIDLQELGKYEQYIKWPWYIWLGFIAGLIAIVMVTIMLCCMTSCCSC
LKGCCSCGSCCKFDEDDSEPVLKGVKLHYT*
**SEQ ID No.28: Nucleotide sequence of S antigen of Ad-S-Delta vaccine** 
ATGTTCGTGTTCCTGGTGCTGCTGCCCCTGGTGAGCAGCCAATGCGTGAACCTGAG
AACAAGAACACAGCTGCCCCCCGCCTACACCAACAGCTTCACAAGAGGGGTCTACTAC
CCCGACAAGGTGTTCAGAAGCAGCGTGCTGCACAGCACCCAAGACCTGTTCCTGCCCT
TCTTCTCCAACGTGACCTGGTTCCACGCCATCCACGTGAGCGGCACCAACGGCACCAAG
AGATTCGACAACCCCGTGCTGCCCTTCAACGACGGCGTGTACTTCGCTAGCACCGAGAA
GAGCAACATCATCAGAGGCTGGATCTTCGGCACCACCCTGGACAGCAAGACACAGAGC
CTGCTGATTGTGAACAACGCTACCAACGTGGTGATCAAGGTGTGCGAGTTTCAGTTCTG
CAACGACCCCTTCCTGGGGGTGTACTACCACAAAAACAACAAGAGCTGGATGGAGAGC
GGCGTGTACAGCAGCGCCAACAACTGCACCTTCGAGTACGTGAGCCAACCCTTCCTGAT
GGACCTGGAGGGCAAGCAAGGCAACTTCAAGAACCTCAGAGAGTTCGTGTTCAAGAA
CATCGACGGCTACTTCAAGATCTACAGCAAGCACACCCCCATCAACCTGGTGAGAGACC
TGCCCCAAGGCTTCAGCGCCCTGGAGCCCCTGGTGGACCTGCCCATCGGCATCAACATC
ACAAGATTTCAGACCCTGCTGGCCCTGCACAGAAGCTACCTGACACCCGGCGACAGCA
GCAGCGGGTGGACAGCCGGCGCTGCCGCTTACTACGTGGGCTACCTGCAGCCTAGAAC
CTTCCTGCTGAAGTACAACGAGAACGGCACCATTACCGACGCCGTCGACTGCGCCCTGG
ACCCCCTGAGCGAGACCAAGTGCACCCTGAAGAGCTTCACCGTGGAGAAGGGCATCTA
TCAGACAAGCAACTTCAGAGTGCAGCCCACCGAGAGCATCGTGAGATTCCCCAACATC
ACCAACCTGTGCCCCTTCGGCGAGGTGTTCAACGCCACAAGATTCGCTAGCGTGTACGC
CTGGAACAGAAAGAGAATCAGCAACTGCGTGGCCGACTACAGCGTGCTGTACAACAGC
GCTAGCTTCAGCACCTTCAAATGCTACGGGGTCAGCCCCACCAAGCTGAACGACCTGTG
CTTCACCAACGTGTACGCCGACAGCTTCGTGATCAGAGGCGACGAGGTGAGACAGATC
 
GCCCCCGGGCAGACCGGCAAGATCGCCGACTACAACTACAAGCTGCCCGACGACTTCA
CCGGCTGCGTGATCGCCTGGAACTCCAACAACCTGGATAGCAAGGTGGGCGGCAACTA
CAACTATAGATACAGACTGTTCAGAAAGAGCAACCTGAAGCCCTTCGAGAGAGACATCA
GCACCGAGATCTACCAAGCCGGCAGCAAGCCCTGCAACGGCGTGGAGGGCTTCAACTG
CTACTTCCCCCTGCAGAGCTACGGCTTTCAGCCCACCAACGGCGTGGGCTATCAGCCCT
ACAGAGTGGTCGTGCTGAGCTTCGAGCTGCTGCACGCCCCTGCCACCGTGTGCGGCCC
CAAGAAGAGCACCAACCTGGTGAAGAACAAGTGCGTGAACTTCAACTTCAACGGCCTG
ACCGGCACCGGCGTGCTGACCGAGAGCAACAAGAAGTTCCTCCCTTTTCAGCAGTTCG
GCAGAGACATCGCCGACACCACCGATGCCGTGAGAGACCCTCAGACCCTGGAGATCCT
GGACATCACCCCCTGCAGCTTTGGCGGCGTGAGCGTGATCACCCCCGGCACCAACACA
AGCAACCAAGTGGCCGTGCTGTACCAAGGCGTGAACTGCACCGAGGTGCCCGTGGCCA
TCCACGCCGATCAGCTGACCCCCACCTGGAGAGTGTACAGCACCGGCAGCAACGTGTTT
CAGACAAGAGCCGGCTGCCTGATCGGCGCCGAGCACGTGAACAACAGCTACGAGTGCG
ACATCCCCATCGGCGCCGGCATCTGCGCTAGCTATCAGACACAGACCAACAGCAGACGG
AGAGCTAGAAGCGTGGCTAGCCAAAGCATCATCGCCTACACCATGAGCCTGGGCGCCGA
GAACAGCGTGGCCTACAGCAACAACAGCATCGCCATCCCCACCAACTTCACCATCAGCG
TGACCACCGAGATCCTGCCCGTCTCCATGACCAAGACAAGCGTGGACTGCACCATGTAC
ATCTGCGGCGACAGCACCGAGTGCAGCAACCTGCTCCTGCAGTACGGCAGCTTCTGCAC
ACAGCTGAACAGAGCCCTGACCGGCATCGCCGTGGAGCAAGACAAGAACACCCAAGA
GGTGTTCGCCCAAGTGAAGCAGATCTACAAGACCCCCCCCATCAAGGACTTCGGCGGCT
TCAACTTCAGCCAAATCCTGCCCGACCCTAGCAAGCCTAGCAAGAGAAGCTTCATCGAG
GACCTGCTGTTCAACAAGGTGACCCTGGCCGACGCCGGCTTCATCAAGCAGTACGGCG
ACTGCCTGGGCGACATCGCCGCTAGAGACCTGATCTGCGCTCAGAAGTTCAATGGCCTG
ACCGTCCTGCCCCCCCTGCTGACCGACGAGATGATCGCTCAGTACACAAGCGCCCTGCT
GGCCGGCACAATTACAAGCGGCTGGACATTCGGCGCTGGCGCCGCCCTGCAAATCCCCT
TCGCCATGCAGATGGCCTACAGATTCAACGGCATCGGCGTGACACAGAACGTGCTGTAC
GAGAATCAGAAGCTGATCGCCAATCAGTTCAACAGCGCCATCGGCAAGATCCAAGACA
GCCTGAGCAGCACCGCTAGCGCCCTGGGCAAGCTGCAGAACGTGGTGAATCAGAACGC
CCAAGCCCTGAACACCCTGGTGAAGCAGCTGAGCAGCAACTTCGGCGCCATCAGCTCC
GTGCTGAACGATATCCTGAGCAGACTGGACCCCCCCGAGGCCGAGGTGCAGATCGACC
GGCTGATCACCGGCAGACTGCAGAGCCTGCAGACCTACGTGACACAGCAGCTGATCAG
 
AGCCGCCGAGATCAGAGCTAGCGCCAACCTGGCCGCCACCAAGATGAGCGAGTGCGTG
CTGGGGCAGAGCAAGAGAGTGGACTTCTGCGGCAAGGGCTACCACCTGATGAGCTTCC
CTCAGAGCGCCCCCCACGGCGTGGTGTTCCTGCACGTGACCTACGTGCCCGCCCAAGA
GAAGAACTTCACCACCGCCCCCGCCATCTGCCACGACGGCAAGGCCCACTTCCCTAGA
GAGGGCGTGTTCGTGAGCAACGGCACCCACTGGTTCGTGACACAGAGAAACTTCTACG
AGCCTCAGATCATCACCACCGACAACACCTTCGTGAGCGGCAACTGCGACGTGGTGATC
GGCATTGTGAACAATACAGTCTACGACCCTCTGCAGCCCGAGCTGGACAGCTTCAAGGA
GGAGCTGGACAAGTACTTCAAGAATCACACAAGCCCCGACGTGGACCTGGGGGACATC
AGCGGCATCAACGCTAGCGTGGTGAACATTCAGAAGGAGATCGACAGACTGAACGAGG
TGGCCAAGAACCTGAACGAGAGCCTGATCGACCTGCAAGAGCTGGGCAAGTACGAGCA
GTACATCAAGTGGCCCTGGTACATCTGGCTGGGCTTCATCGCCGGCCTGATCGCCATCGT
GATGGTGACCATCATGCTGTGCTGCATGACAAGCTGCTGCAGCTGCCTGAAGGGCTGCT
GCTCCTGCGGCAGCTGCTGCAAGTTCGACGAGGACGACAGCGAGCCCGTGCTGAAGGG
CGTGAAGCTGCACTACACCTGA
**SEQ ID No.29: Amino acid sequence of S antigen of Ad-S-Delta vaccine** 
MFVFLVLLPLVSSQCVNLRTRTQLPPAYTNSFTRGVYYPDKVFRSSVLHSTQDLFLPFFS
NVTWFHAIHVSGTNGTKRFDNPVLPFNDGVYFASTEKSNIIRGWIFGTTLDSKTQSLLIVNN
ATNVVIKVCEFQFCNDPFLGVYYHKNNKSWMESGVYSSANNCTFEYVSQPFLMDLEGKQ
GNFKNLREFVFKNIDGYFKIYSKHTPINLVRDLPQGFSALEPLVDLPIGINITRFQTLLALHRS
YLTPGDSSSGWTAGAAAYYVGYLQPRTFLLKYNENGTITDAVDCALDPLSETKCTLKSFTV
EKGIYQTSNFRVQPTESIVRFPNITNLCPFGEVFNATRFASVYAWNRKRISNCVADYSVLYNS
ASFSTFKCYGVSPTKLNDLCFTNVYADSFVIRGDEVRQIAPGQTGKIADYNYKLPDDFTGC
VIAWNSNNLDSKVGGNYNYRYRLFRKSNLKPFERDISTEIYQAGSKPCNGVEGFNCYFPLQ
SYGFQPTNGVGYQPYRVVVLSFELLHAPATVCGPKKSTNLVKNKCVNFNFNGLTGTGVLTE
SNKKFLPFQQFGRDIADTTDAVRDPQTLEILDITPCSFGGVSVITPGTNTSNQVAVLYQGVNC
TEVPVAIHADQLTPTWRVYSTGSNVFQTRAGCLIGAEHVNNSYECDIPIGAGICASYQTQTN
SRRRARSVASQSIIAYTMSLGAENSVAYSNNSIAIPTNFTISVTTEILPVSMTKTSVDCTMYIC
GDSTECSNLLLQYGSFCTQLNRALTGIAVEQDKNTQEVFAQVKQIYKTPPIKDFGGFNFSQIL
PDPSKPSKRSFIEDLLFNKVTLADAGFIKQYGDCLGDIAARDLICAQKFNGLTVLPPLLTDEM
IAQYTSALLAGTITSGWTFGAGAALQIPFAMQMAYRFNGIGVTQNVLYENQKLIANQFNSAI
 
GKIQDSLSSTASALGKLQNVVNQNAQALNTLVKQLSSNFGAISSVLNDILSRLDPPEAEVQI
DRLITGRLQSLQTYVTQQLIRAAEIRASANLAATKMSECVLGQSKRVDFCGKGYHLMSFPQ
SAPHGVVFLHVTYVPAQEKNFTTAPAICHDGKAHFPREGVFVSNGTHWFVTQRNFYEPQIIT
TDNTFVSGNCDVVIGIVNNTVYDPLQPELDSFKEELDKYFKNHTSPDVDLGDISGINASVVN
IQKEIDRLNEVAKNLNESLIDLQELGKYEQYIKWPWYIWLGFIAGLIAIVMVTIMLCCMTSC
CSCLKGCCSCGSCCKFDEDDSEPVLKGVKLHYT*
**SEQ ID No.30: Nucleotide sequence of S antigen of Ad-S-Beta-Delta vaccine** 
ATGTTCGTGTTCCTGGTGCTGCTGCCCCTGGTGAGCAGCCAATGCGTGAACCTGAC
CACAAGAACACAGCTGCCCCCCGCCTACACCAACAGCTTCACAAGAGGCGTGTATTATC
CCGACAAGGTGTTCAGAAGCAGCGTGCTGCACAGCACCCAAGACCTGTTTCTGCCCTT
CTTCAGCAACGTGACCTGGTTCCACGCCATCCACGTGAGCGGCACCAACGGCACCAAG
AGATTCGCCAACCCCGTGCTGCCCTTCAACGACGGCGTGTACTTCGCTAGCACCGAGAA
GAGCAACATCATCAGAGGCTGGATCTTCGGCACCACCCTGGATAGCAAGACACAGAGC
CTGCTGATCGTGAACAACGCCACCAACGTGGTGATCAAGGTGTGCGAGTTTCAGTTCTG
CAACGACCCCTTCCTGGGCGTGTATTACCACAAGAACAACAAGAGCTGGATGGAGAGC
GAGTTCAGAGTCTACAGCAGCGCCAACAACTGCACCTTCGAGTACGTGAGCCAACCCT
TCCTGATGGACCTGGAGGGCAAGCAAGGCAATTTCAAGAACCTGAGAGAGTTCGTGTT
CAAGAACATCGACGGCTACTTCAAGATCTACAGCAAGCACACCCCCATCAACCTGGTGA
GAGGCCTGCCCCAAGGCTTCAGCGCCCTGGAGCCCCTGGTGGACCTGCCCATCGGCATC
AACATCACAAGATTTCAGACCCTGCACCGGAGCTATCTGACCCCTGGCGACTCCTCCTC
CGGGTGGACCGCTGGCGCCGCCGCTTACTACGTGGGCTACCTGCAGCCTAGAACCTTCC
TGCTGAAGTACAACGAGAACGGCACCATTACAGACGCCGTGGATTGCGCCCTGGACCC
CCTGAGCGAGACCAAGTGCACCCTGAAGAGCTTCACCGTGGAGAAGGGCATCTATCAG
ACAAGCAACTTCAGAGTGCAGCCCACCGAGAGCATCGTGAGATTCCCCAACATCACCA
ACCTGTGCCCCTTCGGCGAGGTGTTCAACGCCACAAGATTCGCTAGCGTGTACGCCTGG
AACAGAAAAAGAATCAGCAACTGCGTGGCCGACTACAGCGTGCTGTACAACAGCGCTA
GCTTCAGCACCTTCAAGTGCTACGGCGTGAGCCCCACCAAGCTGAACGACCTGTGCTTC
ACCAACGTGTACGCCGACAGCTTCGTGATCAGAGGCGACGAGGTGAGACAGATCGCCC
CCGGGCAGACCGGCAACATCGCCGACTACAACTATAAGCTCCCCGACGACTTCACCGGC
TGCGTGATCGCTTGGAACTCCAACAACCTGGACAGCAAGGTGGGCGGCAACTACAACT
 
ACAGATATAGACTGTTCAGAAAGAGCAACCTGAAGCCCTTCGAGAGAGACATCAGCAC
CGAGATCTACCAAGCCGGCAGCAAGCCCTGCAACGGCGTGAAGGGCTTCAACTGCTAC
TTCCCCCTGCAGAGCTACGGCTTTCAGCCCACCTACGGCGTGGGCTATCAGCCCTACAG
AGTGGTCGTGCTGAGCTTCGAGCTGCTGCACGCCCCCGCCACCGTCTGCGGCCCCAAG
AAGAGCACCAACCTGGTGAAGAACAAGTGCGTGAACTTCAACTTCAACGGGCTGACCG
GCACCGGCGTGCTGACCGAGAGCAACAAGAAGTTCCTGCCCTTTCAGCAGTTCGGCAG
AGACATCGCCGACACCACCGACGCCGTGAGAGACCCTCAGACCCTGGAGATCCTGGAC
ATCACACCCTGCAGCTTCGGCGGCGTGAGCGTGATCACCCCCGGCACCAACACAAGCA
ACCAAGTGGCCGTGCTGTACCAAGGCGTGAACTGCACCGAGGTGCCCGTGGCCATCCA
CGCCGATCAGCTGACCCCCACCTGGAGAGTCTATAGCACCGGCAGCAACGTGTTTCAGA
CAAGAGCCGGCTGCCTGATCGGCGCCGAGCACGTGAACAACAGCTACGAGTGCGACAT
CCCCATCGGCGCCGGCATCTGCGCTAGCTATCAGACACAGACCAACAGCCCTAGAAGAG
CTAGAAGCGTGGCTAGCCAAAGCATCATCGCCTACACCATGAGCCTGGGCGTGGAGAAC
AGCGTGGCCTACAGCAACAACAGCATCGCCATCCCCACCAACTTCACCATCAGCGTGAC
CACCGAGATCCTCCCTGTGAGCATGACCAAGACAAGCGTGGACTGCACCATGTACATCT
GCGGCGACAGCACCGAGTGCAGCAACCTGCTCCTGCAGTACGGCAGCTTCTGCACACA
GCTGAACAGAGCCCTGACCGGCATCGCCGTGGAGCAAGACAAGAACACCCAAGAGGT
GTTCGCCCAAGTGAAGCAGATCTACAAGACCCCCCCCATCAAGGACTTCGGCGGCTTCA
ACTTCAGCCAAATCCTGCCCGACCCTAGCAAGCCTAGCAAGAGAAGCTTCATCGAGGAC
CTGCTGTTCAACAAGGTGACCCTGGCCGACGCCGGCTTCATCAAGCAGTACGGCGACTG
CCTGGGCGACATCGCCGCTAGAGACCTGATCTGCGCTCAGAAGTTCAACGGCCTGACCG
TGCTGCCCCCCCTGCTGACCGACGAGATGATCGCTCAATACACAAGCGCCCTCCTGGCC
GGGACAATCACATCCGGCTGGACCTTCGGCGCCGGGGCTGCCCTGCAGATCCCCTTCGC
CATGCAGATGGCCTACAGATTCAACGGCATCGGCGTGACACAGAACGTGCTGTACGAGA
ATCAGAAGCTGATCGCCAATCAGTTCAACAGCGCCATCGGCAAGATCCAAGACAGCCTG
AGCAGCACCGCTAGCGCCCTGGGCAAGCTGCAAGACGTGGTGAATCAGAACGCCCAAG
CCCTGAACACCCTGGTGAAGCAGCTGAGCAGCAACTTCGGCGCCATCAGCAGCGTGCT
GAACGACATCCTGAGCAGACTGGACCCCCCCGAGGCCGAGGTGCAGATCGATAGACTG
ATCACCGGCAGACTGCAGAGCCTGCAGACCTACGTGACACAGCAGCTGATCAGAGCCG
CCGAGATCAGAGCTAGCGCCAACCTGGCCGCCACCAAGATGAGCGAGTGCGTGCTGGG
GCAGAGCAAGAGAGTGGACTTCTGCGGCAAGGGCTACCACCTGATGAGCTTCCCTCAG
 
AGCGCCCCCCACGGCGTGGTGTTCCTGCACGTGACCTACGTGCCCGCCCAAGAGAAGA
ACTTCACCACCGCTCCCGCCATCTGCCACGACGGCAAGGCCCACTTCCCTAGAGAGGGC
GTGTTCGTGAGCAACGGCACCCACTGGTTCGTGACACAGAGAAACTTCTACGAGCCTC
AGATCATCACCACCGACAACACCTTCGTGAGCGGCAACTGCGACGTGGTGATCGGCATC
GTGAACAATACCGTCTACGACCCCCTGCAACCCGAGCTGGACAGCTTCAAGGAGGAGC
TGGACAAGTACTTCAAGAACCACACAAGCCCCGACGTGGACCTGGGCGATATCAGCGG
CATCAACGCTAGCGTGGTGAACATTCAGAAGGAAATCGATCGGCTGAATGAGGTGGCCA
AGAACCTGAACGAGAGCCTGATCGACCTGCAAGAGCTGGGCAAGTACGAGCAGTACAT
CAAGTGGCCCTGGTACATCTGGCTGGGCTTCATCGCCGGCCTGATCGCCATCGTGATGGT
GACCATCATGCTGTGCTGCATGACAAGCTGCTGCAGCTGCCTGAAGGGCTGCTGTAGCT
GTGGCAGCTGCTGCAAGTTCGACGAGGACGACAGCGAGCCCGTGCTGAAGGGCGTGA
AGCTGCACTACACCTGA
**SEQ ID No.31: Amino acid sequence of S antigen of Ad-S-Beta-Delta vaccine** 
MFVFLVLLPLVSSQCVNLTTRTQLPPAYTNSFTRGVYYPDKVFRSSVLHSTQDLFLPFFS
NVTWFHAIHVSGTNGTKRFANPVLPFNDGVYFASTEKSNIIRGWIFGTTLDSKTQSLLIVNN
ATNVVIKVCEFQFCNDPFLGVYYHKNNKSWMESEFRVYSSANNCTFEYVSQPFLMDLEGK
QGNFKNLREFVFKNIDGYFKIYSKHTPINLVRGLPQGFSALEPLVDLPIGINITRFQTLHRSYL
TPGDSSSGWTAGAAAYYVGYLQPRTFLLKYNENGTITDAVDCALDPLSETKCTLKSFTVEK
GIYQTSNFRVQPTESIVRFPNITNLCPFGEVFNATRFASVYAWNRKRISNCVADYSVLYNSAS
FSTFKCYGVSPTKLNDLCFTNVYADSFVIRGDEVRQIAPGQTGNIADYNYKLPDDFTGCVIA
WNSNNLDSKVGGNYNYRYRLFRKSNLKPFERDISTEIYQAGSKPCNGVKGFNCYFPLQSY
GFQPTYGVGYQPYRVVVLSFELLHAPATVCGPKKSTNLVKNKCVNFNFNGLTGTGVLTESN
KKFLPFQQFGRDIADTTDAVRDPQTLEILDITPCSFGGVSVITPGTNTSNQVAVLYQGVNCTE
VPVAIHADQLTPTWRVYSTGSNVFQTRAGCLIGAEHVNNSYECDIPIGAGICASYQTQTNSP
RRARSVASQSIIAYTMSLGVENSVAYSNNSIAIPTNFTISVTTEILPVSMTKTSVDCTMYICGD
STECSNLLLQYGSFCTQLNRALTGIAVEQDKNTQEVFAQVKQIYKTPPIKDFGGFNFSQILPD
PSKPSKRSFIEDLLFNKVTLADAGFIKQYGDCLGDIAARDLICAQKFNGLTVLPPLLTDEMIA
QYTSALLAGTITSGWTFGAGAALQIPFAMQMAYRFNGIGVTQNVLYENQKLIANQFNSAIG
KIQDSLSSTASALGKLQDVVNQNAQALNTLVKQLSSNFGAISSVLNDILSRLDPPEAEVQID
RLITGRLQSLQTYVTQQLIRAAEIRASANLAATKMSECVLGQSKRVDFCGKGYHLMSFPQS
 
APHGVVFLHVTYVPAQEKNFTTAPAICHDGKAHFPREGVFVSNGTHWFVTQRNFYEPQIIT
TDNTFVSGNCDVVIGIVNNTVYDPLQPELDSFKEELDKYFKNHTSPDVDLGDISGINASVVN
IQKEIDRLNEVAKNLNESLIDLQELGKYEQYIKWPWYIWLGFIAGLIAIVMVTIMLCCMTSC
CSCLKGCCSCGSCCKFDEDDSEPVLKGVKLHYT*
**SEQ ID No.32: Nucleotide sequence of S antigen of Ad-S-Omicron-BA.1 vaccine** 
ATGTTCGTGTTCCTGGTGCTGCTGCCCCTGGTGAGCAGCCAATGCGTGAACCTGAC
CACAAGAACACAGCTGCCCCCCGCCTACACCAACAGCTTCACAAGAGGCGTGTACTAC
CCCGACAAGGTGTTCAGAAGCAGCGTCCTCCACAGCACCCAAGACCTGTTCCTGCCCTT
CTTCAGCAACGTGACCTGGTTCCACGTGATCAGCGGCACCAACGGCACCAAGAGATTC
GACAACCCCGTGCTGCCCTTCAACGACGGCGTGTACTTCGCTAGCATCGAGAAGAGCA
ACATCATCAGAGGCTGGATCTTCGGCACCACCCTGGACAGCAAGACACAGAGCCTGCT
GATCGTCAACAACGCCACCAACGTGGTGATCAAGGTGTGCGAGTTTCAGTTCTGCAACG
ACCCCTTCCTGGACCACAAGAACAACAAGAGCTGGATGGAGAGCGAGTTCAGAGTGTA
TAGCAGCGCTAACAACTGCACCTTCGAGTACGTGAGCCAACCCTTCCTGATGGACCTGG
AGGGCAAGCAAGGCAACTTCAAAAACCTGAGAGAGTTCGTGTTCAAGAACATCGACGG
CTACTTCAAGATCTACAGCAAGCACACCCCCATCATCGTGAGAGAGCCCGAGGACCTGC
CCCAAGGCTTCAGCGCCCTGGAGCCCCTGGTGGACCTGCCCATCGGCATCAACATCACA
AGATTTCAGACCCTGCTGGCCCTGCACAGATCCTATCTGACCCCTGGGGACAGCAGCAG
CGGCTGGACCGCTGGCGCTGCCGCCTACTACGTGGGCTACCTGCAGCCTAGAACCTTCC
TGCTGAAGTACAACGAGAACGGCACAATCACCGATGCCGTCGACTGCGCCCTGGACCC
CCTGAGCGAGACCAAGTGCACCCTGAAGAGCTTCACCGTGGAGAAGGGCATCTATCAG
ACAAGCAACTTCAGAGTGCAGCCCACCGAGAGCATCGTGAGATTCCCCAACATCACCA
ACCTGTGCCCCTTCGACGAGGTGTTCAACGCCACAAGATTCGCTAGCGTGTACGCCTGG
AATAGAAAGAGAATCAGCAACTGCGTGGCCGACTACAGCGTGCTGTACAACCTGGCCC
CCTTCTTCACCTTCAAATGCTACGGCGTGAGCCCCACCAAGCTGAACGACCTGTGCTTC
ACCAACGTGTACGCCGACAGCTTCGTGATCAGAGGCGACGAGGTGAGACAGATCGCCC
CCGGGCAGACCGGCAACATCGCCGACTACAACTACAAGCTGCCCGACGACTTCACCGG
CTGCGTGATCGCCTGGAACAGCAACAAGCTCGACAGCAAGGTGAGCGGCAACTACAAC
TACCTGTACAGACTGTTCAGAAAGAGCAACCTGAAGCCCTTCGAGAGAGACATCAGCA
CCGAGATCTACCAAGCCGGCAACAAGCCCTGCAACGGCGTGGCCGGCTTCAACTGCTA
 
CTTCCCCCTGAGAAGCTACAGCTTCAGACCCACCTACGGCGTGGGCCATCAGCCCTACA
GAGTGGTCGTGCTGAGCTTCGAGCTGCTGCACGCCCCCGCCACCGTGTGCGGCCCCAA
GAAGAGCACCAACCTGGTGAAGAACAAGTGCGTGAACTTCAACTTCAACGGCCTGAAG
GGCACCGGCGTGCTGACCGAGAGCAACAAGAAGTTTCTGCCCTTTCAGCAGTTCGGCA
GAGACATCGCCGACACCACCGACGCCGTGAGAGACCCTCAGACCCTGGAGATCCTGGA
CATCACCCCCTGCTCCTTCGGCGGCGTGAGCGTGATCACCCCCGGCACCAACACAAGCA
ACCAAGTGGCCGTGCTGTACCAAGGCGTGAACTGCACCGAGGTGCCCGTGGCCATCCA
CGCCGATCAGCTGACCCCCACCTGGAGAGTGTACAGCACCGGCAGCAACGTGTTTCAG
ACAAGAGCCGGCTGCCTGATCGGCGCCGAGTACGTGAACAACAGCTACGAGTGCGACA
TCCCCATCGGCGCCGGCATCTGCGCTAGCTATCAGACACAGACCAAGAGCCACAGAAG
AGCTAGAAGCGTGGCTAGCCAAAGCATCATCGCCTACACCATGAGCCTGGGCGCCGAGA
ACAGCGTGGCCTACAGCAACAACAGCATCGCCATCCCCACCAACTTCACCATCAGCGTG
ACCACCGAAATTCTGCCCGTGAGCATGACCAAGACAAGCGTGGACTGCACCATGTACAT
CTGCGGCGACAGCACCGAGTGCAGCAACCTGCTCCTGCAGTACGGCAGCTTCTGCACA
CAGCTGAAGAGAGCCCTGACCGGCATCGCCGTGGAGCAAGACAAGAACACCCAAGAG
GTGTTCGCCCAAGTGAAGCAGATCTACAAGACCCCCCCCATCAAGTACTTCGGCGGCTT
CAACTTCAGCCAAATCCTGCCCGACCCTAGCAAGCCTAGCAAGAGAAGCTTCATCGAGG
ACCTGCTGTTCAACAAGGTGACCCTGGCCGACGCCGGCTTCATCAAGCAGTACGGCGA
CTGCCTGGGCGATATTGCCGCTAGAGACCTGATCTGCGCTCAGAAGTTCAAGGGCCTGA
CCGTGCTGCCCCCCCTGCTGACCGACGAGATGATCGCTCAGTATACAAGCGCCCTCCTC
GCTGGGACCATCACAAGCGGCTGGACATTCGGGGCCGGCGCTGCCCTGCAGATCCCCTT
CGCCATGCAGATGGCCTACAGATTCAACGGCATCGGCGTGACACAGAACGTGCTGTACG
AGAATCAGAAGCTGATCGCCAATCAGTTCAACAGCGCCATCGGCAAGATCCAAGACAG
CCTGAGCAGCACCGCTAGCGCCCTGGGCAAGCTGCAAGACGTGGTGAACCACAACGCC
CAAGCCCTGAACACCCTGGTGAAGCAGCTGAGCAGCAAGTTCGGCGCCATCAGCAGCG
TGCTGAACGACATCTTCAGCAGACTGGACCCCCCCGAGGCCGAGGTGCAGATCGACCG
GCTCATCACCGGCAGACTGCAGAGCCTGCAGACCTACGTGACACAGCAGCTGATCAGA
GCCGCCGAGATCAGAGCTAGCGCCAACCTGGCCGCCACCAAGATGAGCGAGTGCGTGC
TGGGGCAGAGCAAGAGAGTGGACTTCTGCGGCAAGGGCTACCACCTGATGAGCTTCCC
TCAGAGCGCCCCCCACGGCGTGGTGTTCCTGCACGTGACCTACGTGCCCGCCCAAGAG
AAGAACTTCACCACCGCTCCCGCCATTTGCCACGACGGCAAGGCCCACTTCCCTAGAGA
 
GGGCGTGTTCGTGAGCAACGGCACCCACTGGTTCGTGACACAGAGAAACTTCTACGAG
CCTCAGATCATCACCACCGACAACACCTTCGTCAGCGGCAACTGTGACGTGGTGATCGG
CATCGTGAACAATACCGTGTACGACCCCCTGCAGCCCGAACTGGACAGCTTCAAGGAG
GAGCTGGACAAGTACTTCAAGAACCACACAAGCCCCGACGTGGACCTGGGCGACATTA
GCGGCATCAACGCTAGCGTGGTGAACATTCAGAAGGAGATTGACAGACTGAACGAGGT
GGCCAAGAACCTGAACGAGAGCCTGATCGACCTGCAAGAGCTGGGCAAGTACGAGCA
GTACATCAAGTGGCCCTGGTACATCTGGCTGGGCTTCATCGCCGGCCTGATCGCCATCGT
GATGGTGACCATCATGCTGTGCTGCATGACAAGCTGCTGTAGCTGCCTGAAAGGCTGCT
GCAGCTGCGGCAGCTGCTGCAAGTTCGACGAGGACGACAGCGAGCCCGTGCTGAAGG
GCGTGAAGCTGCACTACACCTGA
**SEQ ID No.33: Amino acid sequence of S antigen of Ad-S-Omicron-BA.1 vaccine** 
MFVFLVLLPLVSSQCVNLTTRTQLPPAYTNSFTRGVYYPDKVFRSSVLHSTQDLFLPFFS
NVTWFHVISGTNGTKRFDNPVLPFNDGVYFASIEKSNIIRGWIFGTTLDSKTQSLLIVNNATN
VVIKVCEFQFCNDPFLDHKNNKSWMESEFRVYSSANNCTFEYVSQPFLMDLEGKQGNFKN
LREFVFKNIDGYFKIYSKHTPIIVREPEDLPQGFSALEPLVDLPIGINITRFQTLLALHRSYLTP
GDSSSGWTAGAAAYYVGYLQPRTFLLKYNENGTITDAVDCALDPLSETKCTLKSFTVEKGI
YQTSNFRVQPTESIVRFPNITNLCPFDEVFNATRFASVYAWNRKRISNCVADYSVLYNLAPFF
TFKCYGVSPTKLNDLCFTNVYADSFVIRGDEVRQIAPGQTGNIADYNYKLPDDFTGCVIAW
NSNKLDSKVSGNYNYLYRLFRKSNLKPFERDISTEIYQAGNKPCNGVAGFNCYFPLRSYSFR
PTYGVGHQPYRVVVLSFELLHAPATVCGPKKSTNLVKNKCVNFNFNGLKGTGVLTESNKKF
LPFQQFGRDIADTTDAVRDPQTLEILDITPCSFGGVSVITPGTNTSNQVAVLYQGVNCTEVPV
AIHADQLTPTWRVYSTGSNVFQTRAGCLIGAEYVNNSYECDIPIGAGICASYQTQTKSHRRA
RSVASQSIIAYTMSLGAENSVAYSNNSIAIPTNFTISVTTEILPVSMTKTSVDCTMYICGDSTE
CSNLLLQYGSFCTQLKRALTGIAVEQDKNTQEVFAQVKQIYKTPPIKYFGGFNFSQILPDPSK
PSKRSFIEDLLFNKVTLADAGFIKQYGDCLGDIAARDLICAQKFKGLTVLPPLLTDEMIAQYT
SALLAGTITSGWTFGAGAALQIPFAMQMAYRFNGIGVTQNVLYENQKLIANQFNSAIGKIQ
DSLSSTASALGKLQDVVNHNAQALNTLVKQLSSKFGAISSVLNDIFSRLDPPEAEVQIDRLIT
GRLQSLQTYVTQQLIRAAEIRASANLAATKMSECVLGQSKRVDFCGKGYHLMSFPQSAPH
GVVFLHVTYVPAQEKNFTTAPAICHDGKAHFPREGVFVSNGTHWFVTQRNFYEPQIITTDN
TFVSGNCDVVIGIVNNTVYDPLQPELDSFKEELDKYFKNHTSPDVDLGDISGINASVVNIQK
 
EIDRLNEVAKNLNESLIDLQELGKYEQYIKWPWYIWLGFIAGLIAIVMVTIMLCCMTSCCSC
LKGCCSCGSCCKFDEDDSEPVLKGVKLHYT*
**SEQ ID No.34: Nucleotide sequence of S antigen of Ad-S-Omicron-BA.2 vaccine** 
ATGTTCGTGTTCCTGGTGCTGCTGCCCCTGGTGAGCAGCCAATGCGTGAACCTGAT
CACAAGAACACAGAGCTACACCAACAGCTTCACAAGAGGCGTGTACTACCCCGACAAG
GTGTTCAGAAGCAGCGTCCTCCACAGCACCCAAGACCTGTTCCTGCCTTTCTTTAGCAA
CGTGACCTGGTTCCACGCCATCCACGTGAGCGGCACCAACGGCACCAAGAGATTCGAC
AACCCCGTGCTGCCCTTCAACGACGGCGTGTACTTCGCTAGCACCGAGAAGAGCAACAT
CATCAGAGGCTGGATCTTCGGCACCACCCTGGACAGCAAGACACAGAGCCTGCTGATC
GTCAACAACGCCACCAACGTGGTGATCAAGGTGTGCGAGTTTCAGTTCTGCAACGACC
CCTTCCTGGACGTGTACTACCACAAGAACAACAAGAGCTGGATGGAGAGCGAGTTCAG
AGTGTACAGCAGCGCCAACAACTGCACCTTCGAGTACGTGAGCCAACCCTTCCTGATGG
ACCTGGAGGGCAAGCAAGGCAACTTCAAAAACCTGAGAGAGTTCGTGTTCAAGAACAT
CGACGGCTACTTCAAGATCTACAGCAAGCACACCCCCATCAACCTGGGCAGAGACCTGC
CCCAAGGCTTCAGCGCCCTGGAGCCCCTGGTGGACCTGCCCATCGGCATCAACATCACA
AGATTTCAGACCCTGCTGGCCCTGCACAGATCCTACCTCACACCTGGCGACAGCAGCTC
CGGCTGGACCGCTGGCGCTGCCGCTTACTACGTGGGCTACCTGCAGCCTAGAACCTTCC
TGCTGAAGTACAACGAGAACGGCACAATCACCGATGCCGTCGACTGCGCCCTGGACCC
CCTGAGCGAGACCAAGTGCACCCTGAAGAGCTTCACCGTGGAGAAGGGCATCTATCAG
ACAAGCAACTTCAGAGTGCAGCCCACCGAGAGCATCGTGAGATTCCCCAACATCACCA
ACCTGTGCCCCTTCGACGAGGTGTTCAACGCCACAAGATTCGCTAGCGTGTACGCCTGG
AACAGAAAAAGAATCAGCAACTGCGTGGCCGACTACAGCGTGCTGTACAACTTCGCCC
CCTTCTTCGCCTTCAAATGCTACGGCGTGAGCCCCACCAAGCTGAACGACCTGTGCTTC
ACCAACGTGTACGCCGACAGCTTCGTGATCAGAGGCAACGAGGTGAGCCAAATCGCCC
CCGGGCAGACCGGCAACATCGCCGACTACAACTACAAGCTGCCCGACGACTTCACCGG
CTGCGTGATCGCCTGGAACTCCAACAAGCTCGACAGCAAGGTGGGCGGCAACTACAAC
TACCTGTACAGACTGTTCAGAAAGAGCAACCTGAAGCCCTTCGAGAGAGACATCAGCA
CCGAGATCTACCAAGCCGGCAACAAGCCCTGCAACGGCGTGGCCGGCTTCAACTGCTA
CTTCCCCCTGAGAAGCTACGGCTTCAGACCCACCTACGGCGTGGGCCATCAGCCCTACA
GAGTGGTCGTGCTGAGCTTCGAGCTGCTGCACGCCCCCGCCACCGTGTGCGGCCCCAA
 
GAAGAGCACCAACCTGGTGAAGAACAAGTGCGTGAACTTCAACTTCAACGGCCTGACC
GGCACCGGCGTGCTGACCGAGAGCAACAAGAAGTTCCTCCCCTTTCAGCAGTTCGGCA
GAGACATCGCCGACACCACCGACGCCGTGAGAGACCCTCAGACCCTGGAGATCCTGGA
CATCACCCCCTGCAGCTTCGGCGGCGTGAGCGTGATCACCCCCGGCACCAACACAAGC
AACCAAGTGGCCGTGCTGTACCAAGGCGTGAACTGCACCGAGGTGCCCGTGGCCATCC
ACGCCGATCAGCTGACCCCCACCTGGAGAGTGTACTCCACCGGCAGCAACGTGTTTCAG
ACAAGAGCCGGCTGCCTGATCGGCGCCGAGTACGTGAACAACAGCTACGAGTGCGACA
TCCCCATCGGCGCCGGCATCTGCGCTAGCTATCAGACACAGACCAAGAGCCACAGAAG
AGCTAGAAGCGTGGCTAGCCAAAGCATCATCGCCTACACCATGAGCCTGGGCGCCGAGA
ACAGCGTGGCCTACAGCAACAACAGCATCGCCATCCCCACCAACTTCACCATCAGCGTG
ACCACCGAGATCCTGCCTGTGAGCATGACCAAGACAAGCGTGGACTGCACCATGTACAT
CTGCGGCGACAGCACCGAGTGCAGCAACCTGCTCCTGCAGTACGGCAGCTTCTGCACA
CAGCTGAAGAGAGCCCTGACCGGCATCGCCGTGGAGCAAGACAAGAACACCCAAGAG
GTGTTCGCCCAAGTGAAGCAGATCTACAAGACCCCCCCCATCAAGTACTTCGGCGGCTT
CAACTTCAGCCAAATCCTGCCCGACCCTAGCAAGCCTAGCAAGCGGAGCTTCATCGAGG
ACCTGCTGTTCAACAAGGTGACCCTGGCCGACGCCGGCTTCATCAAGCAGTACGGCGA
CTGCCTCGGCGACATCGCCGCTAGAGACCTGATCTGCGCTCAGAAGTTCAATGGCCTGA
CCGTGCTGCCCCCCCTGCTGACCGACGAGATGATCGCTCAGTATACCTCCGCCCTGCTCG
CCGGGACCATCACAAGCGGCTGGACCTTCGGCGCCGGCGCCGCCCTCCAAATCCCCTTC
GCCATGCAGATGGCCTACAGATTCAACGGCATCGGCGTGACACAGAACGTGCTGTACGA
GAATCAGAAGCTGATCGCCAATCAGTTCAACAGCGCCATCGGCAAGATCCAAGACAGC
CTGAGCAGCACCGCTAGCGCCCTGGGCAAGCTGCAAGACGTGGTGAACCACAACGCCC
AAGCCCTGAACACCCTGGTGAAGCAGCTGAGCAGCAAGTTCGGCGCCATCAGCAGCGT
GCTGAACGATATCCTGAGCAGACTGGACCCCCCCGAGGCCGAGGTGCAGATCGATAGAC
TGATCACCGGCAGACTGCAGAGCCTGCAGACCTACGTGACACAGCAGCTGATCAGAGC
CGCCGAGATCAGAGCTAGCGCCAACCTGGCCGCCACCAAGATGAGCGAGTGCGTGCTG
GGGCAGAGCAAGAGAGTGGACTTCTGCGGCAAGGGCTACCACCTGATGAGCTTCCCTC
AGAGCGCCCCCCACGGCGTGGTGTTCCTGCACGTGACCTACGTGCCCGCCCAAGAGAA
GAACTTCACAACCGCCCCCGCCATCTGCCACGACGGCAAGGCCCACTTCCCTAGAGAG
GGCGTGTTCGTGAGCAACGGCACCCACTGGTTCGTGACACAGAGAAACTTCTACGAGC
CTCAGATCATCACCACCGACAACACCTTCGTGAGCGGCAACTGCGACGTGGTGATCGGG
 
ATCGTGAACAACACCGTGTACGATCCCCTGCAGCCCGAGCTGGACAGCTTCAAGGAGG
AGCTGGACAAGTACTTCAAGAACCACACAAGCCCCGACGTGGACCTGGGCGACATTAG
CGGCATCAACGCTAGCGTGGTGAACATTCAGAAGGAGATCGACAGACTGAATGAGGTG
GCCAAGAACCTGAACGAGAGCCTGATCGACCTGCAAGAGCTGGGCAAGTACGAGCAGT
ACATCAAGTGGCCCTGGTACATCTGGCTGGGCTTCATCGCCGGCCTGATCGCCATCGTGA
TGGTGACCATCATGCTGTGCTGCATGACAAGCTGCTGTAGCTGCCTGAAGGGCTGCTGC
AGCTGCGGCTCCTGCTGCAAGTTCGACGAGGACGACAGCGAGCCCGTGCTGAAGGGCG
TGAAGCTGCACTACACCTGA
**SEQ ID No.35: Amino acid sequence of S antigen of Ad-S-Omicron-BA.2 vaccine** 
MFVFLVLLPLVSSQCVNLITRTQSYTNSFTRGVYYPDKVFRSSVLHSTQDLFLPFFSNVT
WFHAIHVSGTNGTKRFDNPVLPFNDGVYFASTEKSNIIRGWIFGTTLDSKTQSLLIVNNATN
VVIKVCEFQFCNDPFLDVYYHKNNKSWMESEFRVYSSANNCTFEYVSQPFLMDLEGKQGN
FKNLREFVFKNIDGYFKIYSKHTPINLGRDLPQGFSALEPLVDLPIGINITRFQTLLALHRSYL
TPGDSSSGWTAGAAAYYVGYLQPRTFLLKYNENGTITDAVDCALDPLSETKCTLKSFTVEK
GIYQTSNFRVQPTESIVRFPNITNLCPFDEVFNATRFASVYAWNRKRISNCVADYSVLYNFAP
FFAFKCYGVSPTKLNDLCFTNVYADSFVIRGNEVSQIAPGQTGNIADYNYKLPDDFTGCVIA
WNSNKLDSKVGGNYNYLYRLFRKSNLKPFERDISTEIYQAGNKPCNGVAGFNCYFPLRSYG
FRPTYGVGHQPYRVVVLSFELLHAPATVCGPKKSTNLVKNKCVNFNFNGLTGTGVLTESNK
KFLPFQQFGRDIADTTDAVRDPQTLEILDITPCSFGGVSVITPGTNTSNQVAVLYQGVNCTEV
PVAIHADQLTPTWRVYSTGSNVFQTRAGCLIGAEYVNNSYECDIPIGAGICASYQTQTKSHR
RARSVASQSIIAYTMSLGAENSVAYSNNSIAIPTNFTISVTTEILPVSMTKTSVDCTMYICGDS
TECSNLLLQYGSFCTQLKRALTGIAVEQDKNTQEVFAQVKQIYKTPPIKYFGGFNFSQILPDP
SKPSKRSFIEDLLFNKVTLADAGFIKQYGDCLGDIAARDLICAQKFNGLTVLPPLLTDEMIAQ
YTSALLAGTITSGWTFGAGAALQIPFAMQMAYRFNGIGVTQNVLYENQKLIANQFNSAIGKI
QDSLSSTASALGKLQDVVNHNAQALNTLVKQLSSKFGAISSVLNDILSRLDPPEAEVQIDRLI
TGRLQSLQTYVTQQLIRAAEIRASANLAATKMSECVLGQSKRVDFCGKGYHLMSFPQSAPH
GVVFLHVTYVPAQEKNFTTAPAICHDGKAHFPREGVFVSNGTHWFVTQRNFYEPQIITTDN
TFVSGNCDVVIGIVNNTVYDPLQPELDSFKEELDKYFKNHTSPDVDLGDISGINASVVNIQK
EIDRLNEVAKNLNESLIDLQELGKYEQYIKWPWYIWLGFIAGLIAIVMVTIMLCCMTSCCSC
LKGCCSCGSCCKFDEDDSEPVLKGVKLHYT*
**SEQ ID No.36: Nucleotide sequence of S antigen of Ad-S-Omicron-BA.2.12.1 vaccine** 
ATGTTCGTGTTCCTGGTGCTGCTGCCCCTGGTGAGCAGCCAATGCGTGAACCTGAT
CACAAGAACACAGAGCTACACCAACAGCTTCACAAGAGGCGTGTACTACCCCGACAAG
GTGTTCAGAAGCAGCGTCCTCCACAGCACCCAAGACCTGTTCCTGCCTTTCTTTAGCAA
CGTGACCTGGTTCCACGCCATCCACGTGAGCGGCACCAACGGCACCAAGAGATTCGAC
AACCCCGTGCTGCCCTTCAACGACGGCGTGTACTTCGCTAGCACCGAGAAGAGCAACAT
CATCAGAGGCTGGATCTTCGGCACCACCCTGGACAGCAAGACACAGAGCCTGCTGATC
GTCAACAACGCCACCAACGTGGTGATCAAGGTGTGCGAGTTTCAGTTCTGCAACGACC
CCTTCCTGGACGTGTACTACCACAAGAACAACAAGAGCTGGATGGAGAGCGAGTTCAG
AGTGTACAGCAGCGCCAACAACTGCACCTTCGAGTACGTGAGCCAACCCTTCCTGATGG
ACCTGGAGGGCAAGCAAGGCAACTTCAAAAACCTGAGAGAGTTCGTGTTCAAGAACAT
CGACGGCTACTTCAAGATCTACAGCAAGCACACCCCCATCAACCTGGGCAGAGACCTGC
CCCAAGGCTTCAGCGCCCTGGAGCCCCTGGTGGACCTGCCCATCGGCATCAACATCACA
AGATTTCAGACCCTGCTGGCCCTGCACAGATCCTACCTCACACCTGGCGACAGCAGCTC
CGGCTGGACCGCTGGCGCTGCCGCTTACTACGTGGGCTACCTGCAGCCTAGAACCTTCC
TGCTGAAGTACAACGAGAACGGCACAATCACCGATGCCGTCGACTGCGCCCTGGACCC
CCTGAGCGAGACCAAGTGCACCCTGAAGAGCTTCACCGTGGAGAAGGGCATCTATCAG
ACAAGCAACTTCAGAGTGCAGCCCACCGAGAGCATCGTGAGATTCCCCAACATCACCA
ACCTGTGCCCCTTCGACGAGGTGTTCAACGCCACAAGATTCGCTAGCGTGTACGCCTGG
AACAGAAAAAGAATCAGCAACTGCGTGGCCGACTACAGCGTGCTGTACAACTTCGCCC
CCTTCTTCGCCTTCAAATGCTACGGCGTGAGCCCCACCAAGCTGAACGACCTGTGCTTC
ACCAACGTGTACGCCGACAGCTTCGTGATCAGAGGCAACGAGGTGAGCCAAATCGCCC
CCGGGCAGACCGGCAACATCGCCGACTACAACTACAAGCTGCCCGACGACTTCACCGG
CTGCGTGATCGCCTGGAACTCCAACAAGCTCGACAGCAAGGTGGGCGGCAACTACAAC
TATCAGTACAGACTGTTCAGAAAGAGCAACCTGAAGCCCTTCGAGAGAGACATCAGCA
CCGAGATCTACCAAGCCGGCAACAAGCCCTGCAACGGCGTGGCCGGCTTCAACTGCTA
CTTCCCCCTGAGAAGCTACGGCTTCAGACCCACCTACGGCGTGGGCCATCAGCCCTACA
GAGTGGTCGTGCTGAGCTTCGAGCTGCTGCACGCCCCCGCCACCGTGTGCGGCCCCAA
GAAGAGCACCAACCTGGTGAAGAACAAGTGCGTGAACTTCAACTTCAACGGCCTGACC
GGCACCGGCGTGCTGACCGAGAGCAACAAGAAGTTCCTCCCCTTTCAGCAGTTCGGCA
 
GAGACATCGCCGACACCACCGACGCCGTGAGAGACCCTCAGACCCTGGAGATCCTGGA
CATCACCCCCTGCAGCTTCGGCGGCGTGAGCGTGATCACCCCCGGCACCAACACAAGC
AACCAAGTGGCCGTGCTGTACCAAGGCGTGAACTGCACCGAGGTGCCCGTGGCCATCC
ACGCCGATCAGCTGACCCCCACCTGGAGAGTGTACTCCACCGGCAGCAACGTGTTTCAG
ACAAGAGCCGGCTGCCTGATCGGCGCCGAGTACGTGAACAACAGCTACGAGTGCGACA
TCCCCATCGGCGCCGGCATCTGCGCTAGCTATCAGACACAGACCAAGAGCCACAGAAG
AGCTAGAAGCGTGGCTAGCCAAAGCATCATCGCCTACACCATGAGCCTGGGCGCCGAGA
ACCTGGTGGCCTACAGCAACAACAGCATCGCCATCCCCACCAACTTCACCATCAGCGTG
ACCACCGAGATCCTGCCTGTGAGCATGACCAAGACAAGCGTGGACTGCACCATGTACAT
CTGCGGCGACAGCACCGAGTGCAGCAACCTGCTCCTGCAGTACGGCAGCTTCTGCACA
CAGCTGAAGAGAGCCCTGACCGGCATCGCCGTGGAGCAAGACAAGAACACCCAAGAG
GTGTTCGCCCAAGTGAAGCAGATCTACAAGACCCCCCCCATCAAGTACTTCGGCGGCTT
CAACTTCAGCCAAATCCTGCCCGACCCTAGCAAGCCTAGCAAGCGGAGCTTCATCGAGG
ACCTGCTGTTCAACAAGGTGACCCTGGCCGACGCCGGCTTCATCAAGCAGTACGGCGA
CTGCCTCGGCGACATCGCCGCTAGAGACCTGATCTGCGCTCAGAAGTTCAATGGCCTGA
CCGTGCTGCCCCCCCTGCTGACCGACGAGATGATCGCTCAGTATACCTCCGCCCTGCTCG
CCGGGACCATCACAAGCGGCTGGACCTTCGGCGCCGGCGCCGCCCTCCAAATCCCCTTC
GCCATGCAGATGGCCTACAGATTCAACGGCATCGGCGTGACACAGAACGTGCTGTACGA
GAATCAGAAGCTGATCGCCAATCAGTTCAACAGCGCCATCGGCAAGATCCAAGACAGC
CTGAGCAGCACCGCTAGCGCCCTGGGCAAGCTGCAAGACGTGGTGAACCACAACGCCC
AAGCCCTGAACACCCTGGTGAAGCAGCTGAGCAGCAAGTTCGGCGCCATCAGCAGCGT
GCTGAACGATATCCTGAGCAGACTGGACCCCCCCGAGGCCGAGGTGCAGATCGATAGAC
TGATCACCGGCAGACTGCAGAGCCTGCAGACCTACGTGACACAGCAGCTGATCAGAGC
CGCCGAGATCAGAGCTAGCGCCAACCTGGCCGCCACCAAGATGAGCGAGTGCGTGCTGG
GGCAGAGCAAGAGAGTGGACTTCTGCGGCAAGGGCTACCACCTGATGAGCTTCCCTC
AGAGCGCCCCCCACGGCGTGGTGTTCCTGCACGTGACCTACGTGCCCGCCCAAGAGAA
GAACTTCACAACCGCCCCCGCCATCTGCCACGACGGCAAGGCCCACTTCCCTAGAGAG
GGCGTGTTCGTGAGCAACGGCACCCACTGGTTCGTGACACAGAGAAACTTCTACGAGC
CTCAGATCATCACCACCGACAACACCTTCGTGAGCGGCAACTGCGACGTGGTGATCGGG
ATCGTGAACAACACCGTGTACGATCCCCTGCAGCCCGAGCTGGACAGCTTCAAGGAGG
AGCTGGACAAGTACTTCAAGAACCACACAAGCCCCGACGTGGACCTGGGCGACATTAG
 
CGGCATCAACGCTAGCGTGGTGAACATTCAGAAGGAGATCGACAGACTGAATGAGGTG
GCCAAGAACCTGAACGAGAGCCTGATCGACCTGCAAGAGCTGGGCAAGTACGAGCAGT
ACATCAAGTGGCCCTGGTACATCTGGCTGGGCTTCATCGCCGGCCTGATCGCCATCGTGA
TGGTGACCATCATGCTGTGCTGCATGACAAGCTGCTGTAGCTGCCTGAAGGGCTGCTGC
AGCTGCGGCTCCTGCTGCAAGTTCGACGAGGACGACAGCGAGCCCGTGCTGAAGGGCG
TGAAGCTGCACTACACCTGA
**SEQ ID No.37: Amino acid sequence of S antigen of Ad-S-Omicron-BA.2.12.1 vaccine** 
MFVFLVLLPLVSSQCVNLITRTQSYTNSFTRGVYYPDKVFRSSVLHSTQDLFLPFFSNVT
WFHAIHVSGTNGTKRFDNPVLPFNDGVYFASTEKSNIIRGWIFGTTLDSKTQSLLIVNNATN
VVIKVCEFQFCNDPFLDVYYHKNNKSWMESEFRVYSSANNCTFEYVSQPFLMDLEGKQGN
FKNLREFVFKNIDGYFKIYSKHTPINLGRDLPQGFSALEPLVDLPIGINITRFQTLLALHRSYL
TPGDSSSGWTAGAAAYYVGYLQPRTFLLKYNENGTITDAVDCALDPLSETKCTLKSFTVEK
GIYQTSNFRVQPTESIVRFPNITNLCPFDEVFNATRFASVYAWNRKRISNCVADYSVLYNFAP
FFAFKCYGVSPTKLNDLCFTNVYADSFVIRGNEVSQIAPGQTGNIADYNYKLPDDFTGCVIA
WNSNKLDSKVGGNYNYQYRLFRKSNLKPFERDISTEIYQAGNKPCNGVAGFNCYFPLRSY
GFRPTYGVGHQPYRVVVLSFELLHAPATVCGPKKSTNLVKNKCVNFNFNGLTGTGVLTESN
KKFLPFQQFGRDIADTTDAVRDPQTLEILDITPCSFGGVSVITPGTNTSNQVAVLYQGVNCTE
VPVAIHADQLTPTWRVYSTGSNVFQTRAGCLIGAEYVNNSYECDIPIGAGICASYQTQTKSH
RRARSVASQSIIAYTMSLGAENLVAYSNNSIAIPTNFTISVTTEILPVSMTKTSVDCTMYICGD
STECSNLLLQYGSFCTQLKRALTGIAVEQDKNTQEVFAQVKQIYKTPPIKYFGGFNFSQILPD
PSKPSKRSFIEDLLFNKVTLADAGFIKQYGDCLGDIAARDLICAQKFNGLTVLPPLLTDEMIA
QYTSALLAGTITSGWTFGAGAALQIPFAMQMAYRFNGIGVTQNVLYENQKLIANQFNSAIG
KIQDSLSSTASALGKLQDVVNHNAQALNTLVKQLSSKFGAISSVLNDILSRLDPPEAEVQID
RLITGRLQSLQTYVTQQLIRAAEIRASANLAATKMSECVLGQSKRVDFCGKGYHLMSFPQS
APHGVVFLHVTYVPAQEKNFTTAPAICHDGKAHFPREGVFVSNGTHWFVTQRNFYEPQIIT
TDNTFVSGNCDVVIGIVNNTVYDPLQPELDSFKEELDKYFKNHTSPDVDLGDISGINASVVN
IQKEIDRLNEVAKNLNESLIDLQELGKYEQYIKWPWYIWLGFIAGLIAIVMVTIMLCCMTSC
CSCLKGCCSCGSCCKFDEDDSEPVLKGVKLHYT*
**SEQ ID No.38: Nucleotide sequence of S antigen of Ad-S-Omicron-BA.4/5 vaccine** 
ATGTTCGTGTTCCTGGTGCTGCTGCCCCTGGTGAGCAGCCAATGCGTGAACCTGAT
CACAAGAACACAGAGCTACACCAACAGCTTCACAAGAGGCGTGTACTACCCCGACAAG
GTGTTCAGAAGCAGCGTCCTGCATTCCACCCAAGACCTGTTTCTCCCCTTCTTCAGCAA
CGTGACCTGGTTCCACGCCATCAGCGGCACCAACGGCACCAAGAGATTCGACAACCCC
GTGCTGCCCTTCAACGACGGCGTGTACTTCGCTAGCACCGAGAAGAGCAACATCATCAG
AGGCTGGATCTTCGGCACCACCCTGGACAGCAAGACACAGAGCCTGCTGATCGTGAAC
AACGCCACAAACGTGGTGATCAAGGTGTGCGAGTTTCAGTTCTGCAACGACCCCTTCCT
GGACGTGTACTACCACAAGAACAACAAGAGCTGGATGGAGAGCGAGTTCCGGGTGTAC
AGCAGCGCCAACAACTGCACCTTCGAGTACGTGAGCCAACCCTTCCTGATGGACCTGG
AGGGCAAGCAAGGCAACTTCAAGAACCTGAGAGAGTTCGTGTTCAAGAACATCGACGG
CTACTTCAAGATCTACAGCAAGCACACCCCCATCAACCTGGGCAGAGACCTGCCCCAAG
GCTTCAGCGCCCTGGAGCCCCTGGTGGACCTGCCCATCGGCATCAACATCACAAGATTT
CAGACACTGCTCGCCCTGCACCGGAGCTACCTCACCCCTGGCGACAGCAGCTCCGGCT
GGACCGCTGGGGCTGCCGCCTACTACGTGGGCTACCTGCAGCCTAGAACCTTCCTGCTG
AAGTACAACGAGAACGGCACCATCACCGACGCTGTGGACTGTGCCCTGGACCCCCTGA
GCGAGACCAAGTGCACCCTGAAGAGCTTCACCGTGGAGAAGGGCATCTATCAGACAAG
CAACTTCAGAGTGCAGCCCACCGAGAGCATCGTGAGATTCCCCAACATCACCAACCTGT
GCCCCTTCGACGAGGTGTTCAACGCCACAAGATTCGCTAGCGTGTACGCCTGGAACAGA
AAGAGAATCAGCAACTGCGTGGCCGACTACAGCGTGCTGTACAACTTCGCCCCCTTCTT
CGCCTTCAAATGCTACGGCGTCAGCCCCACCAAGCTGAACGACCTGTGCTTCACCAACG
TGTACGCCGACAGCTTCGTGATCAGAGGCAACGAGGTGAGCCAAATCGCCCCCGGGCA
GACCGGCAACATCGCCGACTATAACTACAAACTGCCCGACGACTTCACCGGCTGCGTGA
TCGCCTGGAACTCCAACAAGCTGGACAGCAAAGTGGGCGGCAACTACAACTACAGATA
CAGACTGTTCAGAAAGAGCAACCTGAAGCCCTTCGAGAGAGACATCAGCACCGAGATC
TACCAAGCCGGCAACAAGCCCTGCAACGGCGTGGCCGGCGTGAACTGCTACTTCCCCC
TGCAGAGCTACGGCTTCAGACCCACCTACGGCGTGGGCCATCAGCCCTACAGAGTGGTC
GTGCTGAGCTTCGAGCTGCTGCACGCCCCTGCCACAGTGTGCGGCCCCAAGAAGAGCA
CCAACCTGGTGAAGAACAAGTGCGTGAACTTCAACTTCAACGGCCTGACCGGCACCGG
CGTGCTGACCGAGAGCAACAAGAAGTTCCTGCCCTTTCAGCAGTTCGGCAGAGACATC
GCCGACACCACCGATGCTGTGAGAGACCCTCAGACCCTGGAGATCCTGGACATCACCCC
 
TTGCAGCTTCGGCGGCGTGAGCGTGATCACCCCCGGCACCAACACAAGCAACCAAGTG
GCCGTGCTGTACCAAGGCGTCAACTGCACAGAGGTGCCCGTGGCCATCCACGCCGATCA
GCTGACCCCCACCTGGAGAGTGTACTCCACCGGCAGCAACGTGTTTCAGACAAGAGCC
GGCTGCCTGATCGGCGCCGAGTACGTGAACAACAGCTACGAGTGCGACATCCCCATCGG
CGCCGGCATCTGCGCTAGCTATCAGACACAGACCAAGAGCCACAGAAGAGCTAGAAGC
GTGGCTAGCCAAAGCATCATCGCCTACACCATGAGCCTGGGCGCCGAGAACAGCGTGG
CCTACAGCAACAACAGCATCGCCATCCCCACCAACTTCACCATCAGCGTGACCACCGAG
ATCCTGCCTGTGAGCATGACCAAGACAAGCGTGGACTGCACCATGTACATCTGCGGCGA
CAGCACCGAGTGCAGCAACCTGCTCCTGCAGTACGGCAGCTTCTGCACACAGCTGAAG
AGAGCCCTGACCGGCATCGCCGTGGAGCAAGACAAGAACACCCAAGAGGTGTTCGCCC
AAGTGAAGCAGATCTACAAGACCCCCCCCATCAAGTACTTCGGCGGCTTCAACTTCAGC
CAAATCCTGCCCGACCCTAGCAAGCCTAGCAAGCGGAGCTTCATCGAGGACCTGCTGTT
CAACAAGGTGACCCTGGCCGACGCCGGCTTCATCAAGCAGTACGGCGACTGCCTCGGC
GACATCGCTGCTAGAGACCTGATCTGCGCTCAGAAGTTCAACGGCCTCACAGTGCTGCC
CCCCCTGCTGACCGACGAGATGATCGCTCAGTACACCTCCGCTCTGCTGGCCGGCACAA
TTACATCCGGCTGGACCTTCGGCGCCGGCGCCGCCCTGCAAATCCCCTTCGCCATGCAG
ATGGCCTACAGATTCAACGGCATCGGCGTGACACAGAACGTGCTGTACGAGAATCAGAA
GCTGATCGCCAATCAGTTCAACAGCGCCATCGGCAAGATCCAAGACAGCCTGAGCAGC
ACCGCTAGCGCCCTGGGCAAGCTGCAAGACGTGGTGAACCACAACGCCCAAGCCCTGA
ACACCCTGGTGAAGCAGCTGAGCAGCAAGTTCGGCGCCATCTCCTCCGTCCTGAACGA
CATCCTGAGCAGACTGGACCCCCCCGAGGCCGAGGTGCAGATTGACAGACTGATTACCG
GCAGACTGCAGAGCCTGCAGACCTACGTGACACAGCAGCTGATCAGAGCCGCCGAGAT
CAGAGCTAGCGCCAACCTGGCCGCCACCAAGATGAGCGAGTGCGTGCTGGGGCAGAGC
AAGAGAGTGGACTTCTGCGGCAAGGGCTACCACCTGATGAGCTTCCCTCAGAGCGCCC
CCCACGGCGTGGTGTTCCTGCACGTGACCTACGTGCCCGCCCAAGAGAAGAACTTCAC
CACCGCTCCCGCCATCTGCCACGACGGCAAGGCCCACTTCCCTAGAGAGGGCGTGTTCG
TGAGCAACGGCACCCACTGGTTCGTGACACAGAGAAACTTCTACGAGCCTCAGATCATC
ACCACCGACAACACCTTCGTGAGCGGCAACTGCGACGTGGTGATCGGCATCGTCAACA
ACACCGTGTACGACCCCCTGCAACCCGAGCTGGACAGCTTCAAGGAGGAGCTGGACAA
GTACTTTAAGAACCACACAAGCCCCGACGTGGACCTGGGGGACATCTCCGGCATCAAC
GCTAGCGTGGTGAACATTCAGAAGGAAATTGACAGACTGAATGAGGTGGCCAAGAACC
 
TGAACGAGAGCCTGATCGACCTGCAAGAGCTGGGCAAGTACGAGCAGTACATCAAGTG
GCCCTGGTACATCTGGCTGGGCTTCATCGCCGGCCTGATCGCCATCGTGATGGTGACCAT
CATGCTGTGCTGCATGACAAGCTGCTGCAGCTGTCTGAAGGGCTGCTGCTCCTGCGGCA
GCTGCTGCAAGTTCGACGAGGACGACAGCGAGCCCGTGCTGAAGGGCGTGAAGCTGC
ACTACACCTGA
**SEQ ID No.39: Amino acid sequence of S antigen of Ad-S-Omicron-BA.4/5 vaccine** 
MFVFLVLLPLVSSQCVNLITRTQSYTNSFTRGVYYPDKVFRSSVLHSTQDLFLPFFSNVT
WFHAISGTNGTKRFDNPVLPFNDGVYFASTEKSNIIRGWIFGTTLDSKTQSLLIVNNATNVVI
KVCEFQFCNDPFLDVYYHKNNKSWMESEFRVYSSANNCTFEYVSQPFLMDLEGKQGNFK
NLREFVFKNIDGYFKIYSKHTPINLGRDLPQGFSALEPLVDLPIGINITRFQTLLALHRSYLTP
GDSSSGWTAGAAAYYVGYLQPRTFLLKYNENGTITDAVDCALDPLSETKCTLKSFTVEKGI
YQTSNFRVQPTESIVRFPNITNLCPFDEVFNATRFASVYAWNRKRISNCVADYSVLYNFAPFF
AFKCYGVSPTKLNDLCFTNVYADSFVIRGNEVSQIAPGQTGNIADYNYKLPDDFTGCVIAW
NSNKLDSKVGGNYNYRYRLFRKSNLKPFERDISTEIYQAGNKPCNGVAGVNCYFPLQSYGF
RPTYGVGHQPYRVVVLSFELLHAPATVCGPKKSTNLVKNKCVNFNFNGLTGTGVLTESNKK
FLPFQQFGRDIADTTDAVRDPQTLEILDITPCSFGGVSVITPGTNTSNQVAVLYQGVNCTEVP
VAIHADQLTPTWRVYSTGSNVFQTRAGCLIGAEYVNNSYECDIPIGAGICASYQTQTKSHRR
ARSVASQSIIAYTMSLGAENSVAYSNNSIAIPTNFTISVTTEILPVSMTKTSVDCTMYICGDST
ECSNLLLQYGSFCTQLKRALTGIAVEQDKNTQEVFAQVKQIYKTPPIKYFGGFNFSQILPDPS
KPSKRSFIEDLLFNKVTLADAGFIKQYGDCLGDIAARDLICAQKFNGLTVLPPLLTDEMIAQ
YTSALLAGTITSGWTFGAGAALQIPFAMQMAYRFNGIGVTQNVLYENQKLIANQFNSAIGKI
QDSLSSTASALGKLQDVVNHNAQALNTLVKQLSSKFGAISSVLNDILSRLDPPEAEVQIDRLI
TGRLQSLQTYVTQQLIRAAEIRASANLAATKMSECVLGQSKRVDFCGKGYHLMSFPQSAPH
GVVFLHVTYVPAQEKNFTTAPAICHDGKAHFPREGVFVSNGTHWFVTQRNFYEPQIITTDN
TFVSGNCDVVIGIVNNTVYDPLQPELDSFKEELDKYFKNHTSPDVDLGDISGINASVVNIQK
EIDRLNEVAKNLNESLIDLQELGKYEQYIKWPWYIWLGFIAGLIAIVMVTIMLCCMTSCCSC
LKGCCSCGSCCKFDEDDSEPVLKGVKLHYT*
**SEQ ID No.40: Full-length amino acid sequence of wild S antigen:** Note: the above sequence consists of signal peptide - extracellular domain - transmembrane domain - intracellular domain.
**SEQ ID No.41: full-length nucleic acid sequence of wild S antigen (optimization of CHO cells):** 
ATGTTCGTGTTCCTGGTGCTGCTGCCCCTGGTGAGCAGCCAGTGCGTGAACCTGAC
CACCAGAACCCAGCTGCCCCCCGCCTACACCAACAGCTTCACCAGAGGCGTGTACTACC
CCGACAAGGTGTTCAGAAGCAGCGTGCTGCACAGCACCCAGGACCTGTTCCTGCCCTT
CTTCAGCAACGTGACCTGGTTCCACGCCATCCACGTGAGCGGCACCAACGGCACCAAG
AGATTCGACAACCCCGTGCTGCCCTTCAACGACGGCGTGTACTTCGCCAGCACCGAGAA
GAGCAACATCATCAGAGGCTGGATCTTCGGCACCACCCTGGACAGCAAGACCCAGAGC
 
CTGCTGATCGTGAACAACGCCACCAACGTGGTGATCAAGGTGTGCGAGTTCCAGTTCTG
CAACGACCCCTTCCTGGGCGTGTACTACCACAAGAACAACAAGAGCTGGATGGAGAGC
GAGTTCAGAGTGTACAGCAGCGCCAACAACTGCACCTTCGAGTACGTGAGCCAGCCCT
TCCTGATGGACCTGGAGGGCAAGCAGGGCAACTTCAAGAACCTGAGAGAGTTCGTGTT
CAAGAACATCGACGGCTACTTCAAGATCTACAGCAAGCACACCCCCATCAACCTGGTGA
GAGACCTGCCCCAGGGCTTCAGCGCCCTGGAGCCCCTGGTGGACCTGCCCATCGGCATC
AACATCACCAGATTCCAGACCCTGCTGGCCCTGCACAGAAGCTACCTGACCCCCGGCGA
CAGCAGCAGCGGCTGGACCGCCGGCGCCGCCGCCTACTACGTGGGCTACCTGCAGCCC
AGAACCTTCCTGCTGAAGTACAACGAGAACGGCACCATCACCGACGCCGTGGACTGCG
CCCTGGACCCCCTGAGCGAGACCAAGTGCACCCTGAAGAGCTTCACCGTGGAGAAGGG
CATCTACCAGACCAGCAACTTCAGAGTGCAGCCCACCGAGAGCATCGTGAGATTCCCCA
ACATCACCAACCTGTGCCCCTTCGGCGAGGTGTTCAACGCCACCAGATTCGCCAGCGTG
TACGCCTGGAACAGAAAGAGAATCAGCAACTGCGTGGCCGACTACAGCGTGCTGTACA
ACAGCGCCAGCTTCAGCACCTTCAAGTGCTACGGCGTGAGCCCCACCAAGCTGAACGA
CCTGTGCTTCACCAACGTGTACGCCGACAGCTTCGTGATCAGAGGCGACGAGGTGAGA
CAGATCGCCCCCGGCCAGACCGGCAAGATCGCCGACTACAACTACAAGCTGCCCGACG
ACTTCACCGGCTGCGTGATCGCCTGGAACAGCAACAACCTGGACAGCAAGGTGGGCGG
CAACTACAACTACCTGTACAGACTGTTCAGAAAGAGCAACCTGAAGCCCTTCGAGAGA
GACATCAGCACCGAGATCTACCAGGCCGGCAGCACCCCCTGCAACGGCGTGGAGGGCT
TCAACTGCTACTTCCCCCTGCAGAGCTACGGCTTCCAGCCCACCAACGGCGTGGGCTAC
CAGCCCTACAGAGTGGTGGTGCTGAGCTTCGAGCTGCTGCACGCCCCCGCCACCGTGTG
CGGCCCCAAGAAGAGCACCAACCTGGTGAAGAACAAGTGCGTGAACTTCAACTTCAAC
GGCCTGACCGGCACCGGCGTGCTGACCGAGAGCAACAAGAAGTTCCTGCCCTTCCAGC
AGTTCGGCAGAGACATCGCCGACACCACCGACGCCGTGAGAGACCCCCAGACCCTGGA
GATCCTGGACATCACCCCCTGCAGCTTCGGCGGCGTGAGCGTGATCACCCCCGGCACCA
ACACCAGCAACCAGGTGGCCGTGCTGTACCAGGACGTGAACTGCACCGAGGTGCCCGT
GGCCATCCACGCCGACCAGCTGACCCCCACCTGGAGAGTGTACAGCACCGGCAGCAAC
GTGTTCCAGACCAGAGCCGGCTGCCTGATCGGCGCCGAGCACGTGAACAACAGCTACG
AGTGCGACATCCCCATCGGCGCCGGCATCTGCGCCAGCTACCAGACCCAGACCAACAGC
CCCAGAAGAGCCAGAAGCGTGGCCAGCCAGAGCATCATCGCCTACACCATGAGCCTGG
GCGCCGAGAACAGCGTGGCCTACAGCAACAACAGCATCGCCATCCCCACCAACTTCAC
 
CATCAGCGTGACCACCGAGATCCTGCCCGTGAGCATGACCAAGACCAGCGTGGACTGC
ACCATGTACATCTGCGGCGACAGCACCGAGTGCAGCAACCTGCTGCTGCAGTACGGCA
GCTTCTGCACCCAGCTGAACAGAGCCCTGACCGGCATCGCCGTGGAGCAGGACAAGAA
CACCCAGGAGGTGTTCGCCCAGGTGAAGCAGATCTACAAGACCCCCCCCATCAAGGAC
TTCGGCGGCTTCAACTTCAGCCAGATCCTGCCCGACCCCAGCAAGCCCAGCAAGAGAA
GCTTCATCGAGGACCTGCTGTTCAACAAGGTGACCCTGGCCGACGCCGGCTTCATCAAG
CAGTACGGCGACTGCCTGGGCGACATCGCCGCCAGAGACCTGATCTGCGCCCAGAAGT
TCAACGGCCTGACCGTGCTGCCCCCCCTGCTGACCGACGAGATGATCGCCCAGTACACC
AGCGCCCTGCTGGCCGGCACCATCACCAGCGGCTGGACCTTCGGCGCCGGCGCCGCCC
TGCAGATCCCCTTCGCCATGCAGATGGCCTACAGATTCAACGGCATCGGCGTGACCCAG
AACGTGCTGTACGAGAACCAGAAGCTGATCGCCAACCAGTTCAACAGCGCCATCGGCA
AGATCCAGGACAGCCTGAGCAGCACCGCCAGCGCCCTGGGCAAGCTGCAGGACGTGGT
GAACCAGAACGCCCAGGCCCTGAACACCCTGGTGAAGCAGCTGAGCAGCAACTTCGGC
GCCATCAGCAGCGTGCTGAACGACATCCTGAGCAGACTGGACAAGGTGGAGGCCGAGG
TGCAGATCGACAGACTGATCACCGGCAGACTGCAGAGCCTGCAGACCTACGTGACCCA
GCAGCTGATCAGAGCCGCCGAGATCAGAGCCAGCGCCAACCTGGCCGCCACCAAGATG
AGCGAGTGCGTGCTGGGCCAGAGCAAGAGAGTGGACTTCTGCGGCAAGGGCTACCACC
TGATGAGCTTCCCCCAGAGCGCCCCCCACGGCGTGGTGTTCCTGCACGTGACCTACGTG
CCCGCCCAGGAGAAGAACTTCACCACCGCCCCCGCCATCTGCCACGACGGCAAGGCCC
ACTTCCCCAGAGAGGGCGTGTTCGTGAGCAACGGCACCCACTGGTTCGTGACCCAGAG
AAACTTCTACGAGCCCCAGATCATCACCACCGACAACACCTTCGTGAGCGGCAACTGCG
ACGTGGTGATCGGCATCGTGAACAACACCGTGTACGACCCCCTGCAGCCCGAGCTGGA
CAGCTTCAAGGAGGAGCTGGACAAGTACTTCAAGAACCACACCAGCCCCGACGTGGAC
CTGGGCGACATCAGCGGCATCAACGCCAGCGTGGTGAACATCCAGAAGGAGATCGACA
GACTGAACGAGGTGGCCAAGAACCTGAACGAGAGCCTGATCGACCTGCAGGAGCTGG
GCAAGTACGAGCAGTACATCAAGTGGCCCTGGTACATCTGGCTGGGCTTCATCGCCGGC
CTGATCGCCATCGTGATGGTGACCATCATGCTGTGCTGCATGACCAGCTGCTGCAGCTGC
CTGAAGGGCTGCTGCAGCTGCGGCAGCTGCTGCAAGTTCGACGAGGACGACAGCGAGC
CCGTGCTGAAGGGCGTGAAGCTGCACTACACCTAG
 Note: the above sequence consists of signal peptide - extracellular domain - transmembrane domain - intracellular domain.
**SEQ ID No.42: Full-length nucleic acid sequence of wild S antigen (optimization of insect cells):** 
ATGTTCGTGTTCCTGGTGCTGCTGCCCCTGGTGTCCTCCCAGTGCGTGAACCTGACC
ACCCGTACCCAGCTGCCCCCCGCTTACACCAACTCCTTCACCCGTGGTGTGTACTACCCC
GACAAGGTGTTCCGTTCCTCCGTGCTGCACTCCACCCAGGACCTGTTCCTGCCCTTCTTC
TCCAACGTGACCTGGTTCCACGCTATCCACGTGTCCGGTACCAACGGTACCAAGCGTTT
CGACAACCCCGTGCTGCCCTTCAACGACGGTGTGTACTTCGCTTCCACCGAGAAGTCCA
ACATCATCCGTGGTTGGATCTTCGGTACCACCCTGGACTCCAAGACCCAGTCCCTGCTGA
TCGTGAACAACGCTACCAACGTGGTGATCAAGGTGTGCGAGTTCCAGTTCTGCAACGAC
CCCTTCCTGGGTGTGTACTACCACAAGAACAACAAGTCCTGGATGGAGTCCGAGTTCCG
TGTGTACTCCTCCGCTAACAACTGCACCTTCGAGTACGTGTCCCAGCCCTTCCTGATGGA
CCTGGAGGGTAAGCAGGGTAACTTCAAGAACCTGCGTGAGTTCGTGTTCAAGAACATC
GACGGTTACTTCAAGATCTACTCCAAGCACACCCCCATCAACCTGGTGCGTGACCTGCC
CCAGGGTTTCTCCGCTCTGGAGCCCCTGGTGGACCTGCCCATCGGTATCAACATCACCC
GTTTCCAGACCCTGCTGGCTCTGCACCGTTCCTACCTGACCCCCGGTGACTCCTCCTCCG
GTTGGACCGCTGGTGCTGCTGCTTACTACGTGGGTTACCTGCAGCCCCGTACCTTCCTGC
TGAAGTACAACGAGAACGGTACCATCACCGACGCTGTGGACTGCGCTCTGGACCCCCT
GTCCGAGACCAAGTGCACCCTGAAGTCCTTCACCGTGGAGAAGGGTATCTACCAGACCT
CCAACTTCCGTGTGCAGCCCACCGAGTCCATCGTGCGTTTCCCCAACATCACCAACCTG
TGCCCCTTCGGTGAGGTGTTCAACGCTACCCGTTTCGCTTCCGTGTACGCTTGGAACCGT
AAGCGTATCTCCAACTGCGTGGCTGACTACTCCGTGCTGTACAACTCCGCTTCCTTCTCC
ACCTTCAAGTGCTACGGTGTGTCCCCCACCAAGCTGAACGACCTGTGCTTCACCAACGT
GTACGCTGACTCCTTCGTGATCCGTGGTGACGAGGTGCGTCAGATCGCTCCCGGTCAGA
CCGGTAAGATCGCTGACTACAACTACAAGCTGCCCGACGACTTCACCGGTTGCGTGATC
GCTTGGAACTCCAACAACCTGGACTCCAAGGTGGGTGGTAACTACAACTACCTGTACCG
TCTGTTCCGTAAGTCCAACCTGAAGCCCTTCGAGCGTGACATCTCCACCGAGATCTACC
AGGCTGGTTCCACCCCCTGCAACGGTGTGGAGGGTTTCAACTGCTACTTCCCCCTGCAG
TCCTACGGTTTCCAGCCCACCAACGGTGTGGGTTACCAGCCCTACCGTGTGGTGGTGCT
GTCCTTCGAGCTGCTGCACGCTCCCGCTACCGTGTGCGGTCCCAAGAAGTCCACCAACC
TGGTGAAGAACAAGTGCGTGAACTTCAACTTCAACGGTCTGACCGGTACCGGTGTGCT
 
GACCGAGTCCAACAAGAAGTTCCTGCCCTTCCAGCAGTTCGGTCGTGACATCGCTGACA
CCACCGACGCTGTGCGTGACCCCCAGACCCTGGAGATCCTGGACATCACCCCCTGCTCC
TTCGGTGGTGTGTCCGTGATCACCCCCGGTACCAACACCTCCAACCAGGTGGCTGTGCT
GTACCAGGACGTGAACTGCACCGAGGTGCCCGTGGCTATCCACGCTGACCAGCTGACC
CCCACCTGGCGTGTGTACTCCACCGGTTCCAACGTGTTCCAGACCCGTGCTGGTTGCCT
GATCGGTGCTGAGCACGTGAACAACTCCTACGAGTGCGACATCCCCATCGGTGCTGGTA
TCTGCGCTTCCTACCAGACCCAGACCAACTCCCCCCGTCGTGCTCGTTCCGTGGCTTCCC
AGTCCATCATCGCTTACACCATGTCCCTGGGTGCTGAGAACTCCGTGGCTTACTCCAACA
ACTCCATCGCTATCCCCACCAACTTCACCATCTCCGTGACCACCGAGATCCTGCCCGTGT
CCATGACCAAGACCTCCGTGGACTGCACCATGTACATCTGCGGTGACTCCACCGAGTGC
TCCAACCTGCTGCTGCAGTACGGTTCCTTCTGCACCCAGCTGAACCGTGCTCTGACCGG
TATCGCTGTGGAGCAGGACAAGAACACCCAGGAGGTGTTCGCTCAGGTGAAGCAGATC
TACAAGACCCCCCCCATCAAGGACTTCGGTGGTTTCAACTTCTCCCAGATCCTGCCCGA
CCCCTCCAAGCCCTCCAAGCGTTCCTTCATCGAGGACCTGCTGTTCAACAAGGTGACCC
TGGCTGACGCTGGTTTCATCAAGCAGTACGGTGACTGCCTGGGTGACATCGCTGCTCGT
GACCTGATCTGCGCTCAGAAGTTCAACGGTCTGACCGTGCTGCCCCCCCTGCTGACCGA
CGAGATGATCGCTCAGTACACCTCCGCTCTGCTGGCTGGTACCATCACCTCCGGTTGGAC
CTTCGGTGCTGGTGCTGCTCTGCAGATCCCCTTCGCTATGCAGATGGCTTACCGTTTCAA
CGGTATCGGTGTGACCCAGAACGTGCTGTACGAGAACCAGAAGCTGATCGCTAACCAGT
TCAACTCCGCTATCGGTAAGATCCAGGACTCCCTGTCCTCCACCGCTTCCGCTCTGGGTA
AGCTGCAGGACGTGGTGAACCAGAACGCTCAGGCTCTGAACACCCTGGTGAAGCAGCT
GTCCTCCAACTTCGGTGCTATCTCCTCCGTGCTGAACGACATCCTGTCCCGTCTGGACAA
GGTGGAGGCTGAGGTGCAGATCGACCGTCTGATCACCGGTCGTCTGCAGTCCCTGCAG
ACCTACGTGACCCAGCAGCTGATCCGTGCTGCTGAGATCCGTGCTTCCGCTAACCTGGC
TGCTACCAAGATGTCCGAGTGCGTGCTGGGTCAGTCCAAGCGTGTGGACTTCTGCGGTA
AGGGTTACCACCTGATGTCCTTCCCCCAGTCCGCTCCCCACGGTGTGGTGTTCCTGCAC
GTGACCTACGTGCCCGCTCAGGAGAAGAACTTCACCACCGCTCCCGCTATCTGCCACGA
CGGTAAGGCTCACTTCCCCCGTGAGGGTGTGTTCGTGTCCAACGGTACCCACTGGTTCG
TGACCCAGCGTAACTTCTACGAGCCCCAGATCATCACCACCGACAACACCTTCGTGTCC
GGTAACTGCGACGTGGTGATCGGTATCGTGAACAACACCGTGTACGACCCCCTGCAGCC
CGAGCTGGACTCCTTCAAGGAGGAGCTGGACAAGTACTTCAAGAACCACACCTCCCCC
 
GACGTGGACCTGGGTGACATCTCCGGTATCAACGCTTCCGTGGTGAACATCCAGAAGGA
GATCGACCGTCTGAACGAGGTGGCTAAGAACCTGAACGAGTCCCTGATCGACCTGCAG
GAGCTGGGTAAGTACGAGCAGTACATCAAGTGGCCCTGGTACATCTGGCTGGGTTTCAT
CGCTGGTCTGATCGCTATCGTGATGGTGACCATCATGCTGTGCTGCATGACCTCCTGCTG
CTCCTGCCTGAAGGGTTGCTGCTCCTGCGGTTCCTGCTGCAAGTTCGACGAGGACGACT
CCGAGCCCGTGCTGAAGGGTGTGAAGCTGCACTACACCTAG
 Note: the above sequence consists of signal peptide - extracellular domain - transmembrane domain - intracellular domain.
**SEQ ID No.43: Extracellular amino acid sequence of wild S antigen:** 
MFVFLVLLPLVSSQCVNLTTRTQLPPAYTNSFTRGVYYPDKVFRSSVLHSTQDLFLPFFS
NVTWFHAIHVSGTNGTKRFDNPVLPFNDGVYFASTEKSNIIRGWIFGTTLDSKTQSLLIVNN
ATNVVIKVCEFQFCNDPFLGVYYHKNNKSWMESEFRVYSSANNCTFEYVSQPFLMDLEGK
QGNFKNLREFVFKNIDGYFKIYSKHTPINLVRDLPQGFSALEPLVDLPIGINITRFQTLLALHR
SYLTPGDSSSGWTAGAAAYYVGYLQPRTFLLKYNENGTITDAVDCALDPLSETKCTLKSFT
VEKGIYQTSNFRVQPTESIVRFPNITNLCPFGEVFNATRFASVYAWNRKRISNCVADYSVLYN
SASFSTFKCYGVSPTKLNDLCFTNVYADSFVIRGDEVRQIAPGQTGKIADYNYKLPDDFTGC
VIAWNSNNLDSKVGGNYNYLYRLFRKSNLKPFERDISTEIYQAGSTPCNGVEGFNCYFPLQS
YGFQPTNGVGYQPYRVVVLSFELLHAPATVCGPKKSTNLVKNKCVNFNFNGLTGTGVLTES
NKKFLPFQQFGRDIADTTDAVRDPQTLEILDITPCSFGGVSVITPGTNTSNQVAVLYQDVNCT
EVPVAIHADQLTPTWRVYSTGSNVFQTRAGCLIGAEHVNNSYECDIPIGAGICASYQTQTNS
PRRARSVASQSIIAYTMSLGAENSVAYSNNSIAIPTNFTISVTTEILPVSMTKTSVDCTMYICG
DSTECSNLLLQYGSFCTQLNRALTGIAVEQDKNTQEVFAQVKQIYKTPPIKDFGGFNFSQILP
DPSKPSKRSFIEDLLFNKVTLADAGFIKQYGDCLGDIAARDLICAQKFNGLTVLPPLLTDEMI
AQYTSALLAGTITSGWTFGAGAALQIPFAMQMAYRFNGIGVTQNVLYENQKLIANQFNSAI
GKIQDSLSSTASALGKLQDVVNQNAQALNTLVKQLSSNFGAISSVLNDILSRLDKVEAEVQI
DRLITGRLQSLQTYVTQQLIRAAEIRASANLAATKMSECVLGQSKRVDFCGKGYHLMSFPQ
SAPHGVVFLHVTYVPAQEKNFTTAPAICHDGKAHFPREGVFVSNGTHWFVTQRNFYEPQIIT
TDNTFVSGNCDVVIGIVNNTVYDPLQPELDSFKEELDKYFKNHTSPDVDLGDISGINASVVN
IQKEIDRLNEVAKNLNESLIDLQELGKYEQYIKWP
 Note: the above sequence consists of signal peptide - extracellular domain.
**SEQ ID No.44: Extracellular nucleic acid sequence of wild S antigen (optimization of CHO cells):** 
ATGTTCGTGTTCCTGGTGCTGCTGCCCCTGGTGAGCAGCCAGTGCGTGAACCTGAC
CACCAGAACCCAGCTGCCCCCCGCCTACACCAACAGCTTCACCAGAGGCGTGTACTACC
CCGACAAGGTGTTCAGAAGCAGCGTGCTGCACAGCACCCAGGACCTGTTCCTGCCCTT
CTTCAGCAACGTGACCTGGTTCCACGCCATCCACGTGAGCGGCACCAACGGCACCAAG
AGATTCGACAACCCCGTGCTGCCCTTCAACGACGGCGTGTACTTCGCCAGCACCGAGAA
GAGCAACATCATCAGAGGCTGGATCTTCGGCACCACCCTGGACAGCAAGACCCAGAGC
CTGCTGATCGTGAACAACGCCACCAACGTGGTGATCAAGGTGTGCGAGTTCCAGTTCTG
CAACGACCCCTTCCTGGGCGTGTACTACCACAAGAACAACAAGAGCTGGATGGAGAGC
GAGTTCAGAGTGTACAGCAGCGCCAACAACTGCACCTTCGAGTACGTGAGCCAGCCCT
TCCTGATGGACCTGGAGGGCAAGCAGGGCAACTTCAAGAACCTGAGAGAGTTCGTGTT
CAAGAACATCGACGGCTACTTCAAGATCTACAGCAAGCACACCCCCATCAACCTGGTGA
GAGACCTGCCCCAGGGCTTCAGCGCCCTGGAGCCCCTGGTGGACCTGCCCATCGGCATC
AACATCACCAGATTCCAGACCCTGCTGGCCCTGCACAGAAGCTACCTGACCCCCGGCGA
CAGCAGCAGCGGCTGGACCGCCGGCGCCGCCGCCTACTACGTGGGCTACCTGCAGCCC
AGAACCTTCCTGCTGAAGTACAACGAGAACGGCACCATCACCGACGCCGTGGACTGCG
CCCTGGACCCCCTGAGCGAGACCAAGTGCACCCTGAAGAGCTTCACCGTGGAGAAGGG
CATCTACCAGACCAGCAACTTCAGAGTGCAGCCCACCGAGAGCATCGTGAGATTCCCCA
ACATCACCAACCTGTGCCCCTTCGGCGAGGTGTTCAACGCCACCAGATTCGCCAGCGTG
TACGCCTGGAACAGAAAGAGAATCAGCAACTGCGTGGCCGACTACAGCGTGCTGTACA
ACAGCGCCAGCTTCAGCACCTTCAAGTGCTACGGCGTGAGCCCCACCAAGCTGAACGA
CCTGTGCTTCACCAACGTGTACGCCGACAGCTTCGTGATCAGAGGCGACGAGGTGAGA
CAGATCGCCCCCGGCCAGACCGGCAAGATCGCCGACTACAACTACAAGCTGCCCGACG
ACTTCACCGGCTGCGTGATCGCCTGGAACAGCAACAACCTGGACAGCAAGGTGGGCGG
CAACTACAACTACCTGTACAGACTGTTCAGAAAGAGCAACCTGAAGCCCTTCGAGAGA
GACATCAGCACCGAGATCTACCAGGCCGGCAGCACCCCCTGCAACGGCGTGGAGGGCT
TCAACTGCTACTTCCCCCTGCAGAGCTACGGCTTCCAGCCCACCAACGGCGTGGGCTAC
CAGCCCTACAGAGTGGTGGTGCTGAGCTTCGAGCTGCTGCACGCCCCCGCCACCGTGTG
CGGCCCCAAGAAGAGCACCAACCTGGTGAAGAACAAGTGCGTGAACTTCAACTTCAAC
 
GGCCTGACCGGCACCGGCGTGCTGACCGAGAGCAACAAGAAGTTCCTGCCCTTCCAGC
AGTTCGGCAGAGACATCGCCGACACCACCGACGCCGTGAGAGACCCCCAGACCCTGGA
GATCCTGGACATCACCCCCTGCAGCTTCGGCGGCGTGAGCGTGATCACCCCCGGCACCA
ACACCAGCAACCAGGTGGCCGTGCTGTACCAGGACGTGAACTGCACCGAGGTGCCCGT
GGCCATCCACGCCGACCAGCTGACCCCCACCTGGAGAGTGTACAGCACCGGCAGCAAC
GTGTTCCAGACCAGAGCCGGCTGCCTGATCGGCGCCGAGCACGTGAACAACAGCTACG
AGTGCGACATCCCCATCGGCGCCGGCATCTGCGCCAGCTACCAGACCCAGACCAACAGC
CCCAGAAGAGCCAGAAGCGTGGCCAGCCAGAGCATCATCGCCTACACCATGAGCCTGG
GCGCCGAGAACAGCGTGGCCTACAGCAACAACAGCATCGCCATCCCCACCAACTTCAC
CATCAGCGTGACCACCGAGATCCTGCCCGTGAGCATGACCAAGACCAGCGTGGACTGC
ACCATGTACATCTGCGGCGACAGCACCGAGTGCAGCAACCTGCTGCTGCAGTACGGCA
GCTTCTGCACCCAGCTGAACAGAGCCCTGACCGGCATCGCCGTGGAGCAGGACAAGAA
CACCCAGGAGGTGTTCGCCCAGGTGAAGCAGATCTACAAGACCCCCCCCATCAAGGAC
TTCGGCGGCTTCAACTTCAGCCAGATCCTGCCCGACCCCAGCAAGCCCAGCAAGAGAA
GCTTCATCGAGGACCTGCTGTTCAACAAGGTGACCCTGGCCGACGCCGGCTTCATCAAG
CAGTACGGCGACTGCCTGGGCGACATCGCCGCCAGAGACCTGATCTGCGCCCAGAAGT
TCAACGGCCTGACCGTGCTGCCCCCCCTGCTGACCGACGAGATGATCGCCCAGTACACC
AGCGCCCTGCTGGCCGGCACCATCACCAGCGGCTGGACCTTCGGCGCCGGCGCCGCCC
TGCAGATCCCCTTCGCCATGCAGATGGCCTACAGATTCAACGGCATCGGCGTGACCCAG
AACGTGCTGTACGAGAACCAGAAGCTGATCGCCAACCAGTTCAACAGCGCCATCGGCA
AGATCCAGGACAGCCTGAGCAGCACCGCCAGCGCCCTGGGCAAGCTGCAGGACGTGGT
GAACCAGAACGCCCAGGCCCTGAACACCCTGGTGAAGCAGCTGAGCAGCAACTTCGGC
GCCATCAGCAGCGTGCTGAACGACATCCTGAGCAGACTGGACAAGGTGGAGGCCGAGG
TGCAGATCGACAGACTGATCACCGGCAGACTGCAGAGCCTGCAGACCTACGTGACCCA
GCAGCTGATCAGAGCCGCCGAGATCAGAGCCAGCGCCAACCTGGCCGCCACCAAGATG
AGCGAGTGCGTGCTGGGCCAGAGCAAGAGAGTGGACTTCTGCGGCAAGGGCTACCACC
TGATGAGCTTCCCCCAGAGCGCCCCCCACGGCGTGGTGTTCCTGCACGTGACCTACGTG
CCCGCCCAGGAGAAGAACTTCACCACCGCCCCCGCCATCTGCCACGACGGCAAGGCCC
ACTTCCCCAGAGAGGGCGTGTTCGTGAGCAACGGCACCCACTGGTTCGTGACCCAGAG
AAACTTCTACGAGCCCCAGATCATCACCACCGACAACACCTTCGTGAGCGGCAACTGCG
ACGTGGTGATCGGCATCGTGAACAACACCGTGTACGACCCCCTGCAGCCCGAGCTGGA
 
CAGCTTCAAGGAGGAGCTGGACAAGTACTTCAAGAACCACACCAGCCCCGACGTGGAC
CTGGGCGACATCAGCGGCATCAACGCCAGCGTGGTGAACATCCAGAAGGAGATCGACA
GACTGAACGAGGTGGCCAAGAACCTGAACGAGAGCCTGATCGACCTGCAGGAGCTGG
GCAAGTACGAGCAGTACATCAAGTGGCCCTAG
 Note: the above sequence consists of signal peptide - extracellular domain.
**SEQ ID No.45: Extracellular nucleic acid sequence of wild S antigen (optimization of insect cells):** 
ATGTTCGTGTTCCTGGTGCTGCTGCCCCTGGTGTCCTCCCAGTGCGTGAACCTGACC
ACCCGTACCCAGCTGCCCCCCGCTTACACCAACTCCTTCACCCGTGGTGTGTACTACCCC
GACAAGGTGTTCCGTTCCTCCGTGCTGCACTCCACCCAGGACCTGTTCCTGCCCTTCTTC
TCCAACGTGACCTGGTTCCACGCTATCCACGTGTCCGGTACCAACGGTACCAAGCGTTT
CGACAACCCCGTGCTGCCCTTCAACGACGGTGTGTACTTCGCTTCCACCGAGAAGTCCA
ACATCATCCGTGGTTGGATCTTCGGTACCACCCTGGACTCCAAGACCCAGTCCCTGCTGA
TCGTGAACAACGCTACCAACGTGGTGATCAAGGTGTGCGAGTTCCAGTTCTGCAACGAC
CCCTTCCTGGGTGTGTACTACCACAAGAACAACAAGTCCTGGATGGAGTCCGAGTTCCG
TGTGTACTCCTCCGCTAACAACTGCACCTTCGAGTACGTGTCCCAGCCCTTCCTGATGGA
CCTGGAGGGTAAGCAGGGTAACTTCAAGAACCTGCGTGAGTTCGTGTTCAAGAACATC
GACGGTTACTTCAAGATCTACTCCAAGCACACCCCCATCAACCTGGTGCGTGACCTGCC
CCAGGGTTTCTCCGCTCTGGAGCCCCTGGTGGACCTGCCCATCGGTATCAACATCACCC
GTTTCCAGACCCTGCTGGCTCTGCACCGTTCCTACCTGACCCCCGGTGACTCCTCCTCCG
GTTGGACCGCTGGTGCTGCTGCTTACTACGTGGGTTACCTGCAGCCCCGTACCTTCCTGC
TGAAGTACAACGAGAACGGTACCATCACCGACGCTGTGGACTGCGCTCTGGACCCCCT
GTCCGAGACCAAGTGCACCCTGAAGTCCTTCACCGTGGAGAAGGGTATCTACCAGACCT
CCAACTTCCGTGTGCAGCCCACCGAGTCCATCGTGCGTTTCCCCAACATCACCAACCTG
TGCCCCTTCGGTGAGGTGTTCAACGCTACCCGTTTCGCTTCCGTGTACGCTTGGAACCGT
AAGCGTATCTCCAACTGCGTGGCTGACTACTCCGTGCTGTACAACTCCGCTTCCTTCTCC
ACCTTCAAGTGCTACGGTGTGTCCCCCACCAAGCTGAACGACCTGTGCTTCACCAACGT
GTACGCTGACTCCTTCGTGATCCGTGGTGACGAGGTGCGTCAGATCGCTCCCGGTCAGA
CCGGTAAGATCGCTGACTACAACTACAAGCTGCCCGACGACTTCACCGGTTGCGTGATC
GCTTGGAACTCCAACAACCTGGACTCCAAGGTGGGTGGTAACTACAACTACCTGTACCG
 
TCTGTTCCGTAAGTCCAACCTGAAGCCCTTCGAGCGTGACATCTCCACCGAGATCTACC
AGGCTGGTTCCACCCCCTGCAACGGTGTGGAGGGTTTCAACTGCTACTTCCCCCTGCAG
TCCTACGGTTTCCAGCCCACCAACGGTGTGGGTTACCAGCCCTACCGTGTGGTGGTGCT
GTCCTTCGAGCTGCTGCACGCTCCCGCTACCGTGTGCGGTCCCAAGAAGTCCACCAACC
TGGTGAAGAACAAGTGCGTGAACTTCAACTTCAACGGTCTGACCGGTACCGGTGTGCT
GACCGAGTCCAACAAGAAGTTCCTGCCCTTCCAGCAGTTCGGTCGTGACATCGCTGACA
CCACCGACGCTGTGCGTGACCCCCAGACCCTGGAGATCCTGGACATCACCCCCTGCTCC
TTCGGTGGTGTGTCCGTGATCACCCCCGGTACCAACACCTCCAACCAGGTGGCTGTGCT
GTACCAGGACGTGAACTGCACCGAGGTGCCCGTGGCTATCCACGCTGACCAGCTGACC
CCCACCTGGCGTGTGTACTCCACCGGTTCCAACGTGTTCCAGACCCGTGCTGGTTGCCT
GATCGGTGCTGAGCACGTGAACAACTCCTACGAGTGCGACATCCCCATCGGTGCTGGTA
TCTGCGCTTCCTACCAGACCCAGACCAACTCCCCCCGTCGTGCTCGTTCCGTGGCTTCCC
AGTCCATCATCGCTTACACCATGTCCCTGGGTGCTGAGAACTCCGTGGCTTACTCCAACA
ACTCCATCGCTATCCCCACCAACTTCACCATCTCCGTGACCACCGAGATCCTGCCCGTGT
CCATGACCAAGACCTCCGTGGACTGCACCATGTACATCTGCGGTGACTCCACCGAGTGC
TCCAACCTGCTGCTGCAGTACGGTTCCTTCTGCACCCAGCTGAACCGTGCTCTGACCGG
TATCGCTGTGGAGCAGGACAAGAACACCCAGGAGGTGTTCGCTCAGGTGAAGCAGATC
TACAAGACCCCCCCCATCAAGGACTTCGGTGGTTTCAACTTCTCCCAGATCCTGCCCGA
CCCCTCCAAGCCCTCCAAGCGTTCCTTCATCGAGGACCTGCTGTTCAACAAGGTGACCC
TGGCTGACGCTGGTTTCATCAAGCAGTACGGTGACTGCCTGGGTGACATCGCTGCTCGT
GACCTGATCTGCGCTCAGAAGTTCAACGGTCTGACCGTGCTGCCCCCCCTGCTGACCGA
CGAGATGATCGCTCAGTACACCTCCGCTCTGCTGGCTGGTACCATCACCTCCGGTTGGAC
CTTCGGTGCTGGTGCTGCTCTGCAGATCCCCTTCGCTATGCAGATGGCTTACCGTTTCAA
CGGTATCGGTGTGACCCAGAACGTGCTGTACGAGAACCAGAAGCTGATCGCTAACCAGT
TCAACTCCGCTATCGGTAAGATCCAGGACTCCCTGTCCTCCACCGCTTCCGCTCTGGGTA
AGCTGCAGGACGTGGTGAACCAGAACGCTCAGGCTCTGAACACCCTGGTGAAGCAGCT
GTCCTCCAACTTCGGTGCTATCTCCTCCGTGCTGAACGACATCCTGTCCCGTCTGGACAA
GGTGGAGGCTGAGGTGCAGATCGACCGTCTGATCACCGGTCGTCTGCAGTCCCTGCAG
ACCTACGTGACCCAGCAGCTGATCCGTGCTGCTGAGATCCGTGCTTCCGCTAACCTGGC
TGCTACCAAGATGTCCGAGTGCGTGCTGGGTCAGTCCAAGCGTGTGGACTTCTGCGGTA
AGGGTTACCACCTGATGTCCTTCCCCCAGTCCGCTCCCCACGGTGTGGTGTTCCTGCAC
 
GTGACCTACGTGCCCGCTCAGGAGAAGAACTTCACCACCGCTCCCGCTATCTGCCACGA
CGGTAAGGCTCACTTCCCCCGTGAGGGTGTGTTCGTGTCCAACGGTACCCACTGGTTCG
TGACCCAGCGTAACTTCTACGAGCCCCAGATCATCACCACCGACAACACCTTCGTGTCC
GGTAACTGCGACGTGGTGATCGGTATCGTGAACAACACCGTGTACGACCCCCTGCAGCC
CGAGCTGGACTCCTTCAAGGAGGAGCTGGACAAGTACTTCAAGAACCACACCTCCCCC
GACGTGGACCTGGGTGACATCTCCGGTATCAACGCTTCCGTGGTGAACATCCAGAAGGA
GATCGACCGTCTGAACGAGGTGGCTAAGAACCTGAACGAGTCCCTGATCGACCTGCAG
GAGCTGGGTAAGTACGAGCAGTACATCAAGTGGCCCTAG
 Note: the above sequence consists of signal peptide - extracellular domain.
**SEQ ID No.46: Full-length amino acid sequence of Beta S antigen:** 
MFVFLVLLPLVSSQCVNLTTRTQLPPAYTNSFTRGVYYPDKVFRSSVLHSTQDLFLPFFS
NVTWFHAIHVSGTNGTKRFANPVLPFNDGVYFASTEKSNIIRGWIFGTTLDSKTQSLLIVNN
ATNVVIKVCEFQFCNDPFLGVYYHKNNKSWMESEFRVYSSANNCTFEYVSQPFLMDLEGK
QGNFKNLREFVFKNIDGYFKIYSKHTPINLVRGLPQGFSALEPLVDLPIGINITRFQTLHRSYL
TPGDSSSGWTAGAAAYYVGYLQPRTFLLKYNENGTITDAVDCALDPLSETKCTLKSFTVEK
GIYQTSNFRVQPTESIVRFPNITNLCPFGEVFNATRFASVYAWNRKRISNCVADYSVLYNSAS
FSTFKCYGVSPTKLNDLCFTNVYADSFVIRGDEVRQIAPGQTGNIADYNYKLPDDFTGCVIA
WNSNNLDSKVGGNYNYLYRLFRKSNLKPFERDISTEIYQAGSTPCNGVKGFNCYFPLQSYG
FQPTYGVGYQPYRVVVLSFELLHAPATVCGPKKSTNLVKNKCVNFNFNGLTGTGVLTESNK
KFLPFQQFGRDIADTTDAVRDPQTLEILDITPCSFGGVSVITPGTNTSNQVAVLYQGVNCTEV
PVAIHADQLTPTWRVYSTGSNVFQTRAGCLIGAEHVNNSYECDIPIGAGICASYQTQTNSPR
RARSVASQSIIAYTMSLGVENSVAYSNNSIAIPTNFTISVTTEILPVSMTKTSVDCTMYICGDS
TECSNLLLQYGSFCTQLNRALTGIAVEQDKNTQEVFAQVKQIYKTPPIKDFGGFNFSQILPDP
SKPSKRSFIEDLLFNKVTLADAGFIKQYGDCLGDIAARDLICAQKFNGLTVLPPLLTDEMIAQ
YTSALLAGTITSGWTFGAGAALQIPFAMQMAYRFNGIGVTQNVLYENQKLIANQFNSAIGKI
QDSLSSTASALGKLQDVVNQNAQALNTLVKQLSSNFGAISSVLNDILSRLDKVEAEVQIDRL
ITGRLQSLQTYVTQQLIRAAEIRASANLAATKMSECVLGQSKRVDFCGKGYHLMSFPQSAP
HGVVFLHVTYVPAQEKNFTTAPAICHDGKAHFPREGVFVSNGTHWFVTQRNFYEPQIITTD
NTFVSGNCDVVIGIVNNTVYDPLQPELDSFKEELDKYFKNHTSPDVDLGDISGINASVVNIQ
KEIDRLNEVAKNLNESLIDLQELGKYEQYIKWPWYIWLGFIAGLIAIVMVTIMLCCMTSCCS
 CLKGCCSCGSCCKFDEDDSEPVLKGVKLHYT Note: the above sequence consists of signal peptide - extracellular domain - transmembrane domain - intracellular domain.
**SEQ ID No.47: Full-length nucleic acid sequence of Beta S antigen (optimization of CHO cells):** 
ATGTTCGTGTTCCTGGTGCTGCTGCCCCTGGTGAGCAGCCAGTGCGTGAACCTGAC
CACCAGAACCCAGCTGCCCCCCGCCTACACCAACAGCTTCACCAGAGGCGTGTACTACC
CCGACAAGGTGTTCAGAAGCAGCGTGCTGCACAGCACCCAGGACCTGTTCCTGCCCTT
CTTCAGCAACGTGACCTGGTTCCACGCCATCCACGTGAGCGGCACCAACGGCACCAAG
AGATTCGCCAACCCCGTGCTGCCCTTCAACGACGGCGTGTACTTCGCCAGCACCGAGAA
GAGCAACATCATCAGAGGCTGGATCTTCGGCACCACCCTGGACAGCAAGACCCAGAGC
CTGCTGATCGTGAACAACGCCACCAACGTGGTGATCAAGGTGTGCGAGTTCCAGTTCTG
CAACGACCCCTTCCTGGGCGTGTACTACCACAAGAACAACAAGAGCTGGATGGAGAGC
GAGTTCAGAGTGTACAGCAGCGCCAACAACTGCACCTTCGAGTACGTGAGCCAGCCCT
TCCTGATGGACCTGGAGGGCAAGCAGGGCAACTTCAAGAACCTGAGAGAGTTCGTGTT
CAAGAACATCGACGGCTACTTCAAGATCTACAGCAAGCACACCCCCATCAACCTGGTGA
GAGGCCTGCCCCAGGGCTTCAGCGCCCTGGAGCCCCTGGTGGACCTGCCCATCGGCATC
AACATCACCAGATTCCAGACCCTGCACAGAAGCTACCTGACCCCCGGCGACAGCAGCA
GCGGCTGGACCGCCGGCGCCGCCGCCTACTACGTGGGCTACCTGCAGCCCAGAACCTTC
CTGCTGAAGTACAACGAGAACGGCACCATCACCGACGCCGTGGACTGCGCCCTGGACC
CCCTGAGCGAGACCAAGTGCACCCTGAAGAGCTTCACCGTGGAGAAGGGCATCTACCA
GACCAGCAACTTCAGAGTGCAGCCCACCGAGAGCATCGTGAGATTCCCCAACATCACC
AACCTGTGCCCCTTCGGCGAGGTGTTCAACGCCACCAGATTCGCCAGCGTGTACGCCTG
GAACAGAAAGAGAATCAGCAACTGCGTGGCCGACTACAGCGTGCTGTACAACAGCGCC
AGCTTCAGCACCTTCAAGTGCTACGGCGTGAGCCCCACCAAGCTGAACGACCTGTGCTT
CACCAACGTGTACGCCGACAGCTTCGTGATCAGAGGCGACGAGGTGAGACAGATCGCC
CCCGGCCAGACCGGCAACATCGCCGACTACAACTACAAGCTGCCCGACGACTTCACCG
GCTGCGTGATCGCCTGGAACAGCAACAACCTGGACAGCAAGGTGGGCGGCAACTACAA
CTACCTGTACAGACTGTTCAGAAAGAGCAACCTGAAGCCCTTCGAGAGAGACATCAGC
ACCGAGATCTACCAGGCCGGCAGCACCCCCTGCAACGGCGTGAAGGGCTTCAACTGCT
 
ACTTCCCCCTGCAGAGCTACGGCTTCCAGCCCACCTACGGCGTGGGCTACCAGCCCTAC
AGAGTGGTGGTGCTGAGCTTCGAGCTGCTGCACGCCCCCGCCACCGTGTGCGGCCCCA
AGAAGAGCACCAACCTGGTGAAGAACAAGTGCGTGAACTTCAACTTCAACGGCCTGAC
CGGCACCGGCGTGCTGACCGAGAGCAACAAGAAGTTCCTGCCCTTCCAGCAGTTCGGC
AGAGACATCGCCGACACCACCGACGCCGTGAGAGACCCCCAGACCCTGGAGATCCTGG
ACATCACCCCCTGCAGCTTCGGCGGCGTGAGCGTGATCACCCCCGGCACCAACACCAG
CAACCAGGTGGCCGTGCTGTACCAGGGCGTGAACTGCACCGAGGTGCCCGTGGCCATC
CACGCCGACCAGCTGACCCCCACCTGGAGAGTGTACAGCACCGGCAGCAACGTGTTCC
AGACCAGAGCCGGCTGCCTGATCGGCGCCGAGCACGTGAACAACAGCTACGAGTGCGA
CATCCCCATCGGCGCCGGCATCTGCGCCAGCTACCAGACCCAGACCAACAGCCCCAGAA
GAGCCAGAAGCGTGGCCAGCCAGAGCATCATCGCCTACACCATGAGCCTGGGCGTGGA
GAACAGCGTGGCCTACAGCAACAACAGCATCGCCATCCCCACCAACTTCACCATCAGCG
TGACCACCGAGATCCTGCCCGTGAGCATGACCAAGACCAGCGTGGACTGCACCATGTAC
ATCTGCGGCGACAGCACCGAGTGCAGCAACCTGCTGCTGCAGTACGGCAGCTTCTGCA
CCCAGCTGAACAGAGCCCTGACCGGCATCGCCGTGGAGCAGGACAAGAACACCCAGG
AGGTGTTCGCCCAGGTGAAGCAGATCTACAAGACCCCCCCCATCAAGGACTTCGGCGG
CTTCAACTTCAGCCAGATCCTGCCCGACCCCAGCAAGCCCAGCAAGAGAAGCTTCATCG
AGGACCTGCTGTTCAACAAGGTGACCCTGGCCGACGCCGGCTTCATCAAGCAGTACGG
CGACTGCCTGGGCGACATCGCCGCCAGAGACCTGATCTGCGCCCAGAAGTTCAACGGC
CTGACCGTGCTGCCCCCCCTGCTGACCGACGAGATGATCGCCCAGTACACCAGCGCCCT
GCTGGCCGGCACCATCACCAGCGGCTGGACCTTCGGCGCCGGCGCCGCCCTGCAGATC
CCCTTCGCCATGCAGATGGCCTACAGATTCAACGGCATCGGCGTGACCCAGAACGTGCT
GTACGAGAACCAGAAGCTGATCGCCAACCAGTTCAACAGCGCCATCGGCAAGATCCAG
GACAGCCTGAGCAGCACCGCCAGCGCCCTGGGCAAGCTGCAGGACGTGGTGAACCAG
AACGCCCAGGCCCTGAACACCCTGGTGAAGCAGCTGAGCAGCAACTTCGGCGCCATCA
GCAGCGTGCTGAACGACATCCTGAGCAGACTGGACAAGGTGGAGGCCGAGGTGCAGAT
CGACAGACTGATCACCGGCAGACTGCAGAGCCTGCAGACCTACGTGACCCAGCAGCTG
ATCAGAGCCGCCGAGATCAGAGCCAGCGCCAACCTGGCCGCCACCAAGATGAGCGAGT
GCGTGCTGGGCCAGAGCAAGAGAGTGGACTTCTGCGGCAAGGGCTACCACCTGATGAG
CTTCCCCCAGAGCGCCCCCCACGGCGTGGTGTTCCTGCACGTGACCTACGTGCCCGCCC
AGGAGAAGAACTTCACCACCGCCCCCGCCATCTGCCACGACGGCAAGGCCCACTTCCC
 
CAGAGAGGGCGTGTTCGTGAGCAACGGCACCCACTGGTTCGTGACCCAGAGAAACTTC
TACGAGCCCCAGATCATCACCACCGACAACACCTTCGTGAGCGGCAACTGCGACGTGGT
GATCGGCATCGTGAACAACACCGTGTACGACCCCCTGCAGCCCGAGCTGGACAGCTTCA
AGGAGGAGCTGGACAAGTACTTCAAGAACCACACCAGCCCCGACGTGGACCTGGGCG
ACATCAGCGGCATCAACGCCAGCGTGGTGAACATCCAGAAGGAGATCGACAGACTGAA
CGAGGTGGCCAAGAACCTGAACGAGAGCCTGATCGACCTGCAGGAGCTGGGCAAGTAC
GAGCAGTACATCAAGTGGCCCTGGTACATCTGGCTGGGCTTCATCGCCGGCCTGATCGC
CATCGTGATGGTGACCATCATGCTGTGCTGCATGACCAGCTGCTGCAGCTGCCTGAAGG
GCTGCTGCAGCTGCGGCAGCTGCTGCAAGTTCGACGAGGACGACAGCGAGCCCGTGCT
GAAGGGCGTGAAGCTGCACTACACCTAG
 Note: the above sequence consists of signal peptide - extracellular domain - transmembrane domain - intracellular domain.
**SEQ ID No.48: Full-length nucleic acid sequence of Beta S antigen (optimization of insect cells):** 
ATGTTCGTGTTCCTGGTGCTGCTGCCCCTGGTGTCCTCCCAGTGCGTGAACCTGACC
ACCCGTACCCAGCTGCCCCCCGCTTACACCAACTCCTTCACCCGTGGTGTGTACTACCCC
GACAAGGTGTTCCGTTCCTCCGTGCTGCACTCCACCCAGGACCTGTTCCTGCCCTTCTTC
TCCAACGTGACCTGGTTCCACGCTATCCACGTGTCCGGTACCAACGGTACCAAGCGTTT
CGCTAACCCCGTGCTGCCCTTCAACGACGGTGTGTACTTCGCTTCCACCGAGAAGTCCA
ACATCATCCGTGGTTGGATCTTCGGTACCACCCTGGACTCCAAGACCCAGTCCCTGCTGA
TCGTGAACAACGCTACCAACGTGGTGATCAAGGTGTGCGAGTTCCAGTTCTGCAACGAC
CCCTTCCTGGGTGTGTACTACCACAAGAACAACAAGTCCTGGATGGAGTCCGAGTTCCG
TGTGTACTCCTCCGCTAACAACTGCACCTTCGAGTACGTGTCCCAGCCCTTCCTGATGGA
CCTGGAGGGTAAGCAGGGTAACTTCAAGAACCTGCGTGAGTTCGTGTTCAAGAACATC
GACGGTTACTTCAAGATCTACTCCAAGCACACCCCCATCAACCTGGTGCGTGGTCTGCC
CCAGGGTTTCTCCGCTCTGGAGCCCCTGGTGGACCTGCCCATCGGTATCAACATCACCC
GTTTCCAGACCCTGCACCGTTCCTACCTGACCCCCGGTGACTCCTCCTCCGGTTGGACC
GCTGGTGCTGCTGCTTACTACGTGGGTTACCTGCAGCCCCGTACCTTCCTGCTGAAGTAC
AACGAGAACGGTACCATCACCGACGCTGTGGACTGCGCTCTGGACCCCCTGTCCGAGA
CCAAGTGCACCCTGAAGTCCTTCACCGTGGAGAAGGGTATCTACCAGACCTCCAACTTC
 
CGTGTGCAGCCCACCGAGTCCATCGTGCGTTTCCCCAACATCACCAACCTGTGCCCCTT
CGGTGAGGTGTTCAACGCTACCCGTTTCGCTTCCGTGTACGCTTGGAACCGTAAGCGTAT
CTCCAACTGCGTGGCTGACTACTCCGTGCTGTACAACTCCGCTTCCTTCTCCACCTTCAA
GTGCTACGGTGTGTCCCCCACCAAGCTGAACGACCTGTGCTTCACCAACGTGTACGCTG
ACTCCTTCGTGATCCGTGGTGACGAGGTGCGTCAGATCGCTCCCGGTCAGACCGGTAAC
ATCGCTGACTACAACTACAAGCTGCCCGACGACTTCACCGGTTGCGTGATCGCTTGGAA
CTCCAACAACCTGGACTCCAAGGTGGGTGGTAACTACAACTACCTGTACCGTCTGTTCC
GTAAGTCCAACCTGAAGCCCTTCGAGCGTGACATCTCCACCGAGATCTACCAGGCTGGT
TCCACCCCCTGCAACGGTGTGAAGGGTTTCAACTGCTACTTCCCCCTGCAGTCCTACGG
TTTCCAGCCCACCTACGGTGTGGGTTACCAGCCCTACCGTGTGGTGGTGCTGTCCTTCGA
GCTGCTGCACGCTCCCGCTACCGTGTGCGGTCCCAAGAAGTCCACCAACCTGGTGAAG
AACAAGTGCGTGAACTTCAACTTCAACGGTCTGACCGGTACCGGTGTGCTGACCGAGT
CCAACAAGAAGTTCCTGCCCTTCCAGCAGTTCGGTCGTGACATCGCTGACACCACCGAC
GCTGTGCGTGACCCCCAGACCCTGGAGATCCTGGACATCACCCCCTGCTCCTTCGGTGG
TGTGTCCGTGATCACCCCCGGTACCAACACCTCCAACCAGGTGGCTGTGCTGTACCAGG
GTGTGAACTGCACCGAGGTGCCCGTGGCTATCCACGCTGACCAGCTGACCCCCACCTGG
CGTGTGTACTCCACCGGTTCCAACGTGTTCCAGACCCGTGCTGGTTGCCTGATCGGTGC
TGAGCACGTGAACAACTCCTACGAGTGCGACATCCCCATCGGTGCTGGTATCTGCGCTT
CCTACCAGACCCAGACCAACTCCCCCCGTCGTGCTCGTTCCGTGGCTTCCCAGTCCATCA
TCGCTTACACCATGTCCCTGGGTGTGGAGAACTCCGTGGCTTACTCCAACAACTCCATC
GCTATCCCCACCAACTTCACCATCTCCGTGACCACCGAGATCCTGCCCGTGTCCATGACC
AAGACCTCCGTGGACTGCACCATGTACATCTGCGGTGACTCCACCGAGTGCTCCAACCT
GCTGCTGCAGTACGGTTCCTTCTGCACCCAGCTGAACCGTGCTCTGACCGGTATCGCTG
TGGAGCAGGACAAGAACACCCAGGAGGTGTTCGCTCAGGTGAAGCAGATCTACAAGAC
CCCCCCCATCAAGGACTTCGGTGGTTTCAACTTCTCCCAGATCCTGCCCGACCCCTCCA
AGCCCTCCAAGCGTTCCTTCATCGAGGACCTGCTGTTCAACAAGGTGACCCTGGCTGAC
GCTGGTTTCATCAAGCAGTACGGTGACTGCCTGGGTGACATCGCTGCTCGTGACCTGAT
CTGCGCTCAGAAGTTCAACGGTCTGACCGTGCTGCCCCCCCTGCTGACCGACGAGATGA
TCGCTCAGTACACCTCCGCTCTGCTGGCTGGTACCATCACCTCCGGTTGGACCTTCGGTG
CTGGTGCTGCTCTGCAGATCCCCTTCGCTATGCAGATGGCTTACCGTTTCAACGGTATCG
GTGTGACCCAGAACGTGCTGTACGAGAACCAGAAGCTGATCGCTAACCAGTTCAACTC
 
CGCTATCGGTAAGATCCAGGACTCCCTGTCCTCCACCGCTTCCGCTCTGGGTAAGCTGCA
GGACGTGGTGAACCAGAACGCTCAGGCTCTGAACACCCTGGTGAAGCAGCTGTCCTCC
AACTTCGGTGCTATCTCCTCCGTGCTGAACGACATCCTGTCCCGTCTGGACAAGGTGGA
GGCTGAGGTGCAGATCGACCGTCTGATCACCGGTCGTCTGCAGTCCCTGCAGACCTACG
TGACCCAGCAGCTGATCCGTGCTGCTGAGATCCGTGCTTCCGCTAACCTGGCTGCTACC
AAGATGTCCGAGTGCGTGCTGGGTCAGTCCAAGCGTGTGGACTTCTGCGGTAAGGGTTA
CCACCTGATGTCCTTCCCCCAGTCCGCTCCCCACGGTGTGGTGTTCCTGCACGTGACCTA
CGTGCCCGCTCAGGAGAAGAACTTCACCACCGCTCCCGCTATCTGCCACGACGGTAAGG
CTCACTTCCCCCGTGAGGGTGTGTTCGTGTCCAACGGTACCCACTGGTTCGTGACCCAG
CGTAACTTCTACGAGCCCCAGATCATCACCACCGACAACACCTTCGTGTCCGGTAACTG
CGACGTGGTGATCGGTATCGTGAACAACACCGTGTACGACCCCCTGCAGCCCGAGCTGG
ACTCCTTCAAGGAGGAGCTGGACAAGTACTTCAAGAACCACACCTCCCCCGACGTGGA
CCTGGGTGACATCTCCGGTATCAACGCTTCCGTGGTGAACATCCAGAAGGAGATCGACC
GTCTGAACGAGGTGGCTAAGAACCTGAACGAGTCCCTGATCGACCTGCAGGAGCTGGG
TAAGTACGAGCAGTACATCAAGTGGCCCTGGTACATCTGGCTGGGTTTCATCGCTGGTCT
GATCGCTATCGTGATGGTGACCATCATGCTGTGCTGCATGACCTCCTGCTGCTCCTGCCT
GAAGGGTTGCTGCTCCTGCGGTTCCTGCTGCAAGTTCGACGAGGACGACTCCGAGCCC
GTGCTGAAGGGTGTGAAGCTGCACTACACCTAG
 Note: the above sequence consists of signal peptide - extracellular domain - transmembrane domain - intracellular domain.
**SEQ ID No.49: Extracellular amino acid sequence of Beta S antigen:** 
MFVFLVLLPLVSSQCVNLTTRTQLPPAYTNSFTRGVYYPDKVFRSSVLHSTQDLFLPFFS
NVTWFHAIHVSGTNGTKRFANPVLPFNDGVYFASTEKSNIIRGWIFGTTLDSKTQSLLIVNN
ATNVVIKVCEFQFCNDPFLGVYYHKNNKSWMESEFRVYSSANNCTFEYVSQPFLMDLEGK
QGNFKNLREFVFKNIDGYFKIYSKHTPINLVRGLPQGFSALEPLVDLPIGINITRFQTLHRSYL
TPGDSSSGWTAGAAAYYVGYLQPRTFLLKYNENGTITDAVDCALDPLSETKCTLKSFTVEK
GIYQTSNFRVQPTESIVRFPNITNLCPFGEVFNATRFASVYAWNRKRISNCVADYSVLYNSAS
FSTFKCYGVSPTKLNDLCFTNVYADSFVIRGDEVRQIAPGQTGNIADYNYKLPDDFTGCVIA
WNSNNLDSKVGGNYNYLYRLFRKSNLKPFERDISTEIYQAGSTPCNGVKGFNCYFPLQSYG
FQPTYGVGYQPYRVVVLSFELLHAPATVCGPKKSTNLVKNKCVNFNFNGLTGTGVLTESNK
 
KFLPFQQFGRDIADTTDAVRDPQTLEILDITPCSFGGVSVITPGTNTSNQVAVLYQGVNCTEV
PVAIHADQLTPTWRVYSTGSNVFQTRAGCLIGAEHVNNSYECDIPIGAGICASYQTQTNSPR
RARSVASQSIIAYTMSLGVENSVAYSNNSIAIPTNFTISVTTEILPVSMTKTSVDCTMYICGDS
TECSNLLLQYGSFCTQLNRALTGIAVEQDKNTQEVFAQVKQIYKTPPIKDFGGFNFSQILPDP
SKPSKRSFIEDLLFNKVTLADAGFIKQYGDCLGDIAARDLICAQKFNGLTVLPPLLTDEMIAQ
YTSALLAGTITSGWTFGAGAALQIPFAMQMAYRFNGIGVTQNVLYENQKLIANQFNSAIGKI
QDSLSSTASALGKLQDVVNQNAQALNTLVKQLSSNFGAISSVLNDILSRLDKVEAEVQIDRL
ITGRLQSLQTYVTQQLIRAAEIRASANLAATKMSECVLGQSKRVDFCGKGYHLMSFPQSAP
HGVVFLHVTYVPAQEKNFTTAPAICHDGKAHFPREGVFVSNGTHWFVTQRNFYEPQIITTD
NTFVSGNCDVVIGIVNNTVYDPLQPELDSFKEELDKYFKNHTSPDVDLGDISGINASVVNIQ
KEIDRLNEVAKNLNESLIDLQELGKYEQYIKWP
 Note: the above sequence consists of signal peptide - extracellular domain.
**SEQ ID No.50: Extracellular nucleic acid sequence of Beta S antigen (optimization of CHO cells):** 
ATGTTCGTGTTCCTGGTGCTGCTGCCCCTGGTGAGCAGCCAGTGCGTGAACCTGAC
CACCAGAACCCAGCTGCCCCCCGCCTACACCAACAGCTTCACCAGAGGCGTGTACTACC
CCGACAAGGTGTTCAGAAGCAGCGTGCTGCACAGCACCCAGGACCTGTTCCTGCCCTT
CTTCAGCAACGTGACCTGGTTCCACGCCATCCACGTGAGCGGCACCAACGGCACCAAG
AGATTCGCCAACCCCGTGCTGCCCTTCAACGACGGCGTGTACTTCGCCAGCACCGAGAA
GAGCAACATCATCAGAGGCTGGATCTTCGGCACCACCCTGGACAGCAAGACCCAGAGC
CTGCTGATCGTGAACAACGCCACCAACGTGGTGATCAAGGTGTGCGAGTTCCAGTTCTG
CAACGACCCCTTCCTGGGCGTGTACTACCACAAGAACAACAAGAGCTGGATGGAGAGC
GAGTTCAGAGTGTACAGCAGCGCCAACAACTGCACCTTCGAGTACGTGAGCCAGCCCT
TCCTGATGGACCTGGAGGGCAAGCAGGGCAACTTCAAGAACCTGAGAGAGTTCGTGTT
CAAGAACATCGACGGCTACTTCAAGATCTACAGCAAGCACACCCCCATCAACCTGGTGA
GAGGCCTGCCCCAGGGCTTCAGCGCCCTGGAGCCCCTGGTGGACCTGCCCATCGGCATC
AACATCACCAGATTCCAGACCCTGCACAGAAGCTACCTGACCCCCGGCGACAGCAGCA
GCGGCTGGACCGCCGGCGCCGCCGCCTACTACGTGGGCTACCTGCAGCCCAGAACCTTC
CTGCTGAAGTACAACGAGAACGGCACCATCACCGACGCCGTGGACTGCGCCCTGGACC
CCCTGAGCGAGACCAAGTGCACCCTGAAGAGCTTCACCGTGGAGAAGGGCATCTACCA
 
GACCAGCAACTTCAGAGTGCAGCCCACCGAGAGCATCGTGAGATTCCCCAACATCACC
AACCTGTGCCCCTTCGGCGAGGTGTTCAACGCCACCAGATTCGCCAGCGTGTACGCCTG
GAACAGAAAGAGAATCAGCAACTGCGTGGCCGACTACAGCGTGCTGTACAACAGCGCC
AGCTTCAGCACCTTCAAGTGCTACGGCGTGAGCCCCACCAAGCTGAACGACCTGTGCTT
CACCAACGTGTACGCCGACAGCTTCGTGATCAGAGGCGACGAGGTGAGACAGATCGCC
CCCGGCCAGACCGGCAACATCGCCGACTACAACTACAAGCTGCCCGACGACTTCACCG
GCTGCGTGATCGCCTGGAACAGCAACAACCTGGACAGCAAGGTGGGCGGCAACTACAA
CTACCTGTACAGACTGTTCAGAAAGAGCAACCTGAAGCCCTTCGAGAGAGACATCAGC
ACCGAGATCTACCAGGCCGGCAGCACCCCCTGCAACGGCGTGAAGGGCTTCAACTGCT
ACTTCCCCCTGCAGAGCTACGGCTTCCAGCCCACCTACGGCGTGGGCTACCAGCCCTAC
AGAGTGGTGGTGCTGAGCTTCGAGCTGCTGCACGCCCCCGCCACCGTGTGCGGCCCCA
AGAAGAGCACCAACCTGGTGAAGAACAAGTGCGTGAACTTCAACTTCAACGGCCTGAC
CGGCACCGGCGTGCTGACCGAGAGCAACAAGAAGTTCCTGCCCTTCCAGCAGTTCGGC
AGAGACATCGCCGACACCACCGACGCCGTGAGAGACCCCCAGACCCTGGAGATCCTGG
ACATCACCCCCTGCAGCTTCGGCGGCGTGAGCGTGATCACCCCCGGCACCAACACCAG
CAACCAGGTGGCCGTGCTGTACCAGGGCGTGAACTGCACCGAGGTGCCCGTGGCCATC
CACGCCGACCAGCTGACCCCCACCTGGAGAGTGTACAGCACCGGCAGCAACGTGTTCC
AGACCAGAGCCGGCTGCCTGATCGGCGCCGAGCACGTGAACAACAGCTACGAGTGCGA
CATCCCCATCGGCGCCGGCATCTGCGCCAGCTACCAGACCCAGACCAACAGCCCCAGAA
GAGCCAGAAGCGTGGCCAGCCAGAGCATCATCGCCTACACCATGAGCCTGGGCGTGGA
GAACAGCGTGGCCTACAGCAACAACAGCATCGCCATCCCCACCAACTTCACCATCAGCG
TGACCACCGAGATCCTGCCCGTGAGCATGACCAAGACCAGCGTGGACTGCACCATGTAC
ATCTGCGGCGACAGCACCGAGTGCAGCAACCTGCTGCTGCAGTACGGCAGCTTCTGCA
CCCAGCTGAACAGAGCCCTGACCGGCATCGCCGTGGAGCAGGACAAGAACACCCAGG
AGGTGTTCGCCCAGGTGAAGCAGATCTACAAGACCCCCCCCATCAAGGACTTCGGCGG
CTTCAACTTCAGCCAGATCCTGCCCGACCCCAGCAAGCCCAGCAAGAGAAGCTTCATCG
AGGACCTGCTGTTCAACAAGGTGACCCTGGCCGACGCCGGCTTCATCAAGCAGTACGG
CGACTGCCTGGGCGACATCGCCGCCAGAGACCTGATCTGCGCCCAGAAGTTCAACGGC
CTGACCGTGCTGCCCCCCCTGCTGACCGACGAGATGATCGCCCAGTACACCAGCGCCCT
GCTGGCCGGCACCATCACCAGCGGCTGGACCTTCGGCGCCGGCGCCGCCCTGCAGATC
CCCTTCGCCATGCAGATGGCCTACAGATTCAACGGCATCGGCGTGACCCAGAACGTGCT
 
GTACGAGAACCAGAAGCTGATCGCCAACCAGTTCAACAGCGCCATCGGCAAGATCCAG
GACAGCCTGAGCAGCACCGCCAGCGCCCTGGGCAAGCTGCAGGACGTGGTGAACCAG
AACGCCCAGGCCCTGAACACCCTGGTGAAGCAGCTGAGCAGCAACTTCGGCGCCATCA
GCAGCGTGCTGAACGACATCCTGAGCAGACTGGACAAGGTGGAGGCCGAGGTGCAGAT
CGACAGACTGATCACCGGCAGACTGCAGAGCCTGCAGACCTACGTGACCCAGCAGCTG
ATCAGAGCCGCCGAGATCAGAGCCAGCGCCAACCTGGCCGCCACCAAGATGAGCGAGT
GCGTGCTGGGCCAGAGCAAGAGAGTGGACTTCTGCGGCAAGGGCTACCACCTGATGAG
CTTCCCCCAGAGCGCCCCCCACGGCGTGGTGTTCCTGCACGTGACCTACGTGCCCGCCC
AGGAGAAGAACTTCACCACCGCCCCCGCCATCTGCCACGACGGCAAGGCCCACTTCCC
CAGAGAGGGCGTGTTCGTGAGCAACGGCACCCACTGGTTCGTGACCCAGAGAAACTTC
TACGAGCCCCAGATCATCACCACCGACAACACCTTCGTGAGCGGCAACTGCGACGTGGT
GATCGGCATCGTGAACAACACCGTGTACGACCCCCTGCAGCCCGAGCTGGACAGCTTCA
AGGAGGAGCTGGACAAGTACTTCAAGAACCACACCAGCCCCGACGTGGACCTGGGCG
ACATCAGCGGCATCAACGCCAGCGTGGTGAACATCCAGAAGGAGATCGACAGACTGAA
CGAGGTGGCCAAGAACCTGAACGAGAGCCTGATCGACCTGCAGGAGCTGGGCAAGTAC
GAGCAGTACATCAAGTGGCCCTAG
 Note: the above sequence consists of signal peptide - extracellular domain.
**SEQ ID No.51: extracellular nucleic acid sequence of Beta S antigen (optimization of insect cells):** 
ATGTTCGTGTTCCTGGTGCTGCTGCCCCTGGTGTCCTCCCAGTGCGTGAACCTGACC
ACCCGTACCCAGCTGCCCCCCGCTTACACCAACTCCTTCACCCGTGGTGTGTACTACCCC
GACAAGGTGTTCCGTTCCTCCGTGCTGCACTCCACCCAGGACCTGTTCCTGCCCTTCTTC
TCCAACGTGACCTGGTTCCACGCTATCCACGTGTCCGGTACCAACGGTACCAAGCGTTT
CGCTAACCCCGTGCTGCCCTTCAACGACGGTGTGTACTTCGCTTCCACCGAGAAGTCCA
ACATCATCCGTGGTTGGATCTTCGGTACCACCCTGGACTCCAAGACCCAGTCCCTGCTGA
TCGTGAACAACGCTACCAACGTGGTGATCAAGGTGTGCGAGTTCCAGTTCTGCAACGAC
CCCTTCCTGGGTGTGTACTACCACAAGAACAACAAGTCCTGGATGGAGTCCGAGTTCCG
TGTGTACTCCTCCGCTAACAACTGCACCTTCGAGTACGTGTCCCAGCCCTTCCTGATGGA
CCTGGAGGGTAAGCAGGGTAACTTCAAGAACCTGCGTGAGTTCGTGTTCAAGAACATC
GACGGTTACTTCAAGATCTACTCCAAGCACACCCCCATCAACCTGGTGCGTGGTCTGCC
 
CCAGGGTTTCTCCGCTCTGGAGCCCCTGGTGGACCTGCCCATCGGTATCAACATCACCC
GTTTCCAGACCCTGCACCGTTCCTACCTGACCCCCGGTGACTCCTCCTCCGGTTGGACC
GCTGGTGCTGCTGCTTACTACGTGGGTTACCTGCAGCCCCGTACCTTCCTGCTGAAGTAC
AACGAGAACGGTACCATCACCGACGCTGTGGACTGCGCTCTGGACCCCCTGTCCGAGA
CCAAGTGCACCCTGAAGTCCTTCACCGTGGAGAAGGGTATCTACCAGACCTCCAACTTC
CGTGTGCAGCCCACCGAGTCCATCGTGCGTTTCCCCAACATCACCAACCTGTGCCCCTT
CGGTGAGGTGTTCAACGCTACCCGTTTCGCTTCCGTGTACGCTTGGAACCGTAAGCGTAT
CTCCAACTGCGTGGCTGACTACTCCGTGCTGTACAACTCCGCTTCCTTCTCCACCTTCAA
GTGCTACGGTGTGTCCCCCACCAAGCTGAACGACCTGTGCTTCACCAACGTGTACGCTG
ACTCCTTCGTGATCCGTGGTGACGAGGTGCGTCAGATCGCTCCCGGTCAGACCGGTAAC
ATCGCTGACTACAACTACAAGCTGCCCGACGACTTCACCGGTTGCGTGATCGCTTGGAA
CTCCAACAACCTGGACTCCAAGGTGGGTGGTAACTACAACTACCTGTACCGTCTGTTCC
GTAAGTCCAACCTGAAGCCCTTCGAGCGTGACATCTCCACCGAGATCTACCAGGCTGGT
TCCACCCCCTGCAACGGTGTGAAGGGTTTCAACTGCTACTTCCCCCTGCAGTCCTACGG
TTTCCAGCCCACCTACGGTGTGGGTTACCAGCCCTACCGTGTGGTGGTGCTGTCCTTCGA
GCTGCTGCACGCTCCCGCTACCGTGTGCGGTCCCAAGAAGTCCACCAACCTGGTGAAG
AACAAGTGCGTGAACTTCAACTTCAACGGTCTGACCGGTACCGGTGTGCTGACCGAGT
CCAACAAGAAGTTCCTGCCCTTCCAGCAGTTCGGTCGTGACATCGCTGACACCACCGAC
GCTGTGCGTGACCCCCAGACCCTGGAGATCCTGGACATCACCCCCTGCTCCTTCGGTGG
TGTGTCCGTGATCACCCCCGGTACCAACACCTCCAACCAGGTGGCTGTGCTGTACCAGG
GTGTGAACTGCACCGAGGTGCCCGTGGCTATCCACGCTGACCAGCTGACCCCCACCTGG
CGTGTGTACTCCACCGGTTCCAACGTGTTCCAGACCCGTGCTGGTTGCCTGATCGGTGC
TGAGCACGTGAACAACTCCTACGAGTGCGACATCCCCATCGGTGCTGGTATCTGCGCTT
CCTACCAGACCCAGACCAACTCCCCCCGTCGTGCTCGTTCCGTGGCTTCCCAGTCCATCA
TCGCTTACACCATGTCCCTGGGTGTGGAGAACTCCGTGGCTTACTCCAACAACTCCATC
GCTATCCCCACCAACTTCACCATCTCCGTGACCACCGAGATCCTGCCCGTGTCCATGACC
AAGACCTCCGTGGACTGCACCATGTACATCTGCGGTGACTCCACCGAGTGCTCCAACCT
GCTGCTGCAGTACGGTTCCTTCTGCACCCAGCTGAACCGTGCTCTGACCGGTATCGCTG
TGGAGCAGGACAAGAACACCCAGGAGGTGTTCGCTCAGGTGAAGCAGATCTACAAGAC
CCCCCCCATCAAGGACTTCGGTGGTTTCAACTTCTCCCAGATCCTGCCCGACCCCTCCA
AGCCCTCCAAGCGTTCCTTCATCGAGGACCTGCTGTTCAACAAGGTGACCCTGGCTGAC
 
GCTGGTTTCATCAAGCAGTACGGTGACTGCCTGGGTGACATCGCTGCTCGTGACCTGAT
CTGCGCTCAGAAGTTCAACGGTCTGACCGTGCTGCCCCCCCTGCTGACCGACGAGATGA
TCGCTCAGTACACCTCCGCTCTGCTGGCTGGTACCATCACCTCCGGTTGGACCTTCGGTG
CTGGTGCTGCTCTGCAGATCCCCTTCGCTATGCAGATGGCTTACCGTTTCAACGGTATCG
GTGTGACCCAGAACGTGCTGTACGAGAACCAGAAGCTGATCGCTAACCAGTTCAACTC
CGCTATCGGTAAGATCCAGGACTCCCTGTCCTCCACCGCTTCCGCTCTGGGTAAGCTGCA
GGACGTGGTGAACCAGAACGCTCAGGCTCTGAACACCCTGGTGAAGCAGCTGTCCTCC
AACTTCGGTGCTATCTCCTCCGTGCTGAACGACATCCTGTCCCGTCTGGACAAGGTGGA
GGCTGAGGTGCAGATCGACCGTCTGATCACCGGTCGTCTGCAGTCCCTGCAGACCTACG
TGACCCAGCAGCTGATCCGTGCTGCTGAGATCCGTGCTTCCGCTAACCTGGCTGCTACC
AAGATGTCCGAGTGCGTGCTGGGTCAGTCCAAGCGTGTGGACTTCTGCGGTAAGGGTTA
CCACCTGATGTCCTTCCCCCAGTCCGCTCCCCACGGTGTGGTGTTCCTGCACGTGACCTA
CGTGCCCGCTCAGGAGAAGAACTTCACCACCGCTCCCGCTATCTGCCACGACGGTAAGG
CTCACTTCCCCCGTGAGGGTGTGTTCGTGTCCAACGGTACCCACTGGTTCGTGACCCAG
CGTAACTTCTACGAGCCCCAGATCATCACCACCGACAACACCTTCGTGTCCGGTAACTG
CGACGTGGTGATCGGTATCGTGAACAACACCGTGTACGACCCCCTGCAGCCCGAGCTGG
ACTCCTTCAAGGAGGAGCTGGACAAGTACTTCAAGAACCACACCTCCCCCGACGTGGA
CCTGGGTGACATCTCCGGTATCAACGCTTCCGTGGTGAACATCCAGAAGGAGATCGACC
GTCTGAACGAGGTGGCTAAGAACCTGAACGAGTCCCTGATCGACCTGCAGGAGCTGGG
TAAGTACGAGCAGTACATCAAGTGGCCCTAG
 Note: the above sequence consists of signal peptide - extracellular domain.
**SEQ ID No.52: Full-length amino acid sequence of Delta S antigen:** 
MFVFLVLLPLVSSQCVNLRTRTQLPPAYTNSFTRGVYYPDKVFRSSVLHSTQDLFLPFFS
NVTWFHAIHVSGTNGTKRFDNPVLPFNDGVYFASTEKSNIIRGWIFGTTLDSKTQSLLIVNN
ATNVVIKVCEFQFCNDPFLDVYYHKNNKSWMESGVYSSANNCTFEYVSQPFLMDLEGKQ
GNFKNLREFVFKNIDGYFKIYSKHTPINLVRDLPQGFSALEPLVDLPIGINITRFQTLLALHRS
YLTPGDSSSGWTAGAAAYYVGYLQPRTFLLKYNENGTITDAVDCALDPLSETKCTLKSFTV
EKGIYQTSNFRVQPTESIVRFPNITNLCPFGEVFNATRFASVYAWNRKRISNCVADYSVLYNS
ASFSTFKCYGVSPTKLNDLCFTNVYADSFVIRGDEVRQIAPGQTGKIADYNYKLPDDFTGC
VIAWNSNNLDSKVGGNYNYRYRLFRKSNLKPFERDISTEIYQAGSKPCNGVEGFNCYFPLQ
 
SYGFQPTNGVGYQPYRVVVLSFELLHAPATVCGPKKSTNLVKNKCVNFNFNGLTGTGVLTE
SNKKFLPFQQFGRDIADTTDAVRDPQTLEILDITPCSFGGVSVITPGTNTSNQVAVLYQGVNC
TEVPVAIHADQLTPTWRVYSTGSNVFQTRAGCLIGAEHVNNSYECDIPIGAGICASYQTQTN
SRRRARSVASQSIIAYTMSLGAENSVAYSNNSIAIPTNFTISVTTEILPVSMTKTSVDCTMYIC
GDSTECSNLLLQYGSFCTQLNRALTGIAVEQDKNTQEVFAQVKQIYKTPPIKDFGGFNFSQIL
PDPSKPSKRSFIEDLLFNKVTLADAGFIKQYGDCLGDIAARDLICAQKFNGLTVLPPLLTDEM
IAQYTSALLAGTITSGWTFGAGAALQIPFAMQMAYRFNGIGVTQNVLYENQKLIANQFNSAI
GKIQDSLSSTASALGKLQNVVNQNAQALNTLVKQLSSNFGAISSVLNDILSRLDKVEAEVQI
DRLITGRLQSLQTYVTQQLIRAAEIRASANLAATKMSECVLGQSKRVDFCGKGYHLMSFPQ
SAPHGVVFLHVTYVPAQEKNFTTAPAICHDGKAHFPREGVFVSNGTHWFVTQRNFYEPQIIT
TDNTFVSGNCDVVIGIVNNTVYDPLQPELDSFKEELDKYFKNHTSPDVDLGDISGINASVVN
IQKEIDRLNEVAKNLNESLIDLQELGKYEQYIKWPWYIWLGFIAGLIAIVMVTIMLCCMTSC
CSCLKGCCSCGSCCKFDEDDSEPVLKGVKLHYT
 Note: the above sequence consists of signal peptide - extracellular domain - transmembrane domain - intracellular domain.
**SEQ ID No.53: Full-length nucleic acid sequence of Delta S antigen (optimization of CHO cells):** 
ATGTTCGTGTTCCTGGTGCTGCTGCCCCTGGTGAGCAGCCAGTGCGTGAACCTGAG
AACCAGAACCCAGCTGCCCCCCGCCTACACCAACAGCTTCACCAGAGGCGTGTACTACC
CCGACAAGGTGTTCAGAAGCAGCGTGCTGCACAGCACCCAGGACCTGTTCCTGCCCTT
CTTCAGCAACGTGACCTGGTTCCACGCCATCCACGTGAGCGGCACCAACGGCACCAAG
AGATTCGACAACCCCGTGCTGCCCTTCAACGACGGCGTGTACTTCGCCAGCACCGAGAA
GAGCAACATCATCAGAGGCTGGATCTTCGGCACCACCCTGGACAGCAAGACCCAGAGC
CTGCTGATCGTGAACAACGCCACCAACGTGGTGATCAAGGTGTGCGAGTTCCAGTTCTG
CAACGACCCCTTCCTGGACGTGTACTACCACAAGAACAACAAGAGCTGGATGGAGAGC
GGCGTGTACAGCAGCGCCAACAACTGCACCTTCGAGTACGTGAGCCAGCCCTTCCTGAT
GGACCTGGAGGGCAAGCAGGGCAACTTCAAGAACCTGAGAGAGTTCGTGTTCAAGAA
CATCGACGGCTACTTCAAGATCTACAGCAAGCACACCCCCATCAACCTGGTGAGAGACC
TGCCCCAGGGCTTCAGCGCCCTGGAGCCCCTGGTGGACCTGCCCATCGGCATCAACATC
ACCAGATTCCAGACCCTGCTGGCCCTGCACAGAAGCTACCTGACCCCCGGCGACAGCA
 
GCAGCGGCTGGACCGCCGGCGCCGCCGCCTACTACGTGGGCTACCTGCAGCCCAGAAC
CTTCCTGCTGAAGTACAACGAGAACGGCACCATCACCGACGCCGTGGACTGCGCCCTG
GACCCCCTGAGCGAGACCAAGTGCACCCTGAAGAGCTTCACCGTGGAGAAGGGCATCT
ACCAGACCAGCAACTTCAGAGTGCAGCCCACCGAGAGCATCGTGAGATTCCCCAACAT
CACCAACCTGTGCCCCTTCGGCGAGGTGTTCAACGCCACCAGATTCGCCAGCGTGTACG
CCTGGAACAGAAAGAGAATCAGCAACTGCGTGGCCGACTACAGCGTGCTGTACAACAG
CGCCAGCTTCAGCACCTTCAAGTGCTACGGCGTGAGCCCCACCAAGCTGAACGACCTG
TGCTTCACCAACGTGTACGCCGACAGCTTCGTGATCAGAGGCGACGAGGTGAGACAGA
TCGCCCCCGGCCAGACCGGCAAGATCGCCGACTACAACTACAAGCTGCCCGACGACTT
CACCGGCTGCGTGATCGCCTGGAACAGCAACAACCTGGACAGCAAGGTGGGCGGCAAC
TACAACTACAGATACAGACTGTTCAGAAAGAGCAACCTGAAGCCCTTCGAGAGAGACA
TCAGCACCGAGATCTACCAGGCCGGCAGCAAGCCCTGCAACGGCGTGGAGGGCTTCAA
CTGCTACTTCCCCCTGCAGAGCTACGGCTTCCAGCCCACCAACGGCGTGGGCTACCAGC
CCTACAGAGTGGTGGTGCTGAGCTTCGAGCTGCTGCACGCCCCCGCCACCGTGTGCGGC
CCCAAGAAGAGCACCAACCTGGTGAAGAACAAGTGCGTGAACTTCAACTTCAACGGCC
TGACCGGCACCGGCGTGCTGACCGAGAGCAACAAGAAGTTCCTGCCCTTCCAGCAGTT
CGGCAGAGACATCGCCGACACCACCGACGCCGTGAGAGACCCCCAGACCCTGGAGATC
CTGGACATCACCCCCTGCAGCTTCGGCGGCGTGAGCGTGATCACCCCCGGCACCAACAC
CAGCAACCAGGTGGCCGTGCTGTACCAGGGCGTGAACTGCACCGAGGTGCCCGTGGCC
ATCCACGCCGACCAGCTGACCCCCACCTGGAGAGTGTACAGCACCGGCAGCAACGTGT
TCCAGACCAGAGCCGGCTGCCTGATCGGCGCCGAGCACGTGAACAACAGCTACGAGTG
CGACATCCCCATCGGCGCCGGCATCTGCGCCAGCTACCAGACCCAGACCAACAGCAGA
AGAAGAGCCAGAAGCGTGGCCAGCCAGAGCATCATCGCCTACACCATGAGCCTGGGCG
CCGAGAACAGCGTGGCCTACAGCAACAACAGCATCGCCATCCCCACCAACTTCACCATC
AGCGTGACCACCGAGATCCTGCCCGTGAGCATGACCAAGACCAGCGTGGACTGCACCA
TGTACATCTGCGGCGACAGCACCGAGTGCAGCAACCTGCTGCTGCAGTACGGCAGCTTC
TGCACCCAGCTGAACAGAGCCCTGACCGGCATCGCCGTGGAGCAGGACAAGAACACCC
AGGAGGTGTTCGCCCAGGTGAAGCAGATCTACAAGACCCCCCCCATCAAGGACTTCGG
CGGCTTCAACTTCAGCCAGATCCTGCCCGACCCCAGCAAGCCCAGCAAGAGAAGCTTC
ATCGAGGACCTGCTGTTCAACAAGGTGACCCTGGCCGACGCCGGCTTCATCAAGCAGTA
CGGCGACTGCCTGGGCGACATCGCCGCCAGAGACCTGATCTGCGCCCAGAAGTTCAAC
 
GGCCTGACCGTGCTGCCCCCCCTGCTGACCGACGAGATGATCGCCCAGTACACCAGCGC
CCTGCTGGCCGGCACCATCACCAGCGGCTGGACCTTCGGCGCCGGCGCCGCCCTGCAG
ATCCCCTTCGCCATGCAGATGGCCTACAGATTCAACGGCATCGGCGTGACCCAGAACGT
GCTGTACGAGAACCAGAAGCTGATCGCCAACCAGTTCAACAGCGCCATCGGCAAGATC
CAGGACAGCCTGAGCAGCACCGCCAGCGCCCTGGGCAAGCTGCAGAACGTGGTGAAC
CAGAACGCCCAGGCCCTGAACACCCTGGTGAAGCAGCTGAGCAGCAACTTCGGCGCCA
TCAGCAGCGTGCTGAACGACATCCTGAGCAGACTGGACAAGGTGGAGGCCGAGGTGCA
GATCGACAGACTGATCACCGGCAGACTGCAGAGCCTGCAGACCTACGTGACCCAGCAG
CTGATCAGAGCCGCCGAGATCAGAGCCAGCGCCAACCTGGCCGCCACCAAGATGAGCG
AGTGCGTGCTGGGCCAGAGCAAGAGAGTGGACTTCTGCGGCAAGGGCTACCACCTGAT
GAGCTTCCCCCAGAGCGCCCCCCACGGCGTGGTGTTCCTGCACGTGACCTACGTGCCCG
CCCAGGAGAAGAACTTCACCACCGCCCCCGCCATCTGCCACGACGGCAAGGCCCACTT
CCCCAGAGAGGGCGTGTTCGTGAGCAACGGCACCCACTGGTTCGTGACCCAGAGAAAC
TTCTACGAGCCCCAGATCATCACCACCGACAACACCTTCGTGAGCGGCAACTGCGACGT
GGTGATCGGCATCGTGAACAACACCGTGTACGACCCCCTGCAGCCCGAGCTGGACAGC
TTCAAGGAGGAGCTGGACAAGTACTTCAAGAACCACACCAGCCCCGACGTGGACCTGG
GCGACATCAGCGGCATCAACGCCAGCGTGGTGAACATCCAGAAGGAGATCGACAGACT
GAACGAGGTGGCCAAGAACCTGAACGAGAGCCTGATCGACCTGCAGGAGCTGGGCAA
GTACGAGCAGTACATCAAGTGGCCCTGGTACATCTGGCTGGGCTTCATCGCCGGCCTGAT
CGCCATCGTGATGGTGACCATCATGCTGTGCTGCATGACCAGCTGCTGCAGCTGCCTGA
AGGGCTGCTGCAGCTGCGGCAGCTGCTGCAAGTTCGACGAGGACGACAGCGAGCCCGT
GCTGAAGGGCGTGAAGCTGCACTACACCTAG
 Note: the above sequence consists of signal peptide - extracellular domain - transmembrane domain - intracellular domain.
**SEQ ID No.54: Full-length nucleic acid sequence of Delta S antigen (optimization of insect cells):** 
ATGTTCGTGTTCCTGGTGCTGCTGCCCCTGGTGTCCTCCCAGTGCGTGAACCTGCGT
ACCCGTACCCAGCTGCCCCCCGCTTACACCAACTCCTTCACCCGTGGTGTGTACTACCCC
GACAAGGTGTTCCGTTCCTCCGTGCTGCACTCCACCCAGGACCTGTTCCTGCCCTTCTTC
TCCAACGTGACCTGGTTCCACGCTATCCACGTGTCCGGTACCAACGGTACCAAGCGTTT
 
CGACAACCCCGTGCTGCCCTTCAACGACGGTGTGTACTTCGCTTCCACCGAGAAGTCCA
ACATCATCCGTGGTTGGATCTTCGGTACCACCCTGGACTCCAAGACCCAGTCCCTGCTGA
TCGTGAACAACGCTACCAACGTGGTGATCAAGGTGTGCGAGTTCCAGTTCTGCAACGAC
CCCTTCCTGGACGTGTACTACCACAAGAACAACAAGTCCTGGATGGAGTCCGGTGTGTA
CTCCTCCGCTAACAACTGCACCTTCGAGTACGTGTCCCAGCCCTTCCTGATGGACCTGG
AGGGTAAGCAGGGTAACTTCAAGAACCTGCGTGAGTTCGTGTTCAAGAACATCGACGG
TTACTTCAAGATCTACTCCAAGCACACCCCCATCAACCTGGTGCGTGACCTGCCCCAGG
GTTTCTCCGCTCTGGAGCCCCTGGTGGACCTGCCCATCGGTATCAACATCACCCGTTTCC
AGACCCTGCTGGCTCTGCACCGTTCCTACCTGACCCCCGGTGACTCCTCCTCCGGTTGG
ACCGCTGGTGCTGCTGCTTACTACGTGGGTTACCTGCAGCCCCGTACCTTCCTGCTGAAG
TACAACGAGAACGGTACCATCACCGACGCTGTGGACTGCGCTCTGGACCCCCTGTCCGA
GACCAAGTGCACCCTGAAGTCCTTCACCGTGGAGAAGGGTATCTACCAGACCTCCAACT
TCCGTGTGCAGCCCACCGAGTCCATCGTGCGTTTCCCCAACATCACCAACCTGTGCCCC
TTCGGTGAGGTGTTCAACGCTACCCGTTTCGCTTCCGTGTACGCTTGGAACCGTAAGCGT
ATCTCCAACTGCGTGGCTGACTACTCCGTGCTGTACAACTCCGCTTCCTTCTCCACCTTC
AAGTGCTACGGTGTGTCCCCCACCAAGCTGAACGACCTGTGCTTCACCAACGTGTACGC
TGACTCCTTCGTGATCCGTGGTGACGAGGTGCGTCAGATCGCTCCCGGTCAGACCGGTA
AGATCGCTGACTACAACTACAAGCTGCCCGACGACTTCACCGGTTGCGTGATCGCTTGG
AACTCCAACAACCTGGACTCCAAGGTGGGTGGTAACTACAACTACCGTTACCGTCTGTT
CCGTAAGTCCAACCTGAAGCCCTTCGAGCGTGACATCTCCACCGAGATCTACCAGGCTG
GTTCCAAGCCCTGCAACGGTGTGGAGGGTTTCAACTGCTACTTCCCCCTGCAGTCCTAC
GGTTTCCAGCCCACCAACGGTGTGGGTTACCAGCCCTACCGTGTGGTGGTGCTGTCCTT
CGAGCTGCTGCACGCTCCCGCTACCGTGTGCGGTCCCAAGAAGTCCACCAACCTGGTG
AAGAACAAGTGCGTGAACTTCAACTTCAACGGTCTGACCGGTACCGGTGTGCTGACCG
AGTCCAACAAGAAGTTCCTGCCCTTCCAGCAGTTCGGTCGTGACATCGCTGACACCACC
GACGCTGTGCGTGACCCCCAGACCCTGGAGATCCTGGACATCACCCCCTGCTCCTTCGG
TGGTGTGTCCGTGATCACCCCCGGTACCAACACCTCCAACCAGGTGGCTGTGCTGTACC
AGGGTGTGAACTGCACCGAGGTGCCCGTGGCTATCCACGCTGACCAGCTGACCCCCAC
CTGGCGTGTGTACTCCACCGGTTCCAACGTGTTCCAGACCCGTGCTGGTTGCCTGATCG
GTGCTGAGCACGTGAACAACTCCTACGAGTGCGACATCCCCATCGGTGCTGGTATCTGC
GCTTCCTACCAGACCCAGACCAACTCCCGTCGTCGTGCTCGTTCCGTGGCTTCCCAGTC
 
CATCATCGCTTACACCATGTCCCTGGGTGCTGAGAACTCCGTGGCTTACTCCAACAACTC
CATCGCTATCCCCACCAACTTCACCATCTCCGTGACCACCGAGATCCTGCCCGTGTCCAT
GACCAAGACCTCCGTGGACTGCACCATGTACATCTGCGGTGACTCCACCGAGTGCTCCA
ACCTGCTGCTGCAGTACGGTTCCTTCTGCACCCAGCTGAACCGTGCTCTGACCGGTATC
GCTGTGGAGCAGGACAAGAACACCCAGGAGGTGTTCGCTCAGGTGAAGCAGATCTACA
AGACCCCCCCCATCAAGGACTTCGGTGGTTTCAACTTCTCCCAGATCCTGCCCGACCCC
TCCAAGCCCTCCAAGCGTTCCTTCATCGAGGACCTGCTGTTCAACAAGGTGACCCTGGC
TGACGCTGGTTTCATCAAGCAGTACGGTGACTGCCTGGGTGACATCGCTGCTCGTGACC
TGATCTGCGCTCAGAAGTTCAACGGTCTGACCGTGCTGCCCCCCCTGCTGACCGACGAG
ATGATCGCTCAGTACACCTCCGCTCTGCTGGCTGGTACCATCACCTCCGGTTGGACCTTC
GGTGCTGGTGCTGCTCTGCAGATCCCCTTCGCTATGCAGATGGCTTACCGTTTCAACGGT
ATCGGTGTGACCCAGAACGTGCTGTACGAGAACCAGAAGCTGATCGCTAACCAGTTCA
ACTCCGCTATCGGTAAGATCCAGGACTCCCTGTCCTCCACCGCTTCCGCTCTGGGTAAGC
TGCAGAACGTGGTGAACCAGAACGCTCAGGCTCTGAACACCCTGGTGAAGCAGCTGTC
CTCCAACTTCGGTGCTATCTCCTCCGTGCTGAACGACATCCTGTCCCGTCTGGACAAGGT
GGAGGCTGAGGTGCAGATCGACCGTCTGATCACCGGTCGTCTGCAGTCCCTGCAGACCT
ACGTGACCCAGCAGCTGATCCGTGCTGCTGAGATCCGTGCTTCCGCTAACCTGGCTGCT
ACCAAGATGTCCGAGTGCGTGCTGGGTCAGTCCAAGCGTGTGGACTTCTGCGGTAAGG
GTTACCACCTGATGTCCTTCCCCCAGTCCGCTCCCCACGGTGTGGTGTTCCTGCACGTGA
CCTACGTGCCCGCTCAGGAGAAGAACTTCACCACCGCTCCCGCTATCTGCCACGACGGT
AAGGCTCACTTCCCCCGTGAGGGTGTGTTCGTGTCCAACGGTACCCACTGGTTCGTGAC
CCAGCGTAACTTCTACGAGCCCCAGATCATCACCACCGACAACACCTTCGTGTCCGGTA
ACTGCGACGTGGTGATCGGTATCGTGAACAACACCGTGTACGACCCCCTGCAGCCCGAG
CTGGACTCCTTCAAGGAGGAGCTGGACAAGTACTTCAAGAACCACACCTCCCCCGACG
TGGACCTGGGTGACATCTCCGGTATCAACGCTTCCGTGGTGAACATCCAGAAGGAGATC
GACCGTCTGAACGAGGTGGCTAAGAACCTGAACGAGTCCCTGATCGACCTGCAGGAGC
TGGGTAAGTACGAGCAGTACATCAAGTGGCCCTGGTACATCTGGCTGGGTTTCATCGCT
GGTCTGATCGCTATCGTGATGGTGACCATCATGCTGTGCTGCATGACCTCCTGCTGCTCC
TGCCTGAAGGGTTGCTGCTCCTGCGGTTCCTGCTGCAAGTTCGACGAGGACGACTCCGA
GCCCGTGCTGAAGGGTGTGAAGCTGCACTACACCTAG
 Note: the above sequence consists of signal peptide - extracellular domain - transmembrane domain - intracellular domain.
**SEQ ID No.55: Extracellular amino acid sequence of Delta S antigen:** 
MFVFLVLLPLVSSQCVNLRTRTQLPPAYTNSFTRGVYYPDKVFRSSVLHSTQDLFLPFFS
NVTWFHAIHVSGTNGTKRFDNPVLPFNDGVYFASTEKSNIIRGWIFGTTLDSKTQSLLIVNN
ATNVVIKVCEFQFCNDPFLDVYYHKNNKSWMESGVYSSANNCTFEYVSQPFLMDLEGKQ
GNFKNLREFVFKNIDGYFKIYSKHTPINLVRDLPQGFSALEPLVDLPIGINITRFQTLLALHRS
YLTPGDSSSGWTAGAAAYYVGYLQPRTFLLKYNENGTITDAVDCALDPLSETKCTLKSFTV
EKGIYQTSNFRVQPTESIVRFPNITNLCPFGEVFNATRFASVYAWNRKRISNCVADYSVLYNS
ASFSTFKCYGVSPTKLNDLCFTNVYADSFVIRGDEVRQIAPGQTGKIADYNYKLPDDFTGC
VIAWNSNNLDSKVGGNYNYRYRLFRKSNLKPFERDISTEIYQAGSKPCNGVEGFNCYFPLQ
SYGFQPTNGVGYQPYRVVVLSFELLHAPATVCGPKKSTNLVKNKCVNFNFNGLTGTGVLTE
SNKKFLPFQQFGRDIADTTDAVRDPQTLEILDITPCSFGGVSVITPGTNTSNQVAVLYQGVNC
TEVPVAIHADQLTPTWRVYSTGSNVFQTRAGCLIGAEHVNNSYECDIPIGAGICASYQTQTN
SRRRARSVASQSIIAYTMSLGAENSVAYSNNSIAIPTNFTISVTTEILPVSMTKTSVDCTMYIC
GDSTECSNLLLQYGSFCTQLNRALTGIAVEQDKNTQEVFAQVKQIYKTPPIKDFGGFNFSQIL
PDPSKPSKRSFIEDLLFNKVTLADAGFIKQYGDCLGDIAARDLICAQKFNGLTVLPPLLTDEM
IAQYTSALLAGTITSGWTFGAGAALQIPFAMQMAYRFNGIGVTQNVLYENQKLIANQFNSAI
GKIQDSLSSTASALGKLQNVVNQNAQALNTLVKQLSSNFGAISSVLNDILSRLDKVEAEVQI
DRLITGRLQSLQTYVTQQLIRAAEIRASANLAATKMSECVLGQSKRVDFCGKGYHLMSFPQ
SAPHGVVFLHVTYVPAQEKNFTTAPAICHDGKAHFPREGVFVSNGTHWFVTQRNFYEPQIIT
TDNTFVSGNCDVVIGIVNNTVYDPLQPELDSFKEELDKYFKNHTSPDVDLGDISGINASVVN
IQKEIDRLNEVAKNLNESLIDLQELGKYEQYIKWP
 Note: the above sequence consists of signal peptide - extracellular domain.
**SEQ ID No.56: Extracellular nucleic acid sequence of Delta S antigen (optimization of CHO cells):** 
ATGTTCGTGTTCCTGGTGCTGCTGCCCCTGGTGAGCAGCCAGTGCGTGAACCTGAG
AACCAGAACCCAGCTGCCCCCCGCCTACACCAACAGCTTCACCAGAGGCGTGTACTACC
CCGACAAGGTGTTCAGAAGCAGCGTGCTGCACAGCACCCAGGACCTGTTCCTGCCCTT
CTTCAGCAACGTGACCTGGTTCCACGCCATCCACGTGAGCGGCACCAACGGCACCAAG
 
AGATTCGACAACCCCGTGCTGCCCTTCAACGACGGCGTGTACTTCGCCAGCACCGAGAA
GAGCAACATCATCAGAGGCTGGATCTTCGGCACCACCCTGGACAGCAAGACCCAGAGC
CTGCTGATCGTGAACAACGCCACCAACGTGGTGATCAAGGTGTGCGAGTTCCAGTTCTG
CAACGACCCCTTCCTGGACGTGTACTACCACAAGAACAACAAGAGCTGGATGGAGAGC
GGCGTGTACAGCAGCGCCAACAACTGCACCTTCGAGTACGTGAGCCAGCCCTTCCTGAT
GGACCTGGAGGGCAAGCAGGGCAACTTCAAGAACCTGAGAGAGTTCGTGTTCAAGAA
CATCGACGGCTACTTCAAGATCTACAGCAAGCACACCCCCATCAACCTGGTGAGAGACC
TGCCCCAGGGCTTCAGCGCCCTGGAGCCCCTGGTGGACCTGCCCATCGGCATCAACATC
ACCAGATTCCAGACCCTGCTGGCCCTGCACAGAAGCTACCTGACCCCCGGCGACAGCA
GCAGCGGCTGGACCGCCGGCGCCGCCGCCTACTACGTGGGCTACCTGCAGCCCAGAAC
CTTCCTGCTGAAGTACAACGAGAACGGCACCATCACCGACGCCGTGGACTGCGCCCTG
GACCCCCTGAGCGAGACCAAGTGCACCCTGAAGAGCTTCACCGTGGAGAAGGGCATCT
ACCAGACCAGCAACTTCAGAGTGCAGCCCACCGAGAGCATCGTGAGATTCCCCAACAT
CACCAACCTGTGCCCCTTCGGCGAGGTGTTCAACGCCACCAGATTCGCCAGCGTGTACG
CCTGGAACAGAAAGAGAATCAGCAACTGCGTGGCCGACTACAGCGTGCTGTACAACAG
CGCCAGCTTCAGCACCTTCAAGTGCTACGGCGTGAGCCCCACCAAGCTGAACGACCTG
TGCTTCACCAACGTGTACGCCGACAGCTTCGTGATCAGAGGCGACGAGGTGAGACAGA
TCGCCCCCGGCCAGACCGGCAAGATCGCCGACTACAACTACAAGCTGCCCGACGACTT
CACCGGCTGCGTGATCGCCTGGAACAGCAACAACCTGGACAGCAAGGTGGGCGGCAAC
TACAACTACAGATACAGACTGTTCAGAAAGAGCAACCTGAAGCCCTTCGAGAGAGACA
TCAGCACCGAGATCTACCAGGCCGGCAGCAAGCCCTGCAACGGCGTGGAGGGCTTCAA
CTGCTACTTCCCCCTGCAGAGCTACGGCTTCCAGCCCACCAACGGCGTGGGCTACCAGC
CCTACAGAGTGGTGGTGCTGAGCTTCGAGCTGCTGCACGCCCCCGCCACCGTGTGCGGC
CCCAAGAAGAGCACCAACCTGGTGAAGAACAAGTGCGTGAACTTCAACTTCAACGGCC
TGACCGGCACCGGCGTGCTGACCGAGAGCAACAAGAAGTTCCTGCCCTTCCAGCAGTT
CGGCAGAGACATCGCCGACACCACCGACGCCGTGAGAGACCCCCAGACCCTGGAGATC
CTGGACATCACCCCCTGCAGCTTCGGCGGCGTGAGCGTGATCACCCCCGGCACCAACAC
CAGCAACCAGGTGGCCGTGCTGTACCAGGGCGTGAACTGCACCGAGGTGCCCGTGGCC
ATCCACGCCGACCAGCTGACCCCCACCTGGAGAGTGTACAGCACCGGCAGCAACGTGT
TCCAGACCAGAGCCGGCTGCCTGATCGGCGCCGAGCACGTGAACAACAGCTACGAGTG
CGACATCCCCATCGGCGCCGGCATCTGCGCCAGCTACCAGACCCAGACCAACAGCAGA
 
AGAAGAGCCAGAAGCGTGGCCAGCCAGAGCATCATCGCCTACACCATGAGCCTGGGCG
CCGAGAACAGCGTGGCCTACAGCAACAACAGCATCGCCATCCCCACCAACTTCACCATC
AGCGTGACCACCGAGATCCTGCCCGTGAGCATGACCAAGACCAGCGTGGACTGCACCA
TGTACATCTGCGGCGACAGCACCGAGTGCAGCAACCTGCTGCTGCAGTACGGCAGCTTC
TGCACCCAGCTGAACAGAGCCCTGACCGGCATCGCCGTGGAGCAGGACAAGAACACCC
AGGAGGTGTTCGCCCAGGTGAAGCAGATCTACAAGACCCCCCCCATCAAGGACTTCGG
CGGCTTCAACTTCAGCCAGATCCTGCCCGACCCCAGCAAGCCCAGCAAGAGAAGCTTC
ATCGAGGACCTGCTGTTCAACAAGGTGACCCTGGCCGACGCCGGCTTCATCAAGCAGTA
CGGCGACTGCCTGGGCGACATCGCCGCCAGAGACCTGATCTGCGCCCAGAAGTTCAAC
GGCCTGACCGTGCTGCCCCCCCTGCTGACCGACGAGATGATCGCCCAGTACACCAGCGC
CCTGCTGGCCGGCACCATCACCAGCGGCTGGACCTTCGGCGCCGGCGCCGCCCTGCAG
ATCCCCTTCGCCATGCAGATGGCCTACAGATTCAACGGCATCGGCGTGACCCAGAACGT
GCTGTACGAGAACCAGAAGCTGATCGCCAACCAGTTCAACAGCGCCATCGGCAAGATC
CAGGACAGCCTGAGCAGCACCGCCAGCGCCCTGGGCAAGCTGCAGAACGTGGTGAAC
CAGAACGCCCAGGCCCTGAACACCCTGGTGAAGCAGCTGAGCAGCAACTTCGGCGCCA
TCAGCAGCGTGCTGAACGACATCCTGAGCAGACTGGACAAGGTGGAGGCCGAGGTGCA
GATCGACAGACTGATCACCGGCAGACTGCAGAGCCTGCAGACCTACGTGACCCAGCAG
CTGATCAGAGCCGCCGAGATCAGAGCCAGCGCCAACCTGGCCGCCACCAAGATGAGCG
AGTGCGTGCTGGGCCAGAGCAAGAGAGTGGACTTCTGCGGCAAGGGCTACCACCTGAT
GAGCTTCCCCCAGAGCGCCCCCCACGGCGTGGTGTTCCTGCACGTGACCTACGTGCCCG
CCCAGGAGAAGAACTTCACCACCGCCCCCGCCATCTGCCACGACGGCAAGGCCCACTT
CCCCAGAGAGGGCGTGTTCGTGAGCAACGGCACCCACTGGTTCGTGACCCAGAGAAAC
TTCTACGAGCCCCAGATCATCACCACCGACAACACCTTCGTGAGCGGCAACTGCGACGT
GGTGATCGGCATCGTGAACAACACCGTGTACGACCCCCTGCAGCCCGAGCTGGACAGC
TTCAAGGAGGAGCTGGACAAGTACTTCAAGAACCACACCAGCCCCGACGTGGACCTGG
GCGACATCAGCGGCATCAACGCCAGCGTGGTGAACATCCAGAAGGAGATCGACAGACT
GAACGAGGTGGCCAAGAACCTGAACGAGAGCCTGATCGACCTGCAGGAGCTGGGCAA
GTACGAGCAGTACATCAAGTGGCCCTAG
 Note: the above sequence consists of signal peptide - extracellular domain.
**SEQ ID No.57: Extracellular nucleic acid sequence of Delta S antigen (optimization of insect cells):** 
ATGTTCGTGTTCCTGGTGCTGCTGCCCCTGGTGTCCTCCCAGTGCGTGAACCTGCGT
ACCCGTACCCAGCTGCCCCCCGCTTACACCAACTCCTTCACCCGTGGTGTGTACTACCCC
GACAAGGTGTTCCGTTCCTCCGTGCTGCACTCCACCCAGGACCTGTTCCTGCCCTTCTTC
TCCAACGTGACCTGGTTCCACGCTATCCACGTGTCCGGTACCAACGGTACCAAGCGTTT
CGACAACCCCGTGCTGCCCTTCAACGACGGTGTGTACTTCGCTTCCACCGAGAAGTCCA
ACATCATCCGTGGTTGGATCTTCGGTACCACCCTGGACTCCAAGACCCAGTCCCTGCTGA
TCGTGAACAACGCTACCAACGTGGTGATCAAGGTGTGCGAGTTCCAGTTCTGCAACGAC
CCCTTCCTGGACGTGTACTACCACAAGAACAACAAGTCCTGGATGGAGTCCGGTGTGTA
CTCCTCCGCTAACAACTGCACCTTCGAGTACGTGTCCCAGCCCTTCCTGATGGACCTGG
AGGGTAAGCAGGGTAACTTCAAGAACCTGCGTGAGTTCGTGTTCAAGAACATCGACGG
TTACTTCAAGATCTACTCCAAGCACACCCCCATCAACCTGGTGCGTGACCTGCCCCAGG
GTTTCTCCGCTCTGGAGCCCCTGGTGGACCTGCCCATCGGTATCAACATCACCCGTTTCC
AGACCCTGCTGGCTCTGCACCGTTCCTACCTGACCCCCGGTGACTCCTCCTCCGGTTGG
ACCGCTGGTGCTGCTGCTTACTACGTGGGTTACCTGCAGCCCCGTACCTTCCTGCTGAAG
TACAACGAGAACGGTACCATCACCGACGCTGTGGACTGCGCTCTGGACCCCCTGTCCGA
GACCAAGTGCACCCTGAAGTCCTTCACCGTGGAGAAGGGTATCTACCAGACCTCCAACT
TCCGTGTGCAGCCCACCGAGTCCATCGTGCGTTTCCCCAACATCACCAACCTGTGCCCC
TTCGGTGAGGTGTTCAACGCTACCCGTTTCGCTTCCGTGTACGCTTGGAACCGTAAGCGT
ATCTCCAACTGCGTGGCTGACTACTCCGTGCTGTACAACTCCGCTTCCTTCTCCACCTTC
AAGTGCTACGGTGTGTCCCCCACCAAGCTGAACGACCTGTGCTTCACCAACGTGTACGC
TGACTCCTTCGTGATCCGTGGTGACGAGGTGCGTCAGATCGCTCCCGGTCAGACCGGTA
AGATCGCTGACTACAACTACAAGCTGCCCGACGACTTCACCGGTTGCGTGATCGCTTGG
AACTCCAACAACCTGGACTCCAAGGTGGGTGGTAACTACAACTACCGTTACCGTCTGTT
CCGTAAGTCCAACCTGAAGCCCTTCGAGCGTGACATCTCCACCGAGATCTACCAGGCTG
GTTCCAAGCCCTGCAACGGTGTGGAGGGTTTCAACTGCTACTTCCCCCTGCAGTCCTAC
GGTTTCCAGCCCACCAACGGTGTGGGTTACCAGCCCTACCGTGTGGTGGTGCTGTCCTT
CGAGCTGCTGCACGCTCCCGCTACCGTGTGCGGTCCCAAGAAGTCCACCAACCTGGTG
AAGAACAAGTGCGTGAACTTCAACTTCAACGGTCTGACCGGTACCGGTGTGCTGACCG
AGTCCAACAAGAAGTTCCTGCCCTTCCAGCAGTTCGGTCGTGACATCGCTGACACCACC
GACGCTGTGCGTGACCCCCAGACCCTGGAGATCCTGGACATCACCCCCTGCTCCTTCGG
 
TGGTGTGTCCGTGATCACCCCCGGTACCAACACCTCCAACCAGGTGGCTGTGCTGTACC
AGGGTGTGAACTGCACCGAGGTGCCCGTGGCTATCCACGCTGACCAGCTGACCCCCAC
CTGGCGTGTGTACTCCACCGGTTCCAACGTGTTCCAGACCCGTGCTGGTTGCCTGATCG
GTGCTGAGCACGTGAACAACTCCTACGAGTGCGACATCCCCATCGGTGCTGGTATCTGC
GCTTCCTACCAGACCCAGACCAACTCCCGTCGTCGTGCTCGTTCCGTGGCTTCCCAGTC
CATCATCGCTTACACCATGTCCCTGGGTGCTGAGAACTCCGTGGCTTACTCCAACAACTC
CATCGCTATCCCCACCAACTTCACCATCTCCGTGACCACCGAGATCCTGCCCGTGTCCAT
GACCAAGACCTCCGTGGACTGCACCATGTACATCTGCGGTGACTCCACCGAGTGCTCCA
ACCTGCTGCTGCAGTACGGTTCCTTCTGCACCCAGCTGAACCGTGCTCTGACCGGTATC
GCTGTGGAGCAGGACAAGAACACCCAGGAGGTGTTCGCTCAGGTGAAGCAGATCTACA
AGACCCCCCCCATCAAGGACTTCGGTGGTTTCAACTTCTCCCAGATCCTGCCCGACCCC
TCCAAGCCCTCCAAGCGTTCCTTCATCGAGGACCTGCTGTTCAACAAGGTGACCCTGGC
TGACGCTGGTTTCATCAAGCAGTACGGTGACTGCCTGGGTGACATCGCTGCTCGTGACC
TGATCTGCGCTCAGAAGTTCAACGGTCTGACCGTGCTGCCCCCCCTGCTGACCGACGAG
ATGATCGCTCAGTACACCTCCGCTCTGCTGGCTGGTACCATCACCTCCGGTTGGACCTTC
GGTGCTGGTGCTGCTCTGCAGATCCCCTTCGCTATGCAGATGGCTTACCGTTTCAACGGT
ATCGGTGTGACCCAGAACGTGCTGTACGAGAACCAGAAGCTGATCGCTAACCAGTTCA
ACTCCGCTATCGGTAAGATCCAGGACTCCCTGTCCTCCACCGCTTCCGCTCTGGGTAAGC
TGCAGAACGTGGTGAACCAGAACGCTCAGGCTCTGAACACCCTGGTGAAGCAGCTGTC
CTCCAACTTCGGTGCTATCTCCTCCGTGCTGAACGACATCCTGTCCCGTCTGGACAAGGT
GGAGGCTGAGGTGCAGATCGACCGTCTGATCACCGGTCGTCTGCAGTCCCTGCAGACCT
ACGTGACCCAGCAGCTGATCCGTGCTGCTGAGATCCGTGCTTCCGCTAACCTGGCTGCT
ACCAAGATGTCCGAGTGCGTGCTGGGTCAGTCCAAGCGTGTGGACTTCTGCGGTAAGG
GTTACCACCTGATGTCCTTCCCCCAGTCCGCTCCCCACGGTGTGGTGTTCCTGCACGTGA
CCTACGTGCCCGCTCAGGAGAAGAACTTCACCACCGCTCCCGCTATCTGCCACGACGGT
AAGGCTCACTTCCCCCGTGAGGGTGTGTTCGTGTCCAACGGTACCCACTGGTTCGTGAC
CCAGCGTAACTTCTACGAGCCCCAGATCATCACCACCGACAACACCTTCGTGTCCGGTA
ACTGCGACGTGGTGATCGGTATCGTGAACAACACCGTGTACGACCCCCTGCAGCCCGAG
CTGGACTCCTTCAAGGAGGAGCTGGACAAGTACTTCAAGAACCACACCTCCCCCGACG
TGGACCTGGGTGACATCTCCGGTATCAACGCTTCCGTGGTGAACATCCAGAAGGAGATC
GACCGTCTGAACGAGGTGGCTAAGAACCTGAACGAGTCCCTGATCGACCTGCAGGAGC
 TGGGTAAGTACGAGCAGTACATCAAGTGGCCCTAG Note: the above sequence consists of signal peptide - extracellular domain.
**SEQ ID No.58: Full-length amino acid sequence of Omicron-BA.1 S antigen:** 
MFVFLVLLPLVSSQCVNLTTRTQLPPAYTNSFTRGVYYPDKVFRSSVLHSTQDLFLPFFS
NVTWFHVISGTNGTKRFDNPVLPFNDGVYFASIEKSNIIRGWIFGTTLDSKTQSLLIVNNATN
VVIKVCEFQFCNDPFLDHKNNKSWMESEFRVYSSANNCTFEYVSQPFLMDLEGKQGNFKN
LREFVFKNIDGYFKIYSKHTPIIVRDLPQGFSALEPLVDLPIGINITRFQTLLALHRSYLTPGDS
SSGWTAGAAAYYVGYLQPRTFLLKYNENGTITDAVDCALDPLSETKCTLKSFTVEKGIYQT
SNFRVQPTESIVRFPNITNLCPFDEVFNATRFASVYAWNRKRISNCVADYSVLYNLAPFFTFK
CYGVSPTKLNDLCFTNVYADSFVIRGDEVRQIAPGQTGNIADYNYKLPDDFTGCVIAWNSN
KLDSKVSGNYNYLYRLFRKSNLKPFERDISTEIYQAGNKPCNGVAGFNCYFPLRSYSFRPTY
GVGHQPYRVVVLSFELLHAPATVCGPKKSTNLVKNKCVNFNFNGLKGTGVLTESNKKFLPF
QQFGRDIADTTDAVRDPQTLEILDITPCSFGGVSVITPGTNTSNQVAVLYQGVNCTEVPVAIH
ADQLTPTWRVYSTGSNVFQTRAGCLIGAEYVNNSYECDIPIGAGICASYQTQTKSHRRARSV
ASQSIIAYTMSLGAENSVAYSNNSIAIPTNFTISVTTEILPVSMTKTSVDCTMYICGDSTECSN
LLLQYGSFCTQLKRALTGIAVEQDKNTQEVFAQVKQIYKTPPIKYFGGFNFSQILPDPSKPSK
RSFIEDLLFNKVTLADAGFIKQYGDCLGDIAARDLICAQKFKGLTVLPPLLTDEMIAQYTSA
LLAGTITSGWTFGAGAALQIPFAMQMAYRFNGIGVTQNVLYENQKLIANQFNSAIGKIQDSL
SSTASALGKLQDVVNHNAQALNTLVKQLSSKFGAISSVLNDIFSRLDKVEAEVQIDRLITGR
LQSLQTYVTQQLIRAAEIRASANLAATKMSECVLGQSKRVDFCGKGYHLMSFPQSAPHGV
VFLHVTYVPAQEKNFTTAPAICHDGKAHFPREGVFVSNGTHWFVTQRNFYEPQIITTDNTFV
SGNCDVVIGIVNNTVYDPLQPELDSFKEELDKYFKNHTSPDVDLGDISGINASVVNIQKEID
RLNEVAKNLNESLIDLQELGKYEQYIKWPWYIWLGFIAGLIAIVMVTIMLCCMTSCCSCLK
GCCSCGSCCKFDEDDSEPVLKGVKLHYT
 Note: the above sequence consists of signal peptide - extracellular domain - transmembrane domain - intracellular domain.
**SEQ ID No.59: Full-length nucleic acid sequence of Omicron-BA.1 S antigen (optimization of CHO cells):** ATGTTCGTGTTCCTGGTGCTGCTGCCCCTGGTGAGCAGCCAGTGCGTGAACCTGAC 
CACCAGAACCCAGCTGCCCCCCGCCTACACCAACAGCTTCACCAGAGGCGTGTACTACC
CCGACAAGGTGTTCAGAAGCAGCGTGCTGCACAGCACCCAGGACCTGTTCCTGCCCTT
CTTCAGCAACGTGACCTGGTTCCACGTGATCAGCGGCACCAACGGCACCAAGAGATTC
GACAACCCCGTGCTGCCCTTCAACGACGGCGTGTACTTCGCCAGCATCGAGAAGAGCA
ACATCATCAGAGGCTGGATCTTCGGCACCACCCTGGACAGCAAGACCCAGAGCCTGCT
GATCGTGAACAACGCCACCAACGTGGTGATCAAGGTGTGCGAGTTCCAGTTCTGCAAC
GACCCCTTCCTGGACCACAAGAACAACAAGAGCTGGATGGAGAGCGAGTTCAGAGTGT
ACAGCAGCGCCAACAACTGCACCTTCGAGTACGTGAGCCAGCCCTTCCTGATGGACCTG
GAGGGCAAGCAGGGCAACTTCAAGAACCTGAGAGAGTTCGTGTTCAAGAACATCGACG
GCTACTTCAAGATCTACAGCAAGCACACCCCCATCATCGTGAGAGACCTGCCCCAGGGC
TTCAGCGCCCTGGAGCCCCTGGTGGACCTGCCCATCGGCATCAACATCACCAGATTCCA
GACCCTGCTGGCCCTGCACAGAAGCTACCTGACCCCCGGCGACAGCAGCAGCGGCTGG
ACCGCCGGCGCCGCCGCCTACTACGTGGGCTACCTGCAGCCCAGAACCTTCCTGCTGAA
GTACAACGAGAACGGCACCATCACCGACGCCGTGGACTGCGCCCTGGACCCCCTGAGC
GAGACCAAGTGCACCCTGAAGAGCTTCACCGTGGAGAAGGGCATCTACCAGACCAGCA
ACTTCAGAGTGCAGCCCACCGAGAGCATCGTGAGATTCCCCAACATCACCAACCTGTGC
CCCTTCGACGAGGTGTTCAACGCCACCAGATTCGCCAGCGTGTACGCCTGGAACAGAA
AGAGAATCAGCAACTGCGTGGCCGACTACAGCGTGCTGTACAACCTGGCCCCCTTCTTC
ACCTTCAAGTGCTACGGCGTGAGCCCCACCAAGCTGAACGACCTGTGCTTCACCAACGT
GTACGCCGACAGCTTCGTGATCAGAGGCGACGAGGTGAGACAGATCGCCCCCGGCCAG
ACCGGCAACATCGCCGACTACAACTACAAGCTGCCCGACGACTTCACCGGCTGCGTGAT
CGCCTGGAACAGCAACAAGCTGGACAGCAAGGTGAGCGGCAACTACAACTACCTGTAC
AGACTGTTCAGAAAGAGCAACCTGAAGCCCTTCGAGAGAGACATCAGCACCGAGATCT
ACCAGGCCGGCAACAAGCCCTGCAACGGCGTGGCCGGCTTCAACTGCTACTTCCCCCT
GAGAAGCTACAGCTTCAGACCCACCTACGGCGTGGGCCACCAGCCCTACAGAGTGGTG
GTGCTGAGCTTCGAGCTGCTGCACGCCCCCGCCACCGTGTGCGGCCCCAAGAAGAGCA
CCAACCTGGTGAAGAACAAGTGCGTGAACTTCAACTTCAACGGCCTGAAGGGCACCGG
CGTGCTGACCGAGAGCAACAAGAAGTTCCTGCCCTTCCAGCAGTTCGGCAGAGACATC
GCCGACACCACCGACGCCGTGAGAGACCCCCAGACCCTGGAGATCCTGGACATCACCC
CCTGCAGCTTCGGCGGCGTGAGCGTGATCACCCCCGGCACCAACACCAGCAACCAGGT
GGCCGTGCTGTACCAGGGCGTGAACTGCACCGAGGTGCCCGTGGCCATCCACGCCGAC
 
CAGCTGACCCCCACCTGGAGAGTGTACAGCACCGGCAGCAACGTGTTCCAGACCAGAG
CCGGCTGCCTGATCGGCGCCGAGTACGTGAACAACAGCTACGAGTGCGACATCCCCATC
GGCGCCGGCATCTGCGCCAGCTACCAGACCCAGACCAAGAGCCACAGAAGAGCCAGA
AGCGTGGCCAGCCAGAGCATCATCGCCTACACCATGAGCCTGGGCGCCGAGAACAGCG
TGGCCTACAGCAACAACAGCATCGCCATCCCCACCAACTTCACCATCAGCGTGACCACC
GAGATCCTGCCCGTGAGCATGACCAAGACCAGCGTGGACTGCACCATGTACATCTGCGG
CGACAGCACCGAGTGCAGCAACCTGCTGCTGCAGTACGGCAGCTTCTGCACCCAGCTG
AAGAGAGCCCTGACCGGCATCGCCGTGGAGCAGGACAAGAACACCCAGGAGGTGTTC
GCCCAGGTGAAGCAGATCTACAAGACCCCCCCCATCAAGTACTTCGGCGGCTTCAACTT
CAGCCAGATCCTGCCCGACCCCAGCAAGCCCAGCAAGAGAAGCTTCATCGAGGACCTG
CTGTTCAACAAGGTGACCCTGGCCGACGCCGGCTTCATCAAGCAGTACGGCGACTGCCT
GGGCGACATCGCCGCCAGAGACCTGATCTGCGCCCAGAAGTTCAAGGGCCTGACCGTG
CTGCCCCCCCTGCTGACCGACGAGATGATCGCCCAGTACACCAGCGCCCTGCTGGCCGG
CACCATCACCAGCGGCTGGACCTTCGGCGCCGGCGCCGCCCTGCAGATCCCCTTCGCCA
TGCAGATGGCCTACAGATTCAACGGCATCGGCGTGACCCAGAACGTGCTGTACGAGAAC
CAGAAGCTGATCGCCAACCAGTTCAACAGCGCCATCGGCAAGATCCAGGACAGCCTGA
GCAGCACCGCCAGCGCCCTGGGCAAGCTGCAGGACGTGGTGAACCACAACGCCCAGG
CCCTGAACACCCTGGTGAAGCAGCTGAGCAGCAAGTTCGGCGCCATCAGCAGCGTGCT
GAACGACATCTTCAGCAGACTGGACAAGGTGGAGGCCGAGGTGCAGATCGACAGACTG
ATCACCGGCAGACTGCAGAGCCTGCAGACCTACGTGACCCAGCAGCTGATCAGAGCCG
CCGAGATCAGAGCCAGCGCCAACCTGGCCGCCACCAAGATGAGCGAGTGCGTGCTGGG
CCAGAGCAAGAGAGTGGACTTCTGCGGCAAGGGCTACCACCTGATGAGCTTCCCCCAG
AGCGCCCCCCACGGCGTGGTGTTCCTGCACGTGACCTACGTGCCCGCCCAGGAGAAGA
ACTTCACCACCGCCCCCGCCATCTGCCACGACGGCAAGGCCCACTTCCCCAGAGAGGG
CGTGTTCGTGAGCAACGGCACCCACTGGTTCGTGACCCAGAGAAACTTCTACGAGCCC
CAGATCATCACCACCGACAACACCTTCGTGAGCGGCAACTGCGACGTGGTGATCGGCAT
CGTGAACAACACCGTGTACGACCCCCTGCAGCCCGAGCTGGACAGCTTCAAGGAGGAG
CTGGACAAGTACTTCAAGAACCACACCAGCCCCGACGTGGACCTGGGCGACATCAGCG
GCATCAACGCCAGCGTGGTGAACATCCAGAAGGAGATCGACAGACTGAACGAGGTGGC
CAAGAACCTGAACGAGAGCCTGATCGACCTGCAGGAGCTGGGCAAGTACGAGCAGTAC
ATCAAGTGGCCCTGGTACATCTGGCTGGGCTTCATCGCCGGCCTGATCGCCATCGTGATG
 
GTGACCATCATGCTGTGCTGCATGACCAGCTGCTGCAGCTGCCTGAAGGGCTGCTGCAG
CTGCGGCAGCTGCTGCAAGTTCGACGAGGACGACAGCGAGCCCGTGCTGAAGGGCGTG
AAGCTGCACTACACCTAG
 Note: the above sequence consists of signal peptide - extracellular domain - transmembrane domain - intracellular domain.
**SEQ ID No.60: Full-length nucleic acid sequence of Omicron-BA.1 S antigen (optimization of insect cells):** 
ATGTTCGTGTTCCTGGTGCTGCTGCCCCTGGTGTCCTCCCAGTGCGTGAACCTGACC
ACCCGTACCCAGCTGCCCCCCGCTTACACCAACTCCTTCACCCGTGGTGTGTACTACCCC
GACAAGGTGTTCCGTTCCTCCGTGCTGCACTCCACCCAGGACCTGTTCCTGCCCTTCTTC
TCCAACGTGACCTGGTTCCACGTGATCTCCGGTACCAACGGTACCAAGCGTTTCGACAA
CCCCGTGCTGCCCTTCAACGACGGTGTGTACTTCGCTTCCATCGAGAAGTCCAACATCAT
CCGTGGTTGGATCTTCGGTACCACCCTGGACTCCAAGACCCAGTCCCTGCTGATCGTGA
ACAACGCTACCAACGTGGTGATCAAGGTGTGCGAGTTCCAGTTCTGCAACGACCCCTTC
CTGGACCACAAGAACAACAAGTCCTGGATGGAGTCCGAGTTCCGTGTGTACTCCTCCGC
TAACAACTGCACCTTCGAGTACGTGTCCCAGCCCTTCCTGATGGACCTGGAGGGTAAGC
AGGGTAACTTCAAGAACCTGCGTGAGTTCGTGTTCAAGAACATCGACGGTTACTTCAAG
ATCTACTCCAAGCACACCCCCATCATCGTGCGTGACCTGCCCCAGGGTTTCTCCGCTCTG
GAGCCCCTGGTGGACCTGCCCATCGGTATCAACATCACCCGTTTCCAGACCCTGCTGGC
TCTGCACCGTTCCTACCTGACCCCCGGTGACTCCTCCTCCGGTTGGACCGCTGGTGCTG
CTGCTTACTACGTGGGTTACCTGCAGCCCCGTACCTTCCTGCTGAAGTACAACGAGAAC
GGTACCATCACCGACGCTGTGGACTGCGCTCTGGACCCCCTGTCCGAGACCAAGTGCAC
CCTGAAGTCCTTCACCGTGGAGAAGGGTATCTACCAGACCTCCAACTTCCGTGTGCAGC
CCACCGAGTCCATCGTGCGTTTCCCCAACATCACCAACCTGTGCCCCTTCGACGAGGTG
TTCAACGCTACCCGTTTCGCTTCCGTGTACGCTTGGAACCGTAAGCGTATCTCCAACTGC
GTGGCTGACTACTCCGTGCTGTACAACCTGGCTCCCTTCTTCACCTTCAAGTGCTACGGT
GTGTCCCCCACCAAGCTGAACGACCTGTGCTTCACCAACGTGTACGCTGACTCCTTCGT
GATCCGTGGTGACGAGGTGCGTCAGATCGCTCCCGGTCAGACCGGTAACATCGCTGACT
ACAACTACAAGCTGCCCGACGACTTCACCGGTTGCGTGATCGCTTGGAACTCCAACAAG
CTGGACTCCAAGGTGTCCGGTAACTACAACTACCTGTACCGTCTGTTCCGTAAGTCCAA
 
CCTGAAGCCCTTCGAGCGTGACATCTCCACCGAGATCTACCAGGCTGGTAACAAGCCCT
GCAACGGTGTGGCTGGTTTCAACTGCTACTTCCCCCTGCGTTCCTACTCCTTCCGTCCCA
CCTACGGTGTGGGTCACCAGCCCTACCGTGTGGTGGTGCTGTCCTTCGAGCTGCTGCAC
GCTCCCGCTACCGTGTGCGGTCCCAAGAAGTCCACCAACCTGGTGAAGAACAAGTGCG
TGAACTTCAACTTCAACGGTCTGAAGGGTACCGGTGTGCTGACCGAGTCCAACAAGAA
GTTCCTGCCCTTCCAGCAGTTCGGTCGTGACATCGCTGACACCACCGACGCTGTGCGTG
ACCCCCAGACCCTGGAGATCCTGGACATCACCCCCTGCTCCTTCGGTGGTGTGTCCGTG
ATCACCCCCGGTACCAACACCTCCAACCAGGTGGCTGTGCTGTACCAGGGTGTGAACTG
CACCGAGGTGCCCGTGGCTATCCACGCTGACCAGCTGACCCCCACCTGGCGTGTGTACT
CCACCGGTTCCAACGTGTTCCAGACCCGTGCTGGTTGCCTGATCGGTGCTGAGTACGTG
AACAACTCCTACGAGTGCGACATCCCCATCGGTGCTGGTATCTGCGCTTCCTACCAGACC
CAGACCAAGTCCCACCGTCGTGCTCGTTCCGTGGCTTCCCAGTCCATCATCGCTTACACC
ATGTCCCTGGGTGCTGAGAACTCCGTGGCTTACTCCAACAACTCCATCGCTATCCCCACC
AACTTCACCATCTCCGTGACCACCGAGATCCTGCCCGTGTCCATGACCAAGACCTCCGT
GGACTGCACCATGTACATCTGCGGTGACTCCACCGAGTGCTCCAACCTGCTGCTGCAGT
ACGGTTCCTTCTGCACCCAGCTGAAGCGTGCTCTGACCGGTATCGCTGTGGAGCAGGAC
AAGAACACCCAGGAGGTGTTCGCTCAGGTGAAGCAGATCTACAAGACCCCCCCCATCA
AGTACTTCGGTGGTTTCAACTTCTCCCAGATCCTGCCCGACCCCTCCAAGCCCTCCAAG
CGTTCCTTCATCGAGGACCTGCTGTTCAACAAGGTGACCCTGGCTGACGCTGGTTTCAT
CAAGCAGTACGGTGACTGCCTGGGTGACATCGCTGCTCGTGACCTGATCTGCGCTCAGA
AGTTCAAGGGTCTGACCGTGCTGCCCCCCCTGCTGACCGACGAGATGATCGCTCAGTAC
ACCTCCGCTCTGCTGGCTGGTACCATCACCTCCGGTTGGACCTTCGGTGCTGGTGCTGCT
CTGCAGATCCCCTTCGCTATGCAGATGGCTTACCGTTTCAACGGTATCGGTGTGACCCAG
AACGTGCTGTACGAGAACCAGAAGCTGATCGCTAACCAGTTCAACTCCGCTATCGGTAA
GATCCAGGACTCCCTGTCCTCCACCGCTTCCGCTCTGGGTAAGCTGCAGGACGTGGTGA
ACCACAACGCTCAGGCTCTGAACACCCTGGTGAAGCAGCTGTCCTCCAAGTTCGGTGCT
ATCTCCTCCGTGCTGAACGACATCTTCTCCCGTCTGGACAAGGTGGAGGCTGAGGTGCA
GATCGACCGTCTGATCACCGGTCGTCTGCAGTCCCTGCAGACCTACGTGACCCAGCAGC
TGATCCGTGCTGCTGAGATCCGTGCTTCCGCTAACCTGGCTGCTACCAAGATGTCCGAGT
GCGTGCTGGGTCAGTCCAAGCGTGTGGACTTCTGCGGTAAGGGTTACCACCTGATGTCC
TTCCCCCAGTCCGCTCCCCACGGTGTGGTGTTCCTGCACGTGACCTACGTGCCCGCTCA
 
GGAGAAGAACTTCACCACCGCTCCCGCTATCTGCCACGACGGTAAGGCTCACTTCCCCC
GTGAGGGTGTGTTCGTGTCCAACGGTACCCACTGGTTCGTGACCCAGCGTAACTTCTAC
GAGCCCCAGATCATCACCACCGACAACACCTTCGTGTCCGGTAACTGCGACGTGGTGAT
CGGTATCGTGAACAACACCGTGTACGACCCCCTGCAGCCCGAGCTGGACTCCTTCAAGG
AGGAGCTGGACAAGTACTTCAAGAACCACACCTCCCCCGACGTGGACCTGGGTGACAT
CTCCGGTATCAACGCTTCCGTGGTGAACATCCAGAAGGAGATCGACCGTCTGAACGAGG
TGGCTAAGAACCTGAACGAGTCCCTGATCGACCTGCAGGAGCTGGGTAAGTACGAGCA
GTACATCAAGTGGCCCTGGTACATCTGGCTGGGTTTCATCGCTGGTCTGATCGCTATCGT
GATGGTGACCATCATGCTGTGCTGCATGACCTCCTGCTGCTCCTGCCTGAAGGGTTGCTG
CTCCTGCGGTTCCTGCTGCAAGTTCGACGAGGACGACTCCGAGCCCGTGCTGAAGGGT
GTGAAGCTGCACTACACCTAG
 Note: the above sequence consists of signal peptide - extracellular domain - transmembrane domain - intracellular domain.
**SEQ ID No.61: Extracellular amino acid sequence of Omicron-BA.1 S antigen:** 
MFVFLVLLPLVSSQCVNLTTRTQLPPAYTNSFTRGVYYPDKVFRSSVLHSTQDLFLPFFS
NVTWFHVISGTNGTKRFDNPVLPFNDGVYFASIEKSNIIRGWIFGTTLDSKTQSLLIVNNATN
VVIKVCEFQFCNDPFLDHKNNKSWMESEFRVYSSANNCTFEYVSQPFLMDLEGKQGNFKN
LREFVFKNIDGYFKIYSKHTPIIVRDLPQGFSALEPLVDLPIGINITRFQTLLALHRSYLTPGDS
SSGWTAGAAAYYVGYLQPRTFLLKYNENGTITDAVDCALDPLSETKCTLKSFTVEKGIYQT
SNFRVQPTESIVRFPNITNLCPFDEVFNATRFASVYAWNRKRISNCVADYSVLYNLAPFFTFK
CYGVSPTKLNDLCFTNVYADSFVIRGDEVRQIAPGQTGNIADYNYKLPDDFTGCVIAWNSN
KLDSKVSGNYNYLYRLFRKSNLKPFERDISTEIYQAGNKPCNGVAGFNCYFPLRSYSFRPTY
GVGHQPYRVVVLSFELLHAPATVCGPKKSTNLVKNKCVNFNFNGLKGTGVLTESNKKFLPF
QQFGRDIADTTDAVRDPQTLEILDITPCSFGGVSVITPGTNTSNQVAVLYQGVNCTEVPVAIH
ADQLTPTWRVYSTGSNVFQTRAGCLIGAEYVNNSYECDIPIGAGICASYQTQTKSHRRARSV
ASQSIIAYTMSLGAENSVAYSNNSIAIPTNFTISVTTEILPVSMTKTSVDCTMYICGDSTECSN
LLLQYGSFCTQLKRALTGIAVEQDKNTQEVFAQVKQIYKTPPIKYFGGFNFSQILPDPSKPSK
RSFIEDLLFNKVTLADAGFIKQYGDCLGDIAARDLICAQKFKGLTVLPPLLTDEMIAQYTSA
LLAGTITSGWTFGAGAALQIPFAMQMAYRFNGIGVTQNVLYENQKLIANQFNSAIGKIQDSL
SSTASALGKLQDVVNHNAQALNTLVKQLSSKFGAISSVLNDIFSRLDKVEAEVQIDRLITGR
 
LQSLQTYVTQQLIRAAEIRASANLAATKMSECVLGQSKRVDFCGKGYHLMSFPQSAPHGV
VFLHVTYVPAQEKNFTTAPAICHDGKAHFPREGVFVSNGTHWFVTQRNFYEPQIITTDNTFV
SGNCDVVIGIVNNTVYDPLQPELDSFKEELDKYFKNHTSPDVDLGDISGINASVVNIQKEID
RLNEVAKNLNESLIDLQELGKYEQYIKWP
 Note: the above sequence consists of signal peptide - extracellular domain.
**SEQ ID No.62: Extracellular nucleic acid sequence of Omicron-BA.1 S antigen (optimization of CHO cells):** 
ATGTTCGTGTTCCTGGTGCTGCTGCCCCTGGTGAGCAGCCAGTGCGTGAACCTGAC
CACCAGAACCCAGCTGCCCCCCGCCTACACCAACAGCTTCACCAGAGGCGTGTACTACC
CCGACAAGGTGTTCAGAAGCAGCGTGCTGCACAGCACCCAGGACCTGTTCCTGCCCTT
CTTCAGCAACGTGACCTGGTTCCACGTGATCAGCGGCACCAACGGCACCAAGAGATTC
GACAACCCCGTGCTGCCCTTCAACGACGGCGTGTACTTCGCCAGCATCGAGAAGAGCA
ACATCATCAGAGGCTGGATCTTCGGCACCACCCTGGACAGCAAGACCCAGAGCCTGCT
GATCGTGAACAACGCCACCAACGTGGTGATCAAGGTGTGCGAGTTCCAGTTCTGCAAC
GACCCCTTCCTGGACCACAAGAACAACAAGAGCTGGATGGAGAGCGAGTTCAGAGTGT
ACAGCAGCGCCAACAACTGCACCTTCGAGTACGTGAGCCAGCCCTTCCTGATGGACCTG
GAGGGCAAGCAGGGCAACTTCAAGAACCTGAGAGAGTTCGTGTTCAAGAACATCGACG
GCTACTTCAAGATCTACAGCAAGCACACCCCCATCATCGTGAGAGACCTGCCCCAGGGC
TTCAGCGCCCTGGAGCCCCTGGTGGACCTGCCCATCGGCATCAACATCACCAGATTCCA
GACCCTGCTGGCCCTGCACAGAAGCTACCTGACCCCCGGCGACAGCAGCAGCGGCTGG
ACCGCCGGCGCCGCCGCCTACTACGTGGGCTACCTGCAGCCCAGAACCTTCCTGCTGAA
GTACAACGAGAACGGCACCATCACCGACGCCGTGGACTGCGCCCTGGACCCCCTGAGC
GAGACCAAGTGCACCCTGAAGAGCTTCACCGTGGAGAAGGGCATCTACCAGACCAGCA
ACTTCAGAGTGCAGCCCACCGAGAGCATCGTGAGATTCCCCAACATCACCAACCTGTGC
CCCTTCGACGAGGTGTTCAACGCCACCAGATTCGCCAGCGTGTACGCCTGGAACAGAA
AGAGAATCAGCAACTGCGTGGCCGACTACAGCGTGCTGTACAACCTGGCCCCCTTCTTC
ACCTTCAAGTGCTACGGCGTGAGCCCCACCAAGCTGAACGACCTGTGCTTCACCAACGT
GTACGCCGACAGCTTCGTGATCAGAGGCGACGAGGTGAGACAGATCGCCCCCGGCCAG
ACCGGCAACATCGCCGACTACAACTACAAGCTGCCCGACGACTTCACCGGCTGCGTGAT
CGCCTGGAACAGCAACAAGCTGGACAGCAAGGTGAGCGGCAACTACAACTACCTGTAC
 
AGACTGTTCAGAAAGAGCAACCTGAAGCCCTTCGAGAGAGACATCAGCACCGAGATCT
ACCAGGCCGGCAACAAGCCCTGCAACGGCGTGGCCGGCTTCAACTGCTACTTCCCCCT
GAGAAGCTACAGCTTCAGACCCACCTACGGCGTGGGCCACCAGCCCTACAGAGTGGTG
GTGCTGAGCTTCGAGCTGCTGCACGCCCCCGCCACCGTGTGCGGCCCCAAGAAGAGCA
CCAACCTGGTGAAGAACAAGTGCGTGAACTTCAACTTCAACGGCCTGAAGGGCACCGG
CGTGCTGACCGAGAGCAACAAGAAGTTCCTGCCCTTCCAGCAGTTCGGCAGAGACATC
GCCGACACCACCGACGCCGTGAGAGACCCCCAGACCCTGGAGATCCTGGACATCACCC
CCTGCAGCTTCGGCGGCGTGAGCGTGATCACCCCCGGCACCAACACCAGCAACCAGGT
GGCCGTGCTGTACCAGGGCGTGAACTGCACCGAGGTGCCCGTGGCCATCCACGCCGAC
CAGCTGACCCCCACCTGGAGAGTGTACAGCACCGGCAGCAACGTGTTCCAGACCAGAG
CCGGCTGCCTGATCGGCGCCGAGTACGTGAACAACAGCTACGAGTGCGACATCCCCATC
GGCGCCGGCATCTGCGCCAGCTACCAGACCCAGACCAAGAGCCACAGAAGAGCCAGA
AGCGTGGCCAGCCAGAGCATCATCGCCTACACCATGAGCCTGGGCGCCGAGAACAGCG
TGGCCTACAGCAACAACAGCATCGCCATCCCCACCAACTTCACCATCAGCGTGACCACC
GAGATCCTGCCCGTGAGCATGACCAAGACCAGCGTGGACTGCACCATGTACATCTGCGG
CGACAGCACCGAGTGCAGCAACCTGCTGCTGCAGTACGGCAGCTTCTGCACCCAGCTG
AAGAGAGCCCTGACCGGCATCGCCGTGGAGCAGGACAAGAACACCCAGGAGGTGTTC
GCCCAGGTGAAGCAGATCTACAAGACCCCCCCCATCAAGTACTTCGGCGGCTTCAACTT
CAGCCAGATCCTGCCCGACCCCAGCAAGCCCAGCAAGAGAAGCTTCATCGAGGACCTG
CTGTTCAACAAGGTGACCCTGGCCGACGCCGGCTTCATCAAGCAGTACGGCGACTGCCT
GGGCGACATCGCCGCCAGAGACCTGATCTGCGCCCAGAAGTTCAAGGGCCTGACCGTG
CTGCCCCCCCTGCTGACCGACGAGATGATCGCCCAGTACACCAGCGCCCTGCTGGCCGG
CACCATCACCAGCGGCTGGACCTTCGGCGCCGGCGCCGCCCTGCAGATCCCCTTCGCCA
TGCAGATGGCCTACAGATTCAACGGCATCGGCGTGACCCAGAACGTGCTGTACGAGAAC
CAGAAGCTGATCGCCAACCAGTTCAACAGCGCCATCGGCAAGATCCAGGACAGCCTGA
GCAGCACCGCCAGCGCCCTGGGCAAGCTGCAGGACGTGGTGAACCACAACGCCCAGG
CCCTGAACACCCTGGTGAAGCAGCTGAGCAGCAAGTTCGGCGCCATCAGCAGCGTGCT
GAACGACATCTTCAGCAGACTGGACAAGGTGGAGGCCGAGGTGCAGATCGACAGACTG
ATCACCGGCAGACTGCAGAGCCTGCAGACCTACGTGACCCAGCAGCTGATCAGAGCCG
CCGAGATCAGAGCCAGCGCCAACCTGGCCGCCACCAAGATGAGCGAGTGCGTGCTGGG
CCAGAGCAAGAGAGTGGACTTCTGCGGCAAGGGCTACCACCTGATGAGCTTCCCCCAG
 
AGCGCCCCCCACGGCGTGGTGTTCCTGCACGTGACCTACGTGCCCGCCCAGGAGAAGA
ACTTCACCACCGCCCCCGCCATCTGCCACGACGGCAAGGCCCACTTCCCCAGAGAGGG
CGTGTTCGTGAGCAACGGCACCCACTGGTTCGTGACCCAGAGAAACTTCTACGAGCCC
CAGATCATCACCACCGACAACACCTTCGTGAGCGGCAACTGCGACGTGGTGATCGGCAT
CGTGAACAACACCGTGTACGACCCCCTGCAGCCCGAGCTGGACAGCTTCAAGGAGGAG
CTGGACAAGTACTTCAAGAACCACACCAGCCCCGACGTGGACCTGGGCGACATCAGCG
GCATCAACGCCAGCGTGGTGAACATCCAGAAGGAGATCGACAGACTGAACGAGGTGGC
CAAGAACCTGAACGAGAGCCTGATCGACCTGCAGGAGCTGGGCAAGTACGAGCAGTAC
ATCAAGTGGCCCTAG
 Note: the above sequence consists of signal peptide - extracellular domain.
**SEQ ID No.63: Extracellular nucleic acid sequence of Omicron-BA.1 S antigen (optimization of insect cells):** 
ATGTTCGTGTTCCTGGTGCTGCTGCCCCTGGTGTCCTCCCAGTGCGTGAACCTGACC
ACCCGTACCCAGCTGCCCCCCGCTTACACCAACTCCTTCACCCGTGGTGTGTACTACCCC
GACAAGGTGTTCCGTTCCTCCGTGCTGCACTCCACCCAGGACCTGTTCCTGCCCTTCTTC
TCCAACGTGACCTGGTTCCACGTGATCTCCGGTACCAACGGTACCAAGCGTTTCGACAA
CCCCGTGCTGCCCTTCAACGACGGTGTGTACTTCGCTTCCATCGAGAAGTCCAACATCAT
CCGTGGTTGGATCTTCGGTACCACCCTGGACTCCAAGACCCAGTCCCTGCTGATCGTGA
ACAACGCTACCAACGTGGTGATCAAGGTGTGCGAGTTCCAGTTCTGCAACGACCCCTTC
CTGGACCACAAGAACAACAAGTCCTGGATGGAGTCCGAGTTCCGTGTGTACTCCTCCGC
TAACAACTGCACCTTCGAGTACGTGTCCCAGCCCTTCCTGATGGACCTGGAGGGTAAGC
AGGGTAACTTCAAGAACCTGCGTGAGTTCGTGTTCAAGAACATCGACGGTTACTTCAAG
ATCTACTCCAAGCACACCCCCATCATCGTGCGTGACCTGCCCCAGGGTTTCTCCGCTCTG
GAGCCCCTGGTGGACCTGCCCATCGGTATCAACATCACCCGTTTCCAGACCCTGCTGGC
TCTGCACCGTTCCTACCTGACCCCCGGTGACTCCTCCTCCGGTTGGACCGCTGGTGCTG
CTGCTTACTACGTGGGTTACCTGCAGCCCCGTACCTTCCTGCTGAAGTACAACGAGAAC
GGTACCATCACCGACGCTGTGGACTGCGCTCTGGACCCCCTGTCCGAGACCAAGTGCAC
CCTGAAGTCCTTCACCGTGGAGAAGGGTATCTACCAGACCTCCAACTTCCGTGTGCAGC
CCACCGAGTCCATCGTGCGTTTCCCCAACATCACCAACCTGTGCCCCTTCGACGAGGTG
TTCAACGCTACCCGTTTCGCTTCCGTGTACGCTTGGAACCGTAAGCGTATCTCCAACTGC
 
GTGGCTGACTACTCCGTGCTGTACAACCTGGCTCCCTTCTTCACCTTCAAGTGCTACGGT
GTGTCCCCCACCAAGCTGAACGACCTGTGCTTCACCAACGTGTACGCTGACTCCTTCGT
GATCCGTGGTGACGAGGTGCGTCAGATCGCTCCCGGTCAGACCGGTAACATCGCTGACT
ACAACTACAAGCTGCCCGACGACTTCACCGGTTGCGTGATCGCTTGGAACTCCAACAAG
CTGGACTCCAAGGTGTCCGGTAACTACAACTACCTGTACCGTCTGTTCCGTAAGTCCAA
CCTGAAGCCCTTCGAGCGTGACATCTCCACCGAGATCTACCAGGCTGGTAACAAGCCCT
GCAACGGTGTGGCTGGTTTCAACTGCTACTTCCCCCTGCGTTCCTACTCCTTCCGTCCCA
CCTACGGTGTGGGTCACCAGCCCTACCGTGTGGTGGTGCTGTCCTTCGAGCTGCTGCAC
GCTCCCGCTACCGTGTGCGGTCCCAAGAAGTCCACCAACCTGGTGAAGAACAAGTGCG
TGAACTTCAACTTCAACGGTCTGAAGGGTACCGGTGTGCTGACCGAGTCCAACAAGAA
GTTCCTGCCCTTCCAGCAGTTCGGTCGTGACATCGCTGACACCACCGACGCTGTGCGTG
ACCCCCAGACCCTGGAGATCCTGGACATCACCCCCTGCTCCTTCGGTGGTGTGTCCGTG
ATCACCCCCGGTACCAACACCTCCAACCAGGTGGCTGTGCTGTACCAGGGTGTGAACTG
CACCGAGGTGCCCGTGGCTATCCACGCTGACCAGCTGACCCCCACCTGGCGTGTGTACT
CCACCGGTTCCAACGTGTTCCAGACCCGTGCTGGTTGCCTGATCGGTGCTGAGTACGTG
AACAACTCCTACGAGTGCGACATCCCCATCGGTGCTGGTATCTGCGCTTCCTACCAGACC
CAGACCAAGTCCCACCGTCGTGCTCGTTCCGTGGCTTCCCAGTCCATCATCGCTTACACC
ATGTCCCTGGGTGCTGAGAACTCCGTGGCTTACTCCAACAACTCCATCGCTATCCCCACC
AACTTCACCATCTCCGTGACCACCGAGATCCTGCCCGTGTCCATGACCAAGACCTCCGT
GGACTGCACCATGTACATCTGCGGTGACTCCACCGAGTGCTCCAACCTGCTGCTGCAGT
ACGGTTCCTTCTGCACCCAGCTGAAGCGTGCTCTGACCGGTATCGCTGTGGAGCAGGAC
AAGAACACCCAGGAGGTGTTCGCTCAGGTGAAGCAGATCTACAAGACCCCCCCCATCA
AGTACTTCGGTGGTTTCAACTTCTCCCAGATCCTGCCCGACCCCTCCAAGCCCTCCAAG
CGTTCCTTCATCGAGGACCTGCTGTTCAACAAGGTGACCCTGGCTGACGCTGGTTTCAT
CAAGCAGTACGGTGACTGCCTGGGTGACATCGCTGCTCGTGACCTGATCTGCGCTCAGA
AGTTCAAGGGTCTGACCGTGCTGCCCCCCCTGCTGACCGACGAGATGATCGCTCAGTAC
ACCTCCGCTCTGCTGGCTGGTACCATCACCTCCGGTTGGACCTTCGGTGCTGGTGCTGCT
CTGCAGATCCCCTTCGCTATGCAGATGGCTTACCGTTTCAACGGTATCGGTGTGACCCAG
AACGTGCTGTACGAGAACCAGAAGCTGATCGCTAACCAGTTCAACTCCGCTATCGGTAA
GATCCAGGACTCCCTGTCCTCCACCGCTTCCGCTCTGGGTAAGCTGCAGGACGTGGTGA
ACCACAACGCTCAGGCTCTGAACACCCTGGTGAAGCAGCTGTCCTCCAAGTTCGGTGCT
 
ATCTCCTCCGTGCTGAACGACATCTTCTCCCGTCTGGACAAGGTGGAGGCTGAGGTGCA
GATCGACCGTCTGATCACCGGTCGTCTGCAGTCCCTGCAGACCTACGTGACCCAGCAGC
TGATCCGTGCTGCTGAGATCCGTGCTTCCGCTAACCTGGCTGCTACCAAGATGTCCGAGT
GCGTGCTGGGTCAGTCCAAGCGTGTGGACTTCTGCGGTAAGGGTTACCACCTGATGTCC
TTCCCCCAGTCCGCTCCCCACGGTGTGGTGTTCCTGCACGTGACCTACGTGCCCGCTCA
GGAGAAGAACTTCACCACCGCTCCCGCTATCTGCCACGACGGTAAGGCTCACTTCCCCC
GTGAGGGTGTGTTCGTGTCCAACGGTACCCACTGGTTCGTGACCCAGCGTAACTTCTAC
GAGCCCCAGATCATCACCACCGACAACACCTTCGTGTCCGGTAACTGCGACGTGGTGAT
CGGTATCGTGAACAACACCGTGTACGACCCCCTGCAGCCCGAGCTGGACTCCTTCAAGG
AGGAGCTGGACAAGTACTTCAAGAACCACACCTCCCCCGACGTGGACCTGGGTGACAT
CTCCGGTATCAACGCTTCCGTGGTGAACATCCAGAAGGAGATCGACCGTCTGAACGAGG
TGGCTAAGAACCTGAACGAGTCCCTGATCGACCTGCAGGAGCTGGGTAAGTACGAGCA
GTACATCAAGTGGCCCTAG
 Note: the above sequence consists of signal peptide - extracellular domain.
**SEQ ID No.64: Full-length amino acid sequence of Omicron-BA.2 S antigen:** 
MFVFLVLLPLVSSQCVNLITRTQSYTNSFTRGVYYPDKVFRSSVLHSTQDLFLPFFSNVT
WFHAIHVSGTNGTKRFDNPVLPFNDGVYFASTEKSNIIRGWIFGTTLDSKTQSLLIVNNATN
VVIKVCEFQFCNDPFLDVYYHKNNKSWMESEFRVYSSANNCTFEYVSQPFLMDLEGKQGN
FKNLREFVFKNIDGYFKIYSKHTPINLGRDLPQGFSALEPLVDLPIGINITRFQTLLALHRSYL
TPGDSSSGWTAGAAAYYVGYLQPRTFLLKYNENGTITDAVDCALDPLSETKCTLKSFTVEK
GIYQTSNFRVQPTESIVRFPNITNLCPFDEVFNATRFASVYAWNRKRISNCVADYSVLYNFAP
FFAFKCYGVSPTKLNDLCFTNVYADSFVIRGNEVSQIAPGQTGNIADYNYKLPDDFTGCVIA
WNSNKLDSKVGGNYNYLYRLFRKSNLKPFERDISTEIYQAGNKPCNGVAGFNCYFPLRSYG
FRPTYGVGHQPYRVVVLSFELLHAPATVCGPKKSTNLVKNKCVNFNFNGLTGTGVLTESNK
KFLPFQQFGRDIADTTDAVRDPQTLEILDITPCSFGGVSVITPGTNTSNQVAVLYQGVNCTEV
PVAIHADQLTPTWRVYSTGSNVFQTRAGCLIGAEYVNNSYECDIPIGAGICASYQTQTKSHR
RARSVASQSIIAYTMSLGAENSVAYSNNSIAIPTNFTISVTTEILPVSMTKTSVDCTMYICGDS
TECSNLLLQYGSFCTQLKRALTGIAVEQDKNTQEVFAQVKQIYKTPPIKYFGGFNFSQILPDP
SKPSKRSFIEDLLFNKVTLADAGFIKQYGDCLGDIAARDLICAQKFNGLTVLPPLLTDEMIAQ
YTSALLAGTITSGWTFGAGAALQIPFAMQMAYRFNGIGVTQNVLYENQKLIANQFNSAIGKI
 
QDSLSSTASALGKLQDVVNHNAQALNTLVKQLSSKFGAISSVLNDILSRLDKVEAEVQIDRL
ITGRLQSLQTYVTQQLIRAAEIRASANLAATKMSECVLGQSKRVDFCGKGYHLMSFPQSAP
HGVVFLHVTYVPAQEKNFTTAPAICHDGKAHFPREGVFVSNGTHWFVTQRNFYEPQIITTD
NTFVSGNCDVVIGIVNNTVYDPLQPELDSFKEELDKYFKNHTSPDVDLGDISGINASVVNIQ
KEIDRLNEVAKNLNESLIDLQELGKYEQYIKWPWYIWLGFIAGLIAIVMVTIMLCCMTSCCS
CLKGCCSCGSCCKFDEDDSEPVLKGVKLHYT
 Note: the above sequence consists of signal peptide - extracellular domain - transmembrane domain - intracellular domain.
**SEQ ID No.65: Full-length nucleic acid sequence of Omicron-BA.2 S antigen (optimization of CHO cells):** 
ATGTTCGTGTTCCTGGTGCTGCTGCCCCTGGTGAGCAGCCAGTGCGTGAACCTGAT
CACCAGAACCCAGAGCTACACCAACAGCTTCACCAGAGGCGTGTACTACCCCGACAAG
GTGTTCAGAAGCAGCGTGCTGCACAGCACCCAGGACCTGTTCCTGCCCTTCTTCAGCAA
CGTGACCTGGTTCCACGCCATCCACGTGAGCGGCACCAACGGCACCAAGAGATTCGAC
AACCCCGTGCTGCCCTTCAACGACGGCGTGTACTTCGCCAGCACCGAGAAGAGCAACA
TCATCAGAGGCTGGATCTTCGGCACCACCCTGGACAGCAAGACCCAGAGCCTGCTGATC
GTGAACAACGCCACCAACGTGGTGATCAAGGTGTGCGAGTTCCAGTTCTGCAACGACC
CCTTCCTGGACGTGTACTACCACAAGAACAACAAGAGCTGGATGGAGAGCGAGTTCAG
AGTGTACAGCAGCGCCAACAACTGCACCTTCGAGTACGTGAGCCAGCCCTTCCTGATGG
ACCTGGAGGGCAAGCAGGGCAACTTCAAGAACCTGAGAGAGTTCGTGTTCAAGAACAT
CGACGGCTACTTCAAGATCTACAGCAAGCACACCCCCATCAACCTGGGCAGAGACCTGC
CCCAGGGCTTCAGCGCCCTGGAGCCCCTGGTGGACCTGCCCATCGGCATCAACATCACC
AGATTCCAGACCCTGCTGGCCCTGCACAGAAGCTACCTGACCCCCGGCGACAGCAGCA
GCGGCTGGACCGCCGGCGCCGCCGCCTACTACGTGGGCTACCTGCAGCCCAGAACCTTC
CTGCTGAAGTACAACGAGAACGGCACCATCACCGACGCCGTGGACTGCGCCCTGGACC
CCCTGAGCGAGACCAAGTGCACCCTGAAGAGCTTCACCGTGGAGAAGGGCATCTACCA
GACCAGCAACTTCAGAGTGCAGCCCACCGAGAGCATCGTGAGATTCCCCAACATCACC
AACCTGTGCCCCTTCGACGAGGTGTTCAACGCCACCAGATTCGCCAGCGTGTACGCCTG
GAACAGAAAGAGAATCAGCAACTGCGTGGCCGACTACAGCGTGCTGTACAACTTCGCC
CCCTTCTTCGCCTTCAAGTGCTACGGCGTGAGCCCCACCAAGCTGAACGACCTGTGCTT
 
CACCAACGTGTACGCCGACAGCTTCGTGATCAGAGGCAACGAGGTGAGCCAGATCGCC
CCCGGCCAGACCGGCAACATCGCCGACTACAACTACAAGCTGCCCGACGACTTCACCG
GCTGCGTGATCGCCTGGAACAGCAACAAGCTGGACAGCAAGGTGGGCGGCAACTACAA
CTACCTGTACAGACTGTTCAGAAAGAGCAACCTGAAGCCCTTCGAGAGAGACATCAGC
ACCGAGATCTACCAGGCCGGCAACAAGCCCTGCAACGGCGTGGCCGGCTTCAACTGCT
ACTTCCCCCTGAGAAGCTACGGCTTCAGACCCACCTACGGCGTGGGCCACCAGCCCTAC
AGAGTGGTGGTGCTGAGCTTCGAGCTGCTGCACGCCCCCGCCACCGTGTGCGGCCCCA
AGAAGAGCACCAACCTGGTGAAGAACAAGTGCGTGAACTTCAACTTCAACGGCCTGAC
CGGCACCGGCGTGCTGACCGAGAGCAACAAGAAGTTCCTGCCCTTCCAGCAGTTCGGC
AGAGACATCGCCGACACCACCGACGCCGTGAGAGACCCCCAGACCCTGGAGATCCTGG
ACATCACCCCCTGCAGCTTCGGCGGCGTGAGCGTGATCACCCCCGGCACCAACACCAG
CAACCAGGTGGCCGTGCTGTACCAGGGCGTGAACTGCACCGAGGTGCCCGTGGCCATC
CACGCCGACCAGCTGACCCCCACCTGGAGAGTGTACAGCACCGGCAGCAACGTGTTCC
AGACCAGAGCCGGCTGCCTGATCGGCGCCGAGTACGTGAACAACAGCTACGAGTGCGA
CATCCCCATCGGCGCCGGCATCTGCGCCAGCTACCAGACCCAGACCAAGAGCCACAGA
AGAGCCAGAAGCGTGGCCAGCCAGAGCATCATCGCCTACACCATGAGCCTGGGCGCCG
AGAACAGCGTGGCCTACAGCAACAACAGCATCGCCATCCCCACCAACTTCACCATCAGC
GTGACCACCGAGATCCTGCCCGTGAGCATGACCAAGACCAGCGTGGACTGCACCATGTA
CATCTGCGGCGACAGCACCGAGTGCAGCAACCTGCTGCTGCAGTACGGCAGCTTCTGC
ACCCAGCTGAAGAGAGCCCTGACCGGCATCGCCGTGGAGCAGGACAAGAACACCCAG
GAGGTGTTCGCCCAGGTGAAGCAGATCTACAAGACCCCCCCCATCAAGTACTTCGGCGG
CTTCAACTTCAGCCAGATCCTGCCCGACCCCAGCAAGCCCAGCAAGAGAAGCTTCATCG
AGGACCTGCTGTTCAACAAGGTGACCCTGGCCGACGCCGGCTTCATCAAGCAGTACGG
CGACTGCCTGGGCGACATCGCCGCCAGAGACCTGATCTGCGCCCAGAAGTTCAACGGC
CTGACCGTGCTGCCCCCCCTGCTGACCGACGAGATGATCGCCCAGTACACCAGCGCCCT
GCTGGCCGGCACCATCACCAGCGGCTGGACCTTCGGCGCCGGCGCCGCCCTGCAGATC
CCCTTCGCCATGCAGATGGCCTACAGATTCAACGGCATCGGCGTGACCCAGAACGTGCT
GTACGAGAACCAGAAGCTGATCGCCAACCAGTTCAACAGCGCCATCGGCAAGATCCAG
GACAGCCTGAGCAGCACCGCCAGCGCCCTGGGCAAGCTGCAGGACGTGGTGAACCAC
AACGCCCAGGCCCTGAACACCCTGGTGAAGCAGCTGAGCAGCAAGTTCGGCGCCATCA
GCAGCGTGCTGAACGACATCCTGAGCAGACTGGACAAGGTGGAGGCCGAGGTGCAGAT
 
CGACAGACTGATCACCGGCAGACTGCAGAGCCTGCAGACCTACGTGACCCAGCAGCTG
ATCAGAGCCGCCGAGATCAGAGCCAGCGCCAACCTGGCCGCCACCAAGATGAGCGAGT
GCGTGCTGGGCCAGAGCAAGAGAGTGGACTTCTGCGGCAAGGGCTACCACCTGATGAG
CTTCCCCCAGAGCGCCCCCCACGGCGTGGTGTTCCTGCACGTGACCTACGTGCCCGCCC
AGGAGAAGAACTTCACCACCGCCCCCGCCATCTGCCACGACGGCAAGGCCCACTTCCC
CAGAGAGGGCGTGTTCGTGAGCAACGGCACCCACTGGTTCGTGACCCAGAGAAACTTC
TACGAGCCCCAGATCATCACCACCGACAACACCTTCGTGAGCGGCAACTGCGACGTGGT
GATCGGCATCGTGAACAACACCGTGTACGACCCCCTGCAGCCCGAGCTGGACAGCTTCA
AGGAGGAGCTGGACAAGTACTTCAAGAACCACACCAGCCCCGACGTGGACCTGGGCG
ACATCAGCGGCATCAACGCCAGCGTGGTGAACATCCAGAAGGAGATCGACAGACTGAA
CGAGGTGGCCAAGAACCTGAACGAGAGCCTGATCGACCTGCAGGAGCTGGGCAAGTAC
GAGCAGTACATCAAGTGGCCCTGGTACATCTGGCTGGGCTTCATCGCCGGCCTGATCGC
CATCGTGATGGTGACCATCATGCTGTGCTGCATGACCAGCTGCTGCAGCTGCCTGAAGG
GCTGCTGCAGCTGCGGCAGCTGCTGCAAGTTCGACGAGGACGACAGCGAGCCCGTGCT
GAAGGGCGTGAAGCTGCACTACACCTAG
 Note: the above sequence consists of signal peptide - extracellular domain - transmembrane domain - intracellular domain.
**SEQ ID No.66: Full-length nucleic acid sequence of Omicron-BA.2 S antigen (optimization of insect cells):** 
ATGTTCGTGTTCCTGGTGCTGCTGCCCCTGGTGTCCTCCCAGTGCGTGAACCTGATC
ACCCGTACCCAGTCCTACACCAACTCCTTCACCCGTGGTGTGTACTACCCCGACAAGGT
GTTCCGTTCCTCCGTGCTGCACTCCACCCAGGACCTGTTCCTGCCCTTCTTCTCCAACGT
GACCTGGTTCCACGCTATCCACGTGTCCGGTACCAACGGTACCAAGCGTTTCGACAACC
CCGTGCTGCCCTTCAACGACGGTGTGTACTTCGCTTCCACCGAGAAGTCCAACATCATC
CGTGGTTGGATCTTCGGTACCACCCTGGACTCCAAGACCCAGTCCCTGCTGATCGTGAA
CAACGCTACCAACGTGGTGATCAAGGTGTGCGAGTTCCAGTTCTGCAACGACCCCTTCC
TGGACGTGTACTACCACAAGAACAACAAGTCCTGGATGGAGTCCGAGTTCCGTGTGTAC
TCCTCCGCTAACAACTGCACCTTCGAGTACGTGTCCCAGCCCTTCCTGATGGACCTGGA
GGGTAAGCAGGGTAACTTCAAGAACCTGCGTGAGTTCGTGTTCAAGAACATCGACGGTT
ACTTCAAGATCTACTCCAAGCACACCCCCATCAACCTGGGTCGTGACCTGCCCCAGGGT
 
TTCTCCGCTCTGGAGCCCCTGGTGGACCTGCCCATCGGTATCAACATCACCCGTTTCCAG
ACCCTGCTGGCTCTGCACCGTTCCTACCTGACCCCCGGTGACTCCTCCTCCGGTTGGAC
CGCTGGTGCTGCTGCTTACTACGTGGGTTACCTGCAGCCCCGTACCTTCCTGCTGAAGTA
CAACGAGAACGGTACCATCACCGACGCTGTGGACTGCGCTCTGGACCCCCTGTCCGAG
ACCAAGTGCACCCTGAAGTCCTTCACCGTGGAGAAGGGTATCTACCAGACCTCCAACTT
CCGTGTGCAGCCCACCGAGTCCATCGTGCGTTTCCCCAACATCACCAACCTGTGCCCCT
TCGACGAGGTGTTCAACGCTACCCGTTTCGCTTCCGTGTACGCTTGGAACCGTAAGCGT
ATCTCCAACTGCGTGGCTGACTACTCCGTGCTGTACAACTTCGCTCCCTTCTTCGCTTTC
AAGTGCTACGGTGTGTCCCCCACCAAGCTGAACGACCTGTGCTTCACCAACGTGTACGC
TGACTCCTTCGTGATCCGTGGTAACGAGGTGTCCCAGATCGCTCCCGGTCAGACCGGTA
ACATCGCTGACTACAACTACAAGCTGCCCGACGACTTCACCGGTTGCGTGATCGCTTGG
AACTCCAACAAGCTGGACTCCAAGGTGGGTGGTAACTACAACTACCTGTACCGTCTGTT
CCGTAAGTCCAACCTGAAGCCCTTCGAGCGTGACATCTCCACCGAGATCTACCAGGCTG
GTAACAAGCCCTGCAACGGTGTGGCTGGTTTCAACTGCTACTTCCCCCTGCGTTCCTAC
GGTTTCCGTCCCACCTACGGTGTGGGTCACCAGCCCTACCGTGTGGTGGTGCTGTCCTT
CGAGCTGCTGCACGCTCCCGCTACCGTGTGCGGTCCCAAGAAGTCCACCAACCTGGTG
AAGAACAAGTGCGTGAACTTCAACTTCAACGGTCTGACCGGTACCGGTGTGCTGACCG
AGTCCAACAAGAAGTTCCTGCCCTTCCAGCAGTTCGGTCGTGACATCGCTGACACCACC
GACGCTGTGCGTGACCCCCAGACCCTGGAGATCCTGGACATCACCCCCTGCTCCTTCGG
TGGTGTGTCCGTGATCACCCCCGGTACCAACACCTCCAACCAGGTGGCTGTGCTGTACC
AGGGTGTGAACTGCACCGAGGTGCCCGTGGCTATCCACGCTGACCAGCTGACCCCCAC
CTGGCGTGTGTACTCCACCGGTTCCAACGTGTTCCAGACCCGTGCTGGTTGCCTGATCG
GTGCTGAGTACGTGAACAACTCCTACGAGTGCGACATCCCCATCGGTGCTGGTATCTGC
GCTTCCTACCAGACCCAGACCAAGTCCCACCGTCGTGCTCGTTCCGTGGCTTCCCAGTC
CATCATCGCTTACACCATGTCCCTGGGTGCTGAGAACTCCGTGGCTTACTCCAACAACTC
CATCGCTATCCCCACCAACTTCACCATCTCCGTGACCACCGAGATCCTGCCCGTGTCCAT
GACCAAGACCTCCGTGGACTGCACCATGTACATCTGCGGTGACTCCACCGAGTGCTCCA
ACCTGCTGCTGCAGTACGGTTCCTTCTGCACCCAGCTGAAGCGTGCTCTGACCGGTATC
GCTGTGGAGCAGGACAAGAACACCCAGGAGGTGTTCGCTCAGGTGAAGCAGATCTACA
AGACCCCCCCCATCAAGTACTTCGGTGGTTTCAACTTCTCCCAGATCCTGCCCGACCCCT
CCAAGCCCTCCAAGCGTTCCTTCATCGAGGACCTGCTGTTCAACAAGGTGACCCTGGCT
 
GACGCTGGTTTCATCAAGCAGTACGGTGACTGCCTGGGTGACATCGCTGCTCGTGACCT
GATCTGCGCTCAGAAGTTCAACGGTCTGACCGTGCTGCCCCCCCTGCTGACCGACGAGA
TGATCGCTCAGTACACCTCCGCTCTGCTGGCTGGTACCATCACCTCCGGTTGGACCTTCG
GTGCTGGTGCTGCTCTGCAGATCCCCTTCGCTATGCAGATGGCTTACCGTTTCAACGGTA
TCGGTGTGACCCAGAACGTGCTGTACGAGAACCAGAAGCTGATCGCTAACCAGTTCAA
CTCCGCTATCGGTAAGATCCAGGACTCCCTGTCCTCCACCGCTTCCGCTCTGGGTAAGCT
GCAGGACGTGGTGAACCACAACGCTCAGGCTCTGAACACCCTGGTGAAGCAGCTGTCC
TCCAAGTTCGGTGCTATCTCCTCCGTGCTGAACGACATCCTGTCCCGTCTGGACAAGGT
GGAGGCTGAGGTGCAGATCGACCGTCTGATCACCGGTCGTCTGCAGTCCCTGCAGACCT
ACGTGACCCAGCAGCTGATCCGTGCTGCTGAGATCCGTGCTTCCGCTAACCTGGCTGCT
ACCAAGATGTCCGAGTGCGTGCTGGGTCAGTCCAAGCGTGTGGACTTCTGCGGTAAGG
GTTACCACCTGATGTCCTTCCCCCAGTCCGCTCCCCACGGTGTGGTGTTCCTGCACGTGA
CCTACGTGCCCGCTCAGGAGAAGAACTTCACCACCGCTCCCGCTATCTGCCACGACGGT
AAGGCTCACTTCCCCCGTGAGGGTGTGTTCGTGTCCAACGGTACCCACTGGTTCGTGAC
CCAGCGTAACTTCTACGAGCCCCAGATCATCACCACCGACAACACCTTCGTGTCCGGTA
ACTGCGACGTGGTGATCGGTATCGTGAACAACACCGTGTACGACCCCCTGCAGCCCGAG
CTGGACTCCTTCAAGGAGGAGCTGGACAAGTACTTCAAGAACCACACCTCCCCCGACG
TGGACCTGGGTGACATCTCCGGTATCAACGCTTCCGTGGTGAACATCCAGAAGGAGATC
GACCGTCTGAACGAGGTGGCTAAGAACCTGAACGAGTCCCTGATCGACCTGCAGGAGC
TGGGTAAGTACGAGCAGTACATCAAGTGGCCCTGGTACATCTGGCTGGGTTTCATCGCT
GGTCTGATCGCTATCGTGATGGTGACCATCATGCTGTGCTGCATGACCTCCTGCTGCTCC
TGCCTGAAGGGTTGCTGCTCCTGCGGTTCCTGCTGCAAGTTCGACGAGGACGACTCCGA
GCCCGTGCTGAAGGGTGTGAAGCTGCACTACACCTAG
 Note: the above sequence consists of signal peptide - extracellular domain - transmembrane domain - intracellular domain.
**SEQ ID No.67: Extracellular amino acid sequence of Omicron-BA.2 S antigen:** 
MFVFLVLLPLVSSQCVNLITRTQSYTNSFTRGVYYPDKVFRSSVLHSTQDLFLPFFSNVT
WFHAIHVSGTNGTKRFDNPVLPFNDGVYFASTEKSNIIRGWIFGTTLDSKTQSLLIVNNATN
VVIKVCEFQFCNDPFLDVYYHKNNKSWMESEFRVYSSANNCTFEYVSQPFLMDLEGKQGN
FKNLREFVFKNIDGYFKIYSKHTPINLGRDLPQGFSALEPLVDLPIGINITRFQTLLALHRSYL
 
TPGDSSSGWTAGAAAYYVGYLQPRTFLLKYNENGTITDAVDCALDPLSETKCTLKSFTVEK
GIYQTSNFRVQPTESIVRFPNITNLCPFDEVFNATRFASVYAWNRKRISNCVADYSVLYNFAP
FFAFKCYGVSPTKLNDLCFTNVYADSFVIRGNEVSQIAPGQTGNIADYNYKLPDDFTGCVIA
WNSNKLDSKVGGNYNYLYRLFRKSNLKPFERDISTEIYQAGNKPCNGVAGFNCYFPLRSYG
FRPTYGVGHQPYRVVVLSFELLHAPATVCGPKKSTNLVKNKCVNFNFNGLTGTGVLTESNK
KFLPFQQFGRDIADTTDAVRDPQTLEILDITPCSFGGVSVITPGTNTSNQVAVLYQGVNCTEV
PVAIHADQLTPTWRVYSTGSNVFQTRAGCLIGAEYVNNSYECDIPIGAGICASYQTQTKSHR
RARSVASQSIIAYTMSLGAENSVAYSNNSIAIPTNFTISVTTEILPVSMTKTSVDCTMYICGDS
TECSNLLLQYGSFCTQLKRALTGIAVEQDKNTQEVFAQVKQIYKTPPIKYFGGFNFSQILPDP
SKPSKRSFIEDLLFNKVTLADAGFIKQYGDCLGDIAARDLICAQKFNGLTVLPPLLTDEMIAQ
YTSALLAGTITSGWTFGAGAALQIPFAMQMAYRFNGIGVTQNVLYENQKLIANQFNSAIGKI
QDSLSSTASALGKLQDVVNHNAQALNTLVKQLSSKFGAISSVLNDILSRLDKVEAEVQIDRL
ITGRLQSLQTYVTQQLIRAAEIRASANLAATKMSECVLGQSKRVDFCGKGYHLMSFPQSAP
HGVVFLHVTYVPAQEKNFTTAPAICHDGKAHFPREGVFVSNGTHWFVTQRNFYEPQIITTD
NTFVSGNCDVVIGIVNNTVYDPLQPELDSFKEELDKYFKNHTSPDVDLGDISGINASVVNIQ
KEIDRLNEVAKNLNESLIDLQELGKYEQYIKWP
 Note: the above sequence consists of signal peptide - extracellular domain.
**SEQ ID No.68: Extracellular nucleic acid sequence of Omicron-BA.2 S antigen (optimization of CHO cells):** 
ATGTTCGTGTTCCTGGTGCTGCTGCCCCTGGTGAGCAGCCAGTGCGTGAACCTGAT
CACCAGAACCCAGAGCTACACCAACAGCTTCACCAGAGGCGTGTACTACCCCGACAAG
GTGTTCAGAAGCAGCGTGCTGCACAGCACCCAGGACCTGTTCCTGCCCTTCTTCAGCAA
CGTGACCTGGTTCCACGCCATCCACGTGAGCGGCACCAACGGCACCAAGAGATTCGAC
AACCCCGTGCTGCCCTTCAACGACGGCGTGTACTTCGCCAGCACCGAGAAGAGCAACA
TCATCAGAGGCTGGATCTTCGGCACCACCCTGGACAGCAAGACCCAGAGCCTGCTGATC
GTGAACAACGCCACCAACGTGGTGATCAAGGTGTGCGAGTTCCAGTTCTGCAACGACC
CCTTCCTGGACGTGTACTACCACAAGAACAACAAGAGCTGGATGGAGAGCGAGTTCAG
AGTGTACAGCAGCGCCAACAACTGCACCTTCGAGTACGTGAGCCAGCCCTTCCTGATGG
ACCTGGAGGGCAAGCAGGGCAACTTCAAGAACCTGAGAGAGTTCGTGTTCAAGAACAT
CGACGGCTACTTCAAGATCTACAGCAAGCACACCCCCATCAACCTGGGCAGAGACCTGC
 
CCCAGGGCTTCAGCGCCCTGGAGCCCCTGGTGGACCTGCCCATCGGCATCAACATCACC
AGATTCCAGACCCTGCTGGCCCTGCACAGAAGCTACCTGACCCCCGGCGACAGCAGCA
GCGGCTGGACCGCCGGCGCCGCCGCCTACTACGTGGGCTACCTGCAGCCCAGAACCTTC
CTGCTGAAGTACAACGAGAACGGCACCATCACCGACGCCGTGGACTGCGCCCTGGACC
CCCTGAGCGAGACCAAGTGCACCCTGAAGAGCTTCACCGTGGAGAAGGGCATCTACCA
GACCAGCAACTTCAGAGTGCAGCCCACCGAGAGCATCGTGAGATTCCCCAACATCACC
AACCTGTGCCCCTTCGACGAGGTGTTCAACGCCACCAGATTCGCCAGCGTGTACGCCTG
GAACAGAAAGAGAATCAGCAACTGCGTGGCCGACTACAGCGTGCTGTACAACTTCGCC
CCCTTCTTCGCCTTCAAGTGCTACGGCGTGAGCCCCACCAAGCTGAACGACCTGTGCTT
CACCAACGTGTACGCCGACAGCTTCGTGATCAGAGGCAACGAGGTGAGCCAGATCGCC
CCCGGCCAGACCGGCAACATCGCCGACTACAACTACAAGCTGCCCGACGACTTCACCG
GCTGCGTGATCGCCTGGAACAGCAACAAGCTGGACAGCAAGGTGGGCGGCAACTACAA
CTACCTGTACAGACTGTTCAGAAAGAGCAACCTGAAGCCCTTCGAGAGAGACATCAGC
ACCGAGATCTACCAGGCCGGCAACAAGCCCTGCAACGGCGTGGCCGGCTTCAACTGCT
ACTTCCCCCTGAGAAGCTACGGCTTCAGACCCACCTACGGCGTGGGCCACCAGCCCTAC
AGAGTGGTGGTGCTGAGCTTCGAGCTGCTGCACGCCCCCGCCACCGTGTGCGGCCCCA
AGAAGAGCACCAACCTGGTGAAGAACAAGTGCGTGAACTTCAACTTCAACGGCCTGAC
CGGCACCGGCGTGCTGACCGAGAGCAACAAGAAGTTCCTGCCCTTCCAGCAGTTCGGC
AGAGACATCGCCGACACCACCGACGCCGTGAGAGACCCCCAGACCCTGGAGATCCTGG
ACATCACCCCCTGCAGCTTCGGCGGCGTGAGCGTGATCACCCCCGGCACCAACACCAG
CAACCAGGTGGCCGTGCTGTACCAGGGCGTGAACTGCACCGAGGTGCCCGTGGCCATC
CACGCCGACCAGCTGACCCCCACCTGGAGAGTGTACAGCACCGGCAGCAACGTGTTCC
AGACCAGAGCCGGCTGCCTGATCGGCGCCGAGTACGTGAACAACAGCTACGAGTGCGA
CATCCCCATCGGCGCCGGCATCTGCGCCAGCTACCAGACCCAGACCAAGAGCCACAGA
AGAGCCAGAAGCGTGGCCAGCCAGAGCATCATCGCCTACACCATGAGCCTGGGCGCCG
AGAACAGCGTGGCCTACAGCAACAACAGCATCGCCATCCCCACCAACTTCACCATCAGC
GTGACCACCGAGATCCTGCCCGTGAGCATGACCAAGACCAGCGTGGACTGCACCATGTA
CATCTGCGGCGACAGCACCGAGTGCAGCAACCTGCTGCTGCAGTACGGCAGCTTCTGC
ACCCAGCTGAAGAGAGCCCTGACCGGCATCGCCGTGGAGCAGGACAAGAACACCCAG
GAGGTGTTCGCCCAGGTGAAGCAGATCTACAAGACCCCCCCCATCAAGTACTTCGGCGG
CTTCAACTTCAGCCAGATCCTGCCCGACCCCAGCAAGCCCAGCAAGAGAAGCTTCATCG
 
AGGACCTGCTGTTCAACAAGGTGACCCTGGCCGACGCCGGCTTCATCAAGCAGTACGG
CGACTGCCTGGGCGACATCGCCGCCAGAGACCTGATCTGCGCCCAGAAGTTCAACGGC
CTGACCGTGCTGCCCCCCCTGCTGACCGACGAGATGATCGCCCAGTACACCAGCGCCCT
GCTGGCCGGCACCATCACCAGCGGCTGGACCTTCGGCGCCGGCGCCGCCCTGCAGATC
CCCTTCGCCATGCAGATGGCCTACAGATTCAACGGCATCGGCGTGACCCAGAACGTGCT
GTACGAGAACCAGAAGCTGATCGCCAACCAGTTCAACAGCGCCATCGGCAAGATCCAG
GACAGCCTGAGCAGCACCGCCAGCGCCCTGGGCAAGCTGCAGGACGTGGTGAACCAC
AACGCCCAGGCCCTGAACACCCTGGTGAAGCAGCTGAGCAGCAAGTTCGGCGCCATCA
GCAGCGTGCTGAACGACATCCTGAGCAGACTGGACAAGGTGGAGGCCGAGGTGCAGAT
CGACAGACTGATCACCGGCAGACTGCAGAGCCTGCAGACCTACGTGACCCAGCAGCTG
ATCAGAGCCGCCGAGATCAGAGCCAGCGCCAACCTGGCCGCCACCAAGATGAGCGAGT
GCGTGCTGGGCCAGAGCAAGAGAGTGGACTTCTGCGGCAAGGGCTACCACCTGATGAG
CTTCCCCCAGAGCGCCCCCCACGGCGTGGTGTTCCTGCACGTGACCTACGTGCCCGCCC
AGGAGAAGAACTTCACCACCGCCCCCGCCATCTGCCACGACGGCAAGGCCCACTTCCC
CAGAGAGGGCGTGTTCGTGAGCAACGGCACCCACTGGTTCGTGACCCAGAGAAACTTC
TACGAGCCCCAGATCATCACCACCGACAACACCTTCGTGAGCGGCAACTGCGACGTGGT
GATCGGCATCGTGAACAACACCGTGTACGACCCCCTGCAGCCCGAGCTGGACAGCTTCA
AGGAGGAGCTGGACAAGTACTTCAAGAACCACACCAGCCCCGACGTGGACCTGGGCG
ACATCAGCGGCATCAACGCCAGCGTGGTGAACATCCAGAAGGAGATCGACAGACTGAA
CGAGGTGGCCAAGAACCTGAACGAGAGCCTGATCGACCTGCAGGAGCTGGGCAAGTAC
GAGCAGTACATCAAGTGGCCCTAG
 Note: the above sequence consists of signal peptide - extracellular domain.
**SEQ ID No.69: Extracellular nucleic acid sequence of Omicron-BA.2 S antigen (optimization of insect cells):** 
ATGTTCGTGTTCCTGGTGCTGCTGCCCCTGGTGTCCTCCCAGTGCGTGAACCTGATC
ACCCGTACCCAGTCCTACACCAACTCCTTCACCCGTGGTGTGTACTACCCCGACAAGGT
GTTCCGTTCCTCCGTGCTGCACTCCACCCAGGACCTGTTCCTGCCCTTCTTCTCCAACGT
GACCTGGTTCCACGCTATCCACGTGTCCGGTACCAACGGTACCAAGCGTTTCGACAACC
CCGTGCTGCCCTTCAACGACGGTGTGTACTTCGCTTCCACCGAGAAGTCCAACATCATC
CGTGGTTGGATCTTCGGTACCACCCTGGACTCCAAGACCCAGTCCCTGCTGATCGTGAA
 
CAACGCTACCAACGTGGTGATCAAGGTGTGCGAGTTCCAGTTCTGCAACGACCCCTTCC
TGGACGTGTACTACCACAAGAACAACAAGTCCTGGATGGAGTCCGAGTTCCGTGTGTAC
TCCTCCGCTAACAACTGCACCTTCGAGTACGTGTCCCAGCCCTTCCTGATGGACCTGGA
GGGTAAGCAGGGTAACTTCAAGAACCTGCGTGAGTTCGTGTTCAAGAACATCGACGGTT
ACTTCAAGATCTACTCCAAGCACACCCCCATCAACCTGGGTCGTGACCTGCCCCAGGGT
TTCTCCGCTCTGGAGCCCCTGGTGGACCTGCCCATCGGTATCAACATCACCCGTTTCCAG
ACCCTGCTGGCTCTGCACCGTTCCTACCTGACCCCCGGTGACTCCTCCTCCGGTTGGAC
CGCTGGTGCTGCTGCTTACTACGTGGGTTACCTGCAGCCCCGTACCTTCCTGCTGAAGTA
CAACGAGAACGGTACCATCACCGACGCTGTGGACTGCGCTCTGGACCCCCTGTCCGAG
ACCAAGTGCACCCTGAAGTCCTTCACCGTGGAGAAGGGTATCTACCAGACCTCCAACTT
CCGTGTGCAGCCCACCGAGTCCATCGTGCGTTTCCCCAACATCACCAACCTGTGCCCCT
TCGACGAGGTGTTCAACGCTACCCGTTTCGCTTCCGTGTACGCTTGGAACCGTAAGCGT
ATCTCCAACTGCGTGGCTGACTACTCCGTGCTGTACAACTTCGCTCCCTTCTTCGCTTTC
AAGTGCTACGGTGTGTCCCCCACCAAGCTGAACGACCTGTGCTTCACCAACGTGTACGC
TGACTCCTTCGTGATCCGTGGTAACGAGGTGTCCCAGATCGCTCCCGGTCAGACCGGTA
ACATCGCTGACTACAACTACAAGCTGCCCGACGACTTCACCGGTTGCGTGATCGCTTGG
AACTCCAACAAGCTGGACTCCAAGGTGGGTGGTAACTACAACTACCTGTACCGTCTGTT
CCGTAAGTCCAACCTGAAGCCCTTCGAGCGTGACATCTCCACCGAGATCTACCAGGCTG
GTAACAAGCCCTGCAACGGTGTGGCTGGTTTCAACTGCTACTTCCCCCTGCGTTCCTAC
GGTTTCCGTCCCACCTACGGTGTGGGTCACCAGCCCTACCGTGTGGTGGTGCTGTCCTT
CGAGCTGCTGCACGCTCCCGCTACCGTGTGCGGTCCCAAGAAGTCCACCAACCTGGTG
AAGAACAAGTGCGTGAACTTCAACTTCAACGGTCTGACCGGTACCGGTGTGCTGACCG
AGTCCAACAAGAAGTTCCTGCCCTTCCAGCAGTTCGGTCGTGACATCGCTGACACCACC
GACGCTGTGCGTGACCCCCAGACCCTGGAGATCCTGGACATCACCCCCTGCTCCTTCGG
TGGTGTGTCCGTGATCACCCCCGGTACCAACACCTCCAACCAGGTGGCTGTGCTGTACC
AGGGTGTGAACTGCACCGAGGTGCCCGTGGCTATCCACGCTGACCAGCTGACCCCCAC
CTGGCGTGTGTACTCCACCGGTTCCAACGTGTTCCAGACCCGTGCTGGTTGCCTGATCG
GTGCTGAGTACGTGAACAACTCCTACGAGTGCGACATCCCCATCGGTGCTGGTATCTGC
GCTTCCTACCAGACCCAGACCAAGTCCCACCGTCGTGCTCGTTCCGTGGCTTCCCAGTC
CATCATCGCTTACACCATGTCCCTGGGTGCTGAGAACTCCGTGGCTTACTCCAACAACTC
CATCGCTATCCCCACCAACTTCACCATCTCCGTGACCACCGAGATCCTGCCCGTGTCCAT
 
GACCAAGACCTCCGTGGACTGCACCATGTACATCTGCGGTGACTCCACCGAGTGCTCCA
ACCTGCTGCTGCAGTACGGTTCCTTCTGCACCCAGCTGAAGCGTGCTCTGACCGGTATC
GCTGTGGAGCAGGACAAGAACACCCAGGAGGTGTTCGCTCAGGTGAAGCAGATCTACA
AGACCCCCCCCATCAAGTACTTCGGTGGTTTCAACTTCTCCCAGATCCTGCCCGACCCCT
CCAAGCCCTCCAAGCGTTCCTTCATCGAGGACCTGCTGTTCAACAAGGTGACCCTGGCT
GACGCTGGTTTCATCAAGCAGTACGGTGACTGCCTGGGTGACATCGCTGCTCGTGACCT
GATCTGCGCTCAGAAGTTCAACGGTCTGACCGTGCTGCCCCCCCTGCTGACCGACGAGA
TGATCGCTCAGTACACCTCCGCTCTGCTGGCTGGTACCATCACCTCCGGTTGGACCTTCG
GTGCTGGTGCTGCTCTGCAGATCCCCTTCGCTATGCAGATGGCTTACCGTTTCAACGGTA
TCGGTGTGACCCAGAACGTGCTGTACGAGAACCAGAAGCTGATCGCTAACCAGTTCAA
CTCCGCTATCGGTAAGATCCAGGACTCCCTGTCCTCCACCGCTTCCGCTCTGGGTAAGCT
GCAGGACGTGGTGAACCACAACGCTCAGGCTCTGAACACCCTGGTGAAGCAGCTGTCC
TCCAAGTTCGGTGCTATCTCCTCCGTGCTGAACGACATCCTGTCCCGTCTGGACAAGGT
GGAGGCTGAGGTGCAGATCGACCGTCTGATCACCGGTCGTCTGCAGTCCCTGCAGACCT
ACGTGACCCAGCAGCTGATCCGTGCTGCTGAGATCCGTGCTTCCGCTAACCTGGCTGCT
ACCAAGATGTCCGAGTGCGTGCTGGGTCAGTCCAAGCGTGTGGACTTCTGCGGTAAGG
GTTACCACCTGATGTCCTTCCCCCAGTCCGCTCCCCACGGTGTGGTGTTCCTGCACGTGA
CCTACGTGCCCGCTCAGGAGAAGAACTTCACCACCGCTCCCGCTATCTGCCACGACGGT
AAGGCTCACTTCCCCCGTGAGGGTGTGTTCGTGTCCAACGGTACCCACTGGTTCGTGAC
CCAGCGTAACTTCTACGAGCCCCAGATCATCACCACCGACAACACCTTCGTGTCCGGTA
ACTGCGACGTGGTGATCGGTATCGTGAACAACACCGTGTACGACCCCCTGCAGCCCGAG
CTGGACTCCTTCAAGGAGGAGCTGGACAAGTACTTCAAGAACCACACCTCCCCCGACG
TGGACCTGGGTGACATCTCCGGTATCAACGCTTCCGTGGTGAACATCCAGAAGGAGATC
GACCGTCTGAACGAGGTGGCTAAGAACCTGAACGAGTCCCTGATCGACCTGCAGGAGC
TGGGTAAGTACGAGCAGTACATCAAGTGGCCCTAG
 Note: the above sequence consists of signal peptide - extracellular domain.
**SEQ ID No.70: Full-length amino acid sequence of Omicron-BA.4/5 S antigen:** 
MFVFLVLLPLVSSQCVNLITRTQSYTNSFTRGVYYPDKVFRSSVLHSTQDLFLPFFSNVT
WFHAISGTNGTKRFDNPVLPFNDGVYFASTEKSNIIRGWIFGTTLDSKTQSLLIVNNATNVVI
KVCEFQFCNDPFLDVYYHKNNKSWMESEFRVYSSANNCTFEYVSQPFLMDLEGKQGNFK
 
NLREFVFKNIDGYFKIYSKHTPINLGRDLPQGFSALEPLVDLPIGINITRFQTLLALHRSYLTP
GDSSSGWTAGAAAYYVGYLQPRTFLLKYNENGTITDAVDCALDPLSETKCTLKSFTVEKGI
YQTSNFRVQPTESIVRFPNITNLCPFDEVFNATRFASVYAWNRKRISNCVADYSVLYNFAPFF
AFKCYGVSPTKLNDLCFTNVYADSFVIRGNEVSQIAPGQTGNIADYNYKLPDDFTGCVIAW
NSNKLDSKVGGNYNYRYRLFRKSNLKPFERDISTEIYQAGNKPCNGVAGVNCYFPLQSYGF
RPTYGVGHQPYRVVVLSFELLHAPATVCGPKKSTNLVKNKCVNFNFNGLTGTGVLTESNKK
FLPFQQFGRDIADTTDAVRDPQTLEILDITPCSFGGVSVITPGTNTSNQVAVLYQGVNCTEVP
VAIHADQLTPTWRVYSTGSNVFQTRAGCLIGAEYVNNSYECDIPIGAGICASYQTQTKSHRR
ARSVASQSIIAYTMSLGAENSVAYSNNSIAIPTNFTISVTTEILPVSMTKTSVDCTMYICGDST
ECSNLLLQYGSFCTQLKRALTGIAVEQDKNTQEVFAQVKQIYKTPPIKYFGGFNFSQILPDPS
KPSKRSFIEDLLFNKVTLADAGFIKQYGDCLGDIAARDLICAQKFNGLTVLPPLLTDEMIAQ
YTSALLAGTITSGWTFGAGAALQIPFAMQMAYRFNGIGVTQNVLYENQKLIANQFNSAIGKI
QDSLSSTASALGKLQDVVNHNAQALNTLVKQLSSKFGAISSVLNDILSRLDKVEAEVQIDRL
ITGRLQSLQTYVTQQLIRAAEIRASANLAATKMSECVLGQSKRVDFCGKGYHLMSFPQSAP
HGVVFLHVTYVPAQEKNFTTAPAICHDGKAHFPREGVFVSNGTHWFVTQRNFYEPQIITTD
NTFVSGNCDVVIGIVNNTVYDPLQPELDSFKEELDKYFKNHTSPDVDLGDISGINASVVNIQ
KEIDRLNEVAKNLNESLIDLQELGKYEQYIKWPWYIWLGFIAGLIAIVMVTIMLCCMTSCCS
CLKGCCSCGSCCKFDEDDSEPVLKGVKLHYT
 Note: the above sequence consists of signal peptide - extracellular domain - transmembrane domain - intracellular domain.
**SEQ ID No.71: Full-length nucleic acid sequence of Omicron-BA.4/5 S antigen (optimization of CHO cells):** 
ATGTTCGTGTTCCTGGTGCTGCTGCCCCTGGTGAGCAGCCAGTGCGTGAACCTGAT
CACCAGAACCCAGAGCTACACCAACAGCTTCACCAGAGGCGTGTACTACCCCGACAAG
GTGTTCAGAAGCAGCGTGCTGCACAGCACCCAGGACCTGTTCCTGCCCTTCTTCAGCAA
CGTGACCTGGTTCCACGCCATCAGCGGCACCAACGGCACCAAGAGATTCGACAACCCC
GTGCTGCCCTTCAACGACGGCGTGTACTTCGCCAGCACCGAGAAGAGCAACATCATCAG
AGGCTGGATCTTCGGCACCACCCTGGACAGCAAGACCCAGAGCCTGCTGATCGTGAAC
AACGCCACCAACGTGGTGATCAAGGTGTGCGAGTTCCAGTTCTGCAACGACCCCTTCCT
GGACGTGTACTACCACAAGAACAACAAGAGCTGGATGGAGAGCGAGTTCAGAGTGTAC
 
AGCAGCGCCAACAACTGCACCTTCGAGTACGTGAGCCAGCCCTTCCTGATGGACCTGG
AGGGCAAGCAGGGCAACTTCAAGAACCTGAGAGAGTTCGTGTTCAAGAACATCGACGG
CTACTTCAAGATCTACAGCAAGCACACCCCCATCAACCTGGGCAGAGACCTGCCCCAGG
GCTTCAGCGCCCTGGAGCCCCTGGTGGACCTGCCCATCGGCATCAACATCACCAGATTC
CAGACCCTGCTGGCCCTGCACAGAAGCTACCTGACCCCCGGCGACAGCAGCAGCGGCT
GGACCGCCGGCGCCGCCGCCTACTACGTGGGCTACCTGCAGCCCAGAACCTTCCTGCTG
AAGTACAACGAGAACGGCACCATCACCGACGCCGTGGACTGCGCCCTGGACCCCCTGA
GCGAGACCAAGTGCACCCTGAAGAGCTTCACCGTGGAGAAGGGCATCTACCAGACCAG
CAACTTCAGAGTGCAGCCCACCGAGAGCATCGTGAGATTCCCCAACATCACCAACCTGT
GCCCCTTCGACGAGGTGTTCAACGCCACCAGATTCGCCAGCGTGTACGCCTGGAACAG
AAAGAGAATCAGCAACTGCGTGGCCGACTACAGCGTGCTGTACAACTTCGCCCCCTTCT
TCGCCTTCAAGTGCTACGGCGTGAGCCCCACCAAGCTGAACGACCTGTGCTTCACCAAC
GTGTACGCCGACAGCTTCGTGATCAGAGGCAACGAGGTGAGCCAGATCGCCCCCGGCC
AGACCGGCAACATCGCCGACTACAACTACAAGCTGCCCGACGACTTCACCGGCTGCGT
GATCGCCTGGAACAGCAACAAGCTGGACAGCAAGGTGGGCGGCAACTACAACTACAGA
TACAGACTGTTCAGAAAGAGCAACCTGAAGCCCTTCGAGAGAGACATCAGCACCGAGA
TCTACCAGGCCGGCAACAAGCCCTGCAACGGCGTGGCCGGCGTGAACTGCTACTTCCC
CCTGCAGAGCTACGGCTTCAGACCCACCTACGGCGTGGGCCACCAGCCCTACAGAGTG
GTGGTGCTGAGCTTCGAGCTGCTGCACGCCCCCGCCACCGTGTGCGGCCCCAAGAAGA
GCACCAACCTGGTGAAGAACAAGTGCGTGAACTTCAACTTCAACGGCCTGACCGGCAC
CGGCGTGCTGACCGAGAGCAACAAGAAGTTCCTGCCCTTCCAGCAGTTCGGCAGAGAC
ATCGCCGACACCACCGACGCCGTGAGAGACCCCCAGACCCTGGAGATCCTGGACATCA
CCCCCTGCAGCTTCGGCGGCGTGAGCGTGATCACCCCCGGCACCAACACCAGCAACCA
GGTGGCCGTGCTGTACCAGGGCGTGAACTGCACCGAGGTGCCCGTGGCCATCCACGCC
GACCAGCTGACCCCCACCTGGAGAGTGTACAGCACCGGCAGCAACGTGTTCCAGACCA
GAGCCGGCTGCCTGATCGGCGCCGAGTACGTGAACAACAGCTACGAGTGCGACATCCC
CATCGGCGCCGGCATCTGCGCCAGCTACCAGACCCAGACCAAGAGCCACAGAAGAGCC
AGAAGCGTGGCCAGCCAGAGCATCATCGCCTACACCATGAGCCTGGGCGCCGAGAACA
GCGTGGCCTACAGCAACAACAGCATCGCCATCCCCACCAACTTCACCATCAGCGTGACC
ACCGAGATCCTGCCCGTGAGCATGACCAAGACCAGCGTGGACTGCACCATGTACATCTG
CGGCGACAGCACCGAGTGCAGCAACCTGCTGCTGCAGTACGGCAGCTTCTGCACCCAG
 
CTGAAGAGAGCCCTGACCGGCATCGCCGTGGAGCAGGACAAGAACACCCAGGAGGTG
TTCGCCCAGGTGAAGCAGATCTACAAGACCCCCCCCATCAAGTACTTCGGCGGCTTCAA
CTTCAGCCAGATCCTGCCCGACCCCAGCAAGCCCAGCAAGAGAAGCTTCATCGAGGAC
CTGCTGTTCAACAAGGTGACCCTGGCCGACGCCGGCTTCATCAAGCAGTACGGCGACTG
CCTGGGCGACATCGCCGCCAGAGACCTGATCTGCGCCCAGAAGTTCAACGGCCTGACC
GTGCTGCCCCCCCTGCTGACCGACGAGATGATCGCCCAGTACACCAGCGCCCTGCTGGC
CGGCACCATCACCAGCGGCTGGACCTTCGGCGCCGGCGCCGCCCTGCAGATCCCCTTCG
CCATGCAGATGGCCTACAGATTCAACGGCATCGGCGTGACCCAGAACGTGCTGTACGAG
AACCAGAAGCTGATCGCCAACCAGTTCAACAGCGCCATCGGCAAGATCCAGGACAGCC
TGAGCAGCACCGCCAGCGCCCTGGGCAAGCTGCAGGACGTGGTGAACCACAACGCCC
AGGCCCTGAACACCCTGGTGAAGCAGCTGAGCAGCAAGTTCGGCGCCATCAGCAGCGT
GCTGAACGACATCCTGAGCAGACTGGACAAGGTGGAGGCCGAGGTGCAGATCGACAG
ACTGATCACCGGCAGACTGCAGAGCCTGCAGACCTACGTGACCCAGCAGCTGATCAGA
GCCGCCGAGATCAGAGCCAGCGCCAACCTGGCCGCCACCAAGATGAGCGAGTGCGTGC
TGGGCCAGAGCAAGAGAGTGGACTTCTGCGGCAAGGGCTACCACCTGATGAGCTTCCC
CCAGAGCGCCCCCCACGGCGTGGTGTTCCTGCACGTGACCTACGTGCCCGCCCAGGAG
AAGAACTTCACCACCGCCCCCGCCATCTGCCACGACGGCAAGGCCCACTTCCCCAGAG
AGGGCGTGTTCGTGAGCAACGGCACCCACTGGTTCGTGACCCAGAGAAACTTCTACGA
GCCCCAGATCATCACCACCGACAACACCTTCGTGAGCGGCAACTGCGACGTGGTGATCG
GCATCGTGAACAACACCGTGTACGACCCCCTGCAGCCCGAGCTGGACAGCTTCAAGGA
GGAGCTGGACAAGTACTTCAAGAACCACACCAGCCCCGACGTGGACCTGGGCGACATC
AGCGGCATCAACGCCAGCGTGGTGAACATCCAGAAGGAGATCGACAGACTGAACGAGG
TGGCCAAGAACCTGAACGAGAGCCTGATCGACCTGCAGGAGCTGGGCAAGTACGAGCA
GTACATCAAGTGGCCCTGGTACATCTGGCTGGGCTTCATCGCCGGCCTGATCGCCATCGT
GATGGTGACCATCATGCTGTGCTGCATGACCAGCTGCTGCAGCTGCCTGAAGGGCTGCT
GCAGCTGCGGCAGCTGCTGCAAGTTCGACGAGGACGACAGCGAGCCCGTGCTGAAGG
GCGTGAAGCTGCACTACACCTAG
 Note: the above sequence consists of signal peptide - extracellular domain - transmembrane domain - intracellular domain.
**SEQ ID No.72: Full-length nucleic acid sequence of Omicron-BA.4/5 S antigen (optimization of insect cells):** 
ATGTTCGTGTTCCTGGTGCTGCTGCCCCTGGTGTCCTCCCAGTGCGTGAACCTGATC
ACCCGTACCCAGTCCTACACCAACTCCTTCACCCGTGGTGTGTACTACCCCGACAAGGT
GTTCCGTTCCTCCGTGCTGCACTCCACCCAGGACCTGTTCCTGCCCTTCTTCTCCAACGT
GACCTGGTTCCACGCTATCTCCGGTACCAACGGTACCAAGCGTTTCGACAACCCCGTGC
TGCCCTTCAACGACGGTGTGTACTTCGCTTCCACCGAGAAGTCCAACATCATCCGTGGT
TGGATCTTCGGTACCACCCTGGACTCCAAGACCCAGTCCCTGCTGATCGTGAACAACGC
TACCAACGTGGTGATCAAGGTGTGCGAGTTCCAGTTCTGCAACGACCCCTTCCTGGACG
TGTACTACCACAAGAACAACAAGTCCTGGATGGAGTCCGAGTTCCGTGTGTACTCCTCC
GCTAACAACTGCACCTTCGAGTACGTGTCCCAGCCCTTCCTGATGGACCTGGAGGGTAA
GCAGGGTAACTTCAAGAACCTGCGTGAGTTCGTGTTCAAGAACATCGACGGTTACTTCA
AGATCTACTCCAAGCACACCCCCATCAACCTGGGTCGTGACCTGCCCCAGGGTTTCTCC
GCTCTGGAGCCCCTGGTGGACCTGCCCATCGGTATCAACATCACCCGTTTCCAGACCCT
GCTGGCTCTGCACCGTTCCTACCTGACCCCCGGTGACTCCTCCTCCGGTTGGACCGCTG
GTGCTGCTGCTTACTACGTGGGTTACCTGCAGCCCCGTACCTTCCTGCTGAAGTACAACG
AGAACGGTACCATCACCGACGCTGTGGACTGCGCTCTGGACCCCCTGTCCGAGACCAA
GTGCACCCTGAAGTCCTTCACCGTGGAGAAGGGTATCTACCAGACCTCCAACTTCCGTG
TGCAGCCCACCGAGTCCATCGTGCGTTTCCCCAACATCACCAACCTGTGCCCCTTCGAC
GAGGTGTTCAACGCTACCCGTTTCGCTTCCGTGTACGCTTGGAACCGTAAGCGTATCTCC
AACTGCGTGGCTGACTACTCCGTGCTGTACAACTTCGCTCCCTTCTTCGCTTTCAAGTGC
TACGGTGTGTCCCCCACCAAGCTGAACGACCTGTGCTTCACCAACGTGTACGCTGACTC
CTTCGTGATCCGTGGTAACGAGGTGTCCCAGATCGCTCCCGGTCAGACCGGTAACATCG
CTGACTACAACTACAAGCTGCCCGACGACTTCACCGGTTGCGTGATCGCTTGGAACTCC
AACAAGCTGGACTCCAAGGTGGGTGGTAACTACAACTACCGTTACCGTCTGTTCCGTAA
GTCCAACCTGAAGCCCTTCGAGCGTGACATCTCCACCGAGATCTACCAGGCTGGTAACA
AGCCCTGCAACGGTGTGGCTGGTGTGAACTGCTACTTCCCCCTGCAGTCCTACGGTTTC
CGTCCCACCTACGGTGTGGGTCACCAGCCCTACCGTGTGGTGGTGCTGTCCTTCGAGCT
GCTGCACGCTCCCGCTACCGTGTGCGGTCCCAAGAAGTCCACCAACCTGGTGAAGAAC
AAGTGCGTGAACTTCAACTTCAACGGTCTGACCGGTACCGGTGTGCTGACCGAGTCCA
ACAAGAAGTTCCTGCCCTTCCAGCAGTTCGGTCGTGACATCGCTGACACCACCGACGCT
GTGCGTGACCCCCAGACCCTGGAGATCCTGGACATCACCCCCTGCTCCTTCGGTGGTGT
 
GTCCGTGATCACCCCCGGTACCAACACCTCCAACCAGGTGGCTGTGCTGTACCAGGGTG
TGAACTGCACCGAGGTGCCCGTGGCTATCCACGCTGACCAGCTGACCCCCACCTGGCGT
GTGTACTCCACCGGTTCCAACGTGTTCCAGACCCGTGCTGGTTGCCTGATCGGTGCTGA
GTACGTGAACAACTCCTACGAGTGCGACATCCCCATCGGTGCTGGTATCTGCGCTTCCTA
CCAGACCCAGACCAAGTCCCACCGTCGTGCTCGTTCCGTGGCTTCCCAGTCCATCATCG
CTTACACCATGTCCCTGGGTGCTGAGAACTCCGTGGCTTACTCCAACAACTCCATCGCTA
TCCCCACCAACTTCACCATCTCCGTGACCACCGAGATCCTGCCCGTGTCCATGACCAAG
ACCTCCGTGGACTGCACCATGTACATCTGCGGTGACTCCACCGAGTGCTCCAACCTGCT
GCTGCAGTACGGTTCCTTCTGCACCCAGCTGAAGCGTGCTCTGACCGGTATCGCTGTGG
AGCAGGACAAGAACACCCAGGAGGTGTTCGCTCAGGTGAAGCAGATCTACAAGACCCC
CCCCATCAAGTACTTCGGTGGTTTCAACTTCTCCCAGATCCTGCCCGACCCCTCCAAGCC
CTCCAAGCGTTCCTTCATCGAGGACCTGCTGTTCAACAAGGTGACCCTGGCTGACGCTG
GTTTCATCAAGCAGTACGGTGACTGCCTGGGTGACATCGCTGCTCGTGACCTGATCTGC
GCTCAGAAGTTCAACGGTCTGACCGTGCTGCCCCCCCTGCTGACCGACGAGATGATCGC
TCAGTACACCTCCGCTCTGCTGGCTGGTACCATCACCTCCGGTTGGACCTTCGGTGCTGG
TGCTGCTCTGCAGATCCCCTTCGCTATGCAGATGGCTTACCGTTTCAACGGTATCGGTGT
GACCCAGAACGTGCTGTACGAGAACCAGAAGCTGATCGCTAACCAGTTCAACTCCGCTA
TCGGTAAGATCCAGGACTCCCTGTCCTCCACCGCTTCCGCTCTGGGTAAGCTGCAGGAC
GTGGTGAACCACAACGCTCAGGCTCTGAACACCCTGGTGAAGCAGCTGTCCTCCAAGT
TCGGTGCTATCTCCTCCGTGCTGAACGACATCCTGTCCCGTCTGGACAAGGTGGAGGCT
GAGGTGCAGATCGACCGTCTGATCACCGGTCGTCTGCAGTCCCTGCAGACCTACGTGAC
CCAGCAGCTGATCCGTGCTGCTGAGATCCGTGCTTCCGCTAACCTGGCTGCTACCAAGA
TGTCCGAGTGCGTGCTGGGTCAGTCCAAGCGTGTGGACTTCTGCGGTAAGGGTTACCAC
CTGATGTCCTTCCCCCAGTCCGCTCCCCACGGTGTGGTGTTCCTGCACGTGACCTACGTG
CCCGCTCAGGAGAAGAACTTCACCACCGCTCCCGCTATCTGCCACGACGGTAAGGCTCA
CTTCCCCCGTGAGGGTGTGTTCGTGTCCAACGGTACCCACTGGTTCGTGACCCAGCGTA
ACTTCTACGAGCCCCAGATCATCACCACCGACAACACCTTCGTGTCCGGTAACTGCGAC
GTGGTGATCGGTATCGTGAACAACACCGTGTACGACCCCCTGCAGCCCGAGCTGGACTC
CTTCAAGGAGGAGCTGGACAAGTACTTCAAGAACCACACCTCCCCCGACGTGGACCTG
GGTGACATCTCCGGTATCAACGCTTCCGTGGTGAACATCCAGAAGGAGATCGACCGTCT
GAACGAGGTGGCTAAGAACCTGAACGAGTCCCTGATCGACCTGCAGGAGCTGGGTAAG
 
TACGAGCAGTACATCAAGTGGCCCTGGTACATCTGGCTGGGTTTCATCGCTGGTCTGATC
GCTATCGTGATGGTGACCATCATGCTGTGCTGCATGACCTCCTGCTGCTCCTGCCTGAAG
GGTTGCTGCTCCTGCGGTTCCTGCTGCAAGTTCGACGAGGACGACTCCGAGCCCGTGCT
GAAGGGTGTGAAGCTGCACTACACCTAG
 Note: the above sequence consists of signal peptide - extracellular domain - transmembrane domain - intracellular domain.
**SEQ ID No.73: Extracellular amino acid sequence of Omicron-BA.4 5 S antigen:** 
MFVFLVLLPLVSSQCVNLITRTQSYTNSFTRGVYYPDKVFRSSVLHSTQDLFLPFFSNVT
WFHAISGTNGTKRFDNPVLPFNDGVYFASTEKSNIIRGWIFGTTLDSKTQSLLIVNNATNVVI
KVCEFQFCNDPFLDVYYHKNNKSWMESEFRVYSSANNCTFEYVSQPFLMDLEGKQGNFK
NLREFVFKNIDGYFKIYSKHTPINLGRDLPQGFSALEPLVDLPIGINITRFQTLLALHRSYLTP
GDSSSGWTAGAAAYYVGYLQPRTFLLKYNENGTITDAVDCALDPLSETKCTLKSFTVEKGI
YQTSNFRVQPTESIVRFPNITNLCPFDEVFNATRFASVYAWNRKRISNCVADYSVLYNFAPFF
AFKCYGVSPTKLNDLCFTNVYADSFVIRGNEVSQIAPGQTGNIADYNYKLPDDFTGCVIAW
NSNKLDSKVGGNYNYRYRLFRKSNLKPFERDISTEIYQAGNKPCNGVAGVNCYFPLQSYGF
RPTYGVGHQPYRVVVLSFELLHAPATVCGPKKSTNLVKNKCVNFNFNGLTGTGVLTESNKK
FLPFQQFGRDIADTTDAVRDPQTLEILDITPCSFGGVSVITPGTNTSNQVAVLYQGVNCTEVP
VAIHADQLTPTWRVYSTGSNVFQTRAGCLIGAEYVNNSYECDIPIGAGICASYQTQTKSHRR
ARSVASQSIIAYTMSLGAENSVAYSNNSIAIPTNFTISVTTEILPVSMTKTSVDCTMYICGDST
ECSNLLLQYGSFCTQLKRALTGIAVEQDKNTQEVFAQVKQIYKTPPIKYFGGFNFSQILPDPS
KPSKRSFIEDLLFNKVTLADAGFIKQYGDCLGDIAARDLICAQKFNGLTVLPPLLTDEMIAQ
YTSALLAGTITSGWTFGAGAALQIPFAMQMAYRFNGIGVTQNVLYENQKLIANQFNSAIGKI
QDSLSSTASALGKLQDVVNHNAQALNTLVKQLSSKFGAISSVLNDILSRLDKVEAEVQIDRL
ITGRLQSLQTYVTQQLIRAAEIRASANLAATKMSECVLGQSKRVDFCGKGYHLMSFPQSAP
HGVVFLHVTYVPAQEKNFTTAPAICHDGKAHFPREGVFVSNGTHWFVTQRNFYEPQIITTD
NTFVSGNCDVVIGIVNNTVYDPLQPELDSFKEELDKYFKNHTSPDVDLGDISGINASVVNIQ
KEIDRLNEVAKNLNESLIDLQELGKYEQYIKWP
 Note: the above sequence consists of signal peptide - extracellular domain.
**SEQ ID No.74: Extracellular nucleic acid sequence of Omicron-BA.4/5 S antigen (optimization of CHO cells):** 
ATGTTCGTGTTCCTGGTGCTGCTGCCCCTGGTGAGCAGCCAGTGCGTGAACCTGAT
CACCAGAACCCAGAGCTACACCAACAGCTTCACCAGAGGCGTGTACTACCCCGACAAG
GTGTTCAGAAGCAGCGTGCTGCACAGCACCCAGGACCTGTTCCTGCCCTTCTTCAGCAA
CGTGACCTGGTTCCACGCCATCAGCGGCACCAACGGCACCAAGAGATTCGACAACCCC
GTGCTGCCCTTCAACGACGGCGTGTACTTCGCCAGCACCGAGAAGAGCAACATCATCAG
AGGCTGGATCTTCGGCACCACCCTGGACAGCAAGACCCAGAGCCTGCTGATCGTGAAC
AACGCCACCAACGTGGTGATCAAGGTGTGCGAGTTCCAGTTCTGCAACGACCCCTTCCT
GGACGTGTACTACCACAAGAACAACAAGAGCTGGATGGAGAGCGAGTTCAGAGTGTAC
AGCAGCGCCAACAACTGCACCTTCGAGTACGTGAGCCAGCCCTTCCTGATGGACCTGG
AGGGCAAGCAGGGCAACTTCAAGAACCTGAGAGAGTTCGTGTTCAAGAACATCGACGG
CTACTTCAAGATCTACAGCAAGCACACCCCCATCAACCTGGGCAGAGACCTGCCCCAGG
GCTTCAGCGCCCTGGAGCCCCTGGTGGACCTGCCCATCGGCATCAACATCACCAGATTC
CAGACCCTGCTGGCCCTGCACAGAAGCTACCTGACCCCCGGCGACAGCAGCAGCGGCT
GGACCGCCGGCGCCGCCGCCTACTACGTGGGCTACCTGCAGCCCAGAACCTTCCTGCTG
AAGTACAACGAGAACGGCACCATCACCGACGCCGTGGACTGCGCCCTGGACCCCCTGA
GCGAGACCAAGTGCACCCTGAAGAGCTTCACCGTGGAGAAGGGCATCTACCAGACCAG
CAACTTCAGAGTGCAGCCCACCGAGAGCATCGTGAGATTCCCCAACATCACCAACCTGT
GCCCCTTCGACGAGGTGTTCAACGCCACCAGATTCGCCAGCGTGTACGCCTGGAACAG
AAAGAGAATCAGCAACTGCGTGGCCGACTACAGCGTGCTGTACAACTTCGCCCCCTTCT
TCGCCTTCAAGTGCTACGGCGTGAGCCCCACCAAGCTGAACGACCTGTGCTTCACCAAC
GTGTACGCCGACAGCTTCGTGATCAGAGGCAACGAGGTGAGCCAGATCGCCCCCGGCC
AGACCGGCAACATCGCCGACTACAACTACAAGCTGCCCGACGACTTCACCGGCTGCGT
GATCGCCTGGAACAGCAACAAGCTGGACAGCAAGGTGGGCGGCAACTACAACTACAGA
TACAGACTGTTCAGAAAGAGCAACCTGAAGCCCTTCGAGAGAGACATCAGCACCGAGA
TCTACCAGGCCGGCAACAAGCCCTGCAACGGCGTGGCCGGCGTGAACTGCTACTTCCC
CCTGCAGAGCTACGGCTTCAGACCCACCTACGGCGTGGGCCACCAGCCCTACAGAGTG
GTGGTGCTGAGCTTCGAGCTGCTGCACGCCCCCGCCACCGTGTGCGGCCCCAAGAAGA
GCACCAACCTGGTGAAGAACAAGTGCGTGAACTTCAACTTCAACGGCCTGACCGGCAC
CGGCGTGCTGACCGAGAGCAACAAGAAGTTCCTGCCCTTCCAGCAGTTCGGCAGAGAC
ATCGCCGACACCACCGACGCCGTGAGAGACCCCCAGACCCTGGAGATCCTGGACATCA
 
CCCCCTGCAGCTTCGGCGGCGTGAGCGTGATCACCCCCGGCACCAACACCAGCAACCA
GGTGGCCGTGCTGTACCAGGGCGTGAACTGCACCGAGGTGCCCGTGGCCATCCACGCC
GACCAGCTGACCCCCACCTGGAGAGTGTACAGCACCGGCAGCAACGTGTTCCAGACCA
GAGCCGGCTGCCTGATCGGCGCCGAGTACGTGAACAACAGCTACGAGTGCGACATCCC
CATCGGCGCCGGCATCTGCGCCAGCTACCAGACCCAGACCAAGAGCCACAGAAGAGCC
AGAAGCGTGGCCAGCCAGAGCATCATCGCCTACACCATGAGCCTGGGCGCCGAGAACA
GCGTGGCCTACAGCAACAACAGCATCGCCATCCCCACCAACTTCACCATCAGCGTGACC
ACCGAGATCCTGCCCGTGAGCATGACCAAGACCAGCGTGGACTGCACCATGTACATCTG
CGGCGACAGCACCGAGTGCAGCAACCTGCTGCTGCAGTACGGCAGCTTCTGCACCCAG
CTGAAGAGAGCCCTGACCGGCATCGCCGTGGAGCAGGACAAGAACACCCAGGAGGTG
TTCGCCCAGGTGAAGCAGATCTACAAGACCCCCCCCATCAAGTACTTCGGCGGCTTCAA
CTTCAGCCAGATCCTGCCCGACCCCAGCAAGCCCAGCAAGAGAAGCTTCATCGAGGAC
CTGCTGTTCAACAAGGTGACCCTGGCCGACGCCGGCTTCATCAAGCAGTACGGCGACTG
CCTGGGCGACATCGCCGCCAGAGACCTGATCTGCGCCCAGAAGTTCAACGGCCTGACC
GTGCTGCCCCCCCTGCTGACCGACGAGATGATCGCCCAGTACACCAGCGCCCTGCTGGC
CGGCACCATCACCAGCGGCTGGACCTTCGGCGCCGGCGCCGCCCTGCAGATCCCCTTCG
CCATGCAGATGGCCTACAGATTCAACGGCATCGGCGTGACCCAGAACGTGCTGTACGAG
AACCAGAAGCTGATCGCCAACCAGTTCAACAGCGCCATCGGCAAGATCCAGGACAGCC
TGAGCAGCACCGCCAGCGCCCTGGGCAAGCTGCAGGACGTGGTGAACCACAACGCCC
AGGCCCTGAACACCCTGGTGAAGCAGCTGAGCAGCAAGTTCGGCGCCATCAGCAGCGT
GCTGAACGACATCCTGAGCAGACTGGACAAGGTGGAGGCCGAGGTGCAGATCGACAG
ACTGATCACCGGCAGACTGCAGAGCCTGCAGACCTACGTGACCCAGCAGCTGATCAGA
GCCGCCGAGATCAGAGCCAGCGCCAACCTGGCCGCCACCAAGATGAGCGAGTGCGTGC
TGGGCCAGAGCAAGAGAGTGGACTTCTGCGGCAAGGGCTACCACCTGATGAGCTTCCC
CCAGAGCGCCCCCCACGGCGTGGTGTTCCTGCACGTGACCTACGTGCCCGCCCAGGAG
AAGAACTTCACCACCGCCCCCGCCATCTGCCACGACGGCAAGGCCCACTTCCCCAGAG
AGGGCGTGTTCGTGAGCAACGGCACCCACTGGTTCGTGACCCAGAGAAACTTCTACGA
GCCCCAGATCATCACCACCGACAACACCTTCGTGAGCGGCAACTGCGACGTGGTGATCG
GCATCGTGAACAACACCGTGTACGACCCCCTGCAGCCCGAGCTGGACAGCTTCAAGGA
GGAGCTGGACAAGTACTTCAAGAACCACACCAGCCCCGACGTGGACCTGGGCGACATC
AGCGGCATCAACGCCAGCGTGGTGAACATCCAGAAGGAGATCGACAGACTGAACGAGG
 
TGGCCAAGAACCTGAACGAGAGCCTGATCGACCTGCAGGAGCTGGGCAAGTACGAGCA
GTACATCAAGTGGCCCTAG
 Note: the above sequence consists of signal peptide - extracellular domain.
**SEQ ID No.75: Extracellular nucleic acid sequence of Omicron-BA.4/5 S antigen (optimization of insect cells):** 
ATGTTCGTGTTCCTGGTGCTGCTGCCCCTGGTGTCCTCCCAGTGCGTGAACCTGATC
ACCCGTACCCAGTCCTACACCAACTCCTTCACCCGTGGTGTGTACTACCCCGACAAGGT
GTTCCGTTCCTCCGTGCTGCACTCCACCCAGGACCTGTTCCTGCCCTTCTTCTCCAACGT
GACCTGGTTCCACGCTATCTCCGGTACCAACGGTACCAAGCGTTTCGACAACCCCGTGC
TGCCCTTCAACGACGGTGTGTACTTCGCTTCCACCGAGAAGTCCAACATCATCCGTGGT
TGGATCTTCGGTACCACCCTGGACTCCAAGACCCAGTCCCTGCTGATCGTGAACAACGC
TACCAACGTGGTGATCAAGGTGTGCGAGTTCCAGTTCTGCAACGACCCCTTCCTGGACG
TGTACTACCACAAGAACAACAAGTCCTGGATGGAGTCCGAGTTCCGTGTGTACTCCTCC
GCTAACAACTGCACCTTCGAGTACGTGTCCCAGCCCTTCCTGATGGACCTGGAGGGTAA
GCAGGGTAACTTCAAGAACCTGCGTGAGTTCGTGTTCAAGAACATCGACGGTTACTTCA
AGATCTACTCCAAGCACACCCCCATCAACCTGGGTCGTGACCTGCCCCAGGGTTTCTCC
GCTCTGGAGCCCCTGGTGGACCTGCCCATCGGTATCAACATCACCCGTTTCCAGACCCT
GCTGGCTCTGCACCGTTCCTACCTGACCCCCGGTGACTCCTCCTCCGGTTGGACCGCTG
GTGCTGCTGCTTACTACGTGGGTTACCTGCAGCCCCGTACCTTCCTGCTGAAGTACAACG
AGAACGGTACCATCACCGACGCTGTGGACTGCGCTCTGGACCCCCTGTCCGAGACCAA
GTGCACCCTGAAGTCCTTCACCGTGGAGAAGGGTATCTACCAGACCTCCAACTTCCGTG
TGCAGCCCACCGAGTCCATCGTGCGTTTCCCCAACATCACCAACCTGTGCCCCTTCGAC
GAGGTGTTCAACGCTACCCGTTTCGCTTCCGTGTACGCTTGGAACCGTAAGCGTATCTCC
AACTGCGTGGCTGACTACTCCGTGCTGTACAACTTCGCTCCCTTCTTCGCTTTCAAGTGC
TACGGTGTGTCCCCCACCAAGCTGAACGACCTGTGCTTCACCAACGTGTACGCTGACTC
CTTCGTGATCCGTGGTAACGAGGTGTCCCAGATCGCTCCCGGTCAGACCGGTAACATCG
CTGACTACAACTACAAGCTGCCCGACGACTTCACCGGTTGCGTGATCGCTTGGAACTCC
AACAAGCTGGACTCCAAGGTGGGTGGTAACTACAACTACCGTTACCGTCTGTTCCGTAA
GTCCAACCTGAAGCCCTTCGAGCGTGACATCTCCACCGAGATCTACCAGGCTGGTAACA
AGCCCTGCAACGGTGTGGCTGGTGTGAACTGCTACTTCCCCCTGCAGTCCTACGGTTTC
 
CGTCCCACCTACGGTGTGGGTCACCAGCCCTACCGTGTGGTGGTGCTGTCCTTCGAGCT
GCTGCACGCTCCCGCTACCGTGTGCGGTCCCAAGAAGTCCACCAACCTGGTGAAGAAC
AAGTGCGTGAACTTCAACTTCAACGGTCTGACCGGTACCGGTGTGCTGACCGAGTCCA
ACAAGAAGTTCCTGCCCTTCCAGCAGTTCGGTCGTGACATCGCTGACACCACCGACGCT
GTGCGTGACCCCCAGACCCTGGAGATCCTGGACATCACCCCCTGCTCCTTCGGTGGTGT
GTCCGTGATCACCCCCGGTACCAACACCTCCAACCAGGTGGCTGTGCTGTACCAGGGTG
TGAACTGCACCGAGGTGCCCGTGGCTATCCACGCTGACCAGCTGACCCCCACCTGGCGT
GTGTACTCCACCGGTTCCAACGTGTTCCAGACCCGTGCTGGTTGCCTGATCGGTGCTGA
GTACGTGAACAACTCCTACGAGTGCGACATCCCCATCGGTGCTGGTATCTGCGCTTCCTA
CCAGACCCAGACCAAGTCCCACCGTCGTGCTCGTTCCGTGGCTTCCCAGTCCATCATCG
CTTACACCATGTCCCTGGGTGCTGAGAACTCCGTGGCTTACTCCAACAACTCCATCGCTA
TCCCCACCAACTTCACCATCTCCGTGACCACCGAGATCCTGCCCGTGTCCATGACCAAG
ACCTCCGTGGACTGCACCATGTACATCTGCGGTGACTCCACCGAGTGCTCCAACCTGCT
GCTGCAGTACGGTTCCTTCTGCACCCAGCTGAAGCGTGCTCTGACCGGTATCGCTGTGG
AGCAGGACAAGAACACCCAGGAGGTGTTCGCTCAGGTGAAGCAGATCTACAAGACCCC
CCCCATCAAGTACTTCGGTGGTTTCAACTTCTCCCAGATCCTGCCCGACCCCTCCAAGCC
CTCCAAGCGTTCCTTCATCGAGGACCTGCTGTTCAACAAGGTGACCCTGGCTGACGCTG
GTTTCATCAAGCAGTACGGTGACTGCCTGGGTGACATCGCTGCTCGTGACCTGATCTGC
GCTCAGAAGTTCAACGGTCTGACCGTGCTGCCCCCCCTGCTGACCGACGAGATGATCGC
TCAGTACACCTCCGCTCTGCTGGCTGGTACCATCACCTCCGGTTGGACCTTCGGTGCTGG
TGCTGCTCTGCAGATCCCCTTCGCTATGCAGATGGCTTACCGTTTCAACGGTATCGGTGT
GACCCAGAACGTGCTGTACGAGAACCAGAAGCTGATCGCTAACCAGTTCAACTCCGCTA
TCGGTAAGATCCAGGACTCCCTGTCCTCCACCGCTTCCGCTCTGGGTAAGCTGCAGGAC
GTGGTGAACCACAACGCTCAGGCTCTGAACACCCTGGTGAAGCAGCTGTCCTCCAAGT
TCGGTGCTATCTCCTCCGTGCTGAACGACATCCTGTCCCGTCTGGACAAGGTGGAGGCT
GAGGTGCAGATCGACCGTCTGATCACCGGTCGTCTGCAGTCCCTGCAGACCTACGTGAC
CCAGCAGCTGATCCGTGCTGCTGAGATCCGTGCTTCCGCTAACCTGGCTGCTACCAAGA
TGTCCGAGTGCGTGCTGGGTCAGTCCAAGCGTGTGGACTTCTGCGGTAAGGGTTACCAC
CTGATGTCCTTCCCCCAGTCCGCTCCCCACGGTGTGGTGTTCCTGCACGTGACCTACGTG
CCCGCTCAGGAGAAGAACTTCACCACCGCTCCCGCTATCTGCCACGACGGTAAGGCTCA
CTTCCCCCGTGAGGGTGTGTTCGTGTCCAACGGTACCCACTGGTTCGTGACCCAGCGTA
 
ACTTCTACGAGCCCCAGATCATCACCACCGACAACACCTTCGTGTCCGGTAACTGCGAC
GTGGTGATCGGTATCGTGAACAACACCGTGTACGACCCCCTGCAGCCCGAGCTGGACTC
CTTCAAGGAGGAGCTGGACAAGTACTTCAAGAACCACACCTCCCCCGACGTGGACCTG
GGTGACATCTCCGGTATCAACGCTTCCGTGGTGAACATCCAGAAGGAGATCGACCGTCT
GAACGAGGTGGCTAAGAACCTGAACGAGTCCCTGATCGACCTGCAGGAGCTGGGTAAG
TACGAGCAGTACATCAAGTGGCCCTAG
 Note: the above sequence consists of signal peptide - extracellular domain.
**SEQ ID No.76: Nucleotide sequence of S protein of BQ.1.1 variant strain** 
ATGTTCGTGTTCCTGGTGCTGCTGCCCCTGGTGAGCAGCCAATGCGTGAACCTGAT
CACAAGAACACAGAGCTACACCAACAGCTTCACAAGAGGCGTGTACTACCCCGACAAG
GTGTTCAGAAGCAGCGTGCTGCACAGCACCCAAGACCTGTTTCTCCCCTTCTTCAGCAA
CGTGACCTGGTTCCACGCCATCAGCGGCACCAACGGCACCAAGAGATTCGACAACCCC
GTGCTGCCCTTCAACGACGGCGTGTACTTCGCTAGCACCGAGAAGAGCAACATCATCAG
AGGCTGGATCTTCGGCACCACCCTGGACAGCAAGACACAGAGCCTGCTGATCGTGAATA
ACGCCACCAACGTGGTGATCAAGGTGTGCGAGTTTCAGTTCTGCAACGACCCCTTCCTG
GACGTGTACTACCACAAGAACAACAAGAGCTGGATGGAGAGCGAGTTCAGAGTGTACA
GCAGCGCTAACAACTGCACCTTCGAGTACGTGAGCCAACCCTTCCTGATGGACCTGGAG
GGCAAGCAAGGCAACTTCAAGAACCTGAGAGAGTTCGTGTTCAAGAACATCGACGGCT
ACTTCAAGATCTACAGCAAGCACACCCCCATCAACCTGGGCAGAGACCTGCCCCAAGG
CTTCAGCGCCCTGGAGCCCCTGGTGGACCTGCCCATCGGCATCAACATCACAAGATTTC
AGACCCTGCTGGCCCTGCACAGAAGCTACCTCACCCCTGGGGACAGCAGCAGCGGCTG
GACCGCTGGCGCCGCTGCCTACTACGTGGGCTACCTGCAGCCTAGAACCTTCCTGCTGA
AGTACAACGAGAACGGCACCATTACAGACGCCGTGGACTGCGCCCTGGACCCCCTGAG
CGAGACCAAGTGCACCCTGAAGAGCTTCACCGTGGAGAAGGGCATCTATCAGACAAGC
AACTTCAGAGTGCAGCCCACCGAGAGCATCGTGAGATTCCCCAACATCACCAACCTGTG
CCCCTTCGACGAGGTGTTCAACGCCACCACCTTCGCTAGCGTGTACGCCTGGAATAGAA
AGAGAATCAGCAACTGCGTGGCCGACTACAGCGTGCTGTACAACTTCGCCCCCTTCTTC
GCCTTCAAATGCTATGGCGTGAGCCCCACCAAGCTGAACGACCTGTGCTTCACCAACGT
GTACGCCGACAGCTTCGTGATCAGAGGCAACGAGGTGAGCCAAATCGCCCCTGGGCAG
ACCGGCAACATCGCCGATTACAACTACAAACTGCCCGACGACTTCACCGGCTGCGTGAT
 
CGCCTGGAACAGCAACAAGCTGGACAGCACCGTGGGCGGCAACTACAACTACCGGTAC
AGACTGTTCAGAAAGAGCAAGCTGAAGCCCTTCGAGAGAGACATCAGCACCGAGATCT
ACCAAGCCGGCAACAAGCCCTGCAACGGCGTGGCCGGCGTGAACTGCTACTTCCCCCT
GCAGAGCTACGGCTTCAGACCCACCTACGGCGTGGGCCATCAGCCCTACAGAGTGGTCG
TGCTGAGCTTCGAGCTGCTGCACGCCCCCGCCACCGTGTGCGGCCCCAAGAAGAGCAC
CAACCTGGTGAAGAACAAGTGCGTGAACTTCAACTTCAACGGCCTGACCGGCACCGGC
GTGCTGACCGAGAGCAACAAGAAGTTCCTGCCCTTTCAGCAGTTCGGCAGAGACATCG
CCGACACCACCGACGCCGTGCGGGACCCTCAGACCCTGGAGATCCTGGACATCACCCC
CTGCAGCTTTGGCGGCGTGAGCGTGATCACCCCCGGCACCAACACAAGCAACCAAGTG
GCCGTGCTGTACCAAGGCGTCAATTGCACCGAGGTGCCCGTGGCCATCCACGCCGATCA
GCTGACCCCCACCTGGAGAGTCTACAGCACCGGCAGCAACGTGTTTCAGACAAGAGCC
GGCTGCCTGATCGGCGCCGAGTACGTGAACAACAGCTACGAGTGCGACATCCCCATCGG
CGCCGGCATCTGCGCTAGCTATCAGACACAGACCAAGAGCCACGGCAGCGCTAGCAGC
GTGGCTAGCCAAAGCATCATCGCCTACACCATGAGCCTGGGCGCCGAGAACAGCGTGG
CCTACAGCAACAACAGCATCGCCATCCCCACCAACTTCACCATCAGCGTGACCACCGAG
ATCCTGCCCGTCAGCATGACCAAGACAAGCGTGGACTGCACCATGTACATCTGCGGCGA
CAGCACCGAGTGCAGCAACCTGCTCCTGCAGTACGGCAGCTTCTGCACACAGCTGAAG
AGAGCCCTGACCGGCATCGCCGTGGAGCAAGACAAGAACACCCAAGAGGTGTTCGCCC
AAGTGAAGCAGATCTACAAGACCCCCCCCATCAAGTACTTCGGCGGCTTCAACTTCAGC
CAAATCCTGCCCGATCCTAGCAAGCCTAGCAAGAGAAGCTTCATCGAGGACCTGCTGTT
CAACAAGGTGACCCTGGCCGACGCCGGCTTCATCAAGCAGTACGGCGACTGCCTGGGC
GATATCGCCGCTAGAGACCTGATCTGCGCTCAGAAGTTCAATGGCCTGACCGTGCTGCC
CCCCCTGCTGACCGACGAGATGATCGCTCAGTACACAAGCGCCCTGCTGGCCGGCACCA
TCACAAGCGGCTGGACATTCGGCGCCGGCGCTGCTCTGCAGATCCCCTTCGCCATGCAG
ATGGCCTACAGATTCAACGGCATCGGCGTGACACAGAACGTGCTGTACGAGAATCAGAA
GCTGATCGCCAATCAGTTCAACAGCGCCATCGGCAAGATCCAAGACAGCCTGAGCAGC
ACCGCTAGCGCCCTGGGCAAGCTGCAAGACGTGGTGAACCACAACGCCCAAGCCCTGA
ACACCCTGGTGAAGCAGCTGAGCAGCAAGTTCGGCGCCATCAGCAGCGTGCTGAACGA
TATCCTGAGCAGACTGGACCCCCCCGAGGCCGAGGTGCAGATCGACAGACTGATTACCG
GCAGACTGCAGAGCCTGCAGACCTACGTGACACAGCAGCTGATCAGAGCCGCCGAGAT
CAGAGCTAGCGCCAACCTGGCCGCCACCAAGATGAGCGAGTGCGTGCTGGGGCAGAGC
 
AAGAGAGTGGACTTCTGCGGCAAGGGCTACCACCTGATGAGCTTCCCTCAGAGCGCCC
CCCACGGCGTGGTGTTCCTGCACGTGACCTACGTGCCCGCCCAAGAGAAGAACTTCAC
CACCGCTCCCGCTATCTGCCACGACGGCAAGGCCCACTTCCCTAGAGAGGGCGTGTTCG
TGAGCAACGGCACCCACTGGTTCGTGACACAGAGAAACTTCTACGAGCCTCAGATCATC
ACCACCGACAACACCTTCGTGAGCGGCAACTGCGACGTGGTGATCGGCATCGTGAACA
ACACCGTGTACGACCCCCTGCAACCCGAGCTGGACAGCTTCAAGGAGGAGCTGGACAA
GTACTTTAAGAACCACACAAGCCCCGACGTGGACCTGGGCGACATTAGCGGCATCAACG
CTAGCGTGGTGAACATTCAGAAGGAGATTGACAGACTGAACGAGGTGGCCAAGAACCT
GAACGAGAGCCTGATCGACCTGCAAGAGCTGGGCAAGTACGAGCAGTACATCAAGTGG
CCCTGGTACATCTGGCTGGGCTTCATCGCCGGCCTGATCGCCATCGTGATGGTGACCATC
ATGCTGTGCTGCATGACAAGCTGTTGCAGCTGCCTCAAGGGCTGCTGCAGCTGCGGCAG
CTGCTGCAAGTTCGACGAGGACGACAGCGAGCCCGTGCTGAAGGGCGTGAAGCTGCAC
TACACCTGA
**SEQ ID No.77: Amino acid sequence of S protein of BQ.1.1 variant strain**

MFVFLVLLPLVSSQCVNLITRTQSYTNSFTRGVYYPDKVFRSSVLHSTQDLFLPFFSNVT
WFHAISGTNGTKRFDNPVLPFNDGVYFASTEKSNIIRGWIFGTTLDSKTQSLLIVNNATNVVI
KVCEFQFCNDPFLDVYYHKNNKSWMESEFRVYSSANNCTFEYVSQPFLMDLEGKQGNFK
NLREFVFKNIDGYFKIYSKHTPINLGRDLPQGFSALEPLVDLPIGINITRFQTLLALHRSYLTP
GDSSSGWTAGAAAYYVGYLQPRTFLLKYNENGTITDAVDCALDPLSETKCTLKSFTVEKGI
YQTSNFRVQPTESIVRFPNITNLCPFDEVFNATTFASVYAWNRKRISNCVADYSVLYNFAPFF
AFKCYGVSPTKLNDLCFTNVYADSFVIRGNEVSQIAPGQTGNIADYNYKLPDDFTGCVIAW
NSNKLDSTVGGNYNYRYRLFRKSKLKPFERDISTEIYQAGNKPCNGVAGVNCYFPLQSYGF
RPTYGVGHQPYRVVVLSFELLHAPATVCGPKKSTNLVKNKCVNFNFNGLTGTGVLTESNKK
FLPFQQFGRDIADTTDAVRDPQTLEILDITPCSFGGVSVITPGTNTSNQVAVLYQGVNCTEVP
VAIHADQLTPTWRVYSTGSNVFQTRAGCLIGAEYVNNSYECDIPIGAGICASYQTQTKSHGS
ASSVASQSIIAYTMSLGAENSVAYSNNSIAIPTNFTISVTTEILPVSMTKTSVDCTMYICGDSTE
CSNLLLQYGSFCTQLKRALTGIAVEQDKNTQEVFAQVKQIYKTPPIKYFGGFNFSQILPDPSK
PSKRSFIEDLLFNKVTLADAGFIKQYGDCLGDIAARDLICAQKFNGLTVLPPLLTDEMIAQYT
SALLAGTITSGWTFGAGAALQIPFAMQMAYRFNGIGVTQNVLYENQKLIANQFNSAIGKIQ
DSLSSTASALGKLQDVVNHNAQALNTLVKQLSSKFGAISSVLNDILSRLDPPEAEVQIDRLIT

GRLQSLQTYVTQQLIRAAEIRASANLAATKMSECVLGQSKRVDFCGKGYHLMSFPQSAPH
GVVFLHVTYVPAQEKNFTTAPAICHDGKAHFPREGVFVSNGTHWFVTQRNFYEPQIITTDN
TFVSGNCDVVIGIVNNTVYDPLQPELDSFKEELDKYFKNHTSPDVDLGDISGINASVVNIQK
EIDRLNEVAKNLNESLIDLQELGKYEQYIKWPWYIWLGFIAGLIAIVMVTIMLCCMTSCCSC
LKGCCSCGSCCKFDEDDSEPVLKGVKLHYT

Beneficial effect: the present invention provides a broad-spectrum protein for resisting infection with SARS-CoV-2 or a mutant thereof and a vaccine thereof, and specifically provides a recombinant protein vaccine for resisting infection with SARS-CoV-2 or the mutant thereof, a adenovirus vetor vaccine which takes the human type 5 replication-deficient adenovirus as vector; and the nucleotide sequences encoding the two vaccines are selected from at least of SEQ ID No.10, SEQ ID No.11, SEQ ID No.12, SEQ ID No.13, SEQ ID No.14, SEQ ID No.15, SEQ ID No.16, SEQ ID No.17, SEQ ID No.19, SEQ ID No.21, SEQ ID No.23, SEQ ID No.24, SEQ ID No.26, SEQ ID No.28, SEQ ID No.30, SEQ ID No.32, SEQ ID No.34, SEQ ID No.36, SEQ ID No.38, SEQ ID No.41, SEQ ID No.42, SEQ ID No.44, SEQ ID No.45, SEQ ID No.47, SEQ ID No.48, SEQ ID No.50, SEQ ID No.51, SEQ ID No.53, SEQ ID No.54, SEQ ID No.56, SEQ ID No.57, SEQ ID No.59, SEQ ID No.60, SEQ ID No.62, SEQ ID No.63, SEQ ID No.65, SEQ ID No.66, SEQ ID No.68, SEQ ID No.69, SEQ ID No.71, SEQ ID No.72, SEQ ID No.74, SEQ ID No.75, or SEQ ID No.76. Meanwhile, in the present invention, the two vaccines are combined, where the recombinant protein vaccine is mainly prepared based on the RBD sequence of SARS-CoV-2, the optimized sequence composed of RBD and HR of the SARS-CoV-2 mutant, and the full-length S protein sequence of CHO cell optimized expression; the adenovirus vaccine is constructed by optimizing the full-length sequence of S antigen of SARS-CoV-2 or its mutant, so as to prepare a pharmaceutical composition or combined drug for preventing and/or treating infection with SARS-CoV-2 or its mutant, namely bivalent vaccine. The present invention can induce antibodies and other immune reactions in the body through intramuscular injection, nasal spray administration or combined administration of the two vaccines and block the binding of the S protein of SARS-CoV-2 with the ACE2 receptor of the host cell, thereby helping the host resist coronavirus infection, and especially having better prevention and treatment effect on the mutant virus.

### Brief Description of the Drawings

FIG 1 shows a schematic diagram of construction of a full-length gene of recombinant Ad5_{Beta/Delta} virus and an expression level diagram of spike induced by a recombinant adenovirus in Experiment 1.1.
FIG 2 shows a testing diagram of anti-RBD specific binding antibody levels of recombinant adenovirus vaccines Ad5_{WT}, Ad5_{Beta}, Ad5_{Delta}, Ad5_{Omicron} and Ad5_{Beta/Delta} in the blood of immunized mice in Experiment 1.3.
FIG 3 shows a testing result diagram of neutralizing antibodies of recombinant adenovirus vaccines Ad5_{WT}, Ad5_{Beta}, Ad5_{Delta}, Ad5_{BA.1}, Ad5_{Beta/Delta} and Ad5_{BA.4/5} against pseudovirus in Experiment 1.4.
FIG 4 shows a testing result diagram of anti-RBD specific IgG and IgA antibodies of recombinant adenovirus vaccine Ad5_{Beta/Delta} in bronchoalveolar lavage fluid of immunized mice in Experiment 1.5.
FIG 5 shows a testing result diagram of adenovirus vaccine Ad5_{Beta/Delta} on tissue resident T cells in Experiment 1.6.
FIG 6 shows a testing result diagram of recombinant adenovirus vaccine Ad5_{Beta/Delta} on antigen-specific T cells in lung tissue in Experiment 1.7.
FIG 7 shows a testing result diagram of reactions of a germinal center of recombinant adenovirus vaccine Ad5_{Beta/Delta} in Experiment 1.8.
FIG 8 shows a design diagram of pFastBac1-GP67-Trx-His-EK-S-RBD (Omicron_BA.4/.5)-HR in Embodiment 2.1.
FIG 9 shows a 1% agarose gel electrophoresis map of cloning PCR products at GP67-Trx-His-EK-S-RBD (Omicron_BA.4/.5)-HR fragments 1#-5# in Embodiment 2.1.
FIG 10 shows a 1% agarose gel electrophoresis map of PCR identification of recombinant bacmid in Embodiment 2.1.
FIG 11 shows a flow diagram of packaging and amplification of recombinant baculovirus in Embodiment 2.1.
FIG 12 shows a WB verification result diagram of target protein during baculovirus expansion in Embodiment 2.1.
FIG 13 shows an electrophoresis and WB verification diagram of target protein of CHO cell expression in Embodiment 2.2.
FIG 14 shows a pseudovirus neutralization assay after immunization with a single protein vaccine in Experiment 2.2.
FIG 15 shows a testing result diagram of neutralizing antibodies induced by bivalent RBD protein vaccine in Experiment 2.3.
FIG 16 shows a pseudovirus neutralization assay of S protein vaccines of WT, Beta and Delta strains in Experiment 2.4.
FIG 17 shows a pseudovirus neutralization assay of S protein vaccines of different Omicron variant strains in Experiment 2.4.
FIG 18 shows a flow chart of immunization preparation of a nasal spray prepared by mixing adenovirus Ad5_{BA.4/5} with RDB-HR_{Delta}, and a testing result diagram of expression levels of recombinant Ad5_{BA.4/5} virus-induced spike.
FIG 19 shows a time cycle chart of intranasal immunization for an animal in Experiment 3.2.
FIG 20 shows a testing result diagram of anti-RBD specific antibodies in serum and bronchoalveolar lavage fluid of immunized mice in Experiment 3.3.
FIG 21 shows a testing result diagram of pseudovirus neutralization assay in Experiment 3.4.
FIG 22 shows a pseudovirus neutralization assay of immunization preparation of a nasal spray prepared by mixing Ad5_{WT}+ RDB-WT.
FIG 23 shows a pseudovirus neutralization assay of immunization preparation of a nasal spray prepared by mixing Ad5_{BA.4/5}+S-BA.4/5.
FIG 24 shows a mouse challenge test (Ad5_{BA.4/5}+S-BA.4/5) in Experiment 3.5.
FIG 25 shows a testing result diagram of tissue resident T cells in bronchoalveolar lavage fluid in Experiment 3.6.
FIG 26 shows a testing diagram of antigen-specific T cells in lung tissue in Experiment 3.7.
FIG 27 shows detection of reactions of a germinal center in Experiment 3.8.

### Detailed Description

### Abbreviations of terms:

Monophosphoryl lipid A (MPL), squalene oil-in-water emulsion (MF59), recombinant cholera toxin (rCTB), astragalus polysaccharide (APS), phosphatidylethanolamine (PE), phosphatidylcholine (PC), cholesterol (Chol), dioleoyl phosphatidylethanolamine (DOPE), (2,3-dioleoxypropyl) trimethyl ammonium chloride (DOTAP), N-[1-(2,3- dioleoyl chloride) propyl ]-N,N,N-trimethylamine chloride (DOTMA), cationic cholesterol (DC-Chol), dimethyl-2, 3-dioleoxypropyl-2-(2-arginine formyl amino) ethyl ammonium trimethyldodecylammonium bromide (DOSPA), dodecyltrimethylammonium bromide (DTAB), tetradecyltrimethylammonium bromide (TTAB), hexadecyltrimethylammonium bromide (CTAB), dimethyldioctadecylammonium bromide (DDAB), or CpG ODN (nucleotide sequence containing non-methylated cytosine-phosphate-guanine as core sequence, synthetic CpG).

In the present invention, a recombinant protein vaccine and an adenovirus vaccine for resisting infection with SARS-CoV-2 are designed mainly based on the amino acid sequence at sites 320-545 in the S protein of SARS-CoV-2 or its mutant, heptapeptide repeat region 1 (HR1) and heptapeptide repeat region 2 (HR2). Meanwhile, new sequences are constructed based on RBD sequences, HR1 and HR2 sequences of different SARS-CoV-2 mutants, and finally, drugs for resisting infection with SARS-CoV-2 are constructed based on full-length nucleotides of the S protein of SARS-CoV-2 or its mutant virus, extracellular domains or transmembrane domains or intracellular domains. In order to improve the stability and expression level of the S protein, the amino acid sequence of the S protein of SARS-CoV-2 or its mutant is further artificially mutated when the drug for resisting infection with SARS-CoV-2 is designed.

The present invention further provides a recombinant protein vaccine and an adenovirus vaccine for resisting infection with SARS-CoV-2 or its mutants or a pharmaceutical composition or a combined drug of the two vaccines. This is mainly aimed at the S protein of SARS-CoV-2 virus or its mutants. Particularly, the ACE2 receptor binding domain of the S protein is blocked to induce immune reactions such as antibodies in the body, thereby blocking the binding of the S protein of SARS-CoV-2 or its mutant to the host cell ACE2 receptor, helping the host resist coronavirus infection and especially having better prevention and treatment effect against mutant viruses such as Alpha, Beta, Gamma, Delta, Omicron and Omicron variant strains.

The present invention also proves that a pharmaceutical composition taking a recombinant protein vaccine and an inactivated influenza virus vaccine for resisting infection with SARS-CoV-2 or its mutants as active ingredients or a pharmaceutical composition taking an adenovirus vaccine and an influenza recombinant protein vaccine for resisting infection with SARS-CoV-2 or its mutants as active ingredients has a better prevention and treatment effect on respiratory virus infection or diseases therefrom.

The following will explain the solution of the present invention with reference to embodiments. Those skilled in the art will understand that the following embodiments are used only to illustrate the present invention and should not be regarded as limiting the scope of the present invention. If the specific technologies or conditions are not specified in the embodiments, it shall be carried out according to the technologies or conditions described in the literature in the art or according to the product specification. Reagents or devices used, where the manufacturer is not indicated, are conventional products that can be purchased from the market.

### Part I: immune protection induced by individual adenoviruses

### Embodiment 1.1 Construction and preparation of recombinant adenovirus vaccine

### 1. Optimization and synthesis of S protein gene (wild strains and variant strains) sequences

Wuhan virus strain (GenBank accession number: YP _ 009724390.1) are used as a template to obtain the gene sequence of wild spike (S), and the signal peptide of the S protein is retained. On this basis, in order to improve the expression level of the protein, codons are optimized, and then the optimized sequence is synthesized.

On the basis of the spike (S) sequence of the Wuhan virus strain (GenBank accession number: YP _ 009724390.1), according to the mutation sites of SARS-COV-2 Beta, Delta, Omicron and other variant strains provided on the website of https://covariants.org/, the nucleotide sequence of S protein of each variant strain is obtained, and the signal peptide of each S protein mutant is retained. The gene sequence of Beta-Delta chimeric S protein is based on the mutation site of S protein in Beta variant strain and the mutation site of S protein RBD in Delta variant strain. On this basis, in order to ensure the stability of the protein and improve its expression level, a protein structure is mutated. Firstly, lysine (L) at site 986 is replaced with proline (P), and valine (V) at site 987 is replaced with proline (P) to improve the stability and expression level of the S protein. Secondly, the codons are optimized. Finally, the S protein genes of each variant strain are synthesized according to the mutation and optimized sequence.

The S protein genomes of SARS-CoV-2 and its variant strain synthesized according to the above method comprise WT (wild type), Beta, Delta, Beta/Delta chimeric type, Omicron BA.1 and Omicron BA.4/5, and their nucleotide sequences are as follows: SEQ ID No.24, SEQ ID No.26, SEQ ID No.28, SEQ ID No.30, SEQ ID No.32 and SEQ ID No.38.

### 2. Packaging of recombinant adenovirus SARS-COV-2 vaccine

In a gene synthesis process, S genesare cloned into a pDC316 vector by a recombinant cloning strategy, and the shuttle plasmids (pDC316-S) are obtained. The above-mentioned pDC316-S containing S genes of wild strains and variant strains is co-transfected into HEK293 cells with skeleton plasmids pBHGlox_E1,3Cre of AdMax adenovirus system, respectively; and the recombinant adenovirus is packaged by the following steps:
1) HEK293A cells with 8×10⁵ cells/well are inoculated into a six-well plate, and a high-glucose DMEM+10% FBS culture medium is overnight cultured in a cell incubator with 5% CO₂ at 37°C.
2) Next day, the above solution is replaced with high-glucose DMEM+2% FBS, and the skeleton plasmids (pBHGlox_E1, 3Cre) and the shuttle plasmids are co-transfected into HEK293A cells with lipofectamine3000. Specifically, 4µg skeleton plasmids and 2µg shuttle plasmids are taken for each transfection well and diluted with 125µL Opti-MEM medium; 12µL P3000 reagent is added into the diluted plasmids; another 1.5ml EP tube is taken; 7.5µL lipofectamine3000 is diluted with 125µL Opti-MEM medium; the diluted plasmids and diluted lipofectamine3000 are mixed at the ratio of 1: 1, incubated at room temperature for 10-15 minutes and then added into the cells; the cells continued to be cultured. After the overgrowth of the cells, they are passed on to a 25cm² cell culture bottle, and the virus generation signs of the cells are observed every day; after the overgrowth of the cells at the bottom of the bottle, they are passed on to a 75cm ² cell culture bottle until the cells appeared obvious plaque, and virus is collected when most of the cells are diseased and shed from the bottom.
3) The cell culture with virus is collected and centrifuged at 1200rpm for 3 minutes; a supernatant containing virus is sucked; a cell pellet is re-suspended with the supernatant containing virus at 1/10 culture volume, repeatedly frozen and thawed for three times in a refrigerator at -80°C and a water bath at 37°C, and centrifuged at 3000rpm for 20 minutes; and the supernatant containing virus is collected and mixed with the above supernatant containing virus, and the obtained product is the virus strain of adenovirus vaccine.
4) 50µL vaccine candidate virus strain solution is taken and mixed with 2µL protease K, the mixture is dissociated at 50°C for 30min to release the virus genome, and this is used as a PCR amplification S gene sequence. After the PCR product electrophoresis gel is recovered, it is sequenced and identified. The conditions for PCR amplification are as follows:
   degeneration: 95°C, 10min; degeneration: 95°C, 10s; annealing: 64°C, 30s; elongation: 72°C, 2min; elongation: 72°C, 5min; number of cycles, 40; primers for PCR amplification are as follows:
   pDC516-F1: ACACGTCAATGGGAAGTGAAA (SEQ ID No.78)
   pDC516-R1: GCTAGACGATCCAGACATGAT (SEQ ID No.79)

### 3. Amplification of recombinant adenovirus SARS-COV-2 vaccine

The correctly identified recombinant adenovirus vaccine strains are amplified step by step in 293 cells by the following specific steps: 80%-90% 293 cells overgrown are added according to MOI=10, a virus culture is collected after most of cell viruses become round, and a main virus seed stock and a working virus seed stock are prepared by the above repeated freezing and thawing method. The recombinant adenovirus vaccine is amplified in a cell factory or bioreactor, and the virus culture is collected after most of the cells are diseased. The cell and virus are amplified in the bioreactor by the following steps: 3-5 g/L Cytodex1 microvector is added into the bioreactor and sterilized; a cell culture fluid is added into the bioreactor; when operating conditions are kept at 37°C, pH 7.0, DO 50% and 50rpm, HEK293 cells amplified in cell factory are dissociated and collected and inoculated into the bioreactor, where the inoculated cells have a density of 1.0-5.0×10⁵ cells /ml, and the cell culture fluid is replenished to 5L; when the culture conditions of the cells in the bioreactor are at 37°C, 30-50 rpm, and pH 7.15-7.25, and DO 30% -50%, samples are taken every day to test glucose concentration, cell density and cell morphology on the microvector; when the cell density in the bioreactor reaches 1.0-5.0× 10⁶ cells/ml, the recombinant adenovirus vaccine strains are inoculated in the bioreactor with MOI of 5-30; after inoculation, samples are taken every day to test the glucose concentration, virus titer in a culture supernatant and a cell pellet, and the morphology of cells on the microvector is observed; when most of the cells are shed from the microvector, the culture is terminated, a virus lysate is added into the bioreactor with the final concentration of 0.05%-1% Tween 20, and lysed at 37°C for 2-4 hours, and then the virus solution is collected.

### 4. Purification of recombinant adenovirus SARS-COV-2 vaccine

Collected viruses are purified through cesium chloride ultracentrifugation or ion-exchange column chromatography by the following specific process steps:

### (1) Purification of adenovirus vaccine through cesium chloride ultracentrifugation

1200g collected culture with virus is centrifuged for 10 minutes; a culture supernatant containing virus is sucked; a cell pellet is re-suspended with the supernatant containing virus at 1/10 culture volume and is repeatedly frozen and thawed for three times in a refrigerator at -80°C and a water bath at 37°C, centrifuged for 10-20 minutes at 3000rpm; and then the supernatant is sucked. The culture supernatant containing virus is concentrated 10 times with a 100K-300K ultrafiltration membrane; 1.4g/ml cesium chloride solution (53g cesium chloride +87ml of 10mM Tris-HCl, PH 7.9) and 1.2g/ml cesium chloride solution (26.8g cesium chloride+92ml of 10mM Tris-HCl, PH 7.9) are prepared; 8ml of 1.4g/ml cesium chloride solution and 6ml of 1.2g/ml cesium chloride solution are slowly added into an overspeed tube sequentially; finally, 20ml supernatant containing virus is added at the top of discontinuous gradient and centrifuged at 100000×g at 4°C for 90 minutes after balanced; and after centrifugation, a blue virus band is aspirated by a syringe, and cesium chloride is removed through dialysis and stored at -80°C.

### (2) Purification of adenovirus through ion exchange chromatography

A virus culture is collected, and lysed with0.05%-1% Tween 20at 37°C for 2-4 hours; the lysed culture is filtrated and clarified with 1.2µm and 0.45µm bag filters; the sample is concentrated 10 times with a tangential flow membrane with a molecular weight of 100kD-300kD, and then difiltrated with buffer( 10 times the concentrated sample volume) (50mM Tris-HCl, 2mM MgCl₂, 0mM-500mM NaCl, PH 8.0); the filtrated sample is collected and mixed with nuclease; the mixture has a final concentration of 10U/ml-50U/ml and dissociated at 37°C for 1-3 hours; and the sample is subjected to anion exchange chromatography with Q Sepharose XL, Source 30Q or Source 15Q and other fillers by the following specific steps: balancing the buffer at a flow rate of 20ml/min to balance five column volumes; after balancing, loading the sample at a flow rate of 10ml/min; after sample loading, balancing the buffer to a conductivity level; eluting the sample through linear gradient under the elution conditions of 100% low-salt buffer to 100% high-salt buffer, the elution column volume of 10V and the flow rate of 10ml/min; collecting each elution peak; after elution, regenerating 5-10 column volumes for the column with 2M NaCl buffer solution at a flow rate of 20ml/min; collecting the virus peaks, and then conducting buffer replacement for the eluted virus samples through dialysis or tangential flow filtration (buffer 10mM Tris, 10mM Na-PO4, 150mM NaCl, 2mM MgCl₂, 2% sucrose, 0.15% glycerol and 0.02% Tween 80, PH7.6). The purified adenovirus is filled directly and stored in a dark place at -20°C.

Adenoviruses or vaccines prepared by Embodiment 1 are defined as Ad5_{WT} (SEQ ID No.24), Ad5_{Beta} (SEQ ID No.26) , Ad5_{Delta} (SEQ ID No.28) , Ad5 _{BA.1} (SEQ ID No.32) , Ad5 _{BA.4/5} (SEQ ID No.38) 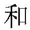 Ad5_{Beta/Delta} (SEQ ID No.30) recombinant adenoviruses, respectively.

The effect of the recombinant adenovirus SARS-COV-2 vaccine is proved by the following experimental examples.

### Experiment 1.1 Identification of recombinant adenovirus vaccine

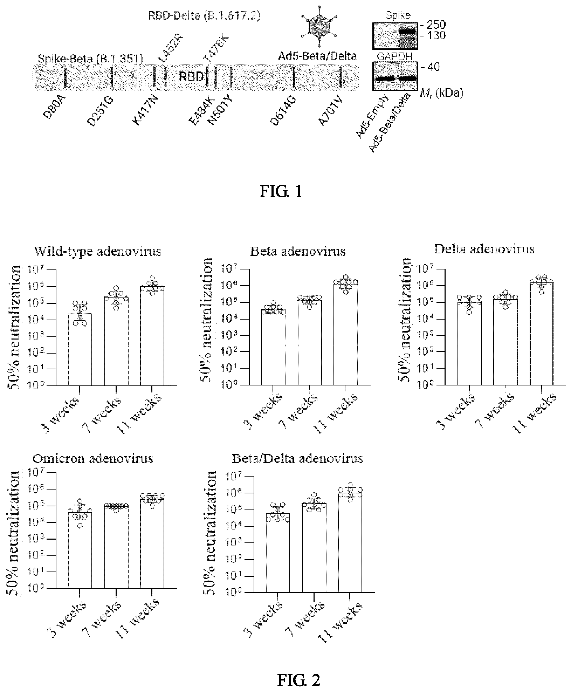

We expressed full-length spike glycoprotein of SARS-CoV-2 original strain, Beta strain, Delta strain, Omicron BA.1 and Omicron BA.4/5 respectively by human replication-deficient Ad5 adenovirus vectors. Meanwhile, we designed and constructed adenovirus Ad5-Beta/Delta (FIG 1) of two mutations L452R and T478K of Delta strain added in the RBD domain based on the full-length spike glycoprotein of Beta strain. The expression level of Spike in 293T cells infected with recombinant Ad5_{Beta/Delta} virus for 48 hours was detected by Western blot. As shown in FIG 1, 293T cells infected with adenovirus Ad5_{Beta/Delta} could induce high level of Spike expression, while such cells infected with empty adenovirus control Ad5_{Empty} could not do this, indicating that our recombinant adenovirus protein vaccine was successfully prepared.

### Experiment 1.2 Animal preparation and mouse immunization and sample collection

Female BALB/c mice at 6-8 weeks of age were purchased from Charles River, and the mice were housed in a pathogen-free environment at the State Key Laboratory of Biotherapy, Sichuan University. Mice were divided into seven groups: (1) PBS group; (2) Ad5_{Empty}; (3) Ad5_{WT}; (4) Ad5_{Beta}; (5) Ad5_{Delta}; (6) Ad5_{BA.1}; and (7) Ad5_{Beta/Delta} group. BALB/c mice were subjected to intranasal immunization respectively at week 0, 4, and 8 at a dose of 5×10⁹ viral particles VP/piece. Blood samples were collected through orbital veins at week 3 and 7; mice were killed at week 11; blood, bronchoalveolar lavage fluid and lung and mediastinal lymph node tissues were collected; and the tissues were prepared into single-cell suspensions and subjected to flow cytometry. The blood was centrifuged for 10 minutes at 4°C and 6000rpm. Serum samples were stored at -20°C prior to use for subsequent detection of binding and neutralizing antibodies in serums.

### Experiment 1.3 Detection of anti-RBD specific antibody

RBD specific antibody in serum was detected by ELISA. We coated 96-well NUNC-MaxiSorp plates (Thermo Fisher Scientific, USA) with recombinant RBD proteins (0.1µg/well) at 4°C for 12 hours. Such plates were washed three times with 1×PBST (1×PBS + 0.1% Tween-20) and blocked with 1% bovine serum albumin (BSA) at 37°C for 1 hour. The culture plates and serum samples with 2x dilution gradient were incubated at room temperature for 1 hour, and then washed three times by 1×PBST. The culture plates were added with 1:10000 diluted horseradish peroxidase (HRP)-goat anti-mouse IgG antibody, incubated at room temperature for 1 hour, and washed five times. The culture plates were added with 3,3', 5,5'-tetramethylbenzidine (TMB) and incubated in a dark place for 10 minutes. The reaction was stopped with 1 M H₂SO₄ (100µl/well) and the absorbance was measured at a position of 450nm.

As shown in FIG 2, recombinant adenovirus vaccines Ad5_{WT}, Ad5_{Beta}, Ad5_{Delta}, Ad5_{BA.1}, and Ad5_{Beta/Delta} all induced the high levels of anti-RBD specific binding antibodies in the blood at week 3, 7, and 11 after vaccination, and thus the levels of the neutralizing antibodies in the serums were further detected to evaluate broad-spectrum neutralizing immunoreaction induced by various recombinant adenovirus vaccines.

### Experiment 1.4 Pseudovirus neutralization assay

In addition to BA.3 and BA.4/5 pseudoviruses purchased from Vazyme Biotech Co., Ltd. (China), we purchased other SARS-COV-2 variant pseudoviruses of SARS-CoV-2 pseudovirus (GFP-Luciferase) from Genomeditech (Shanghai) Co., Ltd. Serum samples collected after the mice were killed at week 11 were inactivated at 60°C for 30 min and then were diluted in a three-fold gradient with DMEM dual culture medium containing serum and antibiotics. Diluted serums were incubated with luciferase pseudoviruses (wild type, B.1.1.7, B.1.351, P.1, B.1.617, C.37, BA.1, BA.2, BA.2.12.1, BA.3, and BA.4/5) for 1 hour at 37°C. 1.2×10⁴ 293T/ACE2 cells were added to each well to express reporter genes. After 48 hours, the supernatant of the infected cells was removed, and 100µl lysis reagent containing luciferase substrate was added to each well. Finally, a multi-mode microplate reader (PerkinElmer, USA) was used for detection.

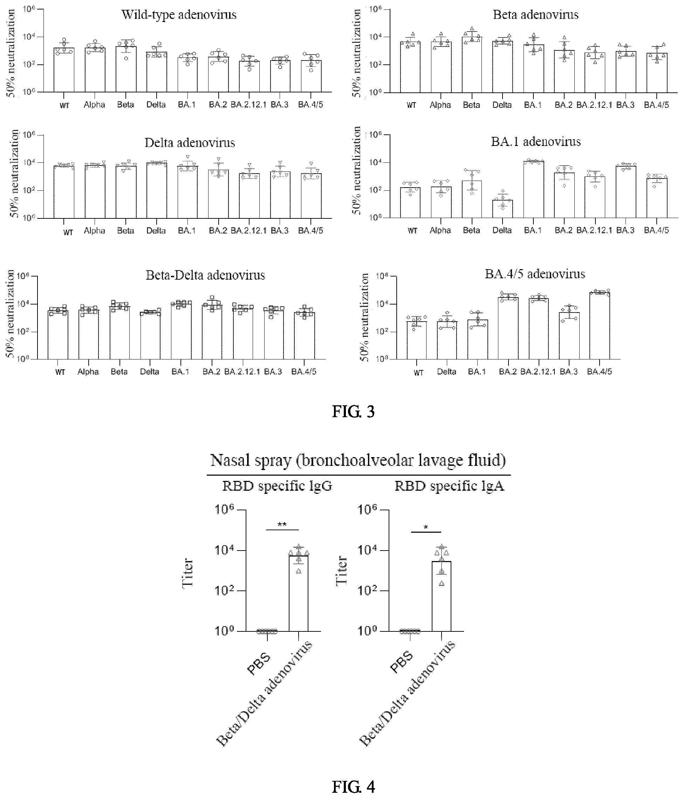

As shown in FIG 3, Ad5_{WT}, Ad5_{Beta}, and Ad5_{Delta} induced high levels of neutralizing antibodies against their own viruses, but such vaccines induced low levels of neutralizing antibodies against Omicron subvariant due to the strong immune escape capability of Omicron strain. Ad5-Omicron vaccine could induce high levels of neutralizing antibodies against the Omicron subvariant, but was unable to induce high levels of neutralizing antibodies against WT, Alpha, Beta, Delta and others. Compared with other adenovirus vaccines, Ad5_{Beta/Delta} could induce high levels of broad-spectrum neutralizing antibodies against a wide range of viruses including WT, Alpha, Beta, Delta, and Omicron BA.1, BA.2, BA.2.12.1, BA.3 and BA.4/5.

### Experiment 1.5 Detection of anti-RBD specific IgG and IgA antibodies in bronchoalveolar lavage fluid

RBD specific antibody IgG and IgA in bronchoalveolar lavage fluid were detected by ELISA. We coated 96-well NUNC-MaxiSorp plates (Thermo Fisher Scientific, USA) with recombinant RBD proteins (0.1µg/well) at 4°C for 12 hours. Such plates were washed three times with 1×PBST (1×PBS + 0.1% Tween-20) and blocked with 1% bovine serum albumin (BSA) at 37°C for 1 hour. The culture plates and bronchoalveolar lavage fluid samples with 2x dilution gradient were incubated at room temperature for 1 hour, and then washed three times by 1×PBST. The culture plates were added with 1:10000 diluted horseradish peroxidase (HRP)-goat anti-mouse IgG or IgA (diluted at 1:5000) antibody, incubated at room temperature for 1 hour, and washed five times. The culture plates were added with 3,3', 5,5'-tetramethylbenzidine (TMB) and incubated in a dark place for 10 minutes. The reaction was stopped with 1 M H₂SO₄ (100µl/well) and the absorbance was measured at a position of 450nm.

As shown in FIG 4, the recombinant adenovirus vaccine Ad5_{Beta/Delta} induced high levels of anti-RBD-specific conjugated antibodies IgG and IgA in the bronchoalveolar lavage fluid at week 11 after vaccination, indicating that our recombinant adenovirus vaccine can induce strong antibody protection in the mucosal portion of the respiratory tract.

### Experiment 1.6 Detection of tissue resident T cells in bronchoalveolar lavage fluid

The number of tissue resident T cells (T_{RM}) in the bronchoalveolar lavage fluid was detected using flow cytometry. 1ml collected bronchoalveolar lavage fluid was centrifuged (400×g, 5 minutes), and the supernatant was removed to obtain the cell pellet. After the cells were re-suspended with 100µl PBS buffer, PerCP/Cyanine5.5-conjugated anti-mouse CD3 (BioLegend, 100718), Brilliant Violet 421-conjugated anti-mouse CD4 (BioLegend, 100412), Brilliant Violet 510-conjugated anti-mouse CD8, PE-conjugated anti-mouse CD44, FITC-conjugated anti-mouse CD69, and APC-conjugated anti-mouse CD103 were added and incubated at 4°C for 30 minutes. The cells were re-selected after being washed once with PBS, and detected by flow cytometry.

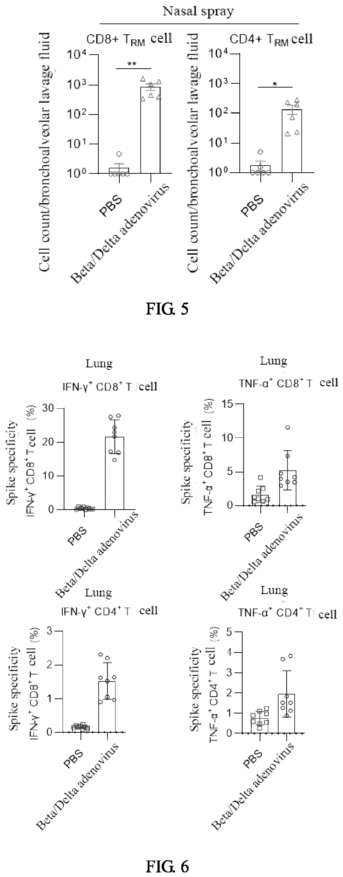

As shown in FIG. 5, when such mice subjected to intranasal immunization with Ad5_{Beta/Delta} were compared with PBS-treated mice, adenovirus vaccine could recruit a large number of tissue resident CD4⁺ and CD8⁺ T cells in lung tissue, resulting in cellular immunoprotection.

### Experiment 1.7 Detection of antigen-specific T cells in lung tissue

Antigen-specific T cells in lung tissue were detected through flow cytometry. After the collected lung tissue was cut to a size of 1mm³, the prepared collagenase digestive fluid was added and incubated at 37°C for 1 hour, and a tissue digestive fluid was filtrated through a 70uM sieve to obtain a single-cell suspension. Single cells of the lung tissue were stimulated and cultured in dual 1640 culture medium containing Spike peptide library for 12 hours, and subjected to intracellular cytokine staining (ICS). The 1640 culture medium was added with 10% fetal bovine serum, 100µg/ml streptomycin, 100U/ml penicillin, 1mM pyruvic acid (all purchased from Gibco), 50µM β-mercaptoethanol and 20U/ml IL-2 (all purchased from Sigma-Aldrich). To block the secretion of intracellular cytokines, brefeldin A (BFA, BD Biosciences) was incubated for 6 hours prior to staining. The cells were washed in a 1× PBS coolant, and stained with PerCP/Cyanine5.5-conjugated anti-mouse CD3 (BioLegend, 100718), APC-conjugated anti-mouse CD4 (BioLegend, 100412), FITC-conjugated anti-mouse CD8, and PE-conjugated anti-mouse CD44 antibodies at 4°C for 30 minutes. Then, the cells were immobilized and perforated, so that they could be stained with PE-Cy7-conjugated anti-mouse IFN-γ and Brilliant Violet 510-conjugated anti-mouse TNF-α (all flow antibodies from BioLegend) after left for 2 hours at room temperature. The cells were washed with 1×PBS and then detected by flow cytometry.

T cell response is a major determinant of clinical outcome and plays an important role in the prevention of SARS-CoV-2. As shown in FIG 6, the cells subjected to intranasal immunization with Ad5_{Beta/Delta} vaccine induce strong antigen-specific cellular immunity, including T cells that secrete IFN-γ and TNF-α.

### Experiment 1.8 Detection of reactions of a germinal center

After a single-cell suspension from mediastinal lymphocyte was prepared, we used PerCP/Cyanine5.5-conjugated anti-mouse CD3, PE-Cy7-conjugated anti-mouse CD45R/B220, Brilliant Violet 421-conjugated anti-mouse GL-7, APC-conjugated anti-mouse CD95, Brilliant Violet 421-conjugated anti-mouse CD19, APC-conjugated anti-mouse CD4, PE-conjugated anti-mouse CXCR5, and Brilliant Violet 510-conjugated PD-1 antibodies to stain Tfh cells and GC B cells (flow cytometry antibodies were derived from BioLegend). The cells were stained in the dark at 4°C. After 30 minutes, the cells were washed with 1×PBS and then detected by a flow cytometer.

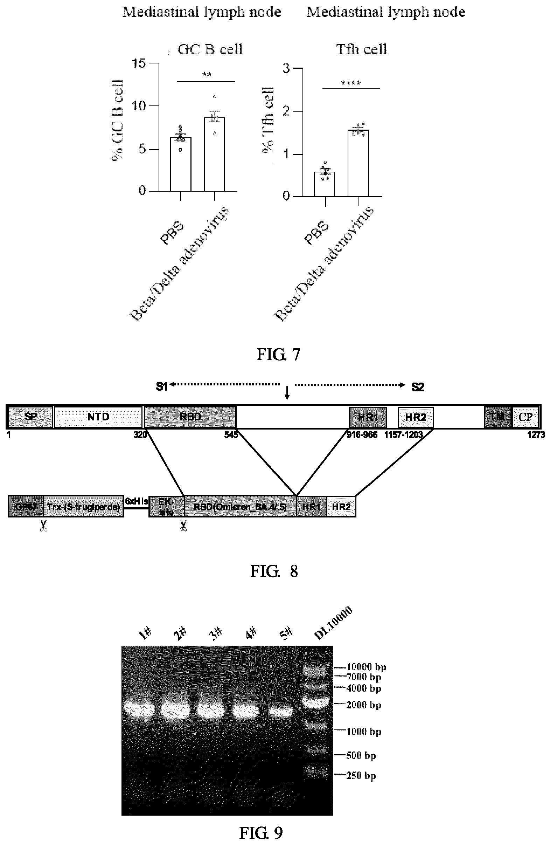

A durable anamnestic reaction provides rapid and effective protective immunity in case of reinfection. The increase of Tfh and GCB cells indicates that the vaccine can provide long-lasting protective immunity to the body. As shown in FIG 7, the cells subjected to intranasal immunization with Ad5_{Beta/Delta} vaccine could induce the significant increase of Tfh and GCB cells in mediastinal lymph nodes, demonstrating that our recombinant adenovirus vaccine can improve long-lasting immunoprotection.

### Part II: immunoprotection induced by individual proteins

### Embodiment 2.1 Preparation of recombinant protein using insect baculovirus expression system (taking S-RBD-HR_{BA.4/5} as an example)

### 1. Construction design of S-RBD (Omicron_BA.4/.5)-HR

The S protein of SARS-CoV-2 is a membrane-localized protein. To ensure the simulation of its secretion process, we added a signal peptide sequence of GP67 at the N-terminus of the protein when constructing the SARS-CoV-2 S-RBD (Omicron_BA.4/.5)-HR protein expression to assist the secretion expression of the protein. This signal peptide will be spontaneously removed by insect cells in a protein secretion process. In addition, we added the thioredoxin (Trx) tag of *Spodoptera frugiperda, S. frugiperda* behind the GP67 signal peptide to assist the folding of the S-RBD (Omicron_BA.4/.5)-HR, and added a 6xhis tag to assist the subsequent purification and an EK restriction enzyme cutting site to remove the Trx and 6xhis tags. The protein expression can remove all non-S-RBD (Omicron_BA,4/ 5)-HR redundant amino acids by EK restriction enzyme. A protein amino acid design sequence is shown in SEQ ID No.6. An expression construction design pattern is shown in FIG 8.

### 2. Identification of recombinant plasmid construction

The designed coding fragments are cloned into pFastBac1 vector plasmids and identifie d through bacterial PCR. Identification results through bacterial PCR show that GP67-Trx-Hi s-EK-S-RBD (Omicron_BA.4/.5)-HR fragments are successfully expanded in all five selected clones, as shown in FIG. 9.

### 3. Identification of recombinant bacmid

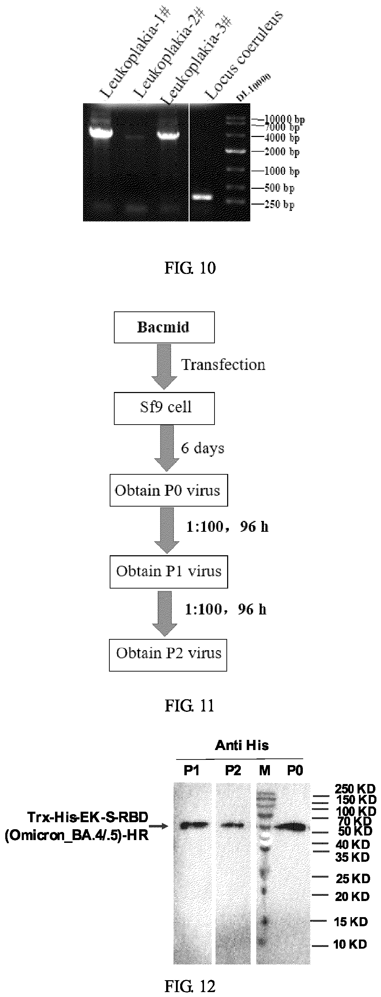

Correct pFastBac1-GP67-Trx-His-EK-S-RBD (Omicron_BA.4/.5)-HR recombinant clones are selected to extract the recombinant plasmids, and DH10b competent cells are transforme d and identified through colony PCR. Colony PCR products are detected through 1% agaros e gel electrophoresis, and the identification results are shown in FIG. 10. Leukoplakia is rec ombinant bacmid clone and locus coeruleus is non-recombinant bacmid clone.

### 4. Packaging of recombinant baculovirus

Recombinant bacmid is transfected into sf9 insect cells. After 5-6 days, recombinant baculovirus P0 is harvested and inoculated into fresh sf9 insect cells at a ratio of 1:100. After 4 days, recombinant baculovirus P1 is harvested and inoculated into fresh sf9 insect cells at the ratio of 1:100. After 4 days, recombinant baculovirus P2 is harvested. A flow diagram of packaging and amplification of recombinant baculovirus is shown in FIG 11.

### 5. Verification of target protein expression

Baculovirus amplification is accompanied by target protein expression. Before the remo val of tags, the target protein contains His tag, so we verify a recombinant protein expressi on through anti-His WB experiment. Verification results show that an obvious band is obser ved between 40 KD and 55 KD Marker bands, and its size is consistent with the size of Trx-His-EK-S-RBD (Omicron_BA.4/.5)-HR protein, indicating that the baculovirus amplificati on and the target protein expression are successful. Detection results are shown in FIG 12.

Recombinant protein vaccines RBD-WT, RBD-HR_{Delta}, RBD-HR_{BA.4/.5}, and RBD-HR_{BA.1/Delta/Beta} are respectively constructed by the construction method of the recombinant protein based on the amino acid sequence of S protein RBD of SARS-CoV-2 or the amino acid sequence of S protein RBD-HR of SARS-CoV-2 mutant: SEQ ID No.1, SEQ ID No.3, SEQ ID No.6 and SEQ ID No.18, all of which are used for animal immunity and other subsequent studies.

### Embodiment 2.2 Preparation of recombinant protein using CHO cell expression system (taking S-BA.4/5 as an example)

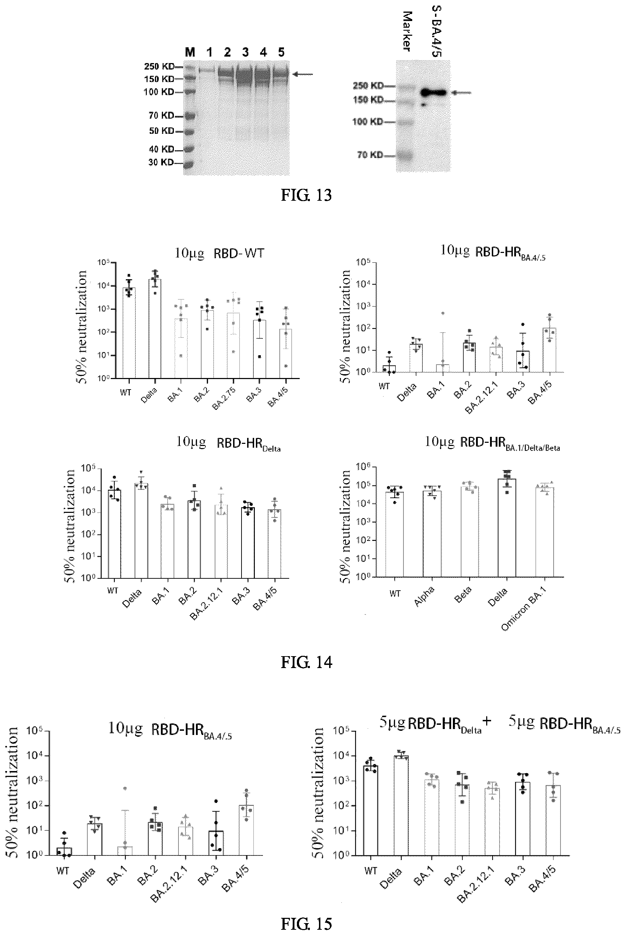

Recombinant protein vaccines produced by CHO cells are mainly targeted at full-length S proteins, and these fragments are genetically synthesized according to codon preference, and polyhistidine is used as a purification tag (6His), and the complete nucleotide sequence is shown in SEQ ID No.71. Subsequently, the high expression vector pTT5 is constructed to express a precursor protein with an amino acid sequence as shown in SEQ ID No.70. After vector construction, CHO cells are transfected, and a harvested solution is taken for SDS-PAGE and Western-blot tests, as shown in FIG 13. This proves that the target protein has been successfully expressed.

Recombinant proteins and vaccines S-WT, S-Beta, S-Delta, S-BA.1, S-BA.2, and S-BA.4/5 are respectively constructed by the construction method of the recombinant protein based on the full-length amino acid sequence of S protein of SARS-CoV-2: SEQ ID No.40, SEQ ID No.46, SEQ ID No.52, SEQ ID No.58, SEQ ID No.64, and SEQ ID No.70, all of which are used for animal immunity and other subsequent studies.

The effect of the recombinant protein vaccine is proved by the following experimental examples.

### Experiment 2.1 Animal preparation and mouse immunization and sample collection

Female BALB/c mice at 6-8 weeks of age were purchased from Charles River, and the mice were housed in a pathogen-free environment at the State Key Laboratory of Biotherapy, Sichuan University.

BALB/c mice were subjected to intranasal immunization at day 0, 14 and 28. Blood samples were collected through orbital veins at day 42; mice were killed at week 11; blood, bronchoalveolar lavage fluid and lung and mediastinal lymph node tissues were collected; and the tissues were prepared into single-cell suspensions subjected to flow cytometry. The blood was centrifuged for 10 minutes at 4°C and 6000rpm. Serum samples were stored at -20°C prior to use for subsequent detection of binding and neutralizing antibodies in serums.

### Experiment 2.2 Pseudovirus neutralization assay after immunization with a single protein vaccine

We used pseudoviruses to detect neutralizing antibodies in serums of immunized mice, where BA.3 and BA.4/5 pseudoviruses were purchased from Vazyme Co., Ltd. (China); and wild-type B.1.1.7, B.1.351, P.1, B.1.617, C.37, BA.1, BA.2, BA.2.12.1 and other pseudoviruses were purchased from Genomeditech (Shanghai) Co., Ltd. Serum samples collected after the mice were killed at week 11 were inactivated at 60°C for 30 min and then were diluted in a three-fold gradient with DMEM dual culture medium containing serum and antibiotics. Diluted serums were incubated with luciferase pseudoviruses (wild type, B.1.1.7, B.1.351, P.1, B.1.617, C.37, BA.1, BA.2, BA.2.12.1, BA.3, and BA.4/5) for 1 hour at 37°C. 1.2×10⁴ 293T/ACE2 cells were added to each well to express reporter genes. After 48 hours, the supernatant of the infected cells was removed, and 100µl lysis reagent containing luciferase substrate was added to each well. Finally, a multi-mode microplate reader (PerkinElmer, USA) was used for detection.

As shown in FIG 14, wild-type RBD-WT, RBD-HR_{Delta}, and RBD-HR_{BA.1/Delta/Beta} proteins induced higher levels of neutralizing antibodies against WT, Alpha, Beta, Delta, and Omicron viruses, where individual RBD-HR_{Delta} protein and chimeric RBD-HR_{BA.1/Delta/Beta} proteins containing Beta and Delta mutants showed strong immunoprotection against WT, Alpha, Beta, Delta, Omicron and other viruses; individual RBD-WT protein showed strong immunoprotection only against WT and Delta mutant strains and weak immunoprotection against Omicron; and individual RBD-HR_{BA.4/.5} protein showed no strong immunoprotection against WT, Delta and Omicron. The RBD-HR_{BA.1/Delta/Beta} protein showed strong immunoprotection against various types of SARS-COV-2.

### Experiment 2.3 Neutralizing antibodies induced by bivalent RBD protein

In the above experiments, we found that individual RBD-HRBA.4/.5 protein had no obvious immunoprotection against WT, Delta, Omicron and other viruses. After mice were immunized with a bivalent combination vaccine (5 micrograms of RBD-HR_{Delta}+5 micrograms of RBD-HR_{BA.4/.5} protein SEQ ID No.3+ SEQ ID No.6), a high-titer neutralizing antibody was induced and had significantly stronger immunoprotection than the individual RBD-HRBA.4/.5 protein, indicating the effectiveness of the bivalent vaccine, as shown in FIG 15.

### Experiment 2.4 Pseudovirus neutralization assay for different S proteins

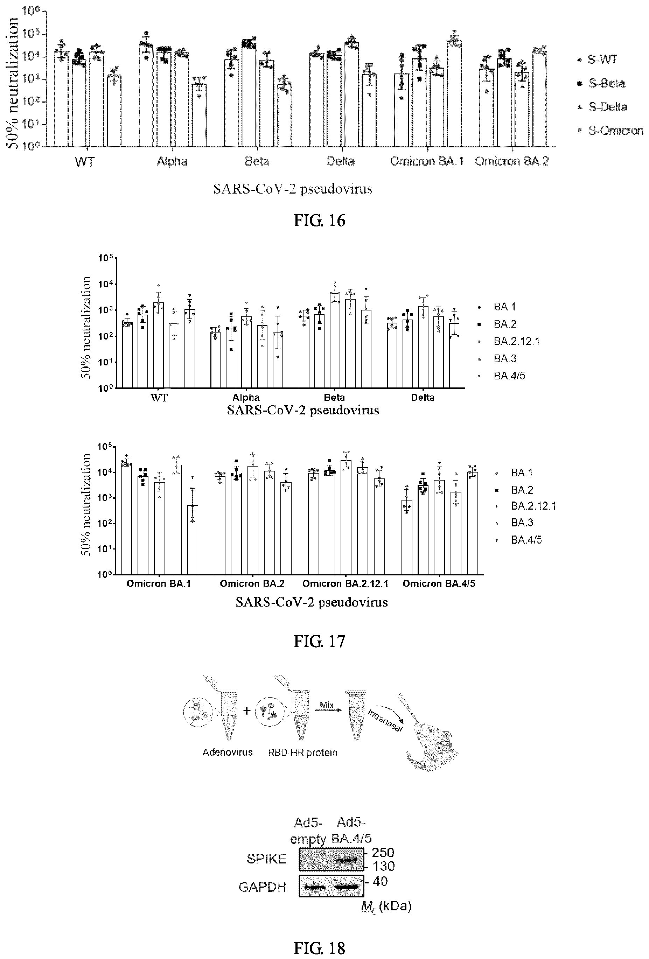

In order to verify the immunoprotection induced by S proteins (full-length trimers) of different virus strains, we immunized mice on day 0, 14, and 28 with prepared S proteins, where S proteins of BA.2.12.1 and BA.3 were commercially available S proteins. S-WT, S-Beta, S-Delta, S-BA.1, S-BA.2 and S-BA.4/5 were all recombinant S proteins prepared with reference to Embodiment 2.2. In an immunization protocol, 10 micrograms of protein +MF59 adjuvant are provided. Pseudovirus neutralizing antibodies were determined by removing the antiserum on day 42, as shown in FIG 16 and FIG 17.

As shown in FIG 16, S proteins of WT, Beta and Delta strains have strong immunoprotection against the WT, Beta and Delta strains, but have weak immunoprotection against Omicron strains. S proteins (S-BA.1 and S-Ba.2) of Omicron strains have significantly stronger immunoprotection against Omicron strains than other strains.

As can be seen from FIG. 17, full-length S proteins of B.1, BA.2, BA.2.12.1, BA.3, and BA.4/5 have significantly stronger immunoprotection against Omicron strains than WT, Alpha, Beta and Delta strains, showing certain specificity.

### Part III: stronger immunoprotection induced by adenovirus combined with trimeric recombinant protein

### Experiment 3.1 Preparation of mixed preparation of adenovirus vaccine and recombinant protein vaccine

We expressed the full-length spike glycoprotein of SARS-CoV-2BA.4/5 strain by human replication-deficient Ad5 adenovirus vectors. The expression level of Spike in 293T cells infected with recombinant Ad5_{BA.4/5} virus for 48 hours was detected by Western blot. The detailed steps are shown in Embodiment 1. As shown in FIG 18, we mixed different doses of adenovirus Ad5_{BA.4/5} with RBD-HR_{Delta} produced in Embodiment 2.1 to prepare the preparation for intranasal immunization. The adenovirus Ad5_{BA.4/5} was prepared based on SEQ ID No.38. The recombinant protein RBD-HR_{Delta} was prepared based on SEQ ID No.3.

Similarly, adenovirus Ad5_{WT} was prepared based on SEQ ID No.24, S-BA.4/5 protein was prepared based on SEQ ID No.71, and RBD-WT protein was prepared based on SEQ ID No.1. We mixed the adenovirus Ad5_{WT} with the RBD-WT protein produced in Embodiment 2 respectively to prepare a preparation. The adenovirus Ad5_{BA.4/5} was mixed with the S-BA.4/5 protein produced in Embodiment 2.2 to prepare a preparation for intranasal immunization.

### Experiment 3.2Animal preparation and mouse immunization and sample collection

Female BALB/c mice at 6-8 weeks of age were purchased from Charles River, and the mice were housed in a pathogen-free environment at the State Key Laboratory of Biotherapy, Sichuan University. The mice were divided into 6 groups: (1) RBD-HR_{Delta} (10µg/piece) control group, (2) Ad5_{empty} (5×10⁹ VP/piece) + RBD-HR_{Delta} (10µg/piece), (3) Ad5_{BA.4/5} (2.5×10⁹ VP/piece) low-dose group, (4) Ad5_{BA.4/5} (2.5×10⁹ VP/piece) low dose + RBD-HR_{Delta} (10µg) group, (5) Ad5_{BA.4/5} (5×10⁹ VP/piece) high-dose group; and (6) Ad5_{BA.4/5} (5×10⁹ VP/piece) high-dose + RBD-HR_{Delta} (10µg) group.

In addition, the experimental grouping of Ad5_{WT}+RBD-WT combined drug was as follows: (a) PBS (5 pieces), (b) RBD-WT vaccine group (10µg/piece, 5 pieces), (c) adenovirus Ad5_{WT} (5×10⁹ VP/piece), and (d) Ad5_{WT} (5×10⁹ VP/piece) + RBD-WT group (10µg/piece) (5 pieces).

The experimental grouping of Ad5_{BA.4/5}+S-BA.4/5 combined drug was as follows: (a) PBS (5 pieces), (b) S-BA.4/5 vaccine group (10µg/piece, 5 pieces), (c) Ad5_{BA.4/5} group (5×10⁹VP/piece, 5 pieces), (d) Ad5_{BA.4/5} (5×10⁹ VP/piece) high dose + S-BA.4/5 (10µg/piece) group (5 pieces).

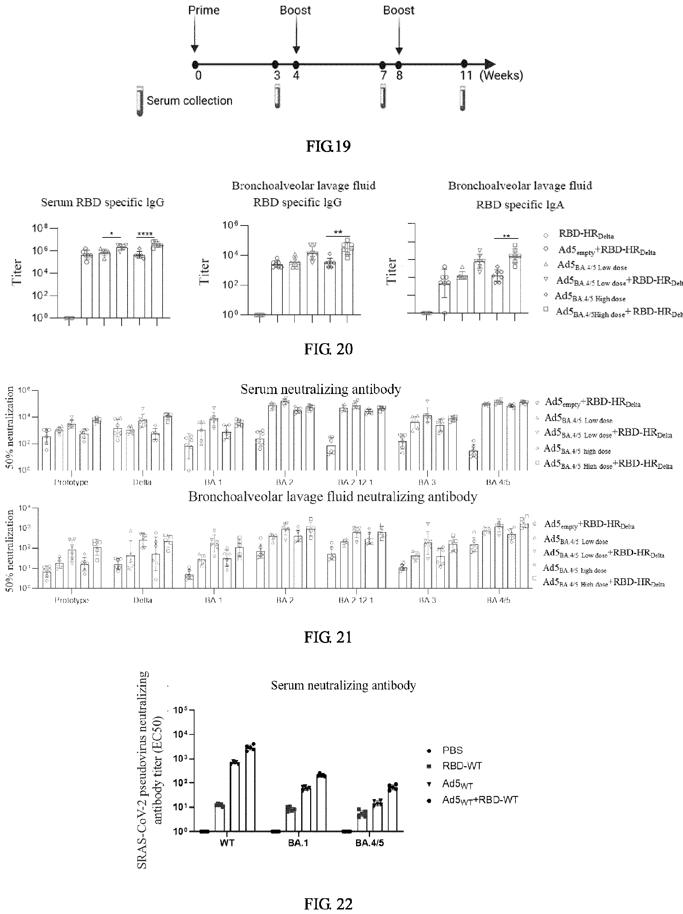

BALB/c mice were subjected to intranasal immunization at weeks 0, 4 and 8. Blood samples were collected through orbital veins at week 3 and 7; mice were killed at week 11; blood, bronchoalveolar lavage fluid and lung and mediastinal lymph node tissues were collected; and the tissues were prepared into single-cell suspensions and subjected to flow cytometry. The blood was centrifuged for 10 minutes at 4°C and 6000rpm. Serum samples were stored at -20°C prior to use for subsequent detection of binding and neutralizing antibodies in serums. The immunization protocol is shown in FIG 19.

### Experiment 3.3 Detection of anti-RBD specific antibodies in serum and bronchoalveolar lavage fluid

RBD specific antibody in serum was detected by ELISA. We coated 96-well NUNC-MaxiSorp plates (Thermo Fisher Scientific, USA) with recombinant RBD proteins (0.1µg/well) at 4°C for 12 hours. Such plates were washed three times with 1×PBST (1×PBS + 0.1% Tween-20) and blocked with 1% bovine serum albumin (BSA) at 37°C for 1 hour. The culture plates and serum samples or bronchoalveolar lavage fluid samples with 2x dilution gradient were incubated at room temperature for 1 hour, and then washed three times by 1×PBST. The culture plates were added with 1:10000 diluted horseradish peroxidase (HRP)-goat anti-mouse IgG antibody, incubated at room temperature for 1 hour, and washed five times. The culture plates were added with 3,3', 5,5'-tetramethylbenzidine (TMB) and incubated in a dark place for 10 minutes. The reaction was stopped with 1 M H₂SO₄ (100µl/well) and the absorbance was measured at a position of 450nm.

As shown in FIG 20, after immunization, a preparation of adenovirus Ad5_{BA.4/5} combined with recombinant RBD-HR_{Delta} induces stronger anti-RBD specific binding antibodies in the serum and bronchoalveolar lavage fluid than that of Ad5_{BA.4/5}, indicating that a preparation of adenovirus combined with recombinant subunit can produce stronger humoral immunoprotection. In addition, intranasal immunization with RBD-HR_{Delta} cannot induce strong blood-conjugated antibodies, but RBD-HR_{Delta} being conjugated with Ad5_{Empty} can significantly increase its immunogenicity. This demonstrates that the adenovirus can be used as an adjuvant of recombinant subunit to enhance the immunogenicity of protein antigens.

### Experiment 3.4 Pseudovirus neutralization assay

In addition to BA.3 and BA.4/5 pseudoviruses purchased from Vazyme Biotech Co., Ltd. (China), we purchased other SARS-COV-2 variant pseudoviruses of SARS-CoV-2 pseudovirus (GFP-Luciferase) from Genomeditech (Shanghai) Co., Ltd. Serum samples collected after the mice were killed at week 11 were inactivated at 60°C for 30 min and then were diluted in a three-fold gradient with DMEM dual culture medium containing serum and antibiotics. Diluted serums were incubated with luciferase pseudoviruses (wild type, B.1.617, BA.1, BA.2, BA.2.12.1, BA.3, and BA.4/5) for 1 hour at 37°C. 1.2×10⁴ 293T/ACE2 cells were added to each well to express reporter genes. After 48 hours, the supernatant of the infected cells was removed, and 100µl lysis reagent containing luciferase substrate was added to each well. Finally, a multi-mode microplate reader (PerkinElmer, USA) was used for detection.

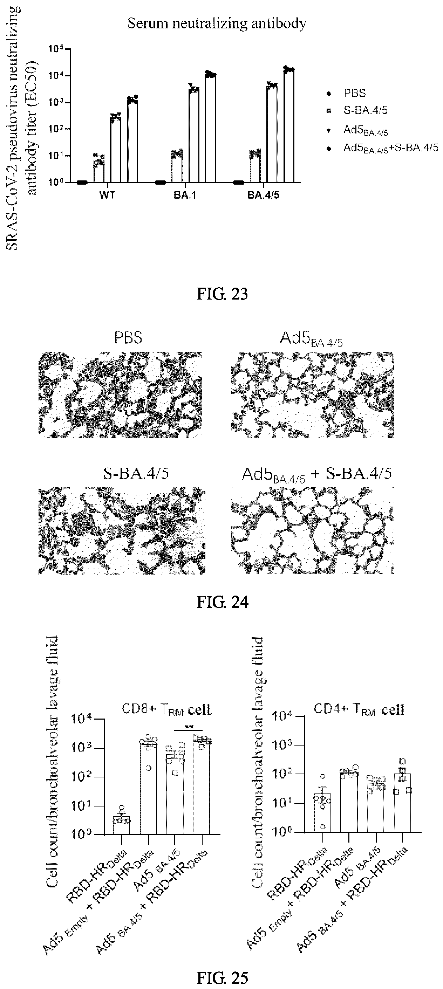

As shown in FIG 21, a preparation of adenovirus Ad5_{BA.4/5} combined with recombinant RBD-HR_{Delta} induced stronger neutralizing antibodies in serum and bronchoalveolar lavage fluid than that of adenovirus Ad5_{BA.4/5}, indicating that a preparation of adenovirus combined with recombinant subunit can produce stronger blood and local mucosal neutralizing protection ability to prevent virus infection. As shown in FIG 22, Ad5_{WT}+ RDB-WT produced serum neutralizing antibodies against viruses such as WT, BA.1 and BA.4/5. As shown in FIG 23, Ad5_{BA.4/5}+S-BA4/5 produced higher-level serum neutralizing antibodies against WT, BA.1, BA.4/5 and other viruses, and an amount of antibodies was significantly higher than that of Ad5_{WT}+RBD-WT, suggesting that Omicron's S protein adenovirus vaccine combined with S protein can produce stronger immunoprotection against Omicron mutant strains.

### Experiment 3.5 Challenge test of mice infected with SARS-CoV-2 virus

Female BALB/c mice at 6-8 weeks of age were purchased from Charles River and tested at the Animal Biosafety Level 4 (ABSL-4) Facility at the National Primate Research Center for Advanced BioSafety in Kunming, Yunnan Province, China. 20 mice were divided into the following groups for challenge test of live SARS-CoV-2: (a) PBS (5 pieces), (b) adenovirus Ad5_{BA.4/5} group (5x10⁹VP/piece, 5 pieces), (c) S-BA.4/5 protein vaccine group (10µg /piece, 5 pieces)and (d) AD5_{BA.4/5} (5×10⁹ VP/piece) high dose + S-BA.4/5 (10µg) group (5 pieces).

Mice were subjected to intranasal immunization at week 0, 4, and 8, and then treated with SARS-CoV-2 for nasal challenge (0.5ml, 10⁶pfu/ml) 21 days after the final immunization. Mouse lung tissues were collected 4 days after virus attack. As shown in FIG 24, after the mice were treated with immune serums induced by Ad5_{BA.4/5} (5×10⁹ VP/ piece) high dose + S-BA.4/5 (10µg) combined vaccine, viral loads in turbinate, trachea and lung tissues were not detected, lung histology was normal, alveolar structure was intact, and no obvious inflammation was present. In the PBS control group, minor pathological changes were observed, including multifocal consolidation area, mild alveolar septum thickening and alveolar congestion. In addition, small patches of inflammation consisting of macrophages, neutrophils, and lymphocytes were occasionally observed near small blood vessels. The effect of adenovirus or protein in the nasal drop is significantly weaker than that of the combined drug of adenovirus and protein.

### Experiment 3.6 Detection of tissue resident T cells in bronchoalveolar lavage fluid

The number of tissue resident T cells (T_{RM}) in the bronchoalveolar lavage fluid was detected using flow cytometry. 1ml collected bronchoalveolar lavage fluid was centrifuged (400×g, 5 minutes), and the supernatant was removed to obtain the cell pellet. After the cells were re-suspended with 100µl PBS buffer, PerCP/Cyanine5.5-conjugated anti-mouse CD3 (BioLegend, 100718), Brilliant Violet 421-conjugated anti-mouse CD4 (BioLegend, 100412), Brilliant Violet 510-conjugated anti-mouse CD8, PE-conjugated anti-mouse CD44, FITC-conjugated anti-mouse CD69, and APC-conjugated anti-mouse CD103 were incubated at 4°C for 30 minutes. The cells were re-selected after being washed once with PBS, and detected by flow cytometry.

As shown in FIG 25, a preparation of high-dose adenovirus Ad5_{BA.4/5} combined with recombinant RBD-HR_{Delta} recruited more lung tissue resident T_{RM} cells than that of adenovirus Ad5_{BA.4/5}. In addition, intranasal immunization with RBD-HR_{Delta} could not induce enough lung tissue T_{RM} cells. However, intranasal immunization with RBD-HR_{Delta} combined with Ad5_{Empty} could significantly improve its immunogenicity and induce more lung tissue T_{RM} cells, indicating that the preparation of adenovirus combined with recombinant subunit can produce stronger local mucosal cell immune response and prevent viral infection.

### Experiment 3.7 Detection of antigen-specific T cells in lung tissue

Antigen-specific T cells in lung tissue were detected through flow cytometry. After the collected lung tissue was cut to a size of 1mm³, the prepared collagenase digestive fluid was added and incubated at 37°C for 1 hour, and a tissue digestive fluid was filtrated through a 70uM sieve to obtain a single-cell suspension. Single cells of the lung tissue were stimulated and cultured in dual 1640 culture medium containing Spike peptide library for 12 hours, and subjected to intracellular cytokine staining (ICS). The 1640 culture medium was added with 10% fetal bovine serum, 100µg/ml streptomycin, 100U/ml penicillin, 1mM pyruvic acid (all purchased from Gibco), 50µM β-mercaptoethanol and 20U/ml IL-2 (all purchased from Sigma-Aldrich). To block the secretion of intracellular cytokines, brefeldin A (BFA, BD Biosciences) was incubated for 6 hours prior to staining. The cells were washed in a 1× PBS coolant, and stained with PerCP/Cyanine5.5-conjugated anti-mouse CD3 (BioLegend, 100718), APC-conjugated anti-mouse CD4 (BioLegend, 100412), FITC-conjugated anti-mouse CD8, and PE-conjugated anti-mouse CD44 antibodies at 4°C for 30 minutes. Then, the cells were immobilized and perforated, so that they could be stained with PE-Cy7-conjugated anti-mouse IFN-γ and Brilliant Violet 510-conjugated anti-mouse TNF-α (all flow antibodies from BioLegend) after left for 2 hours at room temperature. The cells were washed with 1×PBS and then detected by flow cytometry.

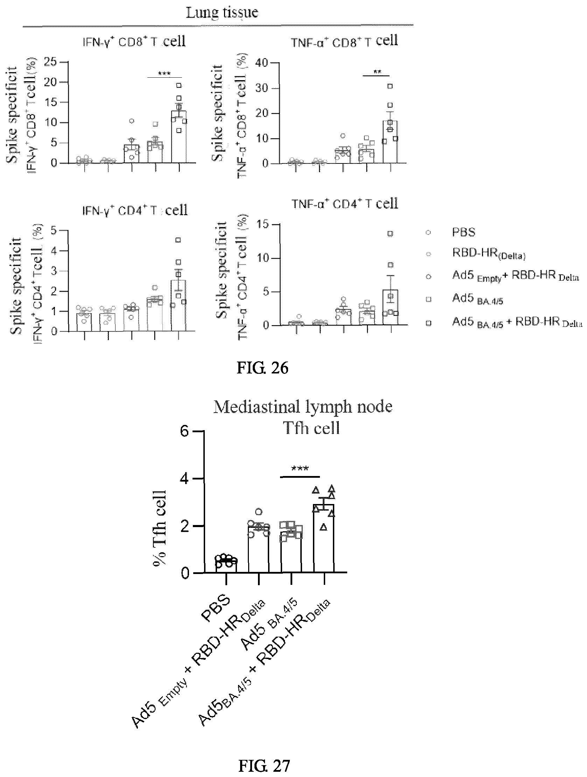

As shown in FIG 26, a preparation of high-dose adenovirus Ad5_{BA.4/5} combined with recombinant RBD-HR_{Delta} recruited more antigen-specific T cells than that of adenovirus Ad5_{BA.4/5}. In addition, intranasal immunization with RBD-HR_{Delta} could not induce enough lung tissue antigen-specific T cells. However, intranasal immunization with RBD-HR_{Delta} combined with Ad5_{Empty} could significantly improve its immunogenicity and induce more lung tissue antigen specificity, indicating that the preparation of adenovirus combined with recombinant subunit can produce stronger local mucosal cell immune response and prevent viral infection.

### Experiment 3.8 Detection of reactions of a germinal center

After a single-cell suspension from mediastinal lymphocyte was prepared, we used PerCP/Cyanine5.5-conjugated anti-mouse CD3, PE-Cy7-conjugated anti-mouse CD45R/B220, Brilliant Violet 421-conjugated anti-mouse GL-7, APC-conjugated anti-mouse CD95, Brilliant Violet 421-conjugated anti-mouse CD19, APC-conjugated anti-mouse CD4, PE-conjugated anti-mouse CXCR5, and Brilliant Violet 510-conjugated PD-1 antibodies to stain Tfh cells and GC B cells (flow cytometry antibodies were derived from BioLegend). The cells were stained in the dark at 4°C. After 30 minutes, the cells were washed with 1×PBS and then detected by a flow cytometer.

A durable anamnestic reaction provides rapid and effective protective immunity in case of reinfection. The increase of Tfh and GCB cells indicates that the vaccine can provide long-lasting protective immunity to the body. As shown in FIG 27, a preparation of high-dose adenovirus Ad5_{BA.4/5} combined with recombinant RBD-HR_{Delta} can induce Tfh cells in mediastinal lymph nodes to increase significantly, demonstrating that adenovirus vaccine combined protein vaccine can improve long-lasting immunoprotection.

## Claims

1. A pharmaceutical composition for preventing and/or treating infection with SARS-CoV-2 or a mutant thereof is **characterized by** being a compound preparation containing active ingredients of a recombinant protein vaccine and an adenovirus vaccine for resisting infection with SARS-CoV-2 or the mutant thereof.

2. A combined drug for preventing and/or treating infection with SARS-CoV-2 or a mutant thereof is **characterized by** containing a recombinant protein vaccine and an adenovirus vaccine for resisting infection with SARS-CoV-2 or the mutant thereof, wherein the recombinant protein vaccine and the adenovirus vaccine are administered separately or simultaneously.

3. The pharmaceutical composition according to claim 1 or the combined drug according to claim 2, **characterized in that** the pharmaceutical composition or the combined drug is an intramuscular injection, a nasal drop, a spray, a nasal spray or inhalation; and preferably, the pharmaceutical composition or the combined drug is a nasal spray.

4. The pharmaceutical composition according to claim 1 or the combined drug according to claim 2, **characterized in that** the recombinant protein vaccine and/or the adenovirus vaccine contain/contains a protein and/or a protein precursor for resisting infection with SARS-CoV-2 or the mutant thereof.

5. The pharmaceutical composition or the combined drug according to claim 4, **characterized in that** the protein and/or the protein precursor contain/contains a full-length S protein or a protein formed by at least one RBD sequence and/or at least one HR sequence in the S protein of SARS-CoV-2 or the mutant thereof;
preferably, the RBD sequence is shown in SEQ ID No.1; or the RBD sequence is a variant that has homology and same or similar biological activity with SEQ ID No.1 and is obtained by substitution and/or deletion and/or insertion of at least one amino acid in the SEQ ID No.1 sequence; preferably, the RBD sequence is a variant obtained by substitution and/or deletion and/or insertion of 1-400 amino acids in the SEQ ID No.1 sequence; and preferably, the RBD sequence is a variant obtained by substitution and/or deletion and/or insertion of 5-30 amino acids in the SEQ ID No.1 sequence.

6. The pharmaceutical composition or the combined drug according to claim 5, **characterized in that** the protein formed by the RBD sequence and the HR sequence in the S protein are capable of spontaneously forming a trimer.

7. The pharmaceutical composition or the combined drug according to claim 5, **characterized in that** the homologous amino acid sequence is selected from at least one of RBD sequences of Alpha, Beta, Gamma, Delta or Omicron.

8. The pharmaceutical composition or the combined drug according to claim 5, **characterized in that** the protein precursor is that a signal peptide and/or a protein tag are/is bonded to the protein for resisting infection with SARS-CoV-2 or the mutant thereof; preferably, the signal peptide comprises the signal peptide of the S protein or the mutant thereof and/or a human tPA signal peptide additionally provided outside the forehead of the self-provided signal peptide; preferably, the protein tag is selected from at least one of a histidine tag, a thioredoxin tag, a glutathione transferase tag, a ubiquitin-like modified protein tag, a maltose-binding protein tag, a c-Myc protein tag or a Avi tag protein tag; and more preferably, the protein tag is a Trx tag and/or a 6His tag.

9. The pharmaceutical composition or the combined drug according to claim 8, **characterized in that** the protein for resisting infection with SARS-CoV-2 or the mutant thereof is further bonded to a protease recognition zone for removing the protein tag; and preferably, the protease is selected from at least one of enterokinase, TEV protease, thrombin, coagulation factor Xa, carboxypeptidase A or rhinovirus 3c protease.

10. The pharmaceutical composition or the combined drug according to claim 5, **characterized in that** the amino acid sequence of the protein and/or the protein precursor are/is selected from at least one of SEQ ID No.1, SEQ ID No.2, SEQ ID No.3, SEQ ID No.4, SEQ ID No.5, SEQ ID No.6, SEQ ID No.7, SEQ ID No.8, SEQ ID No.9, SEQ ID No.18, SEQ ID No.20, SEQ ID No.22, SEQ ID No.40, SEQ ID No.43, SEQ ID No.46, SEQ ID No.49, SEQ ID No.52, SEQ ID No.55, SEQ ID No.58, SEQ ID No.61, SEQ ID No.64, SEQ ID No.67, SEQ ID No.70, or SEQ ID No.73.

11. The pharmaceutical composition or the combined drug according to claim 10, **characterized in that** a nucleotide sequence for encoding the amino acid sequence is shown in SEQ ID No.10, SEQ ID No.11, SEQ ID No.12, SEQ ID No.13, SEQ ID No.14, SEQ ID No.15, SEQ ID No.16, SEQ ID No.17, SEQ ID No.19, SEQ ID No.21, SEQ ID No.23, SEQ ID No.41, SEQ ID No.42, SEQ ID No.44, SEQ ID No.45, SEQ ID No.47, SEQ ID No.48, SEQ ID No.50, SEQ ID No.51, SEQ ID No.53, SEQ ID No.54, SEQ ID No.56, SEQ ID No.57, SEQ ID No.59, SEQ ID No.60, SEQ ID No.62, SEQ ID No.63, SEQ ID No.65, SEQ ID No.66, SEQ ID No.68, SEQ ID No.69, SEQ ID No.71, SEQ ID No.72, SEQ ID No.74, or SEQ ID No.75.

12. The pharmaceutical composition according to claim 1 or the combined drug according to claim 2, **characterized in that** the recombinant protein vaccine and/or the adenovirus vaccine contain/contains a nucleic acid for resisting infection with SARS-CoV-2 or the mutant thereof.

13. The pharmaceutical composition or the combined drug according to claim 12, **characterized in that** the nucleotide sequence of the nucleic acid is shown in SEQ ID No.24, SEQ ID No.26, SEQ ID No.28, SEQ ID No.30, SEQ ID No.32, SEQ ID No.34, SEQ ID No.36, SEQ ID No.38, SEQ ID No.41, SEQ ID No.42, SEQ ID No.44, SEQ ID No.45, SEQ ID No.47, SEQ ID No.48, SEQ ID No.50, SEQ ID No.51, SEQ ID No.53, SEQ ID No.54, SEQ ID No.56, SEQ ID No.57, SEQ ID No.59, SEQ ID No.60, SEQ ID No.62, SEQ ID No.63, SEQ ID No.65, SEQ ID No.66, SEQ ID No.68, SEQ ID No.69, SEQ ID No.71, SEQ ID No.72, SEQ ID No.74, SEQ ID No.75, or SEQ ID No.76.

14. The pharmaceutical composition or the combined drug according to claim 13, **characterized in that** the nucleotide sequence is obtained based on the encoded amino acid sequence SEQ ID No.25, SEQ ID No.27, SEQ ID No.29, SEQ ID No.31, SEQ ID No.33, SEQ ID No.35, SEQ ID No.37, SEQ ID No.39, SEQ ID No.40, SEQ ID No.43, SEQ ID No.46, SEQ ID No.49, SEQ ID No.52, SEQ ID No.55, SEQ ID No.58, SEQ ID No.61, SEQ ID No.64, SEQ ID No.67, SEQ ID No.70, SEQ ID No.73, or SEQ ID No.77 through optimization of codons or cells; and further, the cells are mammalian cells CHO or insect cells.

15. A recombinant vector or an adenovirus vector is **characterized by** containing a polynucleotide sequence in the recombinant protein vaccine or the adenovirus vaccine in the pharmaceutical composition or the combined drug according to any one of claims 1 to 14, wherein the polynucleotide sequence is selected from at least one of SEQ ID No.10, SEQ ID No.11, SEQ ID No.12, SEQ ID No.13, SEQ ID No.14, SEQ ID No.15, SEQ ID No.16, SEQ ID No.17, SEQ ID No.19, SEQ ID No.21, SEQ ID No.23, SEQ ID No.24, SEQ ID No.26, SEQ ID No.28, SEQ ID No.30, SEQ ID No.32, SEQ ID No.34, SEQ ID No.36, SEQ ID No.38, SEQ ID No.41, SEQ ID No.42, SEQ ID No.44, SEQ ID No.45, SEQ ID No.47, SEQ ID No.48, SEQ ID No.50, SEQ ID No.51, SEQ ID No.53, SEQ ID No.54, SEQ ID No.56, SEQ ID No.57, SEQ ID No.59, SEQ ID No.60, SEQ ID No.62, SEQ ID No.63, SEQ ID No.65, SEQ ID No.66, SEQ ID No.68, SEQ ID No.69, SEQ ID No.71, SEQ ID No.72, SEQ ID No.74, SEQ ID No.75, or SEQ ID No.76.

16. The recombinant vector or adenovirus vector according to claim 15, **characterized in that** the recombinant vector is selected from at least one of an insect baculovirus expression vector, a mammalian cell expression vector, an Escherichia coli expression vector or a yeast expression vector; preferably, the insect baculovirus expression vector is pFastBac1; preferably, the mammalian cell expression vector is an CHO cell expression vector; further preferably, the CHO cell expression vector is pTT5 or FTP-002; preferably, the Escherichia coli expression vector is pET32a; and preferably, the yeast expression vector is pPICZaA.

17. The recombinant vector or adenovirus vector according to claim 15, **characterized in that** the adenovirus vector is a human replication-deficient recombinant adenovirus vector; preferably, the adenovirus vector is selected from a human type 5, type 35 or type 26 replication-deficient adenovirus or/and a chimpanzee type AdC68 or AdC7 replication-deficient adenovirus, and more preferably, is selected from a human type 5 replication-deficient adenovirus with combined deletion of E1 and E3.

18. A host cell is **characterized by** containing the recombinant vector or adenovirus vector according to any one of claims 15 to 17.

19. The host cell according to claim 18, **characterized in that** the host cell is selected from at least one of insect cells, mammalian cells, Escherichia coli or yeasts; preferably, the insect cells are selected from at least one of sf9 cells, sf21 cells or Hi5 cells; and preferably, the mammalian cells are CHO cells or HEK293 cells.

20. The pharmaceutical composition according to claim 1 or the combined drug according to claim 2, **characterized in that** the recombinant protein vaccine and/or the adenovirus vaccine further comprise/comprises a pharmaceutically acceptable excipient or a complementary ingredient.

21. The pharmaceutical composition or combined drug according to claim 20, **characterized in that** the complementary ingredient is an immunologic adjuvant; preferably, the immunologic adjuvant is selected from at least one of a squalene oil-in-water emulsion, aluminum salt, calcium salt, plant saponin, plant polysaccharide, monophosphoryl lipid A, muramyl dipeptide, muramyl tripeptide, bacterial toxin, GM-CSF cytokines, lipid or cationic liposome materials; further, the immunologic adjuvant satisfies at least one the squalene oil-in-water emulsion being MF59, the aluminum salt being selected from at least one of aluminum hydroxide or alum, the calcium salt being tricalcium phosphate, the plant saponin being QS-21 or ISCOM, the plant polysaccharide being astragalus polysaccharide, the bacterial toxin being selected from at least one of recombinant cholera toxin or diphtheria toxin, the lipid being selected from at least one of phosphatidylethanolamine, phosphatidylcholine, cholesterol or dioleoyl phosphatidylethanolamine, or the cationic liposome material being selected from at least one of (2,3-dioleoxypropyl) trimethyl ammonium chloride, N-[1-(2,3-dioleoyl chloride) propyl ]-N,N,N-trimethylamine chloride, cationic cholesterol, dimethyl-2, 3-dioleoxypropyl-2-(2-arginine formyl amino) ethyl ammonium trimethyldodecylammonium bromide, dodecyltrimethylammonium bromide, tetradecyltrimethylammonium bromide, hexadecyltrimethylammonium bromide, dimethyldioctadecylammonium bromide, or CpG ODN.

22. The pharmaceutical composition or the combined drug according to claim 20, **characterized in that** the vaccine preparations of the recombinant protein vaccine and the adenovirus vaccine are an intramuscular injection, an injection, a nasal drop, a spray, a nasal spray or inhalation; and preferably, the recombinant protein vaccine or the adenovirus vaccine is a nasal spray.

23. Use of the pharmaceutical composition or the combined drug according to any one of claims 1 to 15 and 20 to 22 in preparation of a drug for preventing and/or treating infection with SARS-CoV-2 or a mutant thereof.

24. Use of the pharmaceutical composition or the combined drug according to any one of claims 1 to 15 and 20 to 22 in preparation of a drug for treating and/or preventing infection or pathopoiesis with a SARS-COV-2 variant strain, wherein the SARS-COV-2 variant strain comprises at least one of Alpha, Beta, Gamma, Delta or Omicron.

25. Use of the pharmaceutical composition or the combined drug according to any one of claims 1 to 15 and 20 to 22 in preparation of a drug for treating and/or preventing respiratory virus infection or pathopoiesis.

26. A pharmaceutical composition is **characterized by** containing the recombinant protein vaccine and an inactivated virus vaccine in the pharmaceutical composition or the combined drug according to claims 1 to 15 and 20 to 22 as active ingredients, wherein the inactivated virus vaccine is an inactivated influenza virus vaccine.

27. A pharmaceutical composition is **characterized by** containing an adenovirus vaccine and an influenza virus recombinant protein vaccine in the pharmaceutical composition or the combined drug according to claims 1 to 15 and 20 to 22 as active ingredients.

28. Use of the pharmaceutical composition according to any one of claim 26 or claim 27 in preparation of a drug for treating and/or preventing respiratory virus infection or pathopoiesis.
